# EUROPEAN PATENT APPLICATION

(11) **EP 4 368 628 A1**
(43) Date of publication of application: **15.05.2024**
(21) Application number: 22837660.4
(22) Date of filing: 05.07.2022
(51) Int. Cl.: C07H 21/02, C12N 15/10

(54) **METHOD FOR PURIFYING NUCLEOTIDES, DEVICE FOR PURIFYING NUCLEOTIDES, HYDROPHOBIC REAGENT, AND HYDROPHOBIC SUBSTRATE**

(30) Priority: 05.07.2021 JP 2021111420
(71) Applicant: Japan Science and Technology Agency, Kawaguchi-shi, Saitama 332-0012 (JP); National University Corporation Tokai National Higher Education and Research System, Nagoya-shi, Aichi 464-8601 (JP)
(72) Inventor: ABE Hiroshi, Nagoya-shi, Aichi 464-8601 (JP); ABE Naoko, Nagoya-shi, Aichi 464-8601 (JP); INAGAKI Masahito, Nagoya-shi, Aichi 464-8601 (JP); KIMURA Yasuaki, Nagoya-shi, Aichi 464-8601 (JP); HASHIYA Fumitaka, Nagoya-shi, Aichi 464-8601 (JP); LI Zhenmin, Nagoya-shi, Aichi 464-8601 (JP); NAKASHIMA Yuko, Nagoya-shi, Aichi 464-8601 (JP)
(74) Representative: Hoffmann Eitle
(86) International application number: PCT/JP2022/026665
(87) International publication number: WO 2023/282245

(57) **Abstract**

The present invention is a method for purifying a nucleotide-based substance, the method including: a protecting group introduction step of introducing a hydrophobic protecting group represented by the following formula (P1) or (P2) into a nucleotide-based substance to produce a hydrophobic nucleotide-based substance; an isolation and purification step of isolating and purifying the hydrophobic nucleotide-based substance under a hydrophobic environment; and a deprotection step of deprotecting the hydrophobic protecting group from the hydrophobic nucleotide-based substance to produce the nucleotide-based substance, 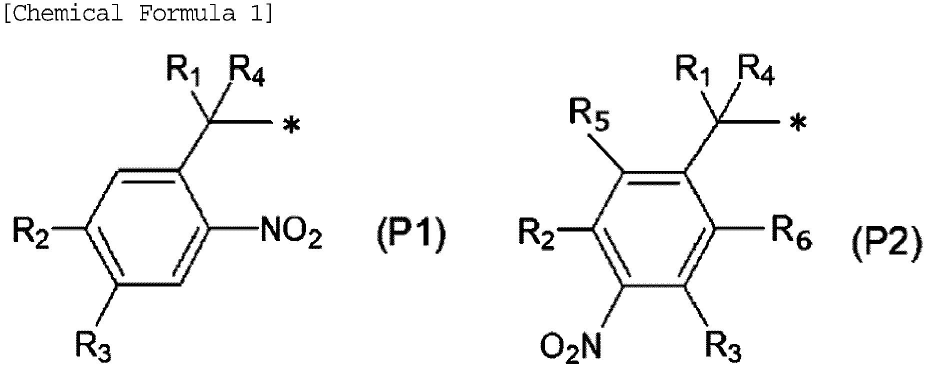 wherein R₁ represents a linear or branched alkyl group having 1 to 30 carbon atoms, R₄ represents hydrogen or a linear or branched alkyl group having 1 to 10 carbon atoms, R₂, R₃, R₅ and R₆ represent hydrogen, a linear or branched alkyl group having 1 to 10 carbon atoms, or the like, and may be the same or different; and * means a bond with a nucleotide-based substance.

## Description

### Technical Field

The present invention relates to a method for purifying a nucleotide-based substance, a device for purifying a nucleotide-based substance, a hydrophobic reagent, and a hydrophobic substrate.

### Background Art

Nucleic acids such as DNA and RNA are important molecules in life science, and have been studied for many years in the medical field and the like. In recent years, pharmaceutical applications of mRNAs have attracted particular attention. In an mRNA drug, a therapeutic effect is obtained by synthesizing a protein in cells, unlike an antisense nucleic acid that inhibits protein synthesis, a small interfering RNA (siRNA) that causes RNA interference, and the like. Therefore, it is possible not only to inhibit abnormal proteins but also to replenish normal proteins, and the realization of mRNA drugs leads to expansion of therapeutic strategies using nucleic acid drugs. In addition, mRNAs do not need to be transported to the nucleus, and have no risk of genomic insertional mutation, and therefore have one aspect of being superior in safety. Furthermore, in recent years, as the development of nucleic acid delivery related technologies has progressed, it has become widely recognized that the low cell membrane permeability of an mRNA can be overcome eventually, and mRNA drugs can be realized.

High-performance liquid chromatography (HPLC) and denaturing polyacrylamide gel electrophoresis have been used for the isolation and purification of mRNAs. So far, an RNA purification method utilizing hydrophobicity has been known. For example, a method has been reported in which a dinitrobenzene protecting group, which is a hydrophobic tag, is introduced to an RNA end synthesized on a solid phase on the basis of the phosphoramidite method, and the RNA is purified by reverse-phase HPLC (see, for example, Non-Patent Literature 1, c of Fig. 5 described later). In this method, a hydrophobically modified RNA is isolated and purified, and then a natural type RNA is obtained by removing a photoprotecting group by ultraviolet photoirradiation. According to this document, a 67mer RNA has been successfully isolated and purified, and the deprotection of a dinitrobenzene group has been achieved in a high yield of 95% or more.

On the other hand, the present inventors have developed primers that are used for amplification of nucleic acids and have a degradable protecting group (see, for example, Patent Literature 1). This degradable protecting group is composed of a nitrobenzyl group or the like, and is introduced into a phosphate group at the 3'-position of the nucleotide at the intermediate position rather than an end position such as the 5'-end or the 3'-end among the nucleotides constituting the nucleic acid.

### Citation List

### Patent Literature

PTL 1:WO 2021/020562 A (claims 1, 4, and 5, paragraphs 0073 to 0088, Figs. 9 and 10, and so on)

### Non-patent Literature

NPL 1:Pradere, U. et al., Chem. Eur. J. 2017, 23, 5210-5213

### Summary of Invention

### Technical Problem

The method of Non-Patent Literature 1 is used only for purification of about 90 residues of chemically synthesized RNA. The dinitrobenzene protecting group in Non-Patent Literature 1 has two nitrobenzyl groups, and the carbon located therebetween is electrophilic and highly reactive. For this reason, the dinitrobenzene protecting group is easily attacked by ammonium or the like and is easily degraded, and therefore stability after introduction of the protecting group is poor, and the yield of an objective nucleotide-based substance is low. In addition, since the dinitrobenzene protecting group of this literature has two highly reactive nitrobenzyl groups in the molecule, it is explosive, and is difficult to handle and dangerous. Furthermore, in this literature, neither purification with a long RNA having 100 or more bases nor purification by a transcription method using a polymerase is described.

An object of the present invention is to provide a method for purifying a nucleotide-based substance and a device for purifying a nucleotide-based substance, with which a nucleotide-based substance with a protecting group introduced is stable, a yield of a nucleotide-based substance after deprotection is high, and safety is high. Another object of the present invention is to provide a hydrophobic reagent and a hydrophobic substrate to be used for such a method or a device for purifying a nucleotide-based substance.

### Solution to Problem

The present inventors have intensively studied to solve the above problems. As a result, the present inventors have found that by employing mononitrobenzene as a protecting group, a nucleotide-based substance into which a protecting group has been introduced become stable, and the yield of a nucleotide-based substance attained through deprotection by light or by a reduction reaction is also improved, thereby having accomplished the present invention. The present invention is as follows.

[1] A method for purifying a nucleotide-based substance having at least one nucleotide and/or a derivative thereof as a constituent unit, the method comprising:
   a protecting group introduction step of introducing a hydrophobic protecting group represented by the following formula (P1) or (P2) into a nucleotide-based substance to produce a hydrophobic nucleotide-based substance;
   an isolation and purification step of isolating and purifying the hydrophobic nucleotide-based substance under a hydrophobic environment; and
   a deprotection step of deprotecting the hydrophobic protecting group from the hydrophobic nucleotide-based substance to produce the nucleotide-based substance, 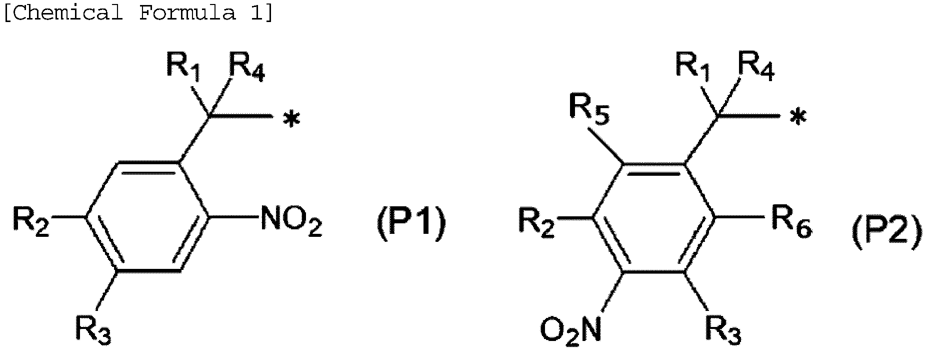
   wherein R₁ represents a linear or branched alkyl group having 1 to 30 carbon atoms, R₄ represents hydrogen or a linear or branched alkyl group having 1 to 10 carbon atoms, R₂, R₃, R₅ and R₆ each represent hydrogen, a linear or branched alkyl group having 1 to 10 carbon atoms, or a linear or branched alkoxy group having 1 to 10 carbon atoms, and R₂, R₃, R₅ and R₆ may be the same or different; and * means a bond with a nucleotide-based substance, and is bonded to an atom constituting the nucleotide-based substance directly or with a linking group selected from -O-C- and -O-C(=O)- interposed therebetween.
[2] The method for purifying a nucleotide-based substance according to the above [1], wherein the hydrophobic protecting group is represented by the following formula (P3), 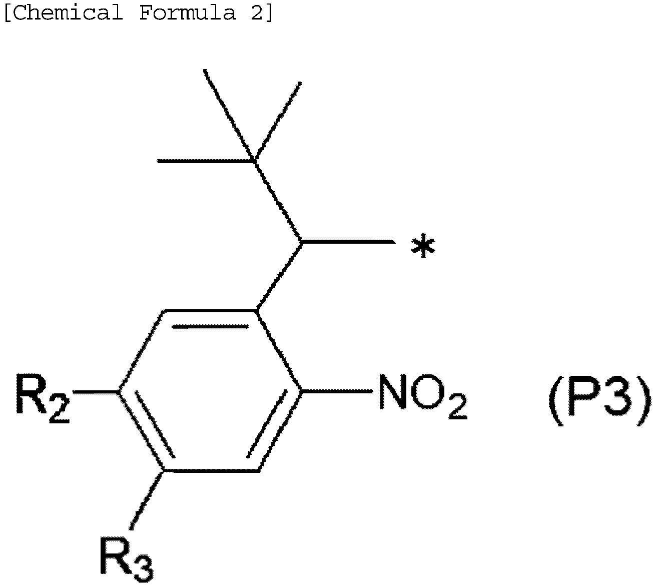 wherein R₂ and R₃ are as defined in [1] .
[3] The method for purifying a nucleotide-based substance according to the above [1], wherein the protecting group introduction step introduces the hydrophobic protecting group into a nucleotide located at the 5'-end of the nucleotide-based substance.
[4] The method for purifying a nucleotide-based substance according to the above [3], wherein the protecting group introduction step introduces the hydrophobic protecting group into a 5'-phosphate group, a 2'-hydroxyl group, or a base of a sugar constituting the nucleotide.
[5] The method for purifying a nucleotide-based substance according to the above [1], wherein in the protecting group introduction step, a protecting group-introduced nucleotide is synthesized by introducing an amidite reagent represented by the following formula (CR1) into a nucleotide, and a mononucleotide is sequentially linked to a 3'-end side of the protecting group-introduced nucleotide by solid phase synthesis to synthesize the hydrophobic nucleotide-based substance, 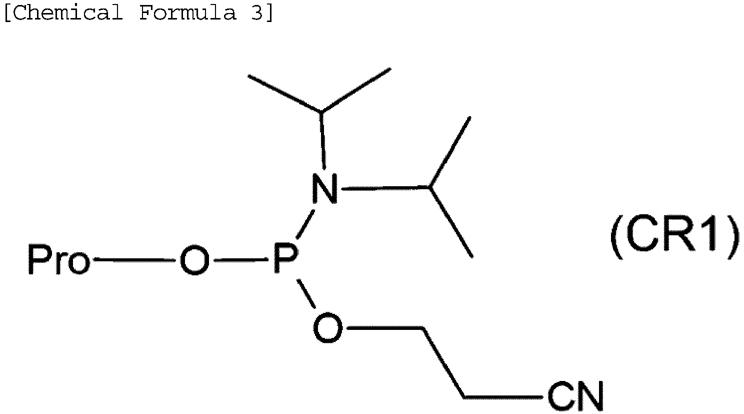 wherein Pro represents the hydrophobic protecting group.
[6] The method for purifying a nucleotide-based substance according to the above [1], wherein in the protecting group introduction step, the hydrophobic nucleotide-based substance is synthesized by employing, as a template, a nucleic acid having a sequence complementary to the nucleotide-based substance, employing, as substrates, a hydrophobic substrate represented by the following formula (ER1) and a nucleoside triphosphate, and transcribing the template by an RNA polymerase, 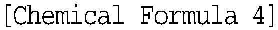 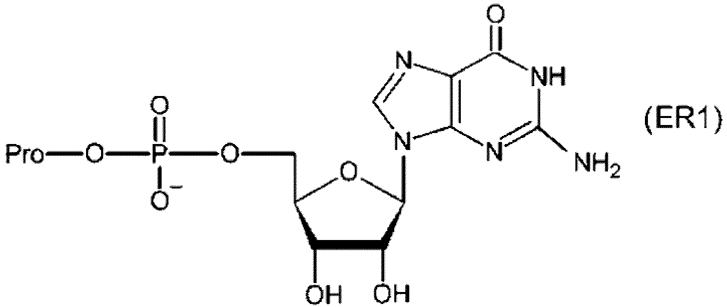 wherein Pro represents the hydrophobic protecting group.
[7] The method for purifying a nucleotide-based substance according to the above [1], wherein in the protecting group introduction step, an adenylation reagent represented by the following formula (CA1) is reacted with a phosphate group at a 5'-end of an oligonucleotide containing the nucleotide-based substance to synthesize the hydrophobic nucleotide-based substance, 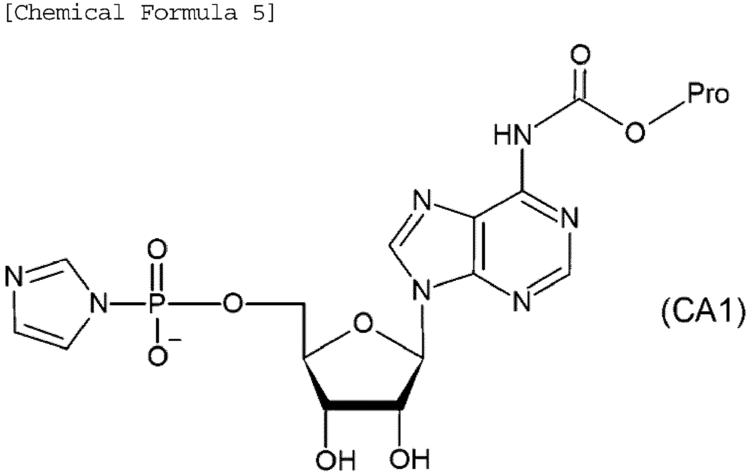 wherein Pro represents the hydrophobic protecting group.
[8] The method for purifying a nucleotide-based substance according to the above [1], wherein in the protecting group introduction step, the hydrophobic nucleotide-based substance is synthesized by employing, as a template, a nucleic acid having a sequence complementary to the nucleotide-based substance, employing, as substrates, a hydrophobic substrate represented by the following formula (EA1) and a nucleoside triphosphate, and transcribing the template by an RNA polymerase, 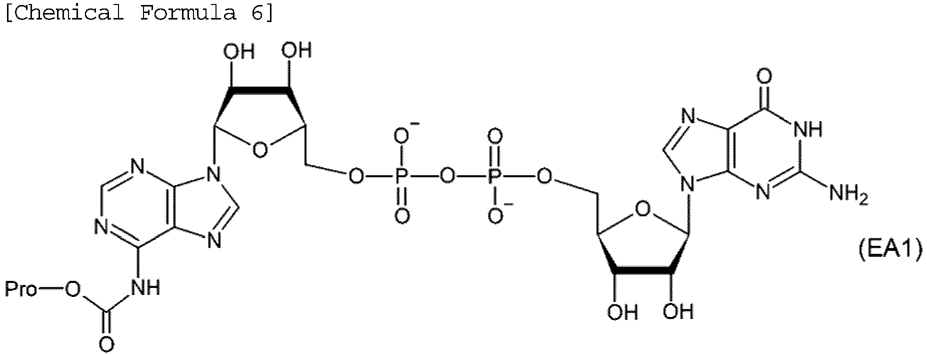 wherein Pro represents the hydrophobic protecting group.
[9] The method for purifying a nucleotide-based substance according to the above [1], wherein in the protecting group introduction step, a capping reagent selected from the group consisting of the following formulas (CC1) to (CC4) is reacted with a phosphate group at a 5'-end of an oligonucleotide containing the nucleotide-based substance to synthesize the hydrophobic nucleotide-based substance, 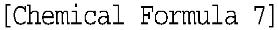 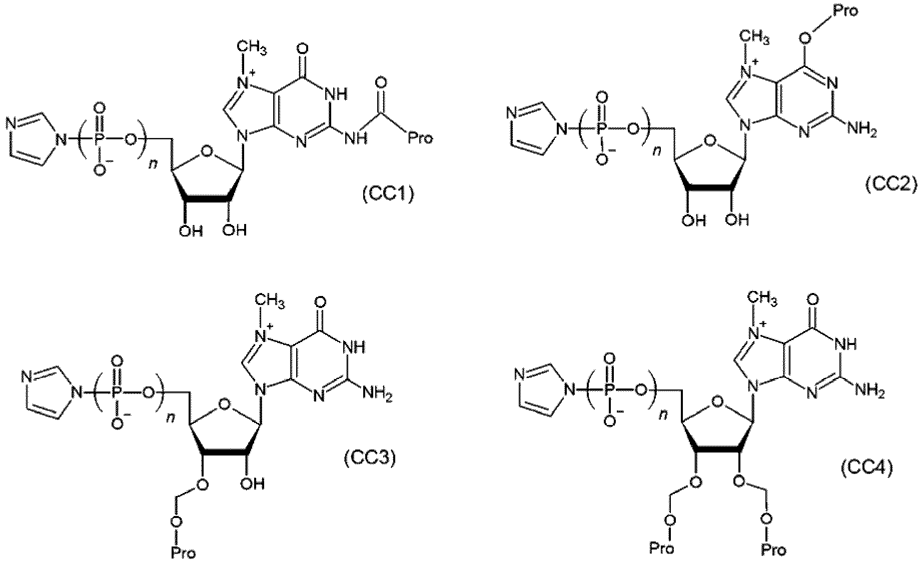 wherein Pro represents the hydrophobic protecting group, and n represents an integer of 1 or 2.
[10] The method for purifying a nucleotide-based substance according to the above [1], wherein in the protecting group introduction step, the hydrophobic nucleotide-based substance is synthesized by employing, as a template, a nucleic acid having a sequence complementary to the nucleotide-based substance, employing, as substrates, a hydrophobic substrate containing a structure of the following formula (EC0) and a nucleoside triphosphate, and transcribing the template by an RNA polymerase, 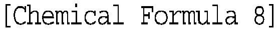 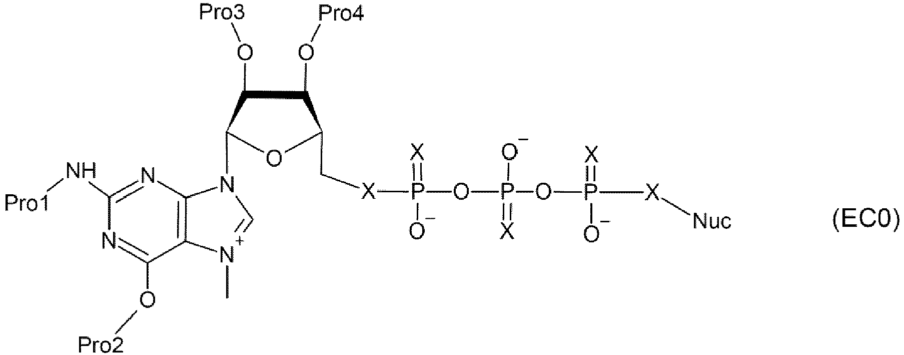 wherein Pro1 to Pro4 each represent the hydrophobic protecting group or hydrogen, provided that at least one of Pro1 to Pro4 is a hydrophobic protecting group, and Pro1 to Pro4 may be the same or different from each other; X is selected from the group consisting of oxygen, sulfur and selenium atoms; and Nuc represents a natural or non-natural nucleoside or one or more natural or non-natural nucleotides on carbon at a 3'-position thereof.
[11] The method for purifying a nucleotide-based substance according to the above [10], wherein the Nuc is a natural or non-natural nucleoside or a natural or non-natural nucleoside in which one or two natural or non-natural nucleotides are linked to the carbon at the 3'-position thereof.
[12] The method for purifying a nucleotide-based substance according to the above [10], wherein the hydrophobic substrate having the structure of the formula (EC0) is selected from the group consisting of the following formulas (EC1) to (EC4) and the following formulas (EC5) to (EC12), 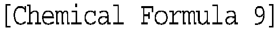 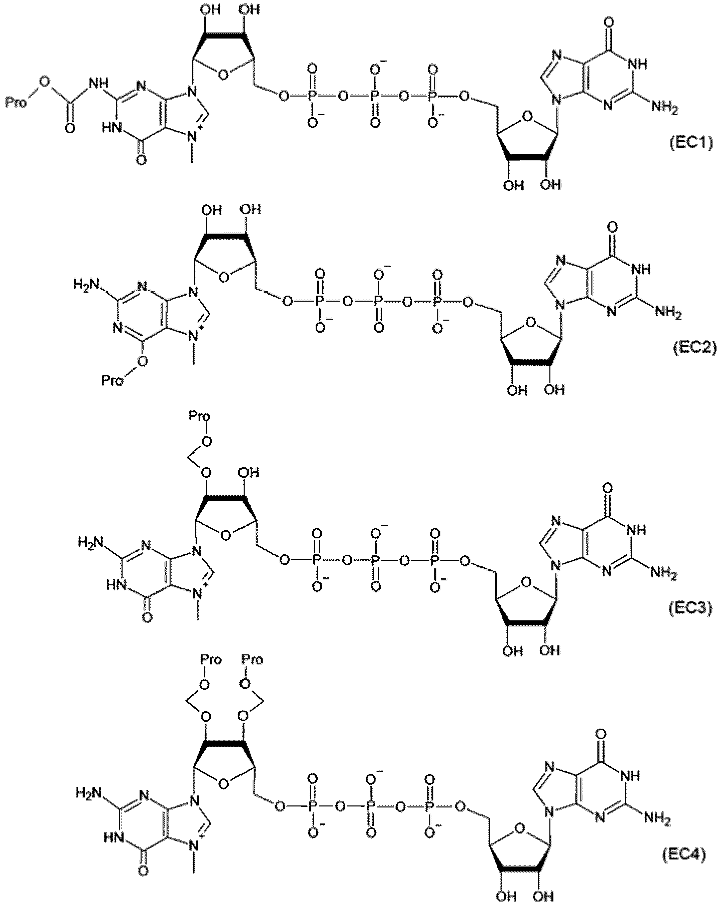
   wherein Pro represents the hydrophobic protecting group, 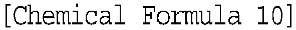 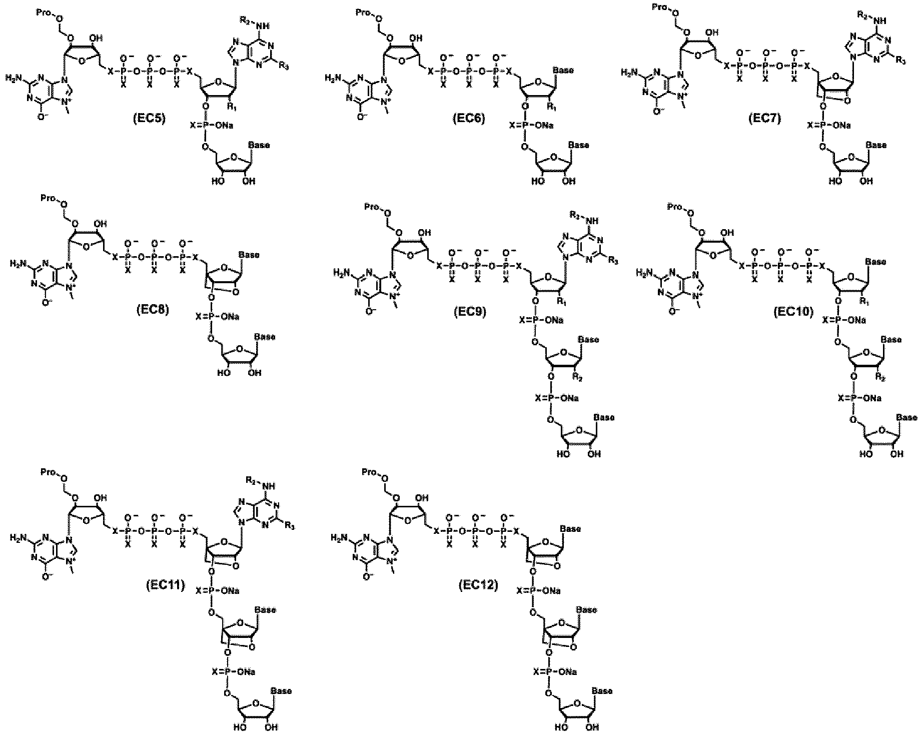
   wherein R₁ represents a substituent selected from among a hydroxy group (OH), a methoxy group (OCH₃), methoxyethyl (-OCH₂OCH₃), fluorine (F), and a formamide group (-NHCHO), R₂ represents hydrogen, a linear or branched alkyl group having 1 to 10 carbon atoms, and R₃ represents hydrogen or an amino group (-NH₂); wherein Base is a natural nucleobase selected from among adenine, cytosine, thymine, uracil, and guanine, or represents a non-natural base; wherein X is selected from the group consisting of oxygen, sulfur, and selenium atoms; and wherein Pro represents a hydrophobic protecting group represented by the formula (P1) or (P2) .
[13] The method for purifying a nucleotide-based substance according to the above [10], wherein the hydrophobic substrate is a cap derivative represented by the formula (EC0), and
   before the deprotection step, translation is performed using the hydrophobic nucleotide-based substance to which the hydrophobic protecting group is bonded.
[14] The method for purifying a nucleotide-based substance according to the above [1], wherein in the isolation and purification step, the hydrophobic nucleotide-based substance is isolated and purified by high-performance liquid chromatography.
[15] The method for purifying a nucleotide-based substance according to the above [1], wherein in the deprotection step, the hydrophobic protecting group is deprotected by photoirradiation.
[16] A device for purifying a nucleotide-based substance having at least one nucleotide and/or a derivative thereof as a constituent unit, the device comprising:
   a protecting group introduction means for introducing a hydrophobic protecting group represented by the following formula (P1) or (P2) into a nucleotide-based substance to produce a hydrophobic nucleotide-based substance;
   an isolation and purification means for isolating and purifying the hydrophobic nucleotide-based substance under a hydrophobic environment; and
   a deprotection means for deprotecting the hydrophobic protecting group from the hydrophobic nucleotide-based substance to produce the nucleotide-based substance, 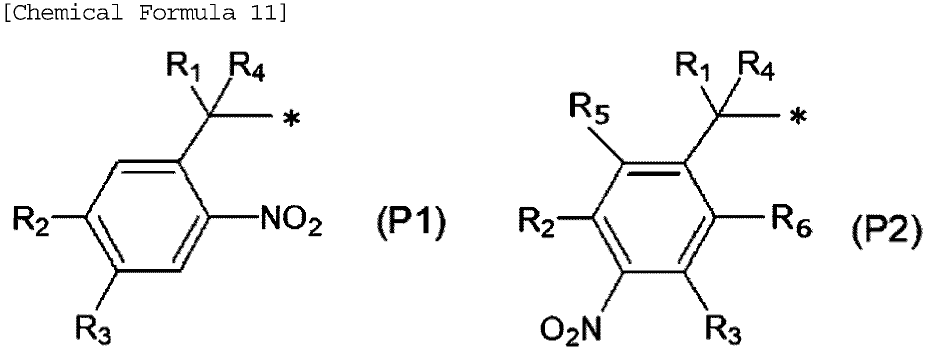
   wherein R₁ represents a linear or branched alkyl group having 1 to 30 carbon atoms, R₄ represents hydrogen or a linear or branched alkyl group having 1 to 10 carbon atoms, R₂, R₃, R₅ and R₆ each represent hydrogen, a linear or branched alkyl group having 1 to 10 carbon atoms, or a linear or branched alkoxy group having 1 to 10 carbon atoms, and R₂, R₃, R₅ and R₆ may be the same or different; and * means a bond with a nucleotide-based substance, and is bonded to an atom constituting the nucleotide-based substance directly or with a linking group selected from -O-C- and -O-C(=O)- interposed therebetween.
[17] A hydrophobic reagent for synthesizing a hydrophobic nucleotide-based substance by chemical synthesis, wherein the hydrophobic reagent is selected from the group consisting of the following formulas (CR1), (CA1), and (CC1) to (CC4), 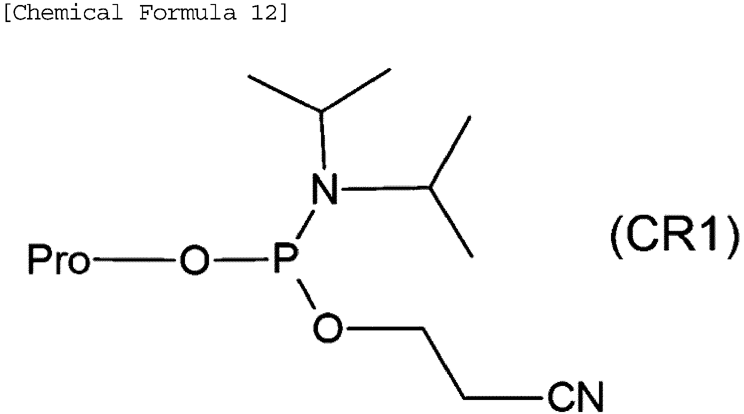 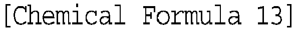 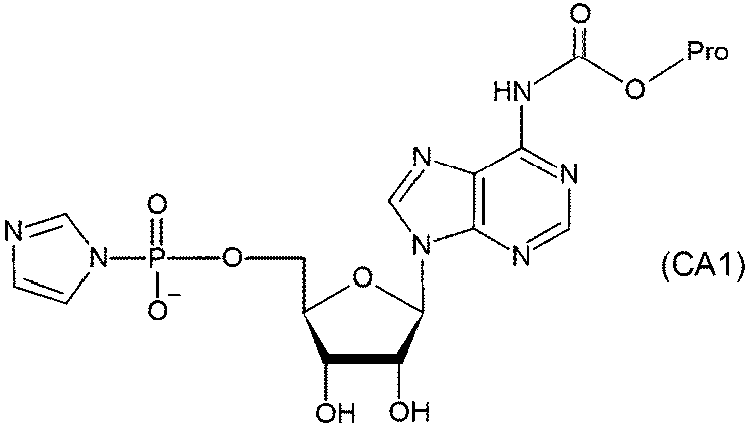 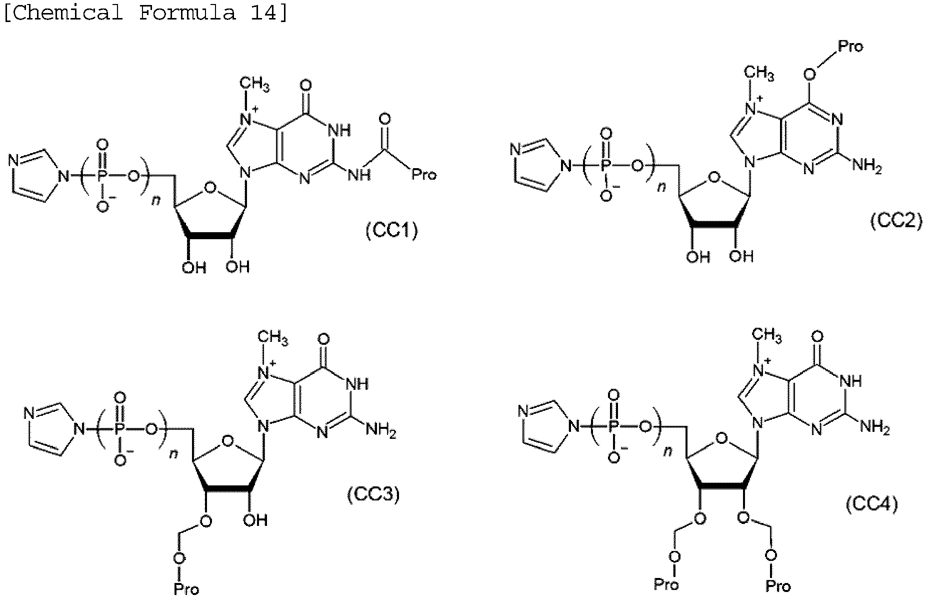
   wherein Pro represents a hydrophobic protecting group represented by the formula (P1) or (P2), and n represents an integer of 1 or 2, 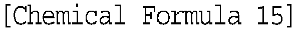 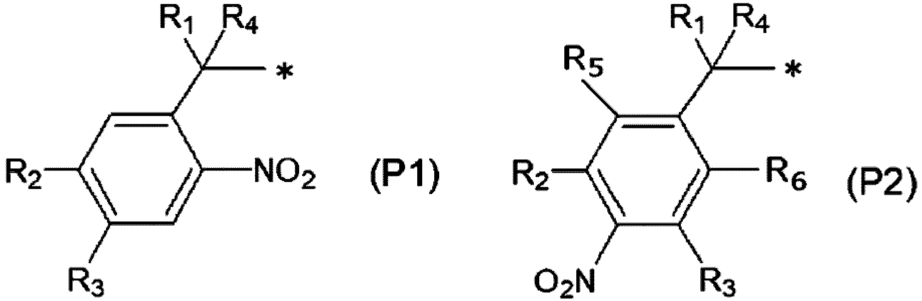
   wherein R₁ represents a linear or branched alkyl group having 1 to 30 carbon atoms, R₄ represents hydrogen or a linear or branched alkyl group having 1 to 10 carbon atoms, R₂, R₃, R₅ and R₆ each represent hydrogen, a linear or branched alkyl group having 1 to 10 carbon atoms, or a linear or branched alkoxy group having 1 to 10 carbon atoms, and R₂, R₃, R₅ and R₆ may be the same or different; and * means a bond.
[18] A hydrophobic substrate for producing a hydrophobic nucleotide-based substance by an RNA polymerase, wherein the hydrophobic substrate is selected from the group consisting of the following formulas (ER1), (EA1), and (EC0), 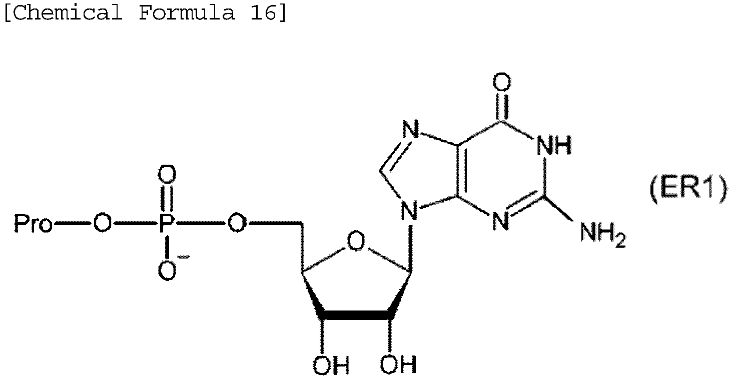
   wherein Pro represents a hydrophobic protecting group represented by the following formula (P1) or (P2), 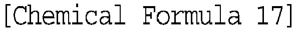 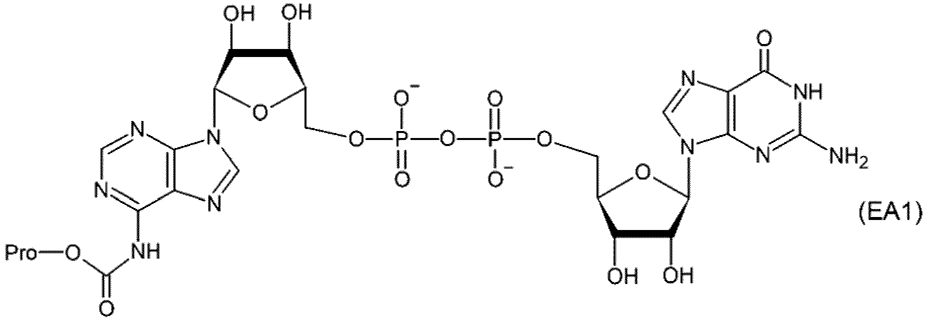
   wherein Pro represents the hydrophobic protecting group represented by the following formula (P1) or (P2), 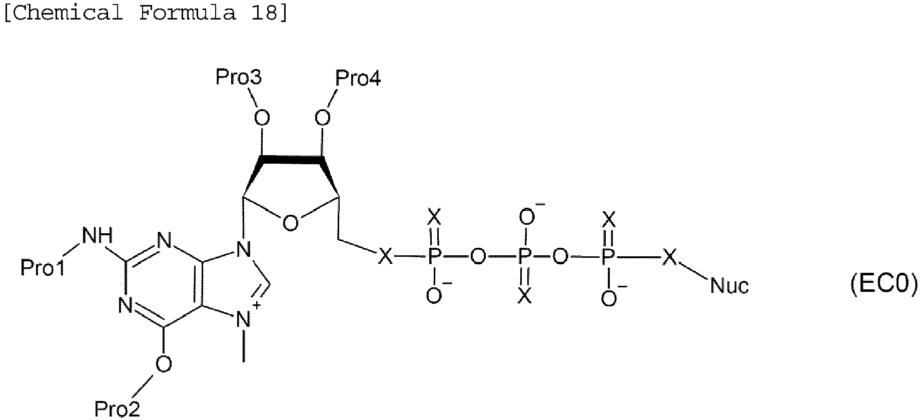
   wherein Pro1 to Pro4 each represent a hydrophobic protecting group represented by the following formula (P1) or (P2) or hydrogen, provided that at least one of Pro1 to Pro4 is a hydrophobic protecting group, and Pro1 to Pro4 may be the same or different from each other; X is selected from the group consisting of oxygen, sulfur and selenium atoms; and Nuc represents a nucleoside, in which one or two natural or unnatural nucleotides may be linked to carbon at a 3'-position. 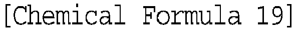 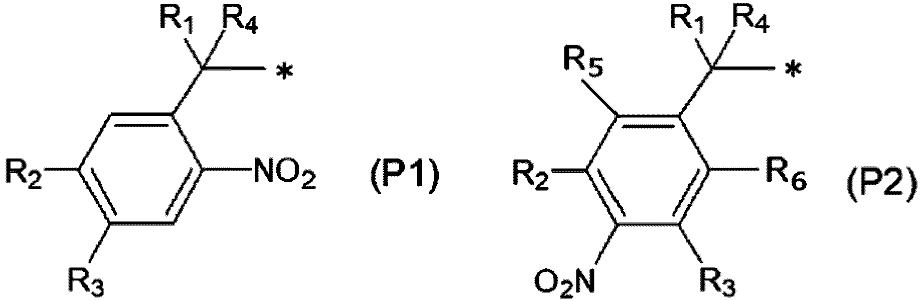
   wherein R₁ represents a linear or branched alkyl group having 1 to 30 carbon atoms, R₄ represents hydrogen or a linear or branched alkyl group having 1 to 10 carbon atoms, R₂, R₃, R₅ and R₆ each represent hydrogen, a linear or branched alkyl group having 1 to 10 carbon atoms, or a linear or branched alkoxy group having 1 to 10 carbon atoms, and R₂, R₃, R₅ and R₆ may be the same or different; and * means a bond.
[19] The hydrophobic substrate according to the above [18], wherein the Nuc is a natural or non-natural nucleoside or a natural or non-natural nucleoside in which one or two natural or non-natural nucleotides are linked to the carbon at the 3'-position thereof.
[20] An mRNA drug comprising a hydrophobic nucleotide-based substance in which a hydrophobic protecting group represented by the following formula (P1) or (P2) is introduced into a nucleotide-based substance having at least one nucleotide and/or a derivative thereof as a constituent unit, 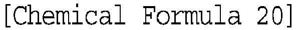 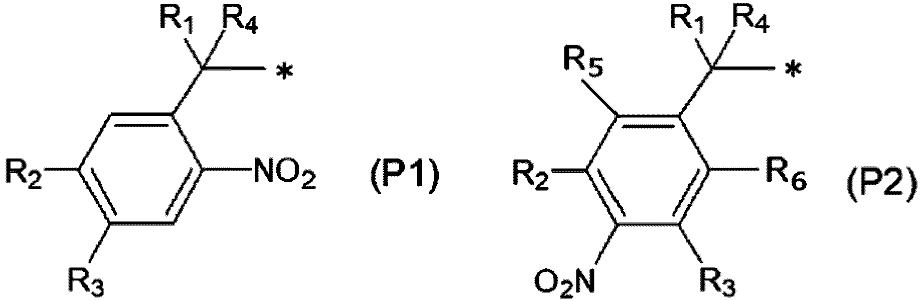 wherein R₁ represents a linear or branched alkyl group having 1 to 30 carbon atoms, R₄ represents hydrogen or a linear or branched alkyl group having 1 to 10 carbon atoms, R₂, R₃, R₅ and R₆ each represent hydrogen, a linear or branched alkyl group having 1 to 10 carbon atoms, or a linear or branched alkoxy group having 1 to 10 carbon atoms, and R₂, R₃, R₅ and R₆ may be the same or different; and * means a bond with a nucleotide-based substance, and is bonded to an atom constituting the nucleotide-based substance directly or with a linking group selected from -O-C- and -O-C(=O)- interposed therebetween.
[21] The mRNA drug according to [20], having a structure in which RNA is linked to carbon at a 3'-position of a nucleotide on a 3'-end side of a hydrophobic substrate containing a structure of the following formula (EC0), 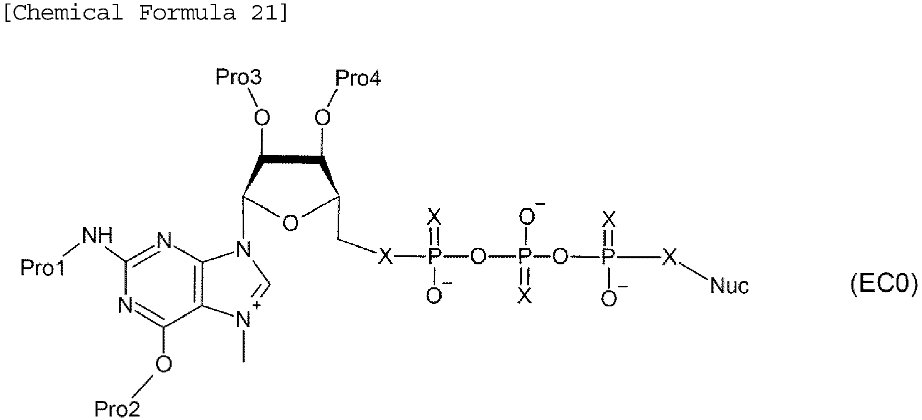 wherein Pro1 to Pro4 each represent the hydrophobic protecting group or hydrogen, provided that at least one of Pro1 to Pro4 is a hydrophobic protecting group, and Pro1 to Pro4 may be the same or different from each other; X is selected from the group consisting of oxygen, sulfur and selenium atoms; and Nuc represents a natural or non-natural nucleoside or one or more natural or non-natural nucleotides on carbon at a 3'-position thereof.
[23] The mRNA drug according to the above [21], wherein the hydrophobic substrate containing the structure of the formula (EC0) is selected from the group consisting of the following formulas (EC1) to (EC12), 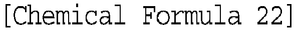 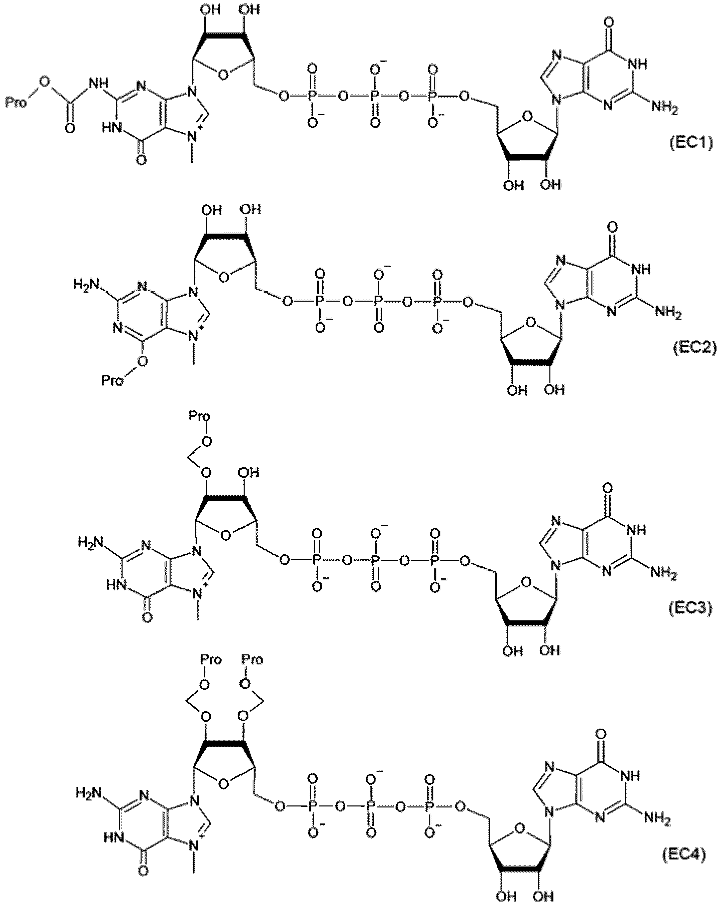
   wherein Pro represents the hydrophobic protecting group. 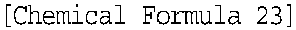 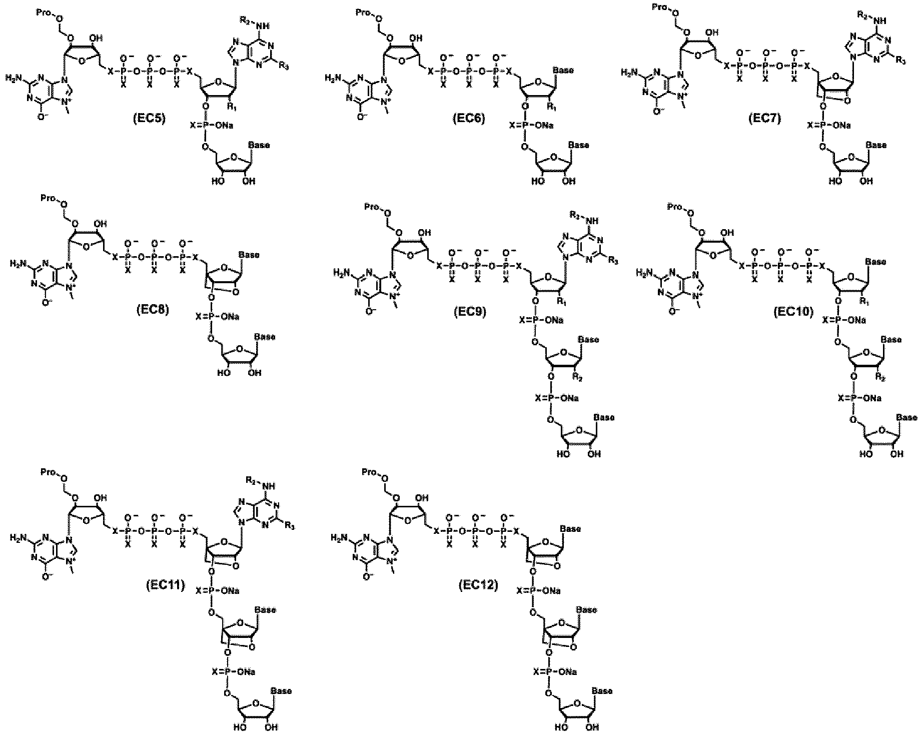
   wherein R₁ represents a substituent selected from among a hydroxy group (OH), a methoxy group (OCH₃), methoxyethyl (-OCH₂OCH₃), fluorine (F), and a formamide group (-NHCHO), R₂ represents hydrogen, a linear or branched alkyl group having 1 to 10 carbon atoms, and R₃ represents hydrogen or an amino group (-NH₂); wherein Base is a natural nucleobase selected from among adenine, cytosine, thymine, uracil, and guanine, or represents a non-natural base; wherein X is selected from the group consisting of oxygen, sulfur, and selenium atoms; and wherein Pro represents a hydrophobic protecting group represented by the formula (P1) or (P2) .
[24] A method for producing the mRNA drug according to the above [20], the method comprising:
   a protecting group introduction step of introducing the hydrophobic protecting group represented by the formula (P1) or (P2) into the nucleotide-based substance to generate a hydrophobic nucleotide-based substance.
[25] The method for purifying the mRNA drug according to the above [21], wherein in the protecting group introduction step, the hydrophobic nucleotide-based substance is synthesized by employing, as a template, a nucleic acid having a sequence complementary to the nucleotide-based substance, employing, as substrates, a hydrophobic substrate having a structure of the formula (EC0) and a nucleoside triphosphate, and transcribing the template by an RNA polymerase.

[Opt 1] An mRNA drug of the above [21], wherein the hydrophobic substrate having the structure of the formula (EC0) is deprotected and Pro1 to Pro4 are hydrogen.

[Opt 2] The mRNA drug according to the above [Opt 1], wherein the hydrophobic substrate containing the structure of the formula (EC0) is selected from the group consisting of the formulas (EC1) to (EC12), the hydrophobic protecting group is deprotected, and Pro is hydrogen.

### Advantageous Effects of Invention

The present invention makes it possible to provide a method for purifying a nucleotide-based substance and a device for purifying a nucleotide-based substance, with which a nucleotide-based substance with a protecting group introduced is stable, a yield of a nucleotide-based substance after deprotection is high, and safety is high. In addition, the present invention makes it possible to provide a hydrophobic reagent and a hydrophobic substrate to be used for such a method or a device for purifying a nucleotide-based substance.

### Brief Description of Drawings

Fig. 1 is a diagram showing a concept of the method for purifying a nucleotide-based substance of the present invention and a production device.
Fig. 2 is a diagram showing the results of reverse-phase HPLC analysis of synthesis and purification of a 5'-phosphorylated oligonucleotide using amidite reagent_1, and a deprotection reaction after the purification.
Fig. 3 is a diagram showing the results of reverse-phase HPLC analysis of synthesis and purification of a 5'-phosphorylated oligonucleotide using amidite reagent_2, and a deprotection reaction after the purification.
Fig. 4 is a diagram showing the results of reverse-phase HPLC analysis of synthesis and purification of a 5'-phosphorylated oligonucleotide using amidite reagent_3, and a deprotection reaction after the purification.
Fig. 5 is a diagram showing the results of synthesis yield comparison by reverse-phase HPLC analysis of 5'-phosphorylated oligonucleotides using amidite reagents_ 1 and 2.
Fig. 6 is a diagram showing the results of reverse-phase HPLC analysis of synthesis and purification of a 5'-phosphorylated oligonucleotide using amidite reagent_1, and a deprotection reaction after the purification.
Fig. 7 is a diagram showing that a 5'-end phosphorylated RNA synthesized using amidite reagent_1 could be quantitatively deprotected by photoirradiation.
Fig. 8 is a diagram showing that a 5'-end phosphorylated RNA synthesized using amidite reagent_1 can be isolated and purified by reverse-phase HPLC, and can be quantitatively deprotected by subsequent photoirradiation.
Fig. 9 is a diagram showing an outline of the methods for transcriptionally synthesizing an RNA in the present invention and the prior art.
Fig. 10 is a diagram showing the result of analysis of GMP and an R-pG reaction liquid by HPLC.
Fig. 11 is a diagram showing the result of electrophoresis of RNAs synthesized by a transcription reaction.
Fig. 12 is a diagram showing the result of analysis of 34 nt RNA with a C18 column.
Fig. 13 is a diagram showing the result of analysis of 34 nt RNA with a C4 column.
Fig. 14 is a diagram showing the result of analysis of 100 nt RNA with a C18 column.
Fig. 15 is a diagram showing the result of analysis of 250 nt RNA with a C18 column.
Fig. 16 is a diagram showing the result of analysis of 250 nt RNA with a C4 column.
Fig. 17 is a diagram showing the result of analysis of 650 nt RNA with a C18 column.
Fig. 18 is a diagram showing the result of analysis of 650 nt RNA with a C4 column.
Fig. 19 is a diagram showing the result of analysis of 1078 nt RNA with a C18 column.
Fig. 20 is a diagram showing the result of analysis of 1078 nt RNA with a C4 column.
Fig. 21 is a diagram showing the result of HPLC analysis and mass spectrometry of 34 nt RNA.
Fig. 22 is a diagram showing the result of HPLC analysis before and after deprotection of 34 nt RNA.
Fig. 23 is a diagram showing the result of HPLC analysis before and after deprotection of 250 nt RNA.
Fig. 24 is a diagram showing the result of synthesis of a branched cap analog compound (precursor).
Fig. 25 is a diagram showing the result of successfully obtaining a protecting group-containing adenylated RNA by adding Compound 11 to an in vitro transcription reaction using T7 RNA polymerase.
Fig. 26 is a diagram showing the result that a protecting group-containing adenylated RNA prepared through a transcription reaction was deprotected by photoirradiation and could be converted into an objective adenylated RNA.
Fig. 27 is a diagram showing the result of various analysis of Cap Analog_1, which is a novel cap analog compound synthesized.
Fig. 28 is a diagram showing the result of various analysis of Cap Analog_2, which is a novel cap analog compound synthesized.
Fig. 29 is a diagram showing that an RNA with a novel cap analog introduced therein could be isolated and purified by reverse-phase HPLC.
Fig. 30 is a diagram showing the evaluation of the translation activity of a NanoLuc luciferase mRNA after reverse-phase HPLC isolation and purification.
Fig. 31 is a diagram showing evaluation of translation activity of a NanoLuc luciferase mRNA after isolation and purification by reverse-phase HPLC, particularly comparison of the activity with the case of using ARCA.
Fig. 32 is a diagram showing the result of a chemical capping reaction of an RNA using a hydrophobic chemical capping reagent.
Fig. 33 is a diagram showing the result of HPLC purification of a co-transcription reaction product mRNA using a cap analog synthesized.
Fig. 34 is a diagram showing the result of HPLC purification of a co-transcription reaction product mRNA using a cap analog synthesized.
Fig. 35 is a diagram showing the result of HPLC purification of a co-transcription reaction product mRNA using a cap analog synthesized.
Fig. 36 is a diagram showing the result of HPLC purification of a co-transcription reaction product mRNA using a cap analog synthesized.
Fig. 37 is a diagram showing the result of evaluation of translation activity in a HeLa cell of RNAs transcriptionally synthesized using a cap analog.
Fig. 38 is a diagram showing the result of evaluation of translation activity in JAWS II cells of an RNA transcriptionally synthesized using a cap analog.
Fig. 39 is a diagram showing a comparison of translation activities of 650-base-long NanoLuc luciferase (Nluc) mRNAs prepared using hydrophobic protecting group-containing dinucleotide-type cap analogs (DiPure, DiPure/3'ome, DiPure/2'ome) and a conventional cap analog (ARCA).
Fig. 40 is a diagram showing a comparison of translation activities of 650-base-long NanoLuc luciferase (Nluc) mRNAs prepared using hydrophobic protecting group-containing trinucleotide type cap analogs (TriPure_0, TriPure_1), tetranucleotide-type cap analogs (TetraPure_2, TetraPure_2/m6A, TetraPure_2/G) and conventional cap analogs (Tri_1, Tetra_2).
Fig. 41 is a diagram showing the result of analysis by reverse-phase HPLC of a capped mRNA prepared by adding a hydrophobic protecting group-containing cap analog (DiPure) at the time of transcription of a 4247-base-long RNA strand.
Fig. 42 is a diagram showing the result that a PureCap type mRNA exhibits a high amount of protein synthesized without introducing methylpseudouridine.
Fig. 43 is a diagram showing the result of evaluation of an intracellular immune response (HEK293 NF-kB cell).
Fig. 44 is a diagram showing the result that a PureCap mRNA with high purity exhibited higher protein expression ability than an ARCA mRNA in an animal individual.
Fig. 45 is a diagram showing the result of evaluation of an intracellular immune response (HEK293 NF-kB cell).

### Description of Embodiments

### 1. Method for purifying nucleotide-based substance

Hereinafter, the method for purifying a nucleotide-based substance of the present invention will be described. The method for purifying a nucleotide-based substance of the present invention is a method for purifying a nucleotide-based substance to be purified, and includes a protecting group introduction step, an isolation and purification step, and a deprotection step. Each will be described below. The "nucleotide-based substance" referred to in the present invention is any compound having at least one nucleotide and/or a derivative thereof as a constituent unit. Nucleotides are composed of a sugar, a base, and a phosphoric acid. Nucleotide-based substances include mononucleotides each composed of one nucleotide, oligonucleotides and polynucleotides in each of which a plurality of nucleotides are linked (the oligonucleotides and the polynucleotides are both referred to as "nucleic acids"), and derivatives thereof. Here, the term oligonucleotides mean nucleic acids (DNA, RNA) having 2 to 20 nucleotides, and the term polynucleotides means nucleic acids having 21 or more nucleotides. In addition, the nucleotide-based substances also include those having a branch structure in which the 5'-end of a nucleotide is branched into two or more, and examples of the branch structure include a bi-branched type structure and a tri-branched type structure.

In addition, examples of the derivative of at least one nucleotide include mononucleotide or nucleic acids (oligonucleotides, polynucleotides) subjected to modification or the like, for example, these nucleotides modified by methylation or the like, and those nucleotides whose 5'-end is adenylated or capped. The sugar constituting the nucleotide-based substance of the present invention may be ribose or deoxyribose. Examples of the base constituting the nucleotide-based substance of the present invention include adenine, guanine, cytosine, thymine, uracil, N-methyladenine, N-benzoyladenine, 2-methylthioadenine, 2-aminoadenine, 7-methylguanine, N-isobutyrylguanine, 5-fluorocytosine, 5-bromocytosine, 5-methylcytosine, 4-N-methylcytosine, 4-N,N-dimethylcytosine, 5-fluorouracil, 5-bromouracil, 5-chlorouracil, and 5,6-dihydrouracil. Furthermore, examples of the phosphoric acid constituting the nucleotide-based substance of the present invention include monophosphoric acid, diphosphoric acid, and triphosphoric acid.

### (1) Protecting group introduction step

The protecting group introduction step is a step of introducing a hydrophobic protecting group (also referred to as "fat-soluble protecting group" or "purification tag") represented by the following formula (P1) or (P2) into a nucleotide-based substance to produce a hydrophobic nucleotide-based substance, 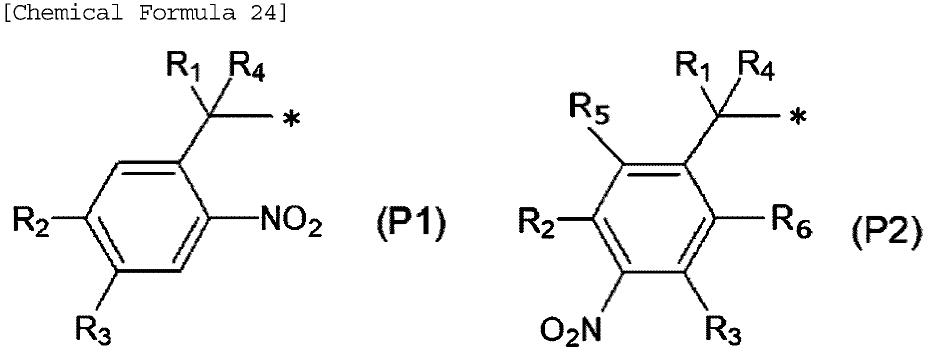 wherein R₁ represents a linear or branched alkyl group having 1 to 30 carbon atoms, R₄ represents hydrogen or a linear or branched alkyl group having 1 to 10 carbon atoms, R₂, R₃, R₅ and R₆ each represent hydrogen, a linear or branched alkyl group having 1 to 10 carbon atoms, or a linear or branched alkoxy group having 1 to 10 carbon atoms, and R₂, R₃, R₅ and R₆ may be the same or different; and * means a bond with a nucleotide-based substance, and is bonded to an atom constituting the nucleotide-based substance directly or with a linking group selected from -O-C- and -O-C(=O)- interposed therebetween.

Among them, R₁ is a linear or branched alkyl group having 1 to 30 carbon atoms, and R₄ is preferably hydrogen. In addition, from the viewpoint of stabilizing a hydrophobic protecting group, R₁ is preferably a branched alkyl group, more preferably a secondary or tertiary alkyl group, and particularly preferably a tertiary alkyl group. On the other hand, the number of carbon atoms of R₁ is preferably 5 or more, more preferably 10 or more from the viewpoint that the hydrophobicity of the hydrophobic protecting group is increased and the compound is easily separated from other compounds. In particular, from the viewpoint of increasing the degradation efficiency of the hydrophobic protecting group, R₄ is preferably hydrogen when the hydrophobic protecting group is photodegradable, and R₄ is preferably an alkyl group when the hydrophobic protecting group is degradable by a reductant.

In particular, as the hydrophobic protecting group, those having a tert-butyl group represented by the following formula (P3) are preferable, 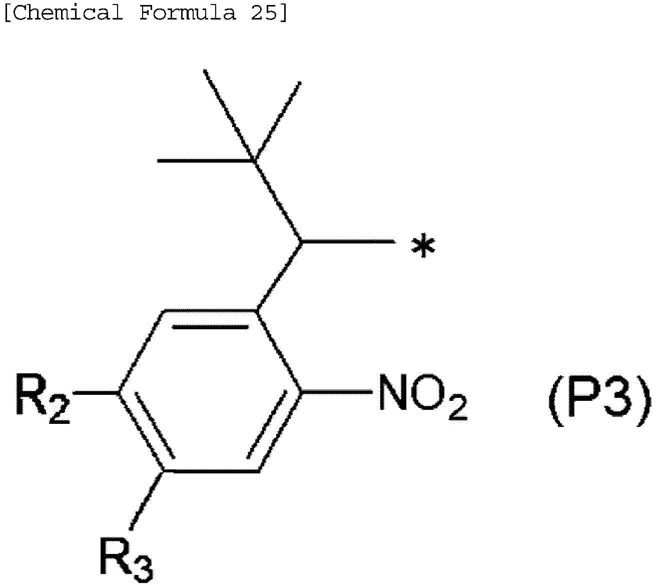 wherein R₂ and R₃ are as defined above.

In this case, in particular, R₂ and R₃ are preferably hydrogen.

When the nucleotide-based substance is a macromolecular compound such as an oligonucleotide or a polynucleotide, the hydrophobic protecting group can be introduced into any nucleotide, but it is particularly preferable to introduce the hydrophobic protecting group into a nucleotide located at the 5'-end of the nucleotide-based substance. The nucleotide located at the 5'-end is a nucleotide located at the most end on the 5'-side in the case of DNA, RNA, or the like. In addition, in the case of adenylation (AMP formation) or capping (CAP formation) described later, the nucleotide located at the 5'-end is the adenosine or the cap (7-methylguanylic acid) bonded to the nucleotide at the 5'-end of DNA or RNA.

In addition, the hydrophobic protecting group may be introduced into an arbitrary position of a nucleotide, and, in particular, is preferably introduced into a 5'-phosphate group, a 2'-hydroxyl group, or a base of a sugar.

When a hydrophobic protecting group is introduced in the 3'-phosphate group of a nucleotide at the intermediate position instead of a nucleotide located at an end as described in Patent Literature 1, the hydrophobic protecting group is present in a hydrophilic region composed of an oligonucleic acid, so that a hydrophobic effect is less likely to be exerted and purification is likely to be difficult. In addition, when a hydrophobic protecting group is introduced in the 3'-phosphate group of a nucleotide at the intermediate position instead of a nucleotide located at an end as described in Patent Literature 1, if the hydrophobic protecting group is degraded in the process until the nucleotide-based substance is purified under hydrophobic conditions, the nucleotide-based substance may be cleaved at an intermediate position thereof. Therefore, the hydrophobic protecting group is preferably introduced to a nucleotide located at the 5'-end, and specifically to a functional group that does not constitute a chain structure of a nucleic acid, such as a 5'-phosphate group, a 2'-hydroxy group, or a base. As a result of introducing a hydrophobic protecting group at an end position of such a nucleic acid, even a nucleic acid having a long hydrophilic moiety such as a long-chain RNA having 1000 or more bases can be easily purified by the hydrophobic protecting group.

When a hydrophobic protecting group is introduced into a phosphate group or a hydroxy group, the hydrophobic protecting group is preferably bonded directly to an oxygen atom constituting the phosphate group or the hydroxy group, or bonded via a linking group selected from -O-C- and -O-C(=O)-. When a hydrophobic protecting group is introduced into a base, the hydrophobic protecting group is preferably bonded to a nitrogen atom of an amino group constituting the base via a linking group selected from -O-C- and -O-C(=O)-. More specifically, from the viewpoint of the degree of the stability and the degree of ease of synthesis of the compound with a hydrophobic protecting group introduced therein, the position to which the hydrophobic protecting group is introduced is more preferably as follows.
· When a hydrophobic protecting group is introduced into an oxygen (=O) of a base (such as a guanine base O6 position), the hydrophobic protecting group is directly introduced.
· When a hydrophobic protecting group is introduced into an amino group (-NH₂) of a base (such as a guanine base portion N2 position), the hydrophobic protecting group is introduced directly or via -(O=)C-O-.
· When a hydrophobic protecting group is introduced into a hydroxy group (-OH) of a sugar moiety (ribose, deoxyribose), the hydrophobic protecting group is introduced via -O-C- (* this is because if the hydrophobic protecting group is introduced via - (O=)C-O-, a rearrangement reaction between adjacent hydroxy groups proceeds).
· When a hydrophobic protecting group is introduced into oxygen (-O-P(=O)(-OH)-O-) of a phosphate group, the hydrophobic protecting group is introduced directly or via -O-C- and -O-C(=O)-.

### (2) Isolation and purification step

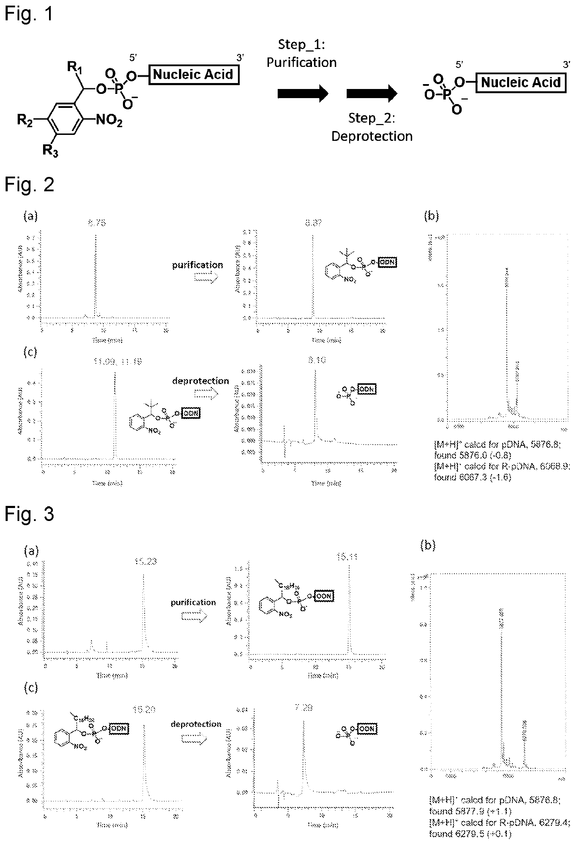

The isolation and purification step is a step of isolating and purifying a hydrophobic nucleotide-based substance under a hydrophobic environment. Fig. 1 shows a step (Step_1: Purification) of separating a nucleic acid with a hydrophobic protecting group introduced therein. The isolation and purification step is not particularly limited as long as it is performed under hydrophobic conditions, and examples thereof include liquid chromatography and membrane separation, and among them, reverse-phase high-performance liquid chromatography (reverse-phase HPLC) is preferable. Reverse-phase HPLC is a method in which a packing material is placed in a column of chromatography and an object to be separated is separated on the basis of a difference in hydrophobic interaction between a mobile phase and the packing material. Silica gel or the like can be used as a stationary phase support of the packing material, and an alkyl group having about 4 to 18 carbon atoms can be used as a binder phase on a surface of the packing material. In addition, water or an organic solvent such as methanol or acetonitrile can be used as a mobile phase of reverse-phase HPLC, and the object to be separated can be separated according to hydrophobicity by gradient elution in which the composition of water/organic solvent (for example, acetonitrile) is continuously changed. The temperature of the isolation and purification step is in the range of 10 to 40°C, and room temperature (25°C) is particularly preferable.

### (3) Deprotection step

The deprotection step is a step of deprotecting a hydrophobic protecting group from a hydrophobic nucleotide-based substance to generate a nucleotide-based substance. Fig. 1 shows a step (Step_2: Deprotection) of deprotecting the hydrophobic nucleotide-based substance purified in the isolation and purification step. Examples of the method for deprotecting the hydrophobic protecting group include photoirradiation, reduction, and alkali degradation, and photoirradiation is particularly preferable. The conditions for photoirradiation may be appropriately set according to the properties of the hydrophobic protecting group and so on, but for example, the wavelength is preferably in the range of 300 to 400 nm, the quantity of light is preferably in the range of 0.5 to 10 mW/cm², and the irradiation time is preferably in the range of 1 to 60 minutes. The conditions of a reduction reaction may be appropriately set according to the properties of the hydrophobic protecting group and so on, and for example, the reaction is performed in a 1 to 100 mM aqueous sodium dithionite solution at 37°C for 30 minutes, and then the reaction is performed at 65°C for 10 minutes to conduct deprotection.

In Examples described later, examples of three types of purification methods, i.e., phosphorylation purification, adenylation purification, and capping purification, are described as the hydrophobic protecting group. Examples of the protecting group introduction step include a method of synthesizing a hydrophobic nucleotide-based substance through chemical synthesis using a hydrophobic reagent having a hydrophobic protecting group (chemical synthesis) and a method of synthesizing a hydrophobic nucleotide-based substance through an enzymatic reaction by a polymerase using a template and a substrate (enzyme synthesis). Hereinafter, chemical synthesis and enzyme synthesis will be described for each of the above three purification methods.

### 2. Phosphorylation purification

Phosphorylation purification is a method in which a hydrophobic protecting group (phosphorylation tag) is bonded to a phosphate group at the 5'-position of an endmost nucleotide on the 5'-side of a nucleotide-based substance, and the nucleotide-based substance is purified utilizing the hydrophobicity of the hydrophobic protecting group.

The phosphorylation purification tag is a hydrophobic protecting group that bonds to the phosphate group at the 5'-position of an endmost nucleotide on the 5'-side of a nucleotide-based substance. Examples of the phosphorylation purification include a method of introducing a hydrophobic protecting group through chemical synthesis and a method of introducing a hydrophobic protecting group through enzyme synthesis. In the following synthesis scheme (hereinafter, "phosphorylation purification scheme"), as one example of the hydrophobic protecting group, an example of using "bNB" in which R₁ is a tert-butyl (t-Bu) group and R₂ to R₄ are hydrogen in the formula (P1) is disclosed. Note that bNB is merely one example of the hydrophobic protecting group of the present invention, and the present invention is not limited thereto, and can be appropriately replaced by another compound included in the formula (P1) or the formula (P2).

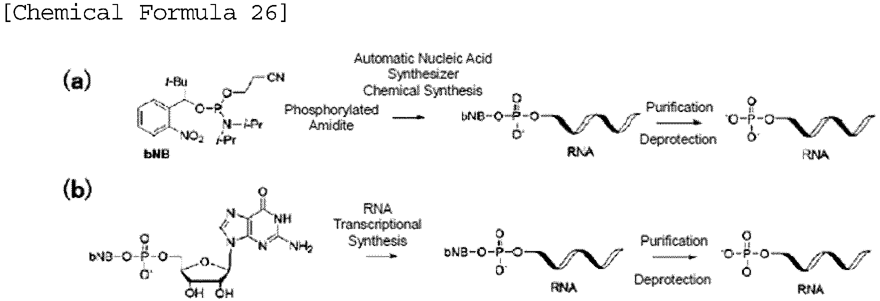

### (1) Chemical synthesis

The upper part (a) of the phosphorylation purification scheme shows a scheme for synthesizing and purifying a hydrophobic nucleotide-based substance by a chemical method. In this method, a hydrophobic nucleotide-based substance having a hydrophobic protecting group of formula (P1) or formula (P2) is synthesized using an amidite reagent represented by the following formula (CR1) as a hydrophobic reagent. 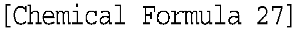 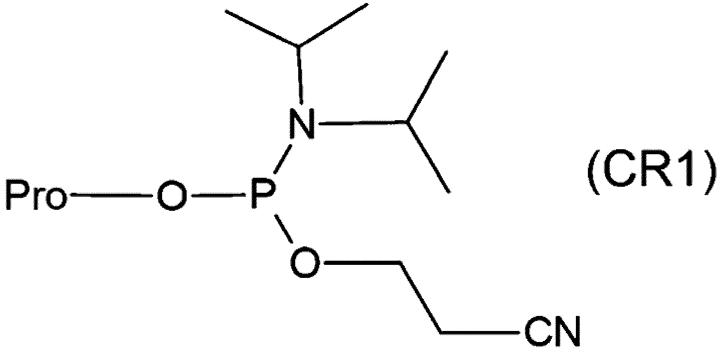 wherein Pro represents the above-mentioned hydrophobic protecting group (namely, the formula (P1) or (P2)).

The amidite reagent represented by the formula (CR1) can be obtained by reacting a phosphoramidite compound such as 2-cyanoethyl-N,N'-diisopropylchlorophosphoramidite with nitrobenzyl alcohol as a starting material.

In the method using the amidite reagent, a protecting group-introduced nucleotide is synthesized in the protecting group introduction step by introducing the amidite reagent into a nucleotide. Next, a mononucleotide is sequentially linked to the 3'-end side of a protecting group-introduced nucleotide by solid phase synthesis, and thereby a hydrophobic nucleotide-based substance is synthesized. The solid phase synthesis can be performed using a publicly known DNA synthesis apparatus. The hydrophobic nucleotide-based substance as a synthetic term can be isolated and purified from other nucleic acids using hydrophobicity (fat-solubility) by reverse-phase HPLC or the like, and further can be deprotected by photoirradiation or the like.

### (2) Enzymatic synthesis

The lower part (b) of the phosphorylation purification scheme shows a scheme for synthesizing a hydrophobic nucleotide-based substance by an enzymatic method. When the nucleotide-based substance is an RNA, the enzyme to be used is preferably an RNA polymerase, and particularly preferably T7 RNA polymerase. The above scheme shows an example in which an RNA is synthesized using T7 RNA polymerase. In this case, in the protecting group introduction step, a nucleic acid (DNA) having a sequence complementary to a nucleotide-based substance on the downstream side of a T7 promoter sequence is used as a template. As substrates for T7 RNA polymerase, a hydrophobic substrate (guanosine monophosphate derivative) of the following formula (ER1) and a nucleoside triphosphate (NTP, i.e., ATP, GTP, CTP, or UTP) are used. 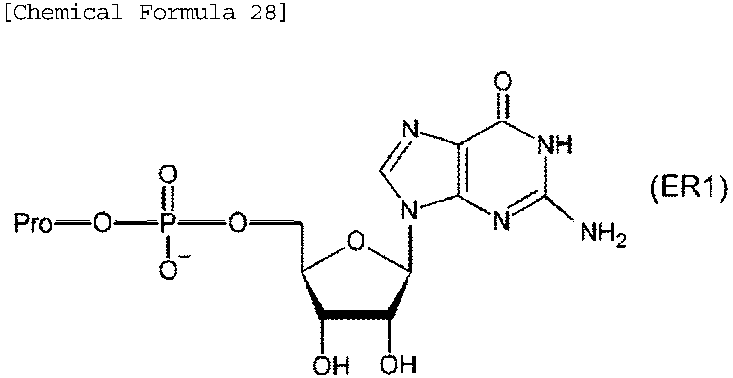 wherein Pro represents the above-mentioned hydrophobic protecting group.

In the reaction of T7 RNA polymerase, transcription is initiated from GTP, and therefore when a guanosine monophosphate derivative and guanosine triphosphate (GTP) are used as substrates, two types of RNA, i.e., one having a guanosine monophosphate derivative at the 5'-end and one having guanosine triphosphate at the 5'-end, are generated in a mixed state. These two types of RNA can be separated according to hydrophobicity by using reverse-phase HPLC or the like, and only a hydrophobic nucleotide-based substance derived from the guanosine monophosphate derivative can be isolated and purified. The transcription of the template by the T7 RNA polymerase can be performed under appropriately set conditions, and for example, the reaction temperature may be set to 30 to 45°C, and the reaction time may be set to 1 to 5 hours. In general, T7, T3, and SP6 RNA polymerases initiate transcription from GTP. T7 RNA polymerase recognizes GTP, and an RNA chain extends to the 2'-hydroxyl group of the GTP. Therefore, when a hydrophobic substrate (guanosine monophosphate derivative) of the above formula (ER1) is used, not only T7 RNA polymerase but also T3 and SP6 RNA polymerases can be used. On the other hand, E. coli RNA polymerase, which is commercially available from New England Biolabs and so on, initiates transcription also from ATP. When this enzyme is used, an RNA chain can be extended from the 2'-hydroxyl group by using an adenosine monophosphate derivative having adenine instead of the guanine of ER1.

### 3. Adenylation purification

Adenylation purification is a method in which an adenylated RNA is generated by a transcription reaction using an adenylation purification tag and is isolated with high purity. An adenylation moiety is co-transcriptionally introduced into the 5'-end of an RNA transcript, and the resulting product can be separated from coexisting RNAs by reverse-phase HPLC or the like utilizing the hydrophobicity of a hydrophobic protecting group (adenylation purification tag) introduced into the adenyl group moiety. Thereafter, the protecting group is removed by photoirradiation or the like, and an objective adenylated RNA can thereby be obtained.

The adenylation purification tag is a hydrophobic tag for obtaining an adenylated nucleic acid. The adenylated nucleic acid has a structure in which an adenosine monophosphate derivative with a hydrophobic protecting group introduced therein is bonded to a nucleotide on the 3'-side with two phosphate groups interposed therebetween. Examples of the adenylation purification as well include a method of introducing a hydrophobic protecting group through chemical synthesis and a method of introducing a hydrophobic protecting group through enzyme synthesis. The following synthesis scheme (hereinafter, "adenylation purification scheme") also shows an example using "bNB". Also in this example, the bNB may be appropriately replaced by another compound of the formula (P1) or the formula (P2).

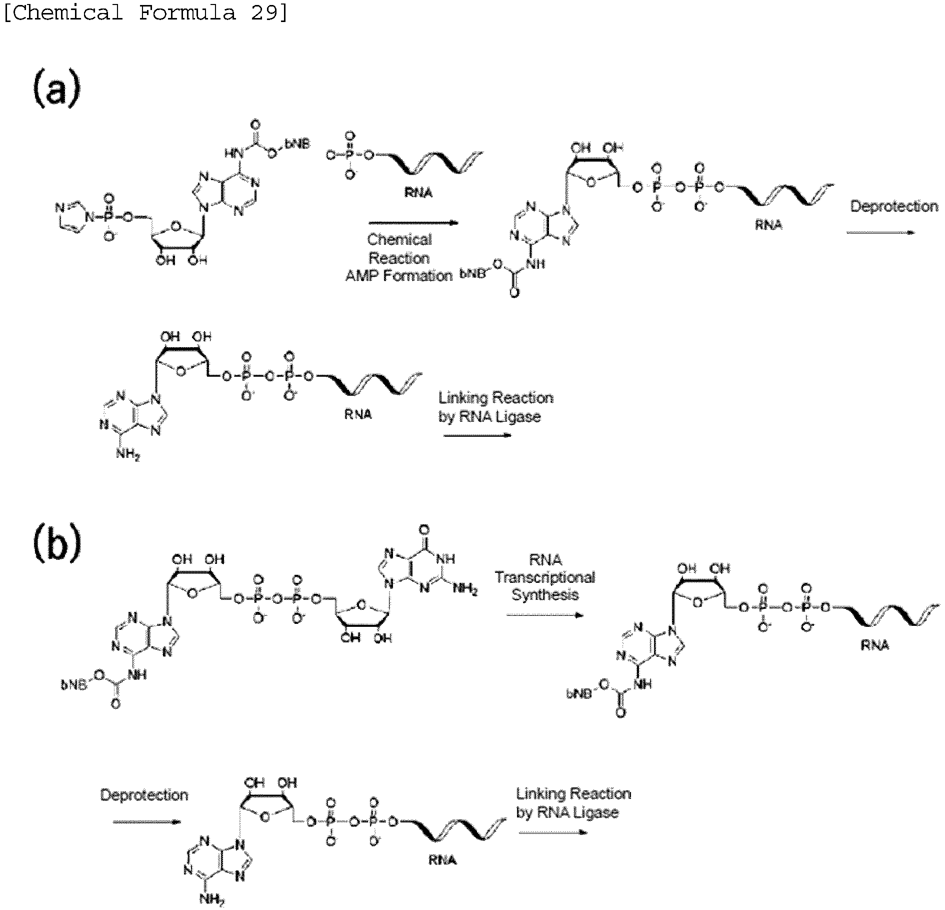

### (1) Chemical synthesis

(a) of the adenylation purification scheme shows a scheme of synthesizing and purifying a hydrophobic nucleotide-based substance by a chemical method. In this method, a hydrophobic nucleotide-based substance is synthesized using an adenylation reagent represented by the following formula (CA1) as a hydrophobic reagent, 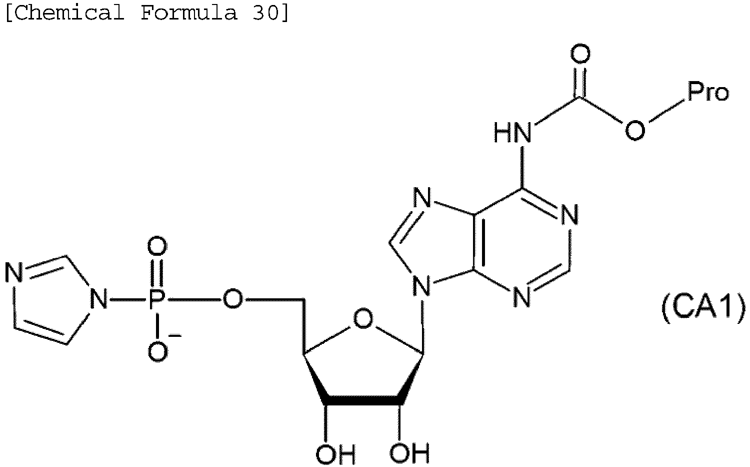 wherein Pro represents the above-mentioned hydrophobic protecting group (namely, the formula (P1) or (P2)).

The adenylation reagent represented by the formula (CA1) can be synthesized by the following procedure. First, a nitrophenylimidazole compound is synthesized using iodonitrobenzene as a starting material, reacted with adenosine in which a hydroxy group or the like is protected with a protecting group such as tert-butyldimethylsilane, and then deprotected to synthesize an adenosine derivative to which a hydrophobic protecting group is bonded. This adenosine derivative is reacted with imidazole, dithiodypyridine, or the like, and thus an adenylation reagent represented by the formula (CA1) can be obtained.

### (2) Enzymatic synthesis

(b) of the adenylation purification scheme shows a scheme of synthesizing and purifying a hydrophobic nucleotide-based substance by an enzymatic method. This scheme shows an example of synthesizing an RNA using T7 RNA polymerase. In this case, in the protecting group introduction step, a nucleic acid (DNA) having a sequence complementary to a nucleotide-based substance on the downstream side of a T7 promoter sequence is used as a template. As substrates for T7 RNA polymerase, a hydrophobic substrate (dinucleotide derivative) of the following formula (EA1) and a nucleoside triphosphate (NTP, i.e., ATP, GTP, CTP, or UTP) are used, 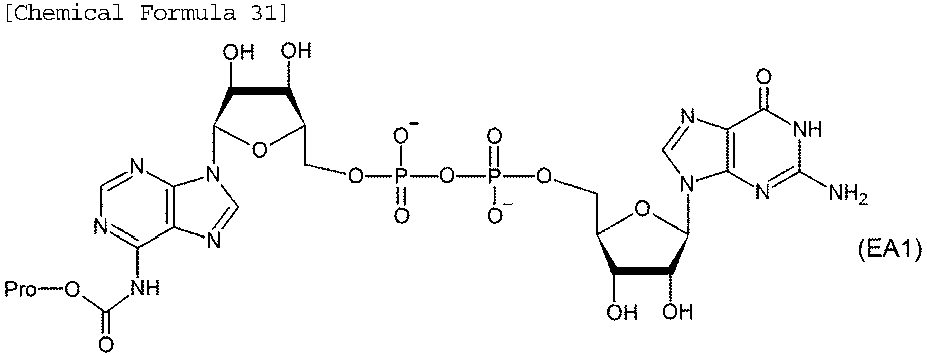 wherein Pro represents the above-mentioned hydrophobic protecting group (namely, the formula (P1) or (P2)).

The dinucleotide derivative represented by the formula (EA1) can be obtained by reacting guanosine 5'-phosphorimidazolide with the adenosine derivative of "(1) Chemical synthesis" described above.

When the dinucleotide derivative and guanosine triphosphate (GTP) are used as substrates, two types of RNA, i.e., an RNA having a protecting group-containing adenylated structure at the 5'-end and an RNA having a triphosphate structure, are generated in accordance with the mechanism of T7 RNA polymerase that initiates a transcription reaction from a guanosine. In the RNA having a protecting group-containing adenylated structure at the 5'-end, an adenosine derivative at the 5'-end and a nucleotide on the 3'-end side thereof are bonded with a diphosphate group interposed therebetween. These two types of RNA can be separated according to hydrophobicity by using reverse-phase HPLC or the like, and only the RNA having the protecting group-containing adenylated structure can be isolated and purified. The transcription of the template by the T7 RNA polymerase can be performed under appropriately set conditions, and for example, the reaction temperature may be set to 30 to 45°C, and the reaction time may be set to 1 to 5 hours.

Documents disclosing existing techniques related to adenylation purification include the following.

Yangming Wang and Scott K. Silverman "Efficient RNA 5'-adenylation by T4 DNA ligase to facilitate practical applications" RNA 2006, 12, 1142-1146. doi: 10.1261/rna.33106

### 4. Capping purification

Eukaryotic mRNA has a cap structure at the 5'-end. When a ribosome bonds to the cap structure, the initiation of translation of an mRNA is strongly promoted. Therefore, when an mRNA molecule is artificially produced, it is essential to provide the cap structure at the 5'-end of the molecule. Generally, a several thousands-base-long mRNA molecule is enzymatically synthesized from a template DNA by transcription with an RNA polymerase. By adding a cap analog compound that is a dinucleotide to the transcription reaction, a cap structure can be co-transcriptionally introduced into the 5'-end of the mRNA. However, since transcription is initiated also from the GTP of a coexisting transcribed substrate, the resulting RNA transcript is a mixture of a desired one having a cap analog at the 5'-end (5'-cap-RNA) and one having a triphosphate group (5'-ppp-RNA). It is known that the RNA having a triphosphate group at the 5'-end has an undesired immune response-inducing activity in vivo, and it is necessary to remove this.

5'-cap-RNA and 5'-ppp-RNA which are prepared using an existing cap compound are very similar in physical properties, and it is very difficult to separate them by a chromatography or the like. An RNA having a triphosphate group at an end (5'-ppp-RNA) can be degraded by an enzyme having a degrading activity against the RNA (RNA 5'-polyphosphatase (epicentre, RP8092H), RNA 5'-pyrophosphohydrolase (RppH) (New England Biolabs, M0356), and the like), and thus can be converted into 5'-monophosphorylated RNA using these enzymes. Furthermore, a monophosphorylated RNA can also be degraded by an enzyme such as XRN-1 (New England Biolabs, M0338) and removed (reference: Chem. Sci., 2021, 12, 4383-4388). Up to date, the isolation and purification of 5'-cap-RNA has taken time and effort as described above.

Then, in the present invention, a novel cap analog compound having a photodegradable or reductive degradable hydrophobic protecting group is used, and an objective capped RNA prepared in a co-transcriptional manner can be isolated from the above-mentioned contaminants by reverse-phase HPLC. The present method makes it possible to obtain an object having a higher purity more simply as compared especially with an enzymatic removal reaction.

The capping purification tag is a hydrophobic tag for obtaining a capped nucleic acid. The capped nucleic acid has a structure in which a cap structure with a hydrophobic protecting group introduced therein is bonded to a nucleotide on the 3'-side with three or four phosphate groups interposed therebetween. Examples of the capping purification as well include a method of introducing a hydrophobic protecting group through chemical synthesis and a method of introducing a hydrophobic protecting group through enzyme synthesis. The following synthesis scheme (hereinafter, "capping purification scheme") also shows an example using "bNB". Also in this example, the bNB may be appropriately replaced by another compound of the formula (P1) or the formula (P2). In the following capping purification scheme, only a scheme using a capping reagent of the formula (CC3) described later is shown. However, the present scheme is not limited thereto, and capping of a nucleic acid and purification of a hydrophobic nucleotide-based substance can be performed by a similar scheme even when a capping reagent of the formula (CC1), (CC2), or (CC4) is used.

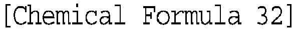

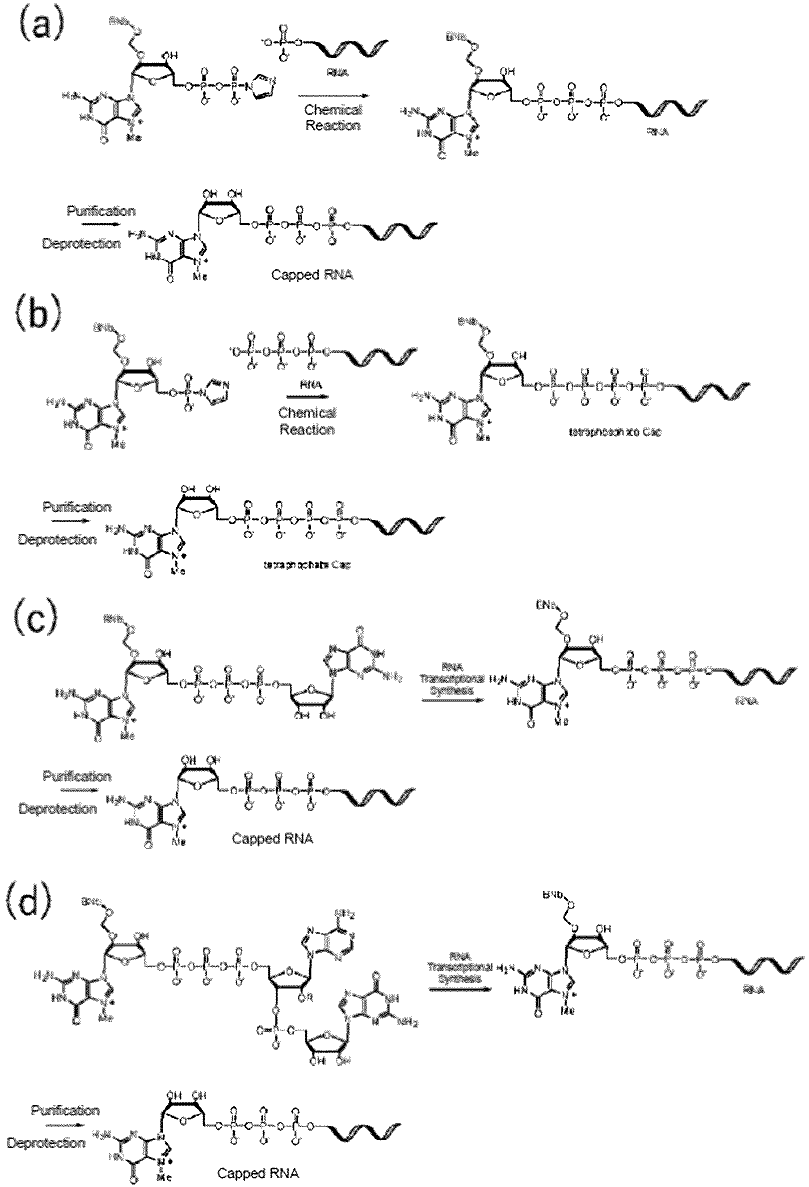

### (1) Chemical synthesis

(a) and (b) of the capping purification scheme each show a scheme of synthesizing and purifying a hydrophobic nucleotide-based substance by a chemical method. In this method, hydrophobic nucleotide-based substances are synthesized using capping reagents represented by the following formulas (CC1) to (CC4) as a hydrophobic reagent, 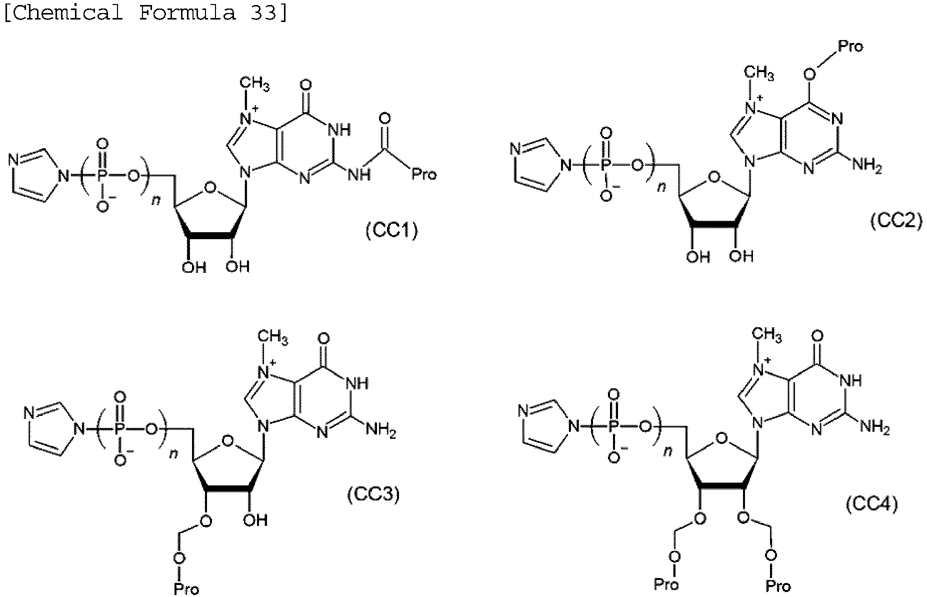 wherein Pro represents the above-mentioned hydrophobic protecting group (namely, the formula (P1) or (P2)), and n represents an integer of 1 or 2.

The capping reagents represented by the formulas (CC1) to (CC4) can be synthesized by the following procedure. First, a nitrophenylimidazole compound is synthesized using iodonitrobenzene as a starting material, reacted with guanosine in which a hydroxy group or the like is protected with a protecting group such as tert-butyldimethylsilane, and then deprotected to synthesize a guanosine derivative to which a hydrophobic protecting group is bonded. Iodomethane, dimethyl sulfoxide (DMSO) or the like is reacted with the guanosine derivative to methylate the 7' position of guanosine, and imidazole, dithiodypyridine or the like is reacted to bond imidazole to a phosphate group, whereby capping reagents represented by the formulas (CC1) to (CC4) can be obtained. Besides imidazole, various leaving groups can be used, and examples of such leaving groups include various imidazole derivatives and nitrogen-containing heteroaromatic compounds described in Japanese Patent Application No. 2020-032889 such as 1-methylimidazole and 4-methylimidazole.

(a) of the capping purification scheme shows a scheme in which an RNA having monophosphoric acid at the 5'-end is reacted with a diphosphate form capping reagent (n in the above formulas (CC1) to (CC4) is an integer of 2). In this case, the hydrophobic nucleotide-based substance to be generated has a structure in which the cap derivative at the 5'-end and the nucleoside at the 3'-end side are bonded with three phosphate groups interposed therebetween. On the other hand, (b) of the capping purification scheme shows a scheme in which an RNA having a triphosphate at the 5'-end is reacted with a monophosphate form capping reagent (n in the above formulas (CC1) to (CC4) is an integer of 1). In this case, the hydrophobic nucleotide-based substance to be generated has a structure in which the cap derivative at the 5'-end and the nucleoside at the 3'-end side are bonded with four phosphate groups interposed therebetween.

### (2) Enzymatic synthesis

(c) of the capping purification scheme shows a scheme of synthesizing and purifying a hydrophobic nucleotide-based substance by an enzymatic method. This scheme shows an example of synthesizing an RNA using T7 RNA polymerase. In this case, in the protecting group introduction step, a nucleic acid (DNA) having a sequence complementary to a nucleotide-based substance on the downstream side of a T7 promoter sequence is used as a template. As substrates for T7 RNA polymerase, a hydrophobic substrate (cap derivative) containing a structure of the following formula (EC0) and a nucleoside triphosphate (NTP, i.e., ATP, GTP, CTP, or UTP) are used.

In US 20180273576 A1 [Compositions and methods for synthesizing 5'-capped RNAs], a transcription reaction is performed using a cap analog having a trinucleotide composition. It is possible to introduce a hydrophobic tag into this analog. Scheme (d) shows a method of introducing a cap purification tag through a transcription reaction using a trinucleotide as a substrate, 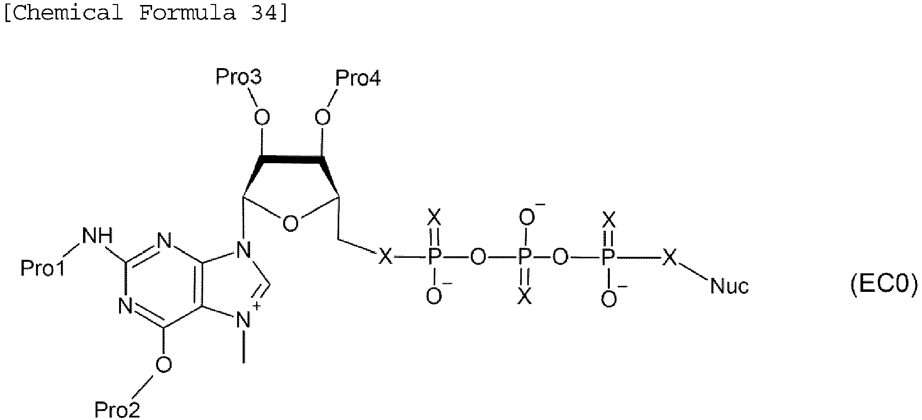 wherein Pro1 to Pro4 each represent the hydrophobic protecting group or hydrogen, provided that at least one of Pro1 to Pro4 is a hydrophobic protecting group, and Pro1 to Pro4 may be the same or different from each other; X is selected from the group consisting of oxygen, sulfur and selenium atoms; and Nuc represents a natural or non-natural nucleoside or one or more natural or non-natural nucleotides on carbon at a 3'-position thereof.

At least one of Pro1 to Pro4 is a hydrophobic protecting group represented by the above formula (P1) or (P2), and the rest are hydrogen. The number of hydrophobic protecting groups as Pro1 to Pro4 is preferably one or two.

The nucleoside constituting Nuc has a structure in which a base is bonded to the carbon at the 1'-position of a ribose, which is a sugar, or a derivative thereof. While a hydroxy group is bonded to the carbon at the 2'-position of the ribose, examples of a ribose derivative include a ribose derivative in which a hydroxy group is substituted with an alkyl group, and a ribose derivative in which oxygen bonded to the carbon at the 2'-position and carbon bonded to the carbon at the 4'-position are bonded to each other to form a heterocyclic ring. The base is the same as the Base described later, and is a natural nucleobase or a non-natural base. Examples of the natural nucleobase include adenine, cytosine, thymine, uracil, and guanine. Examples of the non-natural base may be N-methyladenine, N-benzoyladenine, 2-methylthioadenine, 2-aminoadenine, 7-methylguanine, N-isobutyrylguanine, 5-fluorocytosine, 5-bromocytosine, 5-methylcytosine, 4-N-methylcytosine, 4-N,N-dimethylcytosine, 5-fluorouracil, 5-bromouracil, 5-chlorouracil, and 5,6-dihydrouracil.

To the carbon at the 3'-position of the nucleoside constituting Nuc may be linked one or two or more (preferably two) nucleotides. In this case, it is preferable that the carbon at the 3'-position of the nucleoside and the carbon at the 5'-position of the nucleotide are linked by a phosphodiester linkage. When there are two nucleotides, it is preferable that the nucleotides are 5'-3'-phosphodiester linked. The nucleotides are, similarly to the nucleoside constituting Nuc described above, composed of a sugar and a base, and the sugar is composed of a ribose or a derivative thereof, and the base is composed of a natural nucleobase or a non-natural base. Specific examples of Nuc include DNA, RNA, LNA, 2'OMe-RNA, 2'-methoxyethyl RNA, acyclic nucleosides, and linkers containing a heteroatom.

Examples of the hydrophobic substrate represented by the formula (EC0) include hydrophobic substrates selected from the group consisting of the following formulas (EC1) to (EC4) and the following formulas (EC5) to (EC12). 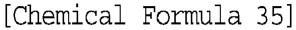 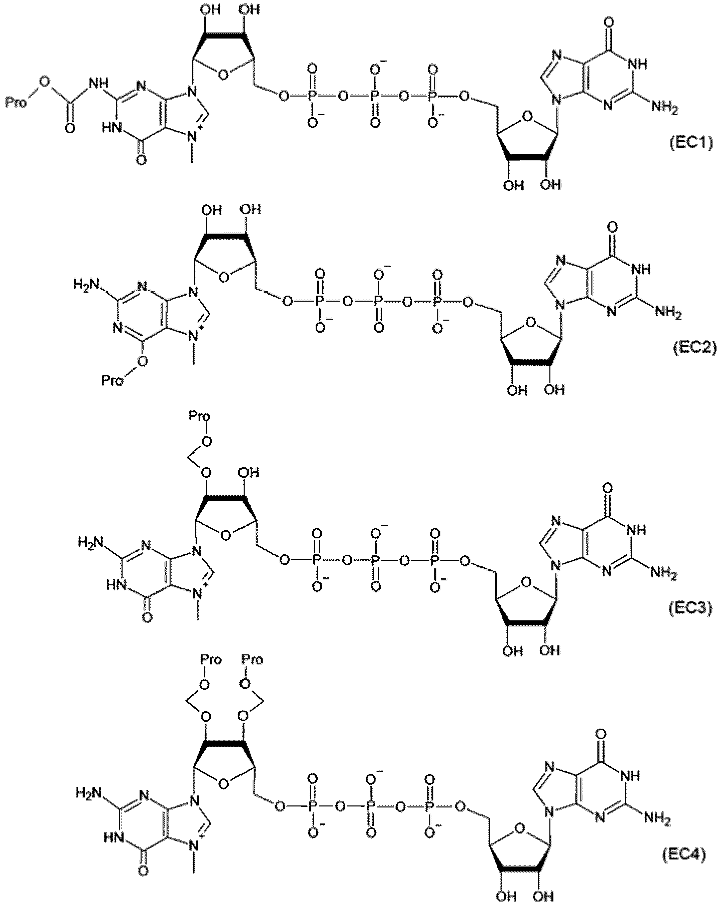 wherein Pro represents the above-mentioned hydrophobic protecting group (namely, the formula (P1) or (P2)).

The cap derivatives represented by the formulas (EC1) to (EC4) can be obtained by reacting guanosine 5'-phosphorimidazolide with the formulas (CA1) to (CA4), which are capping reagents of the "(1) Chemical synthesis" described above, respectively.

As a cap derivative, compounds represented by the following formulas (EC5) to (EC12) can also be used. 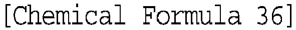 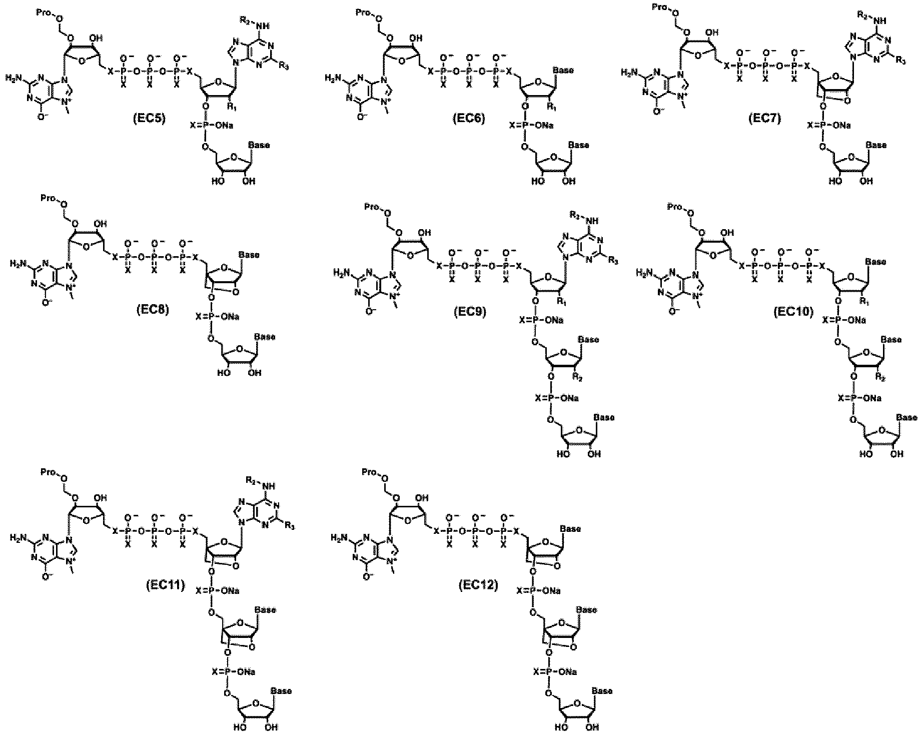 wherein R₁ represents a substituent selected from among a hydroxy group (OH), a methoxy group (OCH₃), methoxyethyl (-OCH₂OCH₃), fluorine (F), and a formamide group (-NHCHO), R₂ represents hydrogen, a linear or branched alkyl group having 1 to 10 carbon atoms, and R₃ represents hydrogen or an amino group (-NH₂); wherein Base is a natural nucleobase selected from among adenine, cytosine, thymine, uracil, and guanine, or represents a non-natural base; wherein X is selected from the group consisting of oxygen, sulfur, and selenium atoms; and wherein Pro represents a hydrophobic protecting group represented by the formula (P1) or (P2) .

Herein, examples of the natural nucleobase and the non-natural base include adenine, guanine, cytosine, thymine, uracil, N-methyladenine, N-benzoyladenine, 2-methylthioadenine, 2-aminoadenine, 7-methylguanine, N-isobutyrylguanine, 5-fluorocytosine, 5-bromocytosine, 5-methylcytosine, 4-N-methylcytosine, 4-N,N-dimethylcytosine, 5-fluorouracil, 5-bromouracil, 5-chlorouracil, and 5,6-dihydrouracil.

When the cap derivative and guanosine triphosphate (GTP) are used as substrates, two types of RNA, i.e., an RNA having a protecting group-containing capped structure at the 5'-end and an RNA having a triphosphate group at the 5'-end, are generated. In the RNA having a protecting group-containing capped structure at the 5'-end, a cap structure at the 5'-end and a nucleotide on the 3'-end side thereof are bonded with a triphosphate group interposed therebetween. These two types of RNA can be separated according to hydrophobicity by using reverse-phase HPLC or the like, and only the RNA having the protecting group-containing capped structure can be isolated and purified. The transcription of the template by the T7 RNA polymerase can be performed under appropriately set conditions, and for example, the reaction temperature may be set to 30 to 45°C, and the reaction time may be set to 1 to 5 hours.

Among the cap derivatives described above, for the formulas (EC3) and (EC5) to (EC12), an mRNA in a state where a hydrophobic protecting group is bonded exhibits a higher translation activity than or equal to that of an mRNA in a state where no hydrophobic protecting group is bonded as shown in Examples described later. Therefore, when a cap derivative of the formula (EC3) is used, translation can be performed before the deprotection step by using a nucleotide-based substance in a state where a hydrophobic protecting group is bonded.

### 5. Device for purifying nucleotide-based substance

Next, the device for purifying a nucleotide-based substance of the present invention will be described. The device for purifying a nucleotide-based substance is a device for carrying out the above-described method for purifying a nucleotide-based substance, and comprises a protecting group introduction means, an isolation and purification means, and a deprotection means.

The protecting group introduction means is a means for carrying out the protecting group introduction step, and introduces a hydrophobic protecting group represented by the formula (P1) or (P2) into a nucleotide-based substance to generate a hydrophobic nucleotide-based substance. In the "chemical synthesis" of the above phosphorylation purification, examples of the protecting group introduction means include an amidite reagent represented by the formula (CR1), and various reagents, reaction devices, and so on to be used for phosphorylation purification. In the "enzyme synthesis" of the above phosphorylation purification, examples of the protecting group introduction means include a hydrophobic substrate represented by the formula (ER1), NTP, and various reagents, reaction devices, and so on to be used for the phosphorylation purification.

In the "chemical synthesis" of the adenylation purification, examples of the protecting group introduction means include adenylation reagents represented by the formulas (CA1) to (CA4), and various reagents, reaction devices, and so on to be used for adenylation purification. In the "enzyme synthesis" of the above adenylation purification, examples of the protecting group introduction means include a hydrophobic substrate represented by the formula (EA1), NTP, and various reagents, reaction devices, and so on to be used for the adenylation purification.

Further, in the "chemical synthesis" of the capping purification, examples of the protecting group introduction means include a capping reagent represented by the formula (CA1), and various reagents, reaction devices, and so on to be used for capping purification. In the "enzyme synthesis" of the above capping purification, examples of the protecting group introduction means include hydrophobic substrates having a structure of the formula (EC0), for example, hydrophobic substrates represented by the formulas (EC1) to (EC4) and the formulas (EC5) to (EC12), NTP, and various reagents, reaction devices, and so on to be used for capping purification.

The isolation and purification means is a means for performing the isolation and purification step described above, and isolates and purifies a hydrophobic nucleotide-based substance under a hydrophobic environment. The isolation and purification device is not particularly limited as long as it is under hydrophobic conditions, and examples thereof include liquid chromatography and membrane separation devices, and the liquid chromatography may be reverse-phase high-performance liquid chromatography.

The deprotection means is a means for performing the above deprotection step, and deprotects a hydrophobic protecting group from a hydrophobic nucleotide-based substance to generate a nucleotide-based substance. Examples of the deprotection means include a device that performs photoirradiation treatment, reduction treatment, or the like. Examples of the device that performs photoirradiation treatment include a light source device that emits light having a wavelength of 300 to 400 nm for 1 to 30 minutes. Examples of the device that performs reduction treatment include a reaction device in which a hydrophobic nucleotide-based substance is treated with a reducing agent such as sodium dithionite (Na₂S₂O₄) at 25 to 80°C for 1 to 30 minutes. When the reaction temperature is excessively high (50°C or higher), the decomposition of sodium dithionite precedes, and the reduction reaction efficiency tends to decrease. Therefore, the reaction temperature is more preferably in the range of 25 to 50°C, and for example, 25°C or 37°C is particularly preferable. Similarly in the other treatments, deprotection is performed using a device for deprotecting a degradable protecting group (deprotection device).

### 6. mRNA drug and method for producing same

The hydrophobic nucleotide-based substance of the present invention is also useful as an mRNA drug. That is, the mRNA drug of the present invention is an mRNA drug comprising a hydrophobic nucleotide-based substance in which a hydrophobic protecting group represented by the following formula (P1) or (P2) is introduced into a nucleotide-based substance having at least one nucleotide and/or a derivative thereof as a constituent unit, 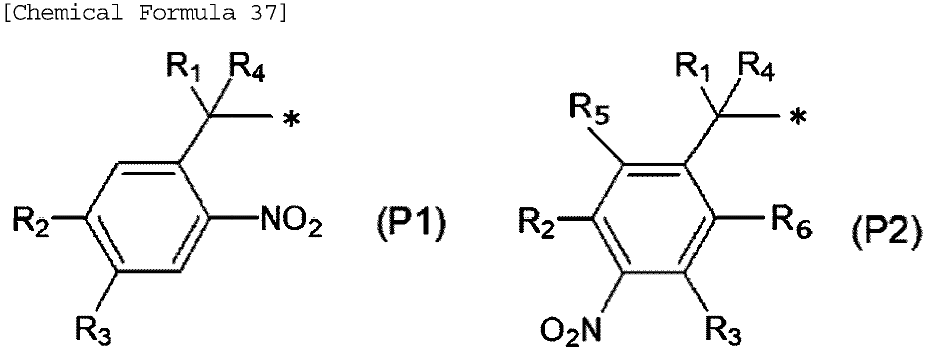 wherein R₁ represents a linear or branched alkyl group having 1 to 30 carbon atoms, R₄ represents hydrogen or a linear or branched alkyl group having 1 to 10 carbon atoms, R₂, R₃, R₅ and R₆ each represent hydrogen, a linear or branched alkyl group having 1 to 10 carbon atoms, or a linear or branched alkoxy group having 1 to 10 carbon atoms, and R₂, R₃, R₅ and R₆ may be the same or different; and * means a bond with a nucleotide-based substance, and is bonded to an atom constituting the nucleotide-based substance directly or with a linking group selected from -O-C- and -O-C(=O)- interposed therebetween.

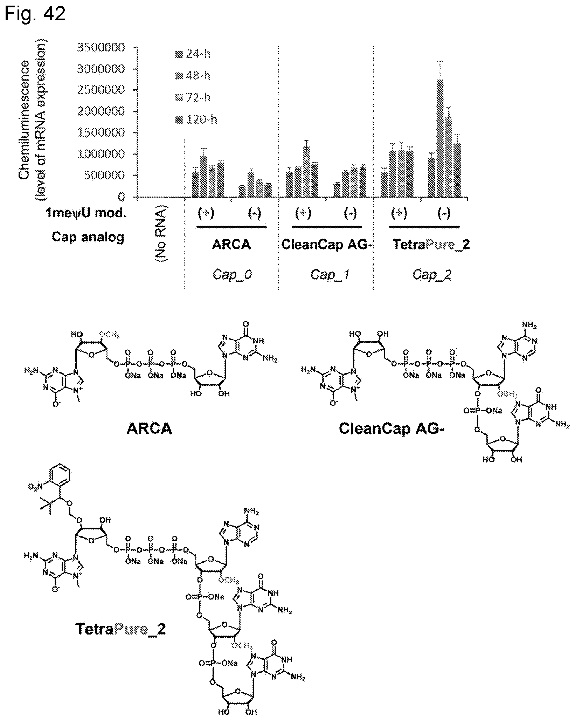

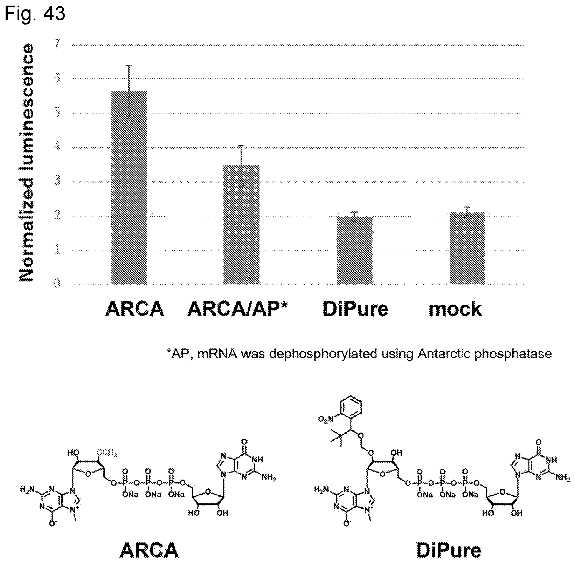

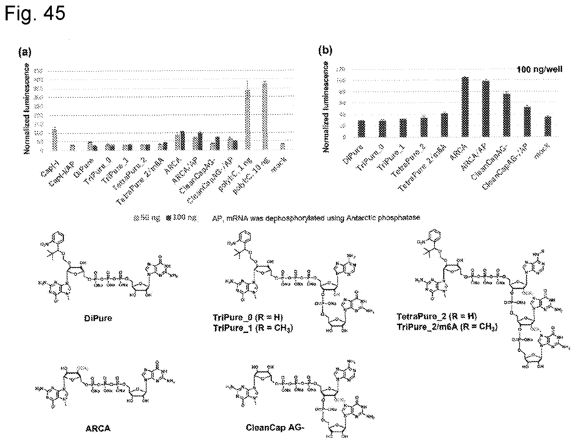

From the viewpoint of the degree of translation activity, the mRNA drug is particularly preferably composed of an mRNA having a cap structure (PureCap type mRNA of Examples described later). Some PureCap type mRNAs exhibit higher translation activity in cells than conventional mRNAs with methylpseudouridine introduced therein as described in Fig. 42 of Examples described later (those using the formulas (EC5) to (EC6) and (EC9) to (EC10), preferably the formulas (EC10) and (EC9)). Furthermore, some PureCap type mRNAs exhibit a lower immune response than conventional mRNAs such as ARCA (Anti-Reverse Cap Analog) as shown in Figs. 43 and 45 (those using the formulas (EC1) to (EC12), preferably the formulas (EC3), (EC6), (EC5), (EC10), and (EC9)). From these viewpoints, PureCap type mRNAs are particularly useful as drugs as compared with conventional mRNAs.

An mRNA drug having a cap structure has a structure in which an RNA is linked to the carbon at the 3'-position of the nucleotide on the 3'-end side of the hydrophobic substrate containing the structure of the formula (EC0), 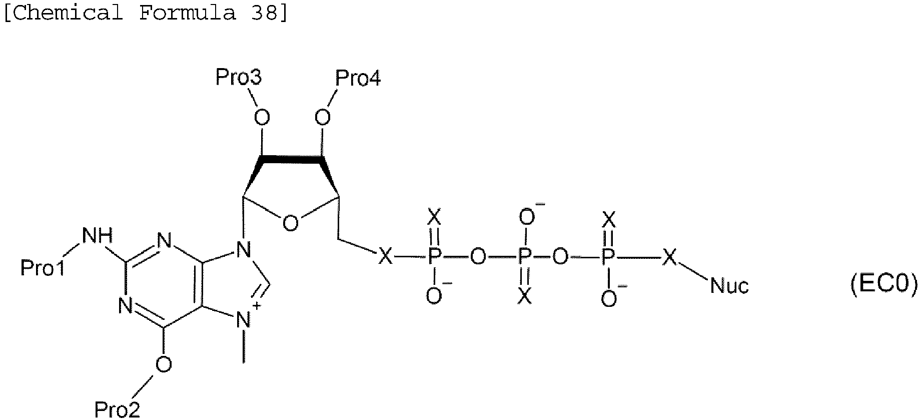 wherein Prol to Pro4 each represent the hydrophobic protecting group or hydrogen, provided that at least one of Prol to Pro4 is a hydrophobic protecting group, and Prol to Pro4 may be the same or different from each other; X is selected from the group consisting of oxygen, sulfur and selenium atoms; and Nuc represents a natural or non-natural nucleoside or one or more natural or non-natural nucleotides on carbon at a 3'-position thereof. Nuc represents a natural or non-natural nucleoside, to the carbon at the 3'-position of which one or two natural or non-natural nucleotides may be linked. Note that details of the formula (EC0) are the same as those described above, and thus detailed description thereof is omitted here.

An RNA has a sequence encoding a protein or peptide having a pharmaceutical effect, and is translated by a ribosome to generate such a useful protein or peptide.

Examples of the hydrophobic substrate represented by the formula (EC0) include hydrophobic substrates selected from the group consisting of the formulas (EC1) to (EC12), 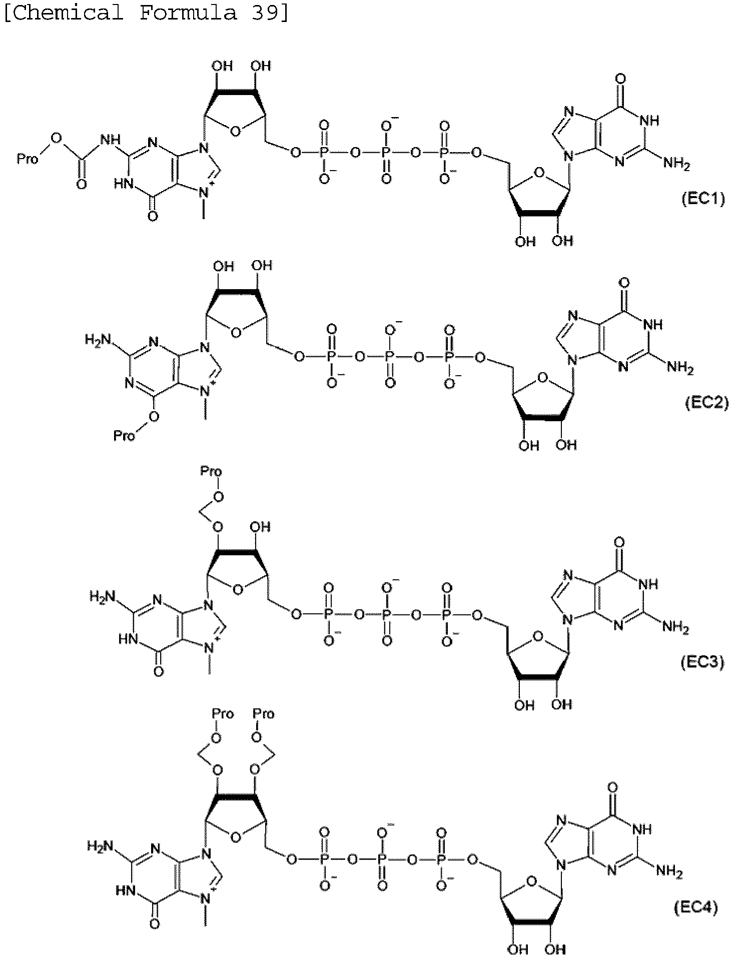 wherein Pro represents the above-mentioned hydrophobic protecting group, 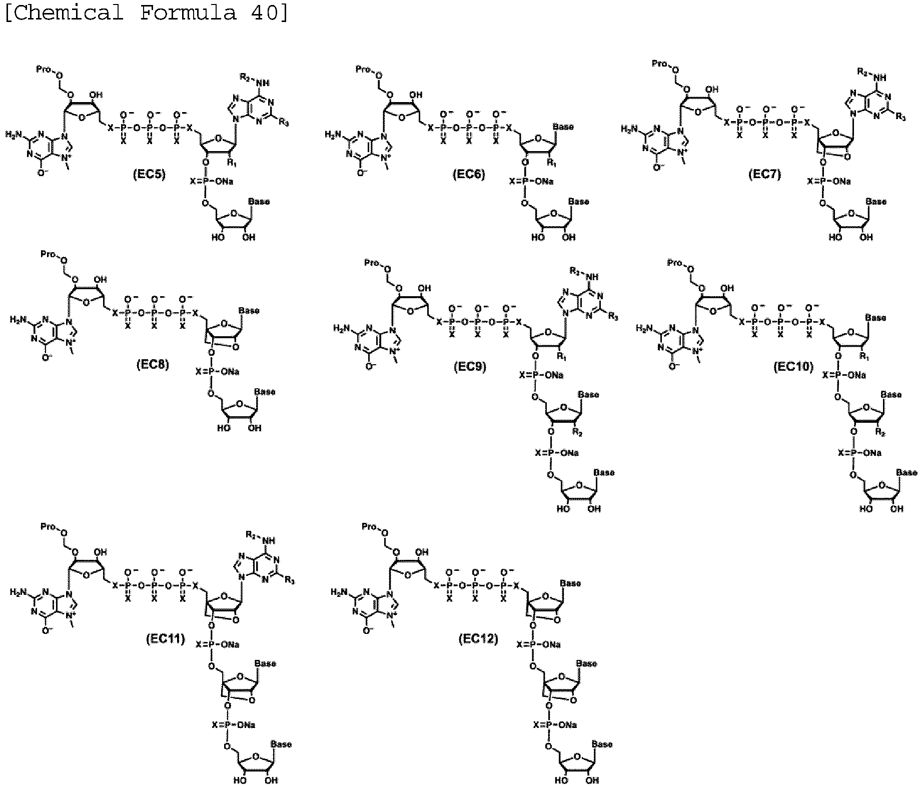 wherein R₁ represents a substituent selected from among a hydroxy group (OH), a methoxy group (OCH₃), methoxyethyl (-OCH₂OCH₃), fluorine (F), and a formamide group (-NHCHO), R₂ represents hydrogen, a linear or branched alkyl group having 1 to 10 carbon atoms, and R₃ represents hydrogen or an amino group (-NH₂); wherein Base is a natural nucleobase selected from among adenine, cytosine, thymine, uracil, and guanine, or represents a non-natural base; wherein X is selected from the group consisting of oxygen, sulfur, and selenium atoms; and wherein Pro represents a hydrophobic protecting group represented by the formula (P1) or (P2) .

A nucleotide-based substance resulting from deprotection of a hydrophobic protecting group of a hydrophobic nucleotide-based substance in a state where the hydrophobic protecting group described above is bonded thereto is also useful as a drug. That is, the mRNA drug of the present invention includes both a hydrophobic nucleotide-based substance and a nucleotide-based substance in a state where a hydrophobic protecting group is deprotected from the hydrophobic nucleotide-based substance. The latter nucleotide-based substance is specifically a nucleotide-based substance having at least one nucleotide and/or a derivative thereof as a constituent unit, and is a nucleotide-based substance in which a hydrophobic protecting group represented by the formula (P1), (P2), or (P3) is deprotected and hydrogen or a substituent is bonded to *, which is a bond with a nucleotide-based substance.

As such a nucleotide-based substance resulting from deprotection of a hydrophobic protecting group, the aforementioned mRNA drug having a cap structure is preferable from the viewpoint of the degree of high translation activity and the degree of low immune responsiveness. A specific example is a nucleotide-based substance in which an RNA is linked to the carbon at the 3'-position of the nucleotide on the 3'-end side of the hydrophobic substrate containing the structure of the formula (EC0), wherein all of Prol to Pro4 of the formula (EC0) are hydrogen as a result of deprotection of the hydrophobic protecting group.

Examples of the nucleotide-based substance represented by the formula (EC0) in a state where the formulas (EC1) to (EC12) are deprotected specifically include nucleotide-based substances of the formulas (EC1) to (EC12) each having a hydrophobic protecting group bonded, wherein Pro is hydrogen as a result of deprotection of the hydrophobic protecting group.

Furthermore, the present invention includes a method for producing the mRNA drug described above, and the production method comprises a protecting group introduction step of introducing a hydrophobic protecting group represented by the following formula (P1) or (P2) into a nucleotide-based substance to produce a hydrophobic nucleotide-based substance.

When the mRNA drug has a cap structure, the protecting group introduction step is a method of synthesizing a hydrophobic nucleotide-based substance by employing, as a template, a nucleic acid having a sequence complementary to the nucleotide-based substance, employing, as substrates, a hydrophobic substrate having a structure of the formula (EC0) and a nucleoside triphosphate, and transcribing the template by an RNA polymerase. Examples of the hydrophobic substrate having the structure of formula (EC0) include hydrophobic substrates selected from the group consisting of (EC1) to (EC12). Since the protecting group introduction step has already been described in detail, detailed description thereof is omitted here.

The mRNA drug of the present invention can be administered to a cell or a tissue by a method of including it in a carrier such as a solid lipid nanoparticle (SNP) or a lipid nanoparticle (LNP) . The lipid nanoparticle is composed of a cationic lipid, a PEGylated lipid such as ALC-0159, DSPE-mPEG, or DMG-mPEG, a neutral phospholipid such as DSPC, DPPC, or DOPE, and cholesterol.

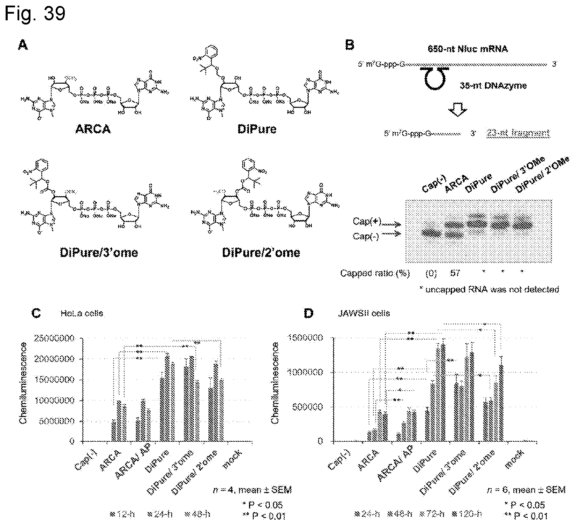

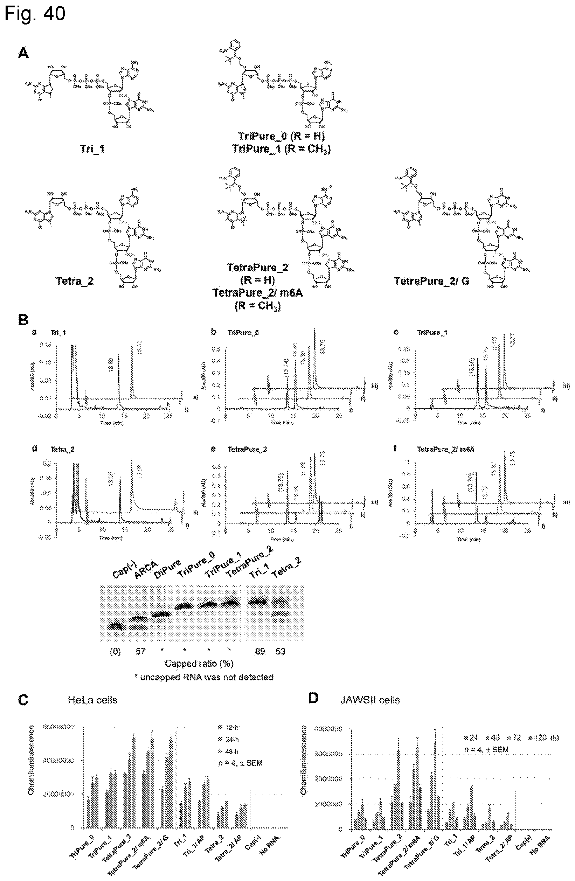

In mRNA synthesis by normal transcription, an uncapped RNA or a by-product generated by transcription may cause an immune response, leading to a decrease in the amount of protein synthesis. As shown in Figs. 39 and 40 of Examples, the mRNA drug of the present invention exhibits a high amount of protein synthesis because by-products are removed with high efficiency by use of a hydrophobic tag.

As described above, a nucleotide-based substance resulting from deprotection of a hydrophobic protecting group is also useful as a drug, and thus the method for producing an mRNA drug of the present invention also includes a method of producing a deprotected nucleotide-based substance through the isolation and purification step and the deprotection step described above. In the deprotection step, the hydrophobic protecting group represented by the formula (P1), (P2) or (P3) is deprotected, and a nucleotide-based substance in which hydrogen or a substituent is bonded to *, which is a bond with a nucleotide-based substance is generated. In the case of an mRNA having a cap structure, a nucleotide-based substance in which all of Prol to Pro4 of the formula (EC0) are hydrogen is generated. As a nucleotide-based substance with the formulas (EC1) to (EC12) deprotected as a specific example, a nucleotide-based substance in which Pro is hydrogen is generated.

### Examples

Hereinafter, the present invention will be specifically described with reference to Examples, but these do not limit the object of the present invention. In the following Examples, the expression "%" is on a mass basis (mass percent) unless otherwise specified.

### 1. Synthesis of amidite reagent and verification by 19-nt oligodeoxynucleotide (ODN) synthesis

### 1-1. Synthesis of amidite reagent

Four chemically phosphorylated amidite reagents (Compounds 1 to 4) having the following structures were synthesized.

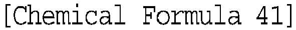

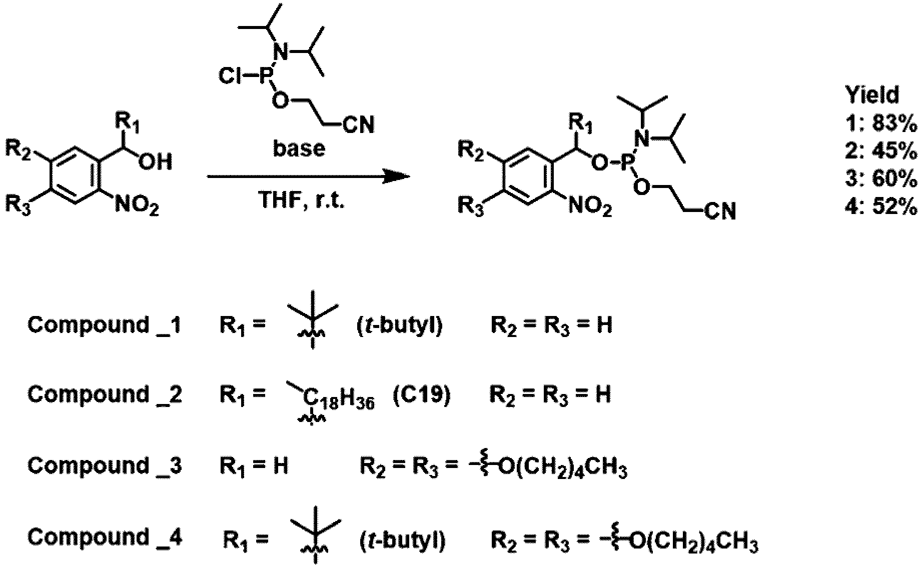

### Related preexisting study

· Chemical synthesis of long RNAs with terminal 5'-phosphate groups. Ugo Pradere, Francois Halloy, Jonathan Hall. Chem Eur J 23, 5210 (2017)
· Novel phosphorylation reagents and uses thereof. WO2018/162610A1

### (1) Synthesis of amidite reagent_1

A synthesis scheme of amidite reagent_1 is shown below.

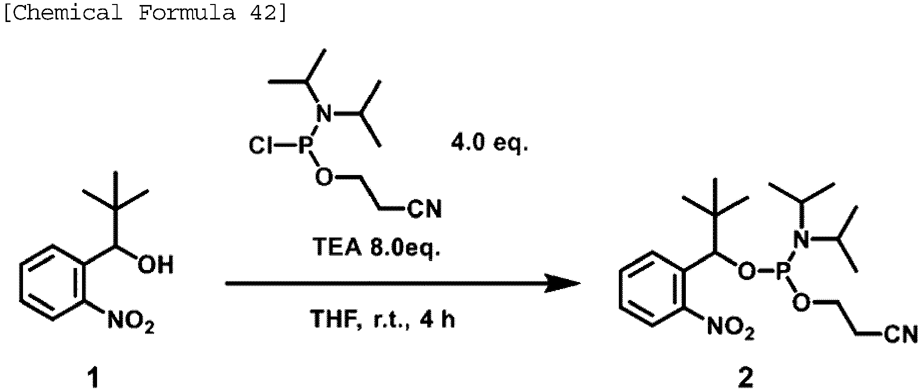

### (a) 2,2-Dimethyl-1-(2-nitrophenyl)propan-1-ol (Compound 1)

2,2-Dimethyl-1-(2-nitrophenyl)propan-1-ol is a known compound and was synthesized by the method of WO 2009152353, p. 76-77.

### (b) 2-Cyanoethyl (2,2-dimethyl-1-(2-nitrophenyl)propyl)diisopropylphosphoramidate (Compound 2)

To a solution of nitrobenzyl alcohol 1 (1.0 g, 4.8 mmol) in dry THF (19 mL) were added triethylamine (5.3 mL, 3.9 g, 38 mmol) and 2-cyanoethyl-N,N'-diisopropylchlorophosphoramidite (4.3 mL, 4.5 g, 11 mmol), and the mixture was stirred at room temperature for 4 hours. The reaction mixture was quenched with saturated aqueous sodium bicarbonate (100 mL). This solution was extracted three times with ethyl acetate (100 mL). The organic phase was dried over sodium sulfate, filtered and purified by silica gel column chromatography (hexane/ethyl acetate = 3/1, containing 1% of triethylamine), affording the corresponding phosphoramidite 2 as a yellow oil (1.6 g, yield 83%).

¹H NMR (400 MHz, CDCl₃) : δ7.77-7.70 (m, 2H), 7.61-7.53 (m, 1H), 7.41-7.36 (m, 1H), 5.45-5.40 (m, 1H), 3.90-3.49 (m, 4H), 2.76-2.62 (m, 1H), 2.36-2.17 (m, 1H), 1.28-1.16 (m, 2H), 1.18-1.13 (m, 6H), 1.00-0.98 (m, 9H), 0.87-0.86 (m, 4H) ppm.

¹³C NMR (400 MHz, CDCl₃) : δ149.78, 149.44, 135.63, 135.51, 131.78, 131.68, 131.07, 130.86, 128.09, 127.87, 123.86, 123.59, 117.75, 77.36, 77.21, 75.83, 75.71, 58.99, 58.76, 58.14, 57.91, 43.57, 43.42, 43.29, 37.23, 37.18, 37.12, 37.05, 25.89, 25.77, 24.83, 24.78, 24.73, 24.70, 24.67, 24.34, 24.27, 20.59, 20.53, 20.17, 20.10 ppm.

³¹P NMR (400 MHz, CDCl₃) : δ152.95, 148.78 ppm. ESI-TOF-MS calcd. for C₂₀H₃₂N₃NaO₄P, 432.2028 [M+Na]⁺; found, 432.1818.

### (2) Synthesis of amidite reagent_2

A synthesis scheme of amidite reagent_2 is shown below.

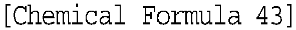

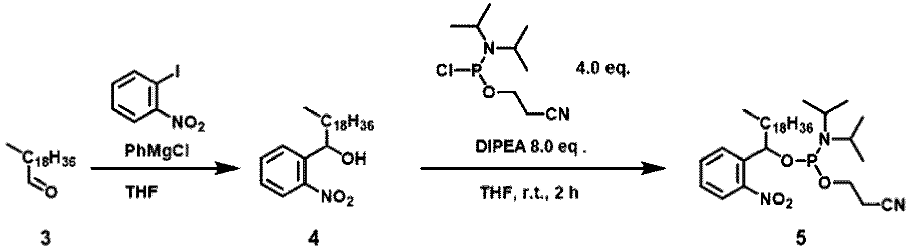

### (a) 1-(2-Nitrophenyl)icosan-1-ol (Compound 4)

1-Iodo-2-nitrobenzene (2.65 g, 10.66 mmol, 1.0 equiv.) was dissolved in THF (25 mL). Phenylmagnesium chloride 2 M in THF (5.86 mL, 11.73 mmol, 1.1 equiv.) was added dropwise at -48°C, and the mixture was stirred at -35°C for 10 minutes. Raw material 3 (3.00 g, 10.12 mmol, 0.95 equiv.) dissolved in THF (15 mL) was added dropwise, and the reaction temperature was gradually raised to room temperature, then the mixture was stirred at room temperature for 15 minutes. A saturated aqueous NH₄Cl solution (10 mL) was added, and liquid-liquid separation was performed using ethyl acetate (100 mL) and saturated saline (50 mL). The organic layer was collected and concentrated. The residue was purified by neutral flash column chromatography (hexane/ethyl acetate = 15/1 → 10/1), affording 3.44 g of intermediate 4 (8.65 mmol, yield 81%) .

¹H-NMR (400 MHz, CDCl₃) δ7.89 (dd, J = 8.2 Hz, 1.6 Hz, 1H), 7.80 (dd, J = 6.4 Hz, 1.6 Hz, 1H), 7.63 (td, J = 6.4 Hz, 1.2 Hz, 1H), 7.41 (td, J = 6.0 Hz, 1.6 Hz, 1H), 5.23 (q, J = 4.0 Hz, 1H), 2.09-1.78 (m, 3H), 1.31-1.21 (m, 33H), 0.88 (t, J = 7.2 Hz, 3H).

¹³C NMR (100 MHz, CDCl₃) δ148.1, 140.3, 133.5, 128.1, 128.0, 124.4, 69.5, 38.3, 32.0, 29.8-29.4, 26.2, 22.8, 14.2.

ESI MS-HR calcd for C₂₆H₄₅NO₃ (M+Na)⁺ 442.3297 found 442.3421.

### (b) 2-Cyanoethyl (1-(2-nitrophenyl)icosyl)diisopropylphosphoramidate (Compound 5)

To a solution of nitrobenzyl alcohol 3 (100 mg, 0.24 mmol) in dry THF (1.0 mL) were added diisopropylethylamine (2.5 mL, 250 mg, 1.3 mmol) and 2-cyanoethyl-N,N'-diisopropylchlorophosphoramidite (0.21 mL, 230 mg, 0.96 mmol), and the mixture was stirred at room temperature for 2 hours. The reaction mixture was quenched with saturated aqueous sodium bicarbonate (20 mL). This solution was extracted three times with ethyl acetate (20 mL). The organic phase was dried over sodium sulfate, filtered and purified by silica gel column chromatography (hexane/ethyl acetate = 3/1, containing 1% of triethylamine), affording the corresponding phosphoramidite 5 as a yellow oil (66 mg, yield 45%).

¹H NMR (400 MHz, CDCl₃) : δ7.89-7.77 (m, 2H), 7.66-7.58 (m, 1H), 7.42-7.36 (m, 1H), 5.44-5.33 (m, 1H), 3.94-3.80 (m, 1H), 3.67-3.42 (m, 3H), 2.72-2.61 (m, 1H), 2.45-2.30 (m, 1H), 1.86-1.70 (m, 2H), 1.55 (s, 2H), 1.36-1.08 (m, 40H), 0.90-0.86 (m, 7H) ppm.

³¹P NMR (400 MHz, CDCl₃) : δ150.68, 148.83 ppm. ESI-TOF-MS calcd. for C₃₅H₆₂KN₃O₄P, 658.4115 [M+K]+; found, 658.4246.

### (3) Synthesis of amidite reagent_3

A synthesis scheme of amidite reagent_3 is shown below.

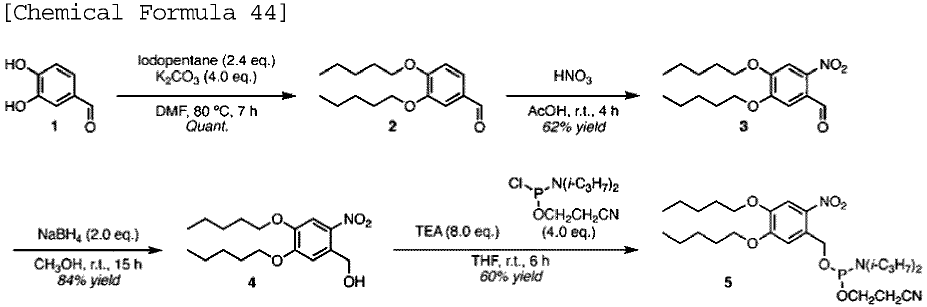

### (a) 3,4-Bis(pentyloxy)benzaldehyde (2)

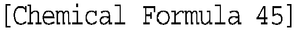

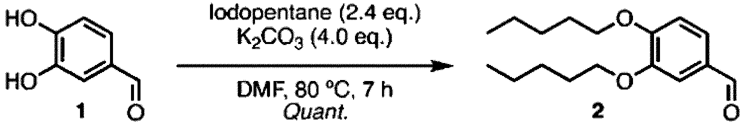

To a mixture of 3,4-dihydroxybenzaldehyde 1 (3.0 g, 22 mmol) and K₂CO₃ (12 g, 88 mmol) in dry DMF (50 mL) was added iodopentane (6.9 mL, 11 g, 53 mmol), and the resulting mixture was heated to 80°C. After stirring at 80°C for 7 hours, the reaction mixture was cooled to room temperature. DMF was removed under reduced pressure and the residue was dissolved in ethyl acetate and washed with water. The organic layer was dried over Na₂SO₄ and concentrated. The crude material was purified by silica gel column chromatography dissolved with 13 → 56% ethyl acetate/hexane, affording Intermediate 2 as a brown solid (6.2 g, quant.).

¹H NMR (600 MHz, CDCl₃) : δ9.80 (s, 1H), 7.39-7.36 (m, 2H), 6.92 (d, J = 8.4 Hz, 1H), 4.09-4.01 (m, 4H), 1.86-1.79 (m, 4H), 1.46-1.35 (m, 8H), 0.92-0.89 (m, 6H) ppm.

¹³C NMR (151 MHz, CDCl₃): δ191.05, 154.73, 149.49, 129.93, 126.69, 111.78, 110.94, 69.16, 60.44, 28.83, 28.74, 28.24, 28.20, 22.51, 22.49, 14.25, 14.09 ppm.

ESI-TOF-MS calcd. for C₁₇H₂₆NaO₃, 301.1774 [M+Na]⁺; found, 301.2054.

### (b) 2-Nitro-4,5-bis(pentyloxy)benzaldehyde (Compound 3)

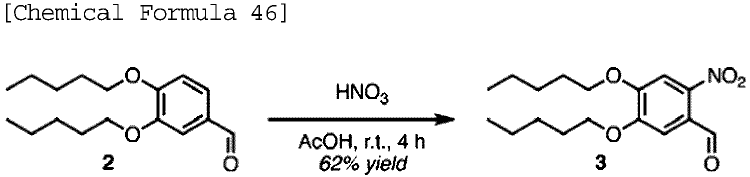

To a solution of Intermediate 2 (4.3 g, 16 mmol) in acetic acid (15 mL) was added HNO₃ (15 mL, up to 70% purity) dropwise over 17 minutes. After stirring at room temperature for 4 hours, ice was added to quench the reaction mixture. The resulting precipitate was collected by filtration and washed with water. The resulting solid was suspended in ethanol and the resulting precipitate was collected by filtration and washed with ethanol, affording Intermediate 3 as a yellow powder (3.1 g, yield: 62%).

¹H NMR (600 MHz, CDCl₃) : δ10.41 (s, 1H), 7.56 (s, 1H), 7.36 (s, 1H), 4.13-4.08 (m, 4H), 1.90-1.83 (m, 5H), 1.49-1.36 (m, 11H), 0.94-0.91 (m, 8H) ppm.

¹³C NMR (151 MHz, CDCl₃): δ188.01, 153.27, 152.37, 151.85, 143.67, 125.34, 110.69, 108.20, 107.94, 70.26, 69.92, 69.78, 28.55, 28.52, 28.46, 28.11, 28.06, 22.42, 22.38, 14.05 ppm.

ESI-TOF-MS calcd. for C₁₇H₂₅NNaO₅, 346.1630 [M+Na]⁺; found, 346.1862.

### (c) (2-Nitro-4,5-bis(pentyloxy)phenyl)methanol (Compound 4)

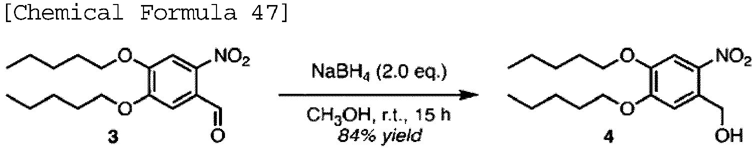

To a suspension of aldehyde 3 (2.5 g, 7.7 mmol) in dry methanol (40 mL) was slowly added NaBH₄ (0.58 g, 15 mmol) . After stirring at room temperature for 15 hours, the reaction mixture was concentrated. The residue was dissolved in dichloromethane and washed with water. The aqueous layer was extracted twice with dichloromethane. The combined organic layers were dried over Na₂SO₄ and concentrated. The residue was purified by silica gel column chromatography dissolved with hexane and then in 12 → 24% ethyl acetate/hexane, affording nitrobenzyl alcohol 4 as a brown solid (2.1 g, yield 84%).

¹H NMR (600 MHz, CDCl₃) : δ7.65 (s, 1H), 7.10 (s, 1H), 4.89 (d, J = 6.0 Hz, 2H), 4.10-4.01 (m, 4H), 1.87-1.81 (m, 4H), 1.47-1.36 (m, 8H), 0.93-0.90 (m, 6H) ppm.

¹³C NMR (151 MHz, CDCl₃): δ154.23, 147.81, 139.52, 132.19, 112.21, 109.86, 69.65, 69.51, 62.97, 28.70, 28.67, 28.17, 28.14, 22.48, 22.45, 14.07 ppm.

ESI-TOF-MS calcd. for C₁₇H₂₇NNaO₅, 348.1787 [M+Na]⁺; found, 348.1994.

### (d) 2-Cyanoethyl (2-nitro-4,5-bis(pentyloxy)benzyl)diisopropylphosphoramidate (Compound 5)

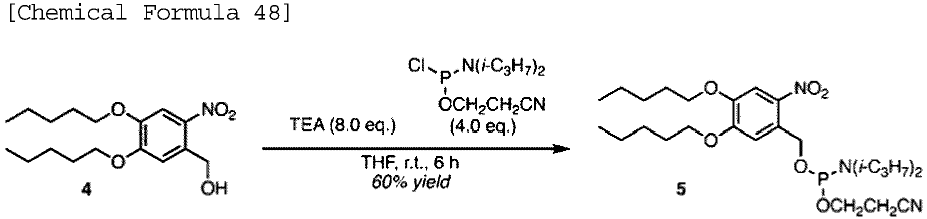

To a solution of nitrobenzyl alcohol 4 (350 mg, 1.1 mmol) in dry THF (5.0 mL) were added triethylamine (1.2 mL, 870 mg, 8.6 mmol) and 2-cyanoethyl-N,N'-isopropylchlorophosphoramidite (0.94 mL, 1.0 g, 4.3 mmol). After stirring at room temperature for 6 hours, the reaction mixture was diluted with ethyl acetate and washed twice with a saturated aqueous NaHCO₃ solution. The organic layer was dried over Na₂SO₄ and concentrated. The residue was purified by silica gel column chromatography by eluting with 1% triethylamine-containing hexanes, and subsequently 1% triethylamine-containing 2 → 6% ethyl acetate/hexane, affording the corresponding phosphoramidite 5 as a yellow oil (340 mg, yield 60%).

¹H NMR (600 MHz, CD₃CN) : δ7.64 (s, 1H), 7.28 (s, 1H), 5.06-4.99 (m, 2H), 4.10-4.00 (m, 4H), 3.87-3.75 (m, 2H), 3.70-3.64 (m, 2H), 2.64-2.62 (m, 2H), 1.92-1.91 (m, 1H), 1.80-1.73 (m, 4H), 1.44-1.33 (m, 8H), 1.19-1.17 (m, 12H), 0.91-0.86 (m, 6H) ppm.

¹³C NMR (151 MHz, CD₃CN): δ153.78, 147.33, 138.93, 130.88, 130.82, 118.52, 110.90, 109.64, 69.39, 69.23, 62.69, 62, 56, 58.69, 58.55, 43.23, 43.16, 28.53, 28.46, 27.97, 27.90, 24.16, 24.11, 23.96, 23.91, 22.20, 20.16, 20.11, 13.42 ppm.

³¹P NMR (243 MHz, CD₃CN) : δ149.03 ppm.

ESI-TOF-MS calcd. for C₂₆H₄₅N₃O₆P, 526.3041 [M+H]⁺; found, 526.3320; calcd. for C₂₆H₄₄N₃NaO₆P, 548.2865 [M+Na]⁺; found, 548.3153.

### (4) Synthesis of amidite reagent_4

The synthesis scheme of amidite reagent_4 is shown below.

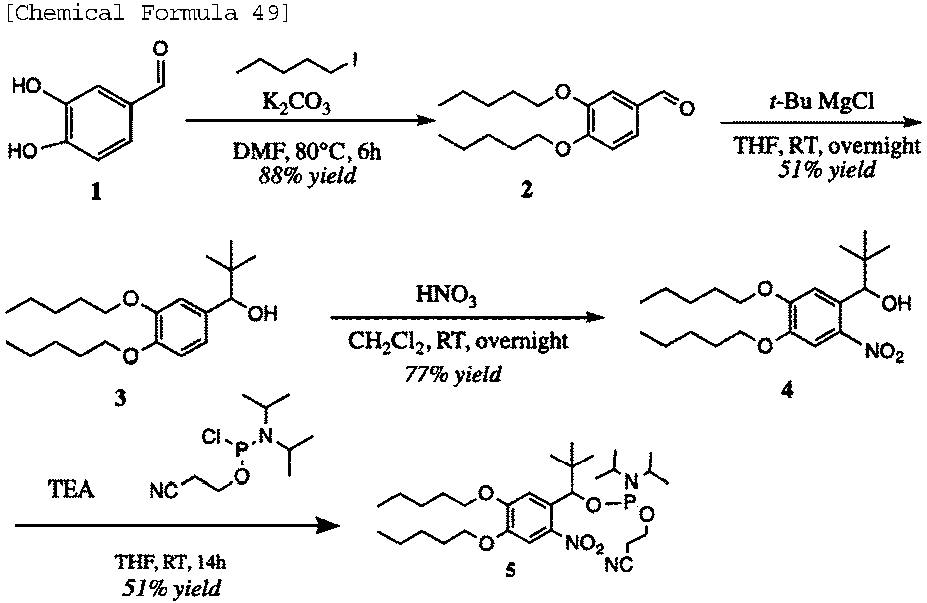

### (a) 3,4-Bis(pentyloxy)benzaldehyde (Compound 2)

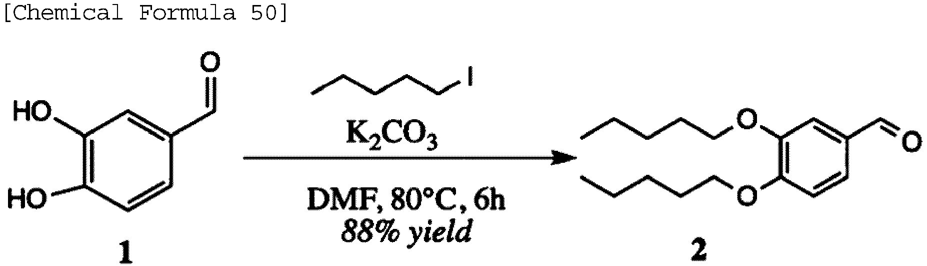

To a mixture of 3,4-dihydroxybenzaldehyde 1 (3.0 g, 22 mmol) and K₂CO₃ (12 g, 88 mmol) in dry DMF (50 mL) was added iodopentane (6.9 mL, 11 g, 53 mmol), and the resulting mixture was heated to 80°C. After stirring at 80°C for 6 hours, the reaction mixture was cooled to room temperature. DMF was removed under reduced pressure and the residue was dissolved in ethyl acetate and washed with water. The organic layer was dried over Na₂SO₄ and concentrated. The crude material was purified by silica gel column chromatography dissolved with 2 → 10% ethyl acetate/hexane, affording Intermediate 2 as a yellow solid (6.0 g, yield 88%) .

¹H NMR (400 MHz, CDCl₃) : δ9.89 (s, 1H) , 7.61-7.12 (m, 2H), 6.98-6.88 (m, 1H), 4.25-3.83 (m, 4H), 2.06-1.18 (m, 14H), 1.02-0.84 (m, 6H) ppm.

ESI-TOF-MS calcd. for C₁₇H₂₆NaO₃, 301.1774 [M+Na]⁺; found, 301.1832.

### (b) 1-(3,4-Bis(pentyloxy)phenyl)-2,2-dimethylpropan-1-ol (Compound 3)

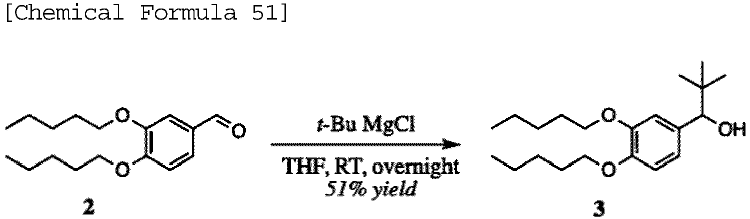

To a suspension of an aldehyde (Compound 2) (5 g, 18 mmol) in dry THF (60 mL) was slowly added tert-butylmagnesium chloride (2.4 g, 1.0 M, 20 mmol) at 0°C. After stirring at 0°C for 30 minutes and at room temperature for 20 hours, 50 mL of an aqueous solution (sat. NH₄Cl was added. The mixed solvent was extracted with ethyl acetate and washed twice with brine. The organic layer was dried over Na₂SO₄ and concentrated. The residue was purified by silica gel column chromatography dissolved with 50% benzene/hexane and then with benzene, affording an alcohol (Compound 3) as a yellow liquid (3.2 g, yield 54%).

¹H NMR (400 MHz, CDCl₃) : δ6.85-6.76 (m, 3H), 4.30 (s, 1H), 4.02-3.91 (m, 4H), 1.84-1.76 (m, 4H), 1.54-1.19 (m, 9H), 1.01-0.85 (m, 15H) ppm.

¹³C NMR (100 MHz, CDCl₃): δ148.45, 148.33, 135.13, 120.20, 113.70, 112.91, 82.23, 77.47, 76.83, 69.39, 69.32, 35.75, 29.13, 28.33, 26.09, 22.59, 14.14 ppm.

ESI-TOF-MS calcd. for C₂₁H₃₆NaO₃, 359.2664 [M+Na]⁺; found, 359.2709.

### (c) 2,2-Dimethyl-1-(2-nitro-4,5-bis(pentyloxy)phenyl)propan-1-ol (Compound 4)

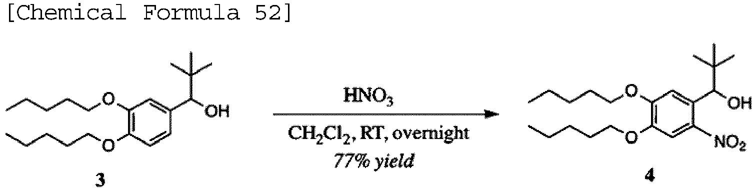

To a suspension of an alcohol (Compound 3) (1 g, 3 mmol) in dry CH₂Cl₂ (30 mL) was slowly added HNO₃ (0.5 mL, up to 70% purity) at 0°C. After stirring at room temperature for 24 hours, 50 mL of water was added. The mixed solvent was extracted with CH₂Cl₂ and washed twice with brine. The organic layer was dried over Na₂SO₄ and concentrated. The residue was purified by silica gel column chromatography dissolved with benzene, affording nitrobenzyl alcohol (Compound 4) as a yellow liquid (0.87 g, yield 77%).

¹H NMR (400 MHz, CDCl₃) : δ7.41 (s, 1H), 7.18 (s, 1H), 5.59 (s, 1H), 5.29 (s, 1H), 4.12-4.00 (m, 4H), 1.87-1.78 (m, 4H), 1.49-1.34 (m, 8H), 0.94-0.84 (m, 15H) ppm.

¹³C NMR (100 MHz, CDCl₃): δ152.23, 147.39, 141.43, 131.67, 112.35, 109.01, 77.48, 76.83, 74.08, 69.47, 69.31, 53.54, 37.05, 28.73, 28.19, 25.72, 22.49, 14.22, 14.06 ppm.

ESI-TOF-MS calcd. for C₂₁H₃₅NNaO₅, 404.2515 [M+Na]⁺; found, 404.2754.

### (d) 2-Cyanoethyl (2,2-dimethyl-1-(2-nitro-4,5-bis(pentyloxy)phenyl)propyl)diisopropylphosphoramidite (Compound 5)

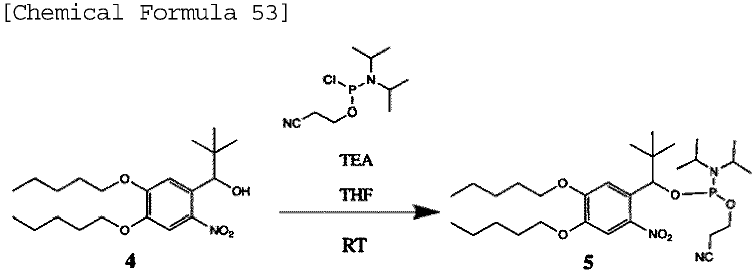

To a solution of nitrobenzyl alcohol (4) (300 mg, 0.79 mmol) in dry THF (10.0 mL) were added triethylamine (0.26 mL, 191 mg, 1.89 mmol) and 2-cyanoethyl-N,N'-diisopropylchlorophosphoramidite (0.23 mL, 223 mg, 1.9 mmol). After stirring at room temperature for 14 hours, the reaction mixture was diluted with ethyl acetate and washed twice with a saturated aqueous NaHCO₃ solution. The organic layer was dried over Na₂SO₄ and concentrated. The residue was purified by silica gel column chromatography on in 3 → 10% ethyl acetate/hexane containing 1% of triethylamine, affording the corresponding phosphoramidite (Compound 5) as a yellow oil (238 mg, 52% yield).

ESI-TOF-MS calcd. for C₃₀H₅₂N₃PNaO₆, 604.3594 [M+Na]⁺; found, 604.3533.

### 1-2. Synthesis of 5'-phosphorylated oligonucleotide using amidite reagent_1 to amidite reagent_3 and purification thereof

### (1) Experiment

### (a) DNA synthesis

A 19-base-long oligodeoxyribonucleotide 5'R-p-TAATACGACTCACTATAGG3'-(SEQ ID NO: 2) having a protected phosphate group at the 5'-end was synthesized by an automatic nucleic acid synthesizer NR-2A7MX (Nihon Techno Service Co., Ltd.) in accordance with a conventional method using amidite reagents_1, 2, and 3 and a commercially available phosphoramidite reagent (The ChemGenes Corporation). After completion of the synthesis, 1 mL of concentrated ammonia water was added to the solid phase carrier, and the solid phase carrier was heated at 55°C for 16 hours to be deprotected. The supernatant was filtered through a Millex LH filter (0.45 um, Merck), and then dried up under reduced pressure using a centrifugal evaporator. The oligonucleotide was re-dissolved in super deionized water, and the absorption at 260 nm was measured to determine the concentration. MALDI-TOF molecular weight measurement of the oligonucleotide was performed using 3-hydroxypicolinic acid as a matrix and using a positive mode of ultrafleXtreme (Bruker Corporation).

### (b) Isolation and purification of object by reverse-phase HPLC

The 19-base-long oligodeoxyribonucleotide synthesized above was isolated and purified by reverse-phase HPLC. Conditions and so on of HPLC were described in "(2) Results" described later.

### (c) Deprotection by photoirradiation

The deprotection reaction of a 5'-phosphate group by photoirradiation was performed as follows. An oligonucleotide (10 µM) was dissolved in a buffer containing 20 mM Tris-HCl (pH 8.5), 10 µL of this solution was added to a transparent 96-multiwell plate, and irradiated with 365 nm light at a quantity of light of 4 mW/cm² for 10 minutes by a MAX-305 light source device (Asahi Spectra Co., Ltd.).

### (2) Results

### (a) Results of amidite reagent_1

Fig. 2 shows the results of reverse-phase HPLC analysis of synthesis and purification of a 5'-phosphorylated oligonucleotide using amidite reagent_1, and a deprotection reaction after the purification.

In (a) of the figure, a 19-base-long oligodeoxyribonucleotide 5'R-p-TAATACGACTCACTATAGG3'-(SEQ ID NO: 2) having a protected phosphate group at the 5'-end was synthesized using amidite reagent_1, and the object was isolated and purified by reverse-phase HPLC. The analysis results of the mixture before purification and the analysis results after isolation and purification are shown.

(b) of the figure shows the result of the MALDI-TOF molecular weight analysis of the oligodeoxynucleotide after isolation and purification. At the same time as a peak of the object was confirmed, a product resulting from the elimination of a protecting group by laser photoirradiation at the time of measurement was confirmed as the main peak.

(c) of the figure shows the results of the reverse-phase HPLC analysis of the oligodeoxynucleotide after the reverse-phase HPLC isolation and purification, and the oligonucleotide after irradiation with 365 nm light. Quantitative progress of the deprotection reaction was confirmed.

HPLC analysis conditions in (a) and (c) of the figure are as follows. System used: Chromaster (Hitachi High-Tech Corporation); column: YMC Hydrosphere C18 (250 × 4.6 mm I.D., YMC); eluent A: 50 mM triethylammonium acetate containing 5% acetonitrile, pH 7.0; eluent B: acetonitrile; gradient condition: 0 to 60% B (0 to 20 min); flow rate: 1.0 mL/min; column temperature: room temperature; detection wavelength: 260 nm.

### (b) Results of amidite reagent_2

Fig. 3 shows the results of reverse-phase HPLC analysis of synthesis and purification of a 5'-phosphorylated oligonucleotide using amidite reagent_2, and a deprotection reaction after the purification.

In (a) of the figure, a 19-base-long oligodeoxyribonucleotide 5'R-p-TAATACGACTCACTATAGG3'-(SEQ ID NO: 2) having a protected phosphate group at the 5'-end was synthesized using amidite reagent_2, and the object was isolated and purified by reverse-phase HPLC. The analysis results of the mixture before purification and the analysis results after isolation and purification are shown.

(b) of the figure shows the result of the MALDI-TOF molecular weight analysis of the oligodeoxynucleotide after isolation and purification. At the same time as a peak of the object was confirmed, a product resulting from the elimination of a protecting group by laser photoirradiation at the time of measurement was confirmed as the main peak.

(c) of the figure shows the results of the reverse-phase HPLC analysis of the oligodeoxynucleotide after the reverse-phase HPLC isolation and purification, and the oligonucleotide after irradiation with 365 nm light. Quantitative progress of the deprotection reaction was confirmed.

HPLC analysis conditions in (a) and (c) of the figure are as follows. System used: Chromaster (Hitachi High-Tech Corporation); column: YMC Hydrosphere C18 (250 × 4.6 mm I.D., YMC); eluent A: 50 mM triethylammonium acetate containing 5% acetonitrile, pH 7.0; eluent B: acetonitrile; gradient condition: 0 to 100% B (0 to 20 min); flow rate: 1.0 mL/min; column temperature: room temperature; detection wavelength: 260 nm.

### (c) Results of amidite reagent_3

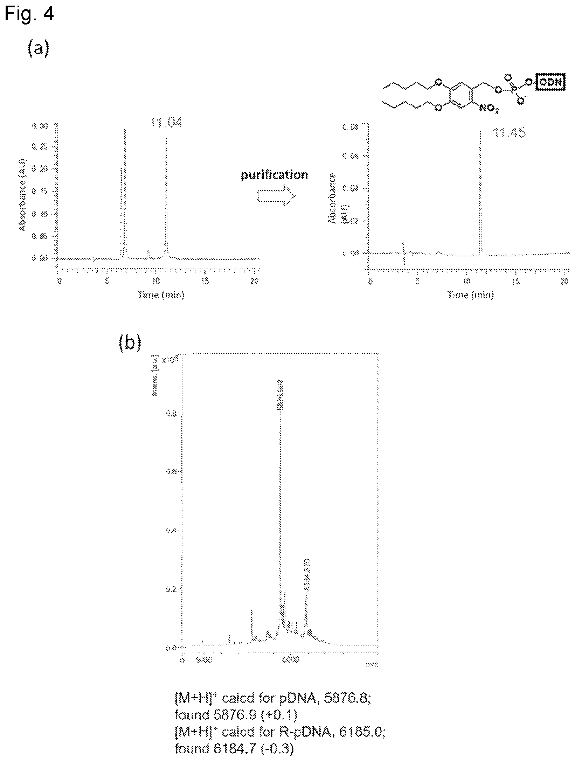

Fig. 4 shows the results of reverse-phase HPLC analysis of synthesis and purification of a 5'-phosphorylated oligonucleotide using amidite reagent_3, and a deprotection reaction after the purification.

In (a) of the figure, a 19-base-long oligodeoxyribonucleotide 5'R-p-TAATACGACTCACTATAGG3'-(SEQ ID NO: 2) having a protected phosphate group at the 5'-end was synthesized using amidite reagent_3, and the object was isolated and purified by reverse-phase HPLC. The analysis results of the mixture before purification and the analysis results after isolation and purification are shown.

(b) of the figure shows the result of the MALDI-TOF molecular weight analysis of the oligodeoxynucleotide after isolation and purification. At the same time as a peak of the object was confirmed, a product resulting from the elimination of a protecting group by laser photoirradiation at the time of measurement was confirmed as the main peak.

HPLC analysis conditions in (a) of the figure are as follows. System used: Chromaster (Hitachi High-Tech Corporation); column: YMC Hydrosphere C18 (250 × 4.6 mm I.D., YMC); eluent A: 50 mM triethylammonium acetate containing 5% acetonitrile, pH 7.0; eluent B: acetonitrile; gradient condition: 0 to 100% B (0 to 20 min); flow rate: 1.0 mL/min; column temperature: room temperature; detection wavelength: 260 nm.

### (d) Comparison of results of amidite reagents_1 and 2 with known compounds (Comparative Examples)

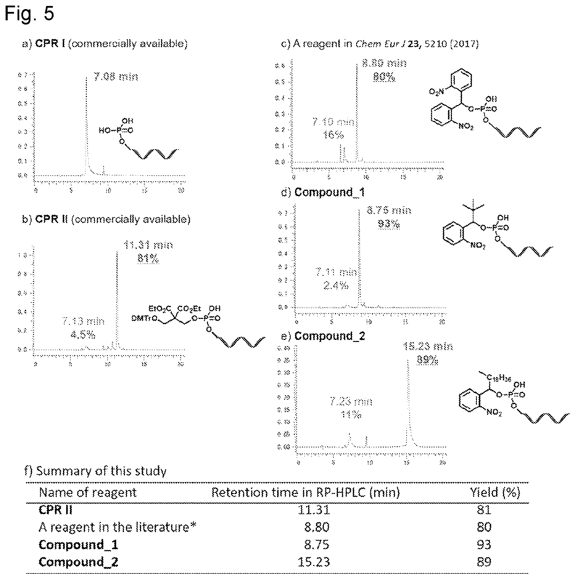

Fig. 5 shows the results of synthesis yield comparison by reverse-phase HPLC analysis of 5'-phosphorylated oligonucleotides using amidite reagents_1 and 2.

(a) to (e) of the figure: a 19-base-long oligodeoxynucleotide 5'R-p-TAATACGACTCACTATAGG3'-(SEQ ID NO: 2) having a protected phosphate group at the 5'-end was synthesized using commercially available CPRI (a), commercially available CPRII (b), a compound known in literature (Chem. Eur. J. 23, 5210 (2017)), amidite reagent_1 (d), or amidite reagent_2 (e), and was deresinized and deprotected using concentrated aqueous ammonia in accordance with a conventional method (55°C, 16 hours). A mixture before purification containing a phosphate group-protected object was analyzed by reverse-phase HPLC to determine a target product content. HPLC analysis conditions are as follows. System used: Chromaster (Hitachi High-Tech Corporation); column: YMC Hydrosphere C18 (250 × 4.6 mm I.D., YMC); eluent A: 50 mM triethylammonium acetate containing 5% acetonitrile, pH 7.0; eluent B: acetonitrile; gradient condition: 0 to 100% B (0 to 20 min); flow rate: 1.0 mL/min; column temperature: room temperature; detection wavelength: 260 nm. In the figure, a schematic diagram illustrating the chemical structure of the object, and its elution time and content% are described.

(f) of the figure is a table summarizing the elution time and the production yield (%) of the objective 5'-end protected phosphate group from the results shown in (a) to (e). * Chem. Eur. J. 23, 5210 (2017) . From this result, it is seen that amidite reagent_1 and amidite reagent_2 of the present invention have a higher yield of the objective nucleic acid than the conventional compounds. In particular, when amidite reagent_1 and amidite reagent_2 are compared with each other, it is found that the yield of amidite reagent_1 in which R₁ is a t-butyl group is higher than the yield of amidite reagent_2 in which R₁ is a C19 linear alkyl group, and amidite reagent_1 is preferable to amidite reagent_2 in terms of yield.

### 2. Application to long-chain RNA oligonucleotide synthesis

### (1) Experiment

### (a) RNA synthesis

107-base-long and 131-base-long oligoribonucleotides whose sequence are shown in the diagram were synthesized by an automatic nucleic acid synthesizer NR-2A7MX (Nihon Techno Service Co., Ltd.) in accordance with a conventional method using amidite reagent_1 and a commercially available phosphoramidite reagent (The ChemGenes Corporation). After completion of the synthesis, 1 mL of a 1:1 mixed solution of concentrated ammonia water and a 40% aqueous methylamine solution was added to the solid phase carrier, and the solid phase carrier was heated at 65°C for 15 minutes to be deprotected. The supernatant was filtered through a Millex LH filter (0.45 um, Merck), and then dried up under reduced pressure using a centrifugal evaporator. 1 mL of a 1 M TBAF solution in THF was added to the residue to dissolve, and the solution was heated at 35°C overnight. 1 mL of a 1 M Tris-HCl (pH 7.5) buffer was added thereto and mixed, and the mixture was concentrated with a centrifugal evaporator and then desalted using an NAP-25 column (GE HealthCare) . To 3 mL of the eluate were added 0.3 mL of a 3 M aqueous ammonium acetate solution (pH 5.2) and 3.3 mL of 2-propanol, and the mixture was cooled at - 30°C overnight, and then the precipitated RNA was centrifuged and collected. The RNA was re-dissolved in super deionized water, and the absorption at 260 nm was measured to determine the nucleic acid concentration.

### (b) Isolation and purification of object by reverse-phase HPLC

The RNA synthesized above was isolated and purified by reverse-phase HPLC. Conditions and so on of HPLC were described in "(2) Results" described later.

### (c) Deprotection by photoirradiation

The deprotection reaction of a 5'-phosphate group by photoirradiation was performed as follows. A solution (10 uL) of an RNA in 50 mM triethylammonium acetate (pH 7.0) was added to a transparent 96-multiwell plate, and irradiated with 365 nm light at a quantity of light of 4 mW/cm² for 10 minutes using a MAX-305 light source device (Asahi Spectra Co., Ltd.).

### (2) Results

### (a) Results of amidite reagent_1

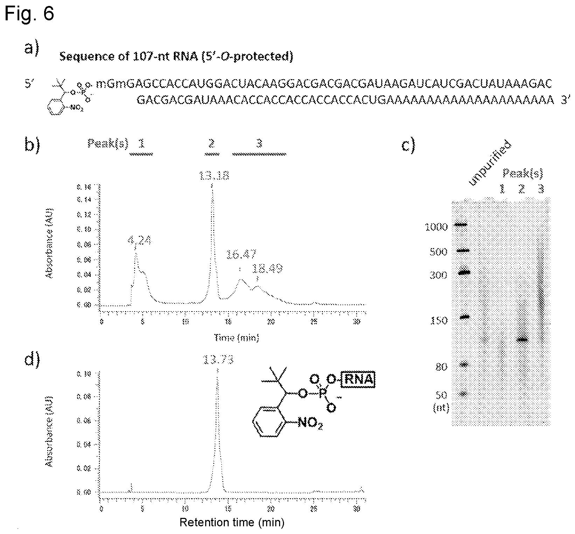

Fig. 6 shows the results of reverse-phase HPLC analysis of synthesis and purification of a 5'-phosphorylated oligonucleotide using amidite reagent_1, and a deprotection reaction after the purification. The 5'-end phosphorylated 107-nucleotide-long RNA could be purified by reverse-phase HPLC after deprotection.

(a) of the figure shows a chemically synthesized RNA sequence, and mG indicates that the hydroxy group at the 2'-position is methylated.

(b) of the figure shows the result of reverse-phase HPLC analysis of a mixture containing a 107-nt RNA after deprotection. The eluted solution was fractionated into three groups (Peak(s)_1, 2, and 3).

(c) of the figure shows the result of analysis of three groups of 5% modified polyacrylamide (containing 7 M urea) fractionated in (b). Peak(s)_2 contained the objective full-length 107-nt RNA. Peak(s)_1 contained by-products generated during synthesis that were not extended to the full length, and Peak(s)_3 contained unidentified by-products. The polyacrylamide gel after electrophoresis was stained with an SYBR Green II nucleic acid staining reagent to visualize an RNA.

(d) of the figure shows the result of reverse-phase HPLC analysis of the object (107-nt RNA) after purification using reverse-phase HPLC.

The HPLC analysis conditions used in (b) and (d) of the figure are as follows. System used: LaChrom Elite (Hitachi High-Tech Corporation); column: YMC Triat Bio C4 (250 × 4.6 mm I.D.); eluent A: 50 mM triethylammonium acetate containing 5% acetonitrile, pH 7.0; eluent B: acetonitrile; gradient condition: 5 to 20% B (0 to 20 min); flow rate: 1.0 mL/min; column temperature: 50°C; detection wavelength: 260 nm.

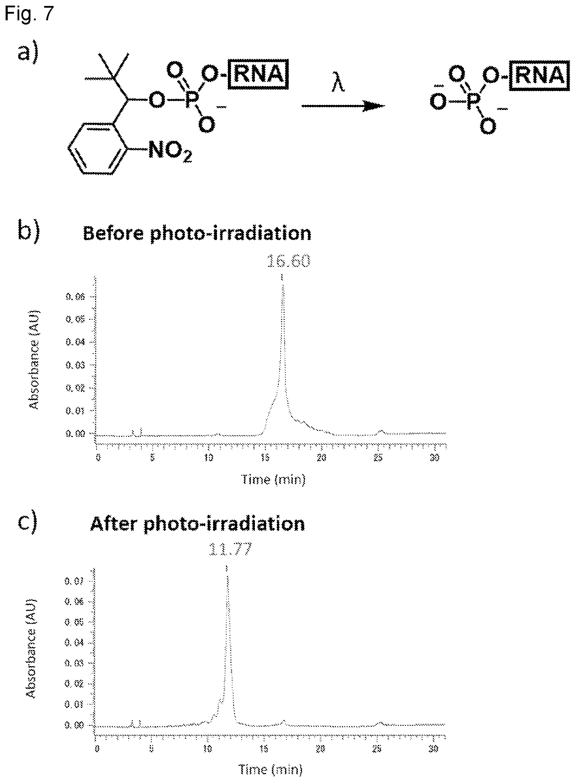

Fig. 7 is a diagram showing that a 5'-end phosphorylated 107-nucleotide-long RNA synthesized using amidite reagent_1 could be quantitatively deprotected by photoirradiation.

(a) of the figure shows a conceptual diagram of the deprotection reaction, and the protecting group of the 5-phosphate group is removed by irradiation with 365 nm light.

(b) and (c) of the figure show the results of reverse-phase HPLC analysis of a 107-nt RNA before photoirradiation (before deprotection); (b) shows the result before photoirradiation (before deprotection), and (c) shows the result after photoirradiation (after deprotection). From these results, quantitative deprotection by photoirradiation was confirmed.

The HPLC analysis conditions used in (b) and (c) of the figure are as follows. System used: LaChrom Elite (Hitachi High-Tech Corporation); column: YMC Hydrosphere C18 (250 × 4.6 mm I.D.); eluent A: 50 mM triethylammonium acetate containing 5% acetonitrile, pH 7.0; eluent B: acetonitrile; gradient condition: 5 to 20% B (0 to 20 min); flow rate: 1.0 mL/min; column temperature: 50°C; detection wavelength: 260 nm.

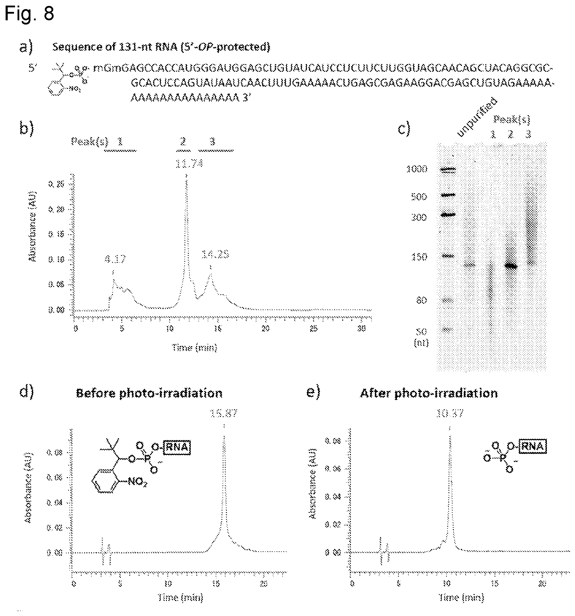

Fig. 8 is a diagram showing that a 5'-end phosphorylated 131-nucleotide-long RNA synthesized using amidite reagent_1 can be isolated and purified by reverse-phase HPLC after deprotection, and can be quantitatively deprotected by subsequent photoirradiation.

(a) of the figure shows a chemically synthesized RNA sequence, and mG indicates that the hydroxy group at the 2'-position is methylated.

(b) of the figure shows the result of reverse-phase HPLC analysis of a mixture containing a 131-nt RNA after deprotection. The eluted solution was fractionated into three groups (Peak(s)_1, 2, and 3) and purified.

(c) of the figure shows the result of analysis of three groups of modified polyacrylamides fractionated in (b). Peak(s)_2 contained the objective full-length 131-nt RNA. Peak(s)_1 contained by-products generated during synthesis that were not extended to the full length, and Peak(s)_3 contained unidentified by-products. The polyacrylamide gel after electrophoresis was stained with an SYBR Green II nucleic acid staining reagent to visualize an RNA.

(d) of the figure shows the result of reverse-phase HPLC analysis of the 5'-protected phosphate group (131-nt RNA) after purification using reverse-phase HPLC.

(e) of the figure shows the result of reverse-phase HPLC analysis of the object (131-nt RNA) purified using reverse-phase HPLC and then deprotected with 365-nm photoirradiation.

The HPLC analysis conditions used in (b) of the figure are as follows. System used: LaChrom Elite (Hitachi High-Tech Corporation); column: YMC Triat Bio C4 (250 × 4.6 mm I.D.); eluent A: 50 mM triethylammonium acetate containing 5% acetonitrile, pH 7.0; eluent B: acetonitrile; gradient condition: 5 to 20% B (0 to 20 min); flow rate: 1.0 mL/min; column temperature: 50°C; detection wavelength: 260 nm. The HPLC analysis conditions used are as follows.

System used: Chromaster (Hitachi High-Tech Corporation); column: YMC Hydrosphere C18 (250 × 4.6 mm I.D.); eluent A: 50 mM triethylammonium acetate containing 5% acetonitrile, pH 7.0; eluent B: acetonitrile; gradient condition: 5 to 15% B (0 to 20 min); flow rate: 1.0 mL/min; column temperature: 50°C; detection wavelength: 260 nm.

### 3. Transcriptional synthesis and isolation of 5'-end monophosphorylated RNA

### (1) Overview

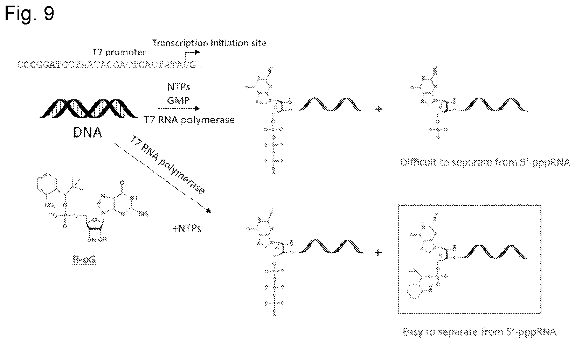

Fig. 9 illustrates the outline of the present experiment. An RNA is transcriptionally synthesized using, as a template, a double-stranded DNA fragment containing a T7 promoter sequence and using T7 RNA polymerase. When GMP is added to the reaction liquid in addition to the substrate NTP (ATP, UTP, GTP, or CTP), a transcription reaction is initiated from either GTP or GMP. Therefore, the 5'-end hydroxy group of an RNA is a mixture of triphosphate and monophosphate according to the mixing ratio of GTP/GMP, but these are difficult to be separated by HPLC or the like (the reaction scheme in the upper part of the figure).

In contrast, in the present invention, R-pG in which a protecting group is bonded to the 5'-end of guanosine is used. When the substrate NTP and the transcribed substrate R-pG coexist in a transcription reaction, the resulting transcript is a mixture of 5'-end triphosphate and protected (R)-monophosphate according to the mixing ratio of GTP/R-pG. Utilizing the hydrophobicity of the protecting group, it was possible to isolate the protected (R)-monophosphorylated RNA from the mixture by reverse-phase HPLC or the like. The protecting group of the phosphate group could be quantitatively removed by photoirradiation after isolation, and this showed that a 5'-end monophosphorylated RNA transcript (34, 100, 250, 650, or 1000-base-long) could be purified with high purity. The experiment and the results thereof are described below.

### (2) Experimental conditions

### (a) HPLC analytical conditions

The analytical conditions of HPLC used in this experiment are shown below.
Column: Hydrosphere C18 (YMC) 4.6 × 250 mm or Triart C4 (YMC) 4.6 × 250 mm
Mobile phase: A) 50 mM TEAA buffer containing 5% acetonitrile (pH 7.0)

### B) Acetonitrile

### (b) Synthesis of transcribed substrate R-pG

3'-TOM-ribo-guanosine CPG 1000 Å was used as a CPG and a transcribed substrate R-pG was synthesized in a nucleic acid synthesizer. After the synthesis, the CPG was transferred to a screw tube with a 1.5 mL O-ring (SARSTEDT), 1.0 mL of 40% aq. methylamine/28% aq. ammonia solution (1:1) was added thereto, and the mixture was incubated at 65°C for 10 minutes. The mixture was allowed to cool to room temperature, then filtered using a Millex LH 0.45 um filter. The filtrate was washed with sterile water (500 µL), and dried up with a centrifugal evaporator. The residue was dissolved in 115 µL of DMSO, and 60 µL of triethylamine was added thereto. 75 µL of triethylamine trihydrofluoride was added thereto, and the mixture was incubated at 65°C for 2 hours. The reaction liquid was analyzed and purified by HPLC, then concentrated with a centrifugal evaporator, and subjected to absorbance measurement at 260 nm to determine the concentration (3.62 mM).
MS (ESI-MS, negativemode), [M-H]- calculated: 611.14 observed: 611.1318
HPLC conditions; column: C18, gradient: B0-80%, flow rate: 3mL

### (c) Transcriptional synthesis of RNA

An in vitro transcription reaction was performed using each template DNA. A transcription reaction liquid (template DNA 5 mg/L, 1 × T7 RNA polymerase buffer (Takara) (Tris-HCl (pH 8.0) 40 mM, MgCl 28 mM, spermidine 2 mM), T7 RNA polymerase (Takara) 2.5 U/µL, DTT 5 mM, ATP 2 mM, UTP 2 mM, GTP 0.5 mM, GMP 2 mM or R-pG 2 mM, CTP 2 mM) was prepared and incubated at 37°C for 2 hours. The reaction liquid was subjected to electrophoresis with a denaturing polyacrylamide gel to confirm the generation of an RNA. After the reaction, a protein was removed by phenolchloroform extraction, and phenol was removed by chloroform extraction. Desalination and concentration were performed using Amicon Ultra (Merck Millipore, 0.5 mL, 10 K). The absorbance of the resulting RNA solution at 260 nm was measured to determine the concentration.

### (3) Results

### (a) Synthesis of transcribed substrate

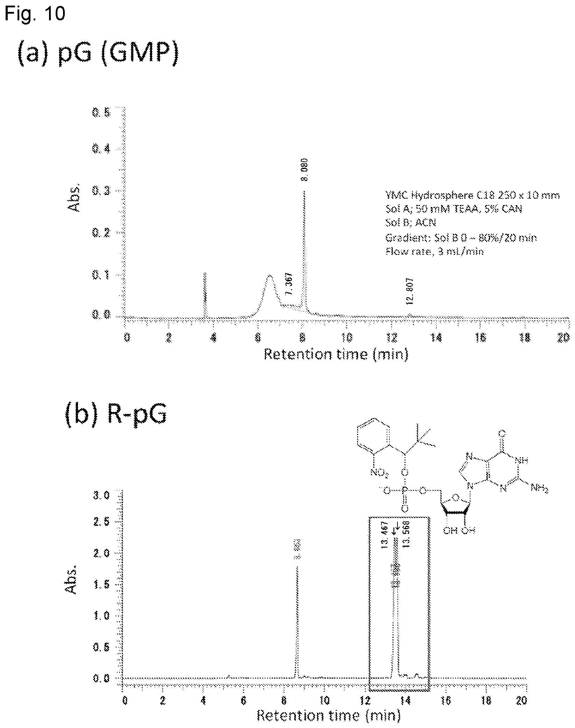

Using amidite reagent_1 and a CPG resin (Glen Research) carrying deoxyguanosine, a transcribed substrate R-pG was synthesized in accordance with a standard phosphoramidite method using an automatic nucleic acid synthesizer. After the synthesis, deprotection was performed, and then analysis and purification were performed by HPLC. The results are shown in Fig. 10.

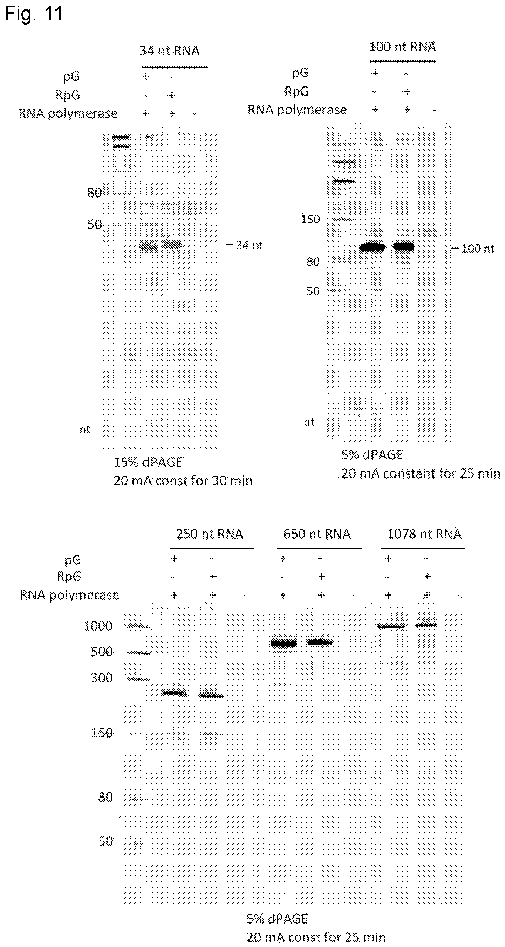

Using the purified RpG, RNAs having five lengths were synthesized through a transcription reaction. The RNAs synthesized by transcription have lengths of 34 nt, 100 nt, 250 nt, 650 nt, and 1078 nt, respectively. Each of the reaction liquid was analyzed by electrophoresis using a denaturing polyacrylamide gel to confirm an RNA having an objective chain length. The results are shown in Fig. 11.

### (b) Analysis by HPLC

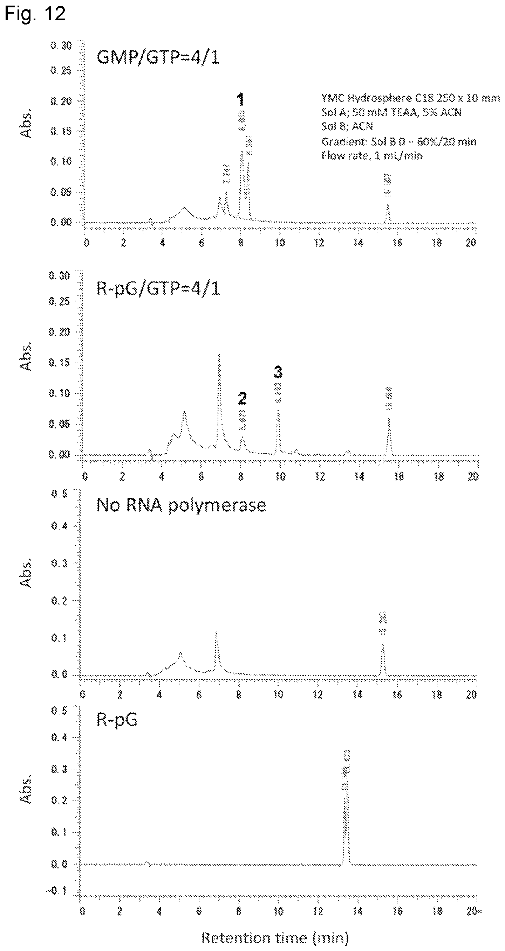

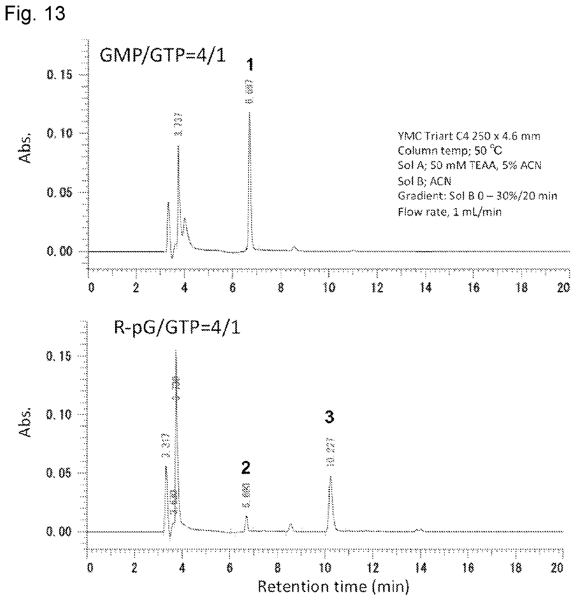

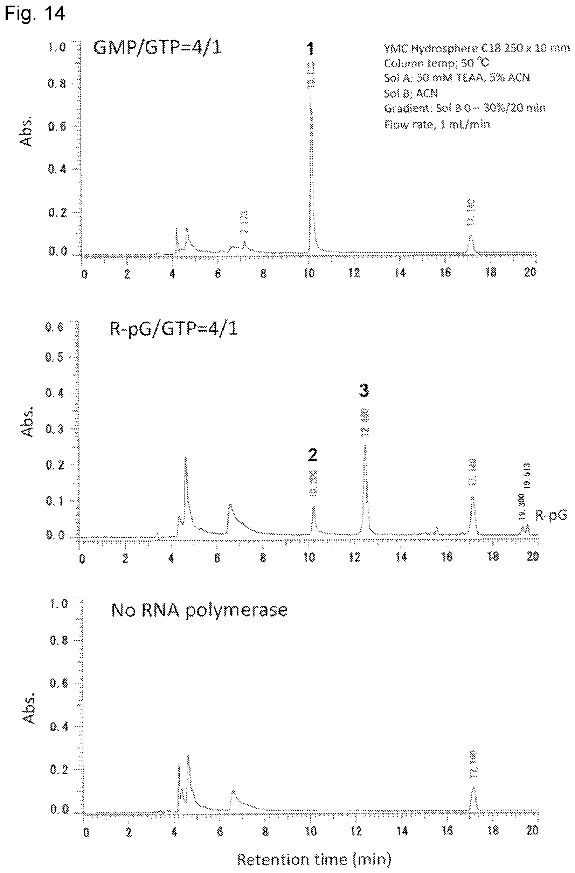

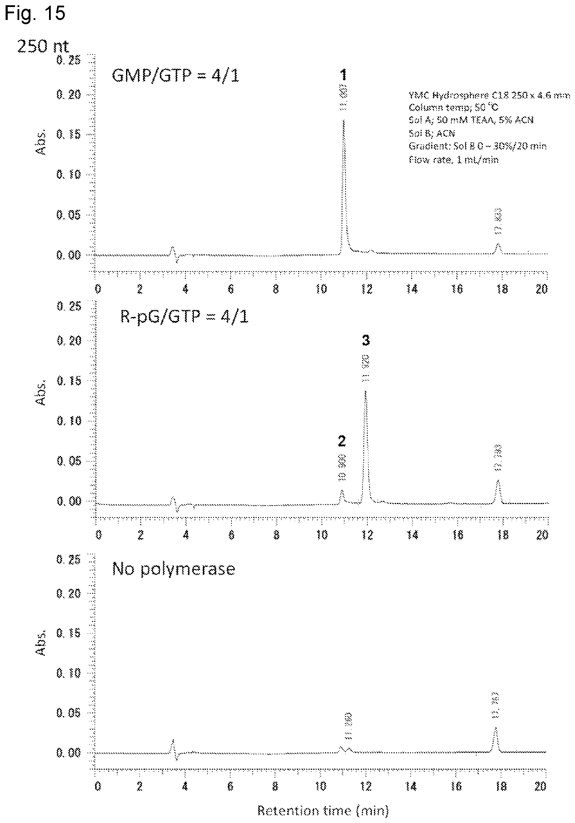

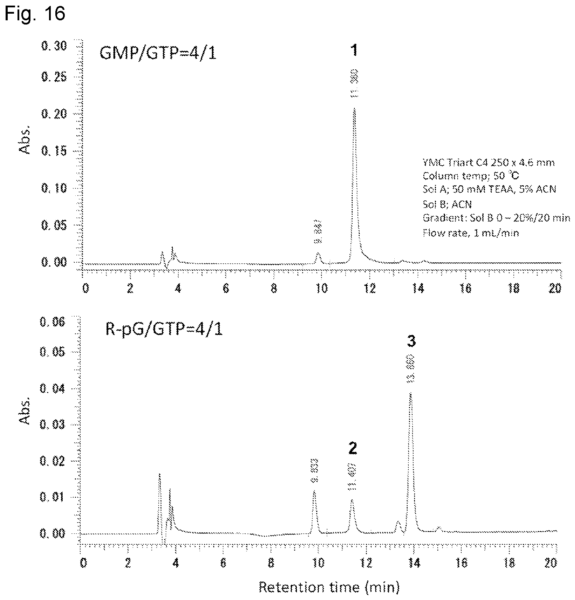

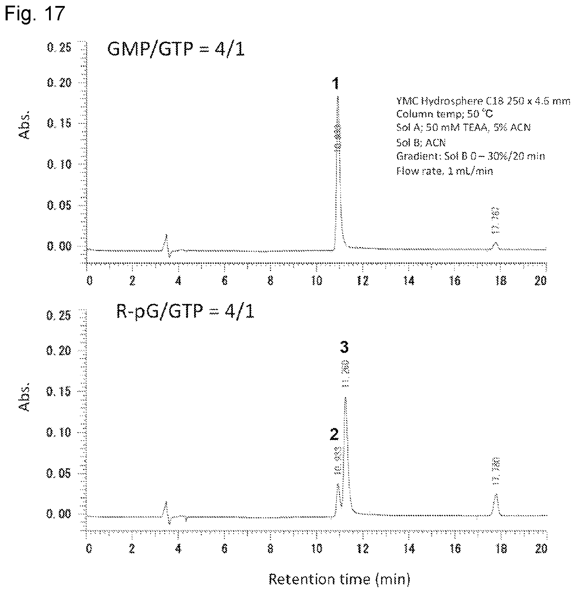

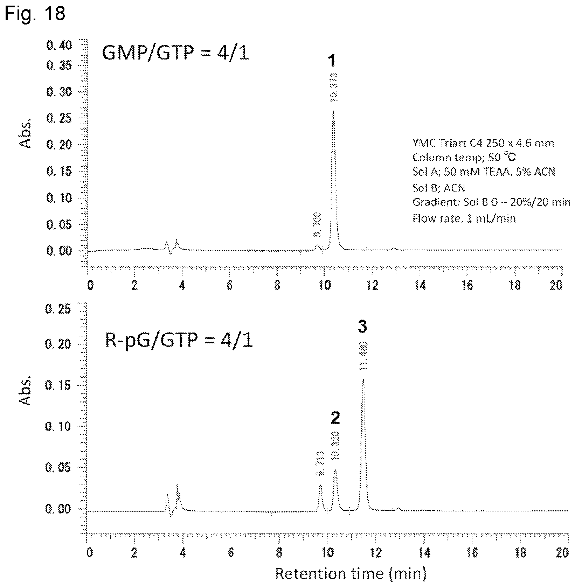

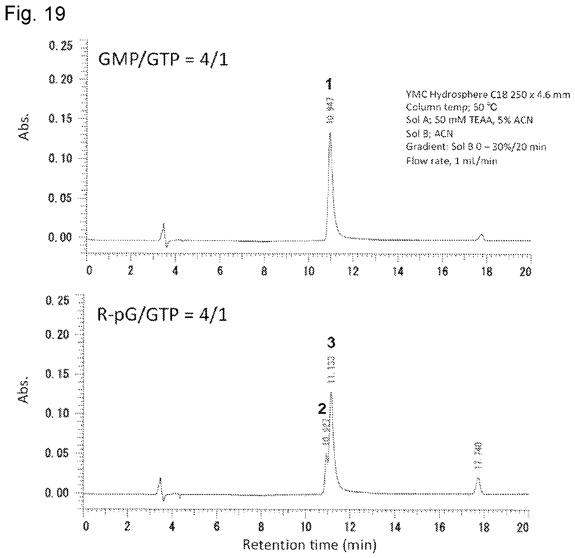

The RNA obtained by transcription was purified and then analyzed by HPLC. The analysis results of RNAs are shown in Figs. 12 to 20. The structures of the RNAs at the respective peaks (peaks 1 to 3) of HPLC are as follows. Peak 1 indicates a mixture of a triphosphate type having no protecting group and a monophosphate type, peak 2 indicates a triphosphate type having no protecting group, and peak 3 indicates a monophosphate type having a protecting group.

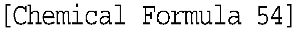

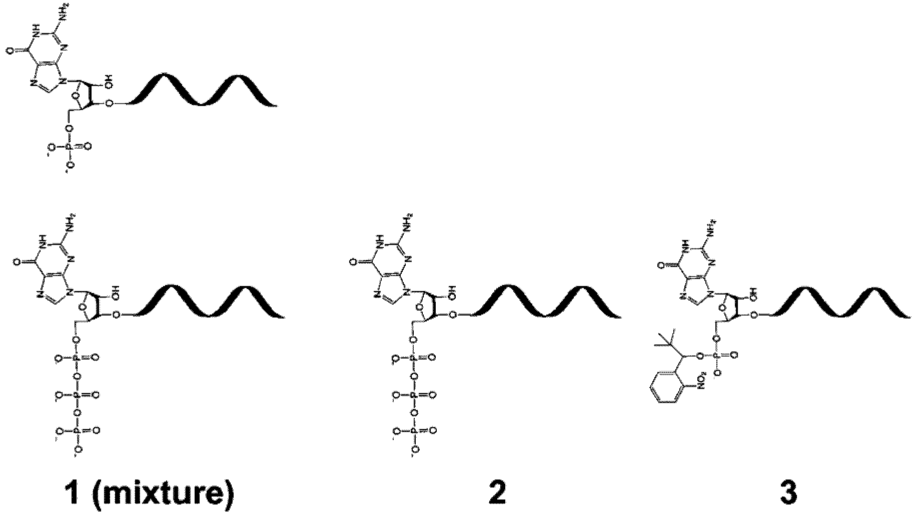

The results summarizing the data are shown in the following table.

**[Table 1]**

| **C18 column** | | | **p-RNA, ppp-RNA** | **Rp-RNA** | | |
|---|---|---|---|---|---|---|
| **Gradient (MeCN/50 mM TEAA, 5% MeCN)** | **Column temp. (°C)** | **Length (nt)** | **Retention time (mln)** | **retention time (min)** | **gap (min)** | **yield (%)** |
| 0-60% | r.t. | 34 | 8.01 | 9.89 | 1.82 | 66.7 |
| 0-30% | 50 | 100 | 10.2 | 12.5 | 2.26 | **14.1** |
| 0-30% | 50 | 250 | 10.9 | 11.9 | 1.02 | 74,1 |
| 0-30% | 50 | 650 | 10.9 | 11.3 | 0.327 | **74** |
| 0-30% | 50 | 1078 | 10.9 | 11.1 | 0.206 | 69.1 |

| C4 column | | | p-RNA. ppp-RNA | Rp-RNA | | |
|---|---|---|---|---|---|---|
| Gradient (MeCN/50 mM TEAA, 5% MeCN) | Column temp. (°C) | Length (nt) | Retention time (min) | retention time (min) | gap (min) | yield (%) |
| 0-30% | 50 | 34 | 6.69 | 10.2 | 3.54 | 68.8 |
| 0-20% | 50 | 250 | 11.4 | 13.9 | 2.45 | 76.9 |
| 0-20% | 50 | 650 | 10.3 | 11.5 | 1.16 | 71.1 |
| 0-20% | 50 | 1078 | 14.0 | 15.2 | 1.25 | 70 |

### (c) Deprotection by photoirradiation and HPLC analysis

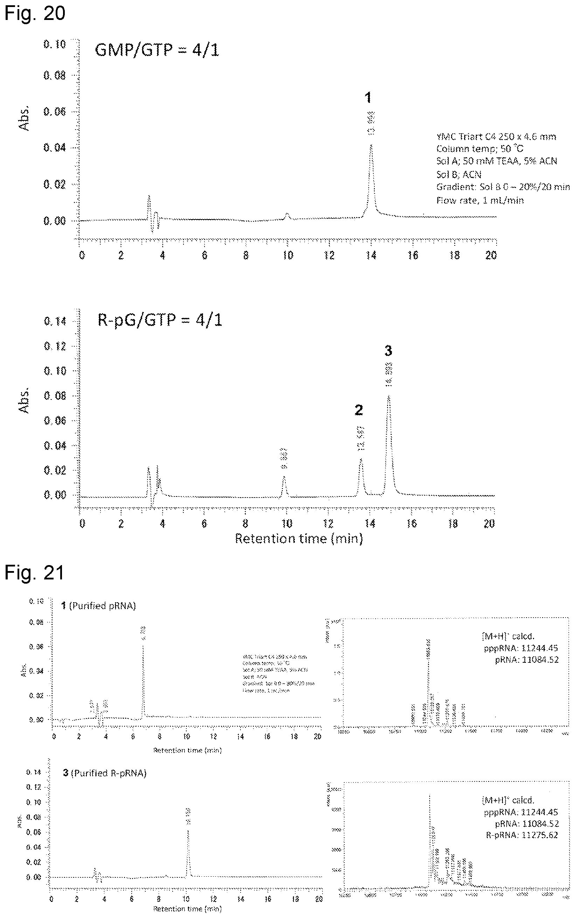

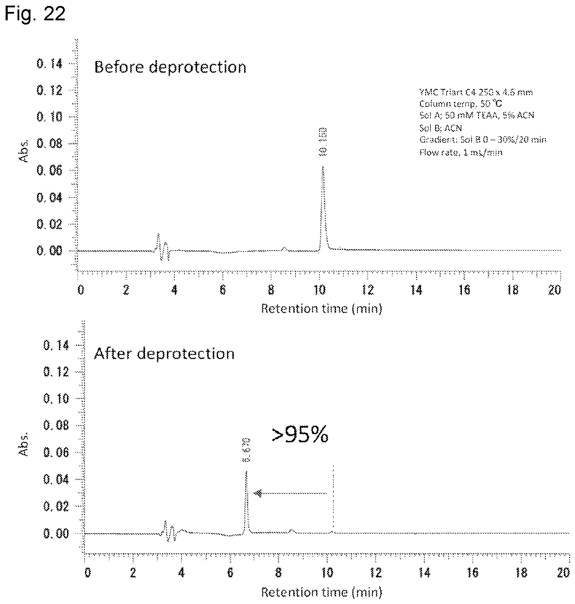

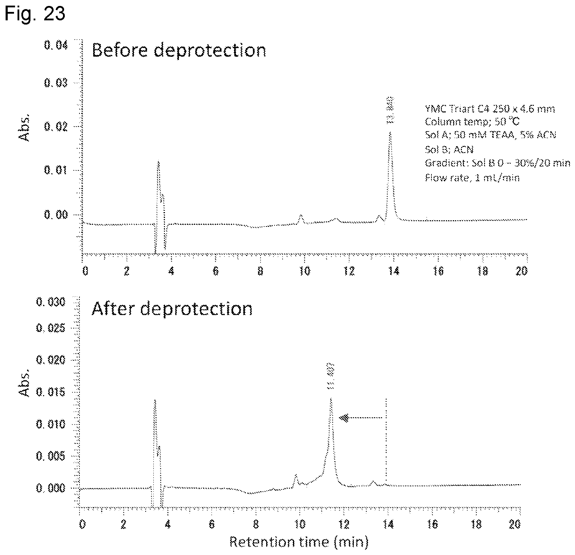

An RNA with a photoprotecting group introduced was purified by HPLC, and deprotection was performed by irradiating the RNA solution with 365 nm light for 10 minutes, followed by analysis by HPLC. The results are shown in Figs. 21 to 23.

### 4. Synthesis of branched cap analog compound (precursor)

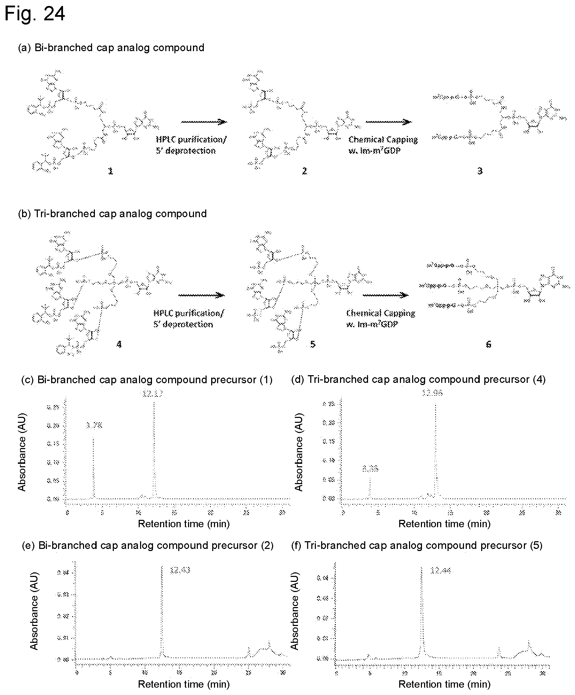

Fig. 24 is a diagram showing an experiment of synthesizing a branched cap analog compound using a novel phosphorylation reagent.

(a) and (b) of the figure illustrate synthesis schemes of bi- and tri-branched cap analog compounds. Compounds 1 and 4 were synthesized using an automatic nucleic acid synthesizer, and purified by reverse-phase HPLC using a hydrophobic protecting group of a novel phosphorylation reagent. The protecting group of the phosphate moiety was removed by photoirradiation, affording capped precursors 2 and 5.

(c) and (d) of the figure show the results of reverse-phase HPLC analysis of the mixtures containing Compounds 1 and 4 after deprotection and dialysis. Since the objects were contained in the peaks at retention times of 12.17 minutes (c) and 12.96 minutes (d), these were isolated.

(e) and (f) of the figure show the results of reverse-phase HPLC analysis of Compounds 2 and 5.

### Experimental section

Guanosine derivatives 1 and 4 were synthesized using a nucleic acid synthesizer at a 1 micromole scale in accordance with a conventional phosphoramidite method. Commercially available branched amidite reagents (Symmetric Doubler Phosphoramidite, Glen Research, cat #10-1920: Trebler Phosphoramidite, Glen Research, cat #10-1922) were each bonded to a CPG solid phase carrier (ChemGenes, cat #N-3203-10) supporting a protected guanosine. Subsequently, rG nucleotide and a novel chemical phosphorylation reagent (Compound 1) were bonded, affording the intended bi-branched compound (1) and the objective tri-branched compound (4). 1 mL of an equal mixture of concentrated ammonia water and a 40% methylamine aqueous solution was added to the solid-phase carrier, and heated at 65°C for 15 minutes. The supernatant was dried up under reduced pressure, the residue was dissolved in 100 µL of dimethyl sulfoxide, 125 µL of triethylamine trihydrofluoride was added thereto, and the mixture was heated at 65°C for 2 hours. To the reaction liquid was added 1 mL of a 0.1 M triethylamine acetate buffer (pH 7.0), and dialysis was carried out overnight using a dialysis membrane (Biotech CE Tubing manufactured by Spectra/Pore; MWCO 500 to 1000 Da). The internal liquid was collected, and the object was isolated by reverse-phase HPLC. The analysis and purification conditions are as follows.

System used: LaChrom Elite (Hitachi High-Tech Corporation); column: YMC Triat Bio C4 (250 × 4.6 mm I.D.); eluent A: 50 mM triethylammonium acetate containing 5% acetonitrile, pH 7.0; eluent B: acetonitrile; gradient condition: 5 to 80% B (0 to 20 min); flow rate: 1.0 mL/min; column temperature: 25°C; detection wavelength: 260 nm.

The object isolated by reverse-phase HPLC was concentrated and then irradiated with 365 nm light to be deprotected. Dialysis was carried out overnight using a dialysis membrane (Biotech CE Tubing manufactured by Spectra/Pore; MWCO 500 to 1000 Da), and the objective Compounds 2 and 5 were obtained in yields of 72 nmol and 96 nmol, respectively. The purities of the objective Compounds 2 and 5 obtained were confirmed by reverse-phase HPLC. The analysis conditions are as follows.

System used: LaChrom Elite (Hitachi High-Tech Corporation); column: YMC Hydrosphere C18 (250 × 4.6 mm I.D.); eluent A: 50 mM triethylammonium acetate containing 5% acetonitrile, pH 7.0; eluent B: acetonitrile; gradient condition: 0 to 20% B (0 to 20 min); flow rate: 1.0 mL/min; column temperature: 25°C; detection wavelength: 260 nm.

The molecular weight of the obtained compound was measured using 3-hydroxypicolinic acid as a matrix and using a positive mode of an ultrafleXtreme MALDI-TOF/TOF mass spectrometer manufactured by Bruker Corporation. The analysis results are as follows.

Bi-branched guanosine derivative 2; [M + H]⁺ calcd: 1486.9, found: 1486.3 (-0.6). Tri-branched guanosine derivative 5; [M + H]⁺ calcd: 1932.2, found: 1931.3 (-0.9).

### 5. Development of adenylated RNA preparation method capable of isolation and purification: synthesis of AppG nucleotide analogue having photodegradable hydrophobic protecting group and introduction into RNA strand using RNA polymerase

A synthesis scheme of the AppG dinucleotide compound having a photodegradable hydrophobic protecting group (Compound 11) is shown below.

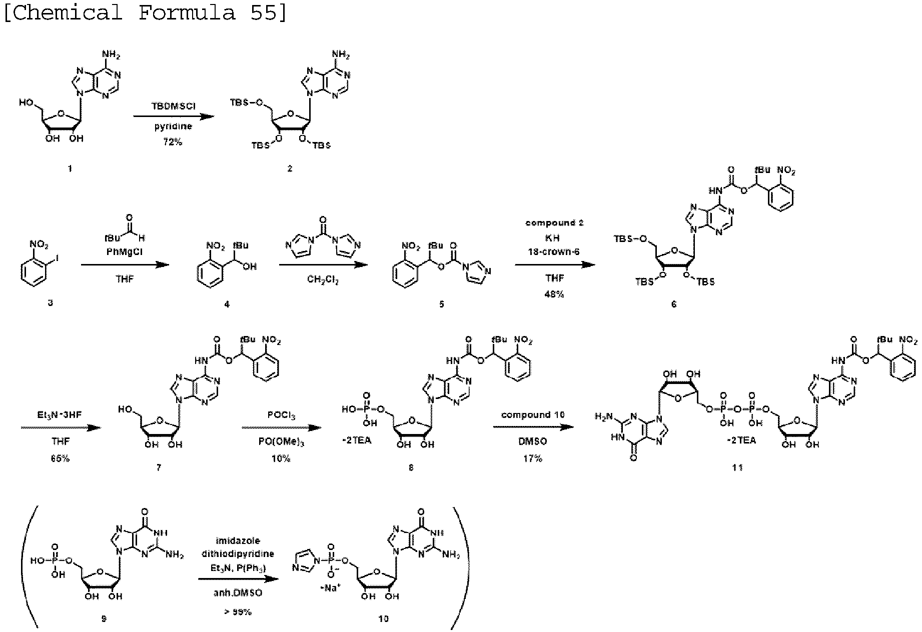

### (1) 2',3',5'-Tri-O-[tert-butyl(dimethyl)silyl]-adenosine (Compound 2)

Adenosine (2.00 g, 7.48 mmol, 1.0 equiv.), tert-butyldimethylsilane (5.64 g, 37.4 mmol, 5.0 equiv.) and imidazole (6.11 g, 89.9 mmol, 12 equiv.) were dissolved in DMF (75.0 mL) and stirred at room temperature overnight. The reaction solution was dissolved in ethyl acetate, and washed with water and saturated saline, then the organic layer was dried over anhydrous sodium sulfate, and the solvent was distilled off under reduced pressure. The residue was purified by neutral flash column chromatography (hexane/ethyl acetate = 3/1), affording 3.30 g of Compound 2 (5.42 mmol, yield 72%).
[¹H NMR] (400 MHz, DMSO-d₆, ppm) δ8.29 (s, 1H), 8.09 (s, 1H), 7.27 (s, 2H), 5.89 (d, 1H, J = 6.4 Hz), 4.87 (dd, 1H, J = 4.4, 6.4 Hz), 4.28 (d, 1H, J = 4.4 Hz), 3.99~3.94 (m, 2H), 3.70 (q, 1H, J = 6.8 Hz), 0.87 (d, 18H, J = 10.8 Hz), 0.67 (s, 9H), 0.08 (d, 6H, J = 7.6 Hz), 0.04 (s, 6H), -0.15 (s, 3H), -0.40 (s, 3H)
[HRMS] (ESI) m/z Cal. for C₂₈H₅₅N₅O₄Si₃Na⁺ [M+Na]⁺; 632.3454 found 632.3486

### (2) 2,2-Dimethyl-1-(2-nitrophenyl)propan-1-ol (Compound 4)

THF (50 mL) added with 1-iodo-2-nitrobenzene (5.0 g, 20 mmol, 1.0 equiv.) was cooled to -40°C, a 2 M phenylmagnesium chloride THF solution (11 mL, 22 mmol, 1.1 equiv.) was slowly added dropwise thereto, and the mixture was stirred at -40°C for 30 minutes. Pivalaldehyde (2.2 mL, 20 mmol, 1.0 equiv.) was slowly added dropwise and the resulting mixture was stirred at - 40°C for 3 hours. Saturated ammonium chloride water (100 mL) and water (100 mL) were added, and the mixture was returned to room temperature with stirring. Saturated saline (200 mL) was added, and the mixture was extracted with ethyl acetate (200 mL × 3). The organic layer was dried over anhydrous sodium sulfate, and distilled off under reduced pressure. The residue was purified by neutral flash column chromatography (hexane/dichloromethane = 2/1 → 1/1 → 1/5), affording objective Compound 4 (2.9 g, 14 mmol, yield 69%).
[¹H NMR] (400 MHz, CDCl₃, ppm) δ7.78 (dd, 1H, J = 8.0, 1.5 Hz), 7.71 (dd, 1H, J = 8.2, 1.2 Hz), 7.54∼7.59 (m, 1H), 7.38 (ddd, 1H, J = 8.4, 7.1, 1.0 Hz), 5.33 (d, 1H, J = 3.1 Hz), 2.35 (d, 1H, J = 3.6 Hz), 0.85 (s, 9H)

### (3) 2',3',5'-Tri-O-[tert-butyl(dimethyl)silyl]-N⁶-[2-nitrobenzyl(tert-butyl)oxycarbonyl]-adenosine (Compound 6)

Compound 4 (0.300 g, 1.43 mmol, 1.0 equiv.) was azeotroped with benzene three times. 1,1'-Carbonyldiimidazole (0.626 g, 3.86 mmol, 2.7 equiv.) was added thereto, dissolved in dichloromethane (7.00 mL), and the mixture was stirred at room temperature for 6.5 hours. After washings with water and saturated saline, the organic layer was dried over anhydrous sodium sulfate, and the solvent was distilled off under reduced pressure. Compound 2 (0.962 g, 1.58 mmol, 1.1 equiv.) and 18-crown-6 (0.455 g, 1.72 mmol, 1.2 equiv.) were added thereto, and the mixture was dissolved in dehydrated THF (7.0 mL). Furthermore, KH (0.0689 g, 1.72 mmol, 1.2 equiv.) was slowly added and the mixture was stirred at 50°C overnight. After the solvent was distilled off under reduced pressure, the residue was dissolved in ethyl acetate and the solution was washed with water and saturated saline. The organic layer was dried over anhydrous sodium sulfate, and the solvent was distilled off under reduced pressure. The residue was purified by neutral flash column chromatography (hexane/ethyl acetate = 3/1), affording 0.585 g of Compound 6 (0.692 mmol, yield 48%).
[¹H NMR] (400 MHz, DMSO-d₆, ppm) diastereomermixture δ10.91 (s, 1H), 8.64∼8.57 (m, 2H), 7.96∼7.93 (m, 1H), 7.83∼7.81 (m, 1H), 7.63~7.50 (m, 2H), 6.22 (s, 1H), 6.01∼6.00 (m, 1H), 4.88∼4.84 (m, 1H), 4.32∼4.30 (m, 1H), 4.01∼3.93 (m, 2H), 3.75∼3.72 (m, 1H), 0.88∼0.85 (m, 27H), 0.664∼0.661 (m, 9H), 0.11∼0.07 (m, 6H), 0.041∼0.039 (m, 6H), -0.14 (s, 3H), -0.415∼0.42 (m, 3H)
[¹³C NMR] (151 MHz, DMSO-d₆, ppm) δ152.30, 152.07, 149.42, 143.14, 137.35, 133.62, 133.42, 133.08, 132.36, 131.81, 130.43, 130.10, 129.98, 129.80, 129.66, 128.97, 128.89, 128.52, 125.21, 125.15, 124.89, 123.74, 123.63, 87.60, 85.97, 80.23, 76.83, 74.93, 72.97, 72.68, 62.94, 36.64, 36.29, 18.55, 18.30, 17.98, - 4.15, -4.31, -4.91, -5.01
[HRMS] (ESI) m/z Cal. for C₄₀H₆₈N₆O₈Si₃Na⁺ [M+Na]⁺; 867.4299 found 867.4305

### (4) N⁶-[2-Nitrobenzyl(tert-butyl)oxycarbonyl]-adenosine (Compound 7)

Compound 6 (0.585 g, 0.692 mmol, 1.0 equiv.) was dissolved in THF (7.00 mL), triethylamine and trihydrochloride (0.282 mL, 1.73 mmol, 2.5 equiv.) was added thereto, and the mixture was stirred at room temperature overnight. The reaction solution was dissolved in ethyl acetate, and washed with water and saturated saline, then the organic layer was dried over anhydrous sodium sulfate, and the solvent was distilled off under reduced pressure. The residue was purified by neutral flash column chromatography (dichloromethane/MeOH = 10/1), affording 0.226 g of Compound 7 (0.450 mmol, yield 65%).
[¹H NMR] (400 MHz, DMSO-d₆, ppm) diastereomer mixture δ9.57∼9.53 (m, 1H), 8.35∼8.33 (m, 1H), 8.07~8.05 (m, 1H), 7.81∼7.79 (m, 1H), 7.61∼7.51 (m, 2H), 7.35∼7.31 (m, 1H), 6.48∼6.46 (m, 1H), 5.83∼5.76 (m, 2H), 5.34 (s, 1H), 4.87∼4.72 (m, 2H), 4.33∼4.14 (m, 2H), 3.78∼3.62 (m, 2H), 0.90∼0.85 (m, 9H)
[¹³C NMR] (101 MHz, DMSO-d₆, ppm) 5151.87, 150.89, 149.95, 149.49, 149.43, 149.18, 149.07, 143.15, 132.91, 132.65, 129.26, 128.76, 124.63, 122.46, 90.42, 87.04, 77.44, 74.25, 71.70, 62.57, 36.29, 25.77
[HRMS] (ESI) m/z Cal. for C₂₂H₂₆N₆O₈Na⁺ [M+Na]⁺; 525.1704 found 525.1709

### (5) Calculation of molar absorbance coefficient of N⁶-[2-nitrobenzyl(tert-butyl)oxycarbonyl]-adenosine (Compound 7)

The molar absorbance coefficient (ε₂₆₀) of Compound 7 was calculated using a UV-vis spectrum. First, 0.990, 1.960, 2.913, and 3.846 × 10⁻⁴ [mol/L] of Compound 7 were prepared, and UV-vis spectra were measured. From ε₂₆₀ at the respective concentrations, an average value was calculated, and ε₂₆₀ of Compound 7 was determined to be 18717 L/mol·cm.

### (6) N⁶-[2-Nitrobenzyl(tert-butyl)oxycarbonyl]-adenosine 5'-monophosphoric acid triethylamine salt (Compound 8)

Compound 7 (0.100 g, 0.199 mmol, 1.0 equiv.) was dissolved in trimethylphosphate (2.00 mL), phosphoryl chloride (22.3 µL, 0.239 mmol, 1.2 equiv.) was added thereto, and then the mixture was stirred at room temperature for 6 hours. A TEAB buffer (2 M, 10.0 mL) was added thereto, the mixture was washed with chloroform, and then the solvent was distilled off under reduced pressure. The residue was purified using DEAE Sephadex A-25 (solvent: (A) water, (B) 1.5 M triethylammonium carbonate buffer 10% ACN, gradient: B conc. 0 to 200 min., 0 to 100%, flow rate: 1.0 mL/min., detection wavelength: 260 nm, size S), affording 8.82 mg of Compound 8 (11.2 µmol, yield 6%).
[¹H NMR] (600 MHz, DMSO-d₆, ppm) diastereomer mixture δ8.80 (s, 1H), 8.57 (s, 1H), 7.94∼7.93 (m, 1H), 7.80∼7.74 (m, 2H), 7.57∼7.54 (m, 1H), 6.23∼6.22 (m, 1H), 6.01∼5.99 (m, 1H), 4.68∼4.66 (m, 1H), 4.20∼4.19 (m, 1H), 4.06∼4.05 (m, 1H), 3.85∼3.81 (m, 2H), 2.47∼2.46 (m, 1H), 1.19 (s, 2H), 1.02∼0.89 (m, 9H)
[¹³C NMR] (151 MHz, DMSO-d₆, ppm) δ152.52, 152.25, 151.75, 149.91, 149.41, 143.22, 133.50, 133.10, 129.79, 129.64, 124.88, 123.38, 87.45, 85.15, 76.72, 74.70, 71.73, 64.63, 36.42, 29.53, 25.98
[³¹P NMR] (243 MHz, DMSO-d₆, ppm) δ0.79
[HRMS] (ESI) m/z Cal. for C₂₂H₂₆N₆O₁₁⁻ [M-H]⁻; 581.1403 found 581.1381

### (7) Sodium salt of guanosine 5'-phosphorimidazolide (Compound 10)

Guanosine 5'-phosphate disodium salt (0.500 g, 1.23 mmol, 1.0 equiv.) was dissolved in water and loaded onto a DOWEX 50W-X8 column in H⁺ type, and the column was washed with water. The eluate was added dropwise to an ethanol solution of triethylamine (0.685 mL, 4.92 mmol, 4.0 equiv.), and the solvent was distilled off under reduced pressure. Imidazole (0.837 g, 12.3 mmol, 10 equiv.) and 2,2'-dipyridyl disulfide (0.811 g, 3.68 mmol, 3.0 equiv.) were added thereto, and the mixture was dissolved in DMSO (10.0 mL). Further, triethylamine (0.372 mL, 3.68 mmol, 3.0 equiv.) and triphenylphosphine (0.965 g, 3.68 mmol, 3.0 equiv.) were added thereto, and the mixture was stirred at room temperature overnight. The reaction was traced by HPLC (column: CoresepSB; solvent: (A) 20 mM ammonium acetate 5% ACN, (B) 400 mM ammonium acetate 5% ACN; gradient: B conc. 2 to 22 min., 0 to 100%, flow rate: 1.0 mL/min., detection wavelength: 260 nm). A solution of sodium perchlorate (4.51 g, 36.8 mmol, 30 equiv.) in dehydrated acetone (200 mL) was added thereto, and the mixture was ice-cooled in an ice bath. The precipitate formed was collected by suction filtration, affording white powdery Compound 10 (0.530 g, 1.22 mmol, yield > 99%). Various spectra agreed well with literature values^{[1]}.

### (8) P¹-Guanosine-5'-P³-(N⁶-[2-nitrobenzyl (tert-butyl) oxycarbonyl] - adenosine-5') diphosphoric acid triethylamine salt (Compound 11)

Compound 8 (8.81 mg, 11.2 µmol, 1.0 equiv.) was dissolved in DMSO (1.00 mL), and guanosine 5'-phosphorimidazolide (24.4 mg, 56.0 µmol, 5.0 equiv.), 1-methylimidazole (4.42 µL, 56.0 µmol, 5.0 equiv.), and CaCl₂ (12.4 mg, 112 µmol, 10 equiv.) were added, then the mixture was stirred at room temperature overnight. The reaction was traced by HPLC (column: Hydrosphere C18; solvent: (A) 20 mM triethylammonium acetate (pH 7.0), (B) ACN; gradient: B conc. 0 to 30 min., 0 to 80%, flow rate: 1.0 mL/min., detection wavelength: 260 nm). After confirming the completion of the reaction progress, purification was performed using HPLC (column: Hydrosphere C18; solvent: (A) 20 mM triethylammonium acetate (pH 7.0), (B) ACN; gradient: B conc. 0 to 30 min., 0 to 80%; flow rate: 10 mL/min.; detection wavelength: 260 nm), affording 2.15 mg of Compound 11 (1.90 µmol, yield 17%).
[¹H NMR] (600 MHz, DMSO-d₆, ppm) diastereomer mixture δ8.34~8.33 (m, 2H), 7.83∼7.81 (m, 1H), 7.69∼7.64 (m, 2H), 7.62∼7.57 (m, 1H), 7.43∼7.40 (m, 1H), 6.25∼6.24 (m, 1H), 5.96∼5.95 (m, 1H), 5.61∼5.57 (m, 1H), 4.50∼4.48 (m, 1H), 4.46∼4.43 (m, 1H), 4.33∼4.31 (m, 1H), 4.27∼4.26 (m, 1H), 4.23∼4.17 (m, 2H), 4.14∼4.11 (m, 2H), 4.08∼4.04 (m, 2H), 0.83∼0.82 (m, 9H)
[¹³C NMR] (151 MHz, DMSO-d₆, ppm) δ157.35, 154.15, 152.14, 151.64, 149.82, 149.39, 142.97, 136.61, 133.52, 133.06, 129.80, 129.62, 124.85, 123.30, 88.12, 87.93, 83.84, 76.74, 74.81, 73.96, 70.57, 70.17, 64.62, 31.23, 27.59, 26.05, 25.97, 22.413
[³¹P NMR] (243 MHz, DMSO-d₆, ppm) δ-10.66
[HRMS] (ESI) m/z Cal. for C₃₂H₃₈N₁₁O₁₈P₂⁻ [M-H]⁻; 926.1877 found 926.1916

### References

1. Marcin Z., Mariusz S., Maciej L., Anna N., Edward D., Robert E. Rhoads, Zbigniew W., Jacek J., RSC Adv. 2013, 3, 20943-20958.

### (9) Transcription by T7 RNA polymerase

Compound 11 synthesized as described above was subjected to a transcription reaction by T7 RNA polymerase. A 1078-base-long protecting group-containing adenylated RNA was prepared by heating, at 37°C for 2 hours, the following reaction liquid: 5 ng/uL dsDNA transcription template (PCR product of 1105-bp containing T7 promoter sequence 5'TAATACGACTCACTATAG3'-(SEQ ID NO: 1)), 2 mM ATP, 2 mM UTP, 2 mM CTP, 0.5 mM GTP, 2 mM Compound 11 (AppG dinucleotide compound having a photodegradable hydrophobic protecting group), 40 mM Tris-HCl (pH 8.0), 8 mM MgCl₂, 2 mM spermidine, 5 mM DTT, 2.5 units/µL T7 RNA polymerase (Takara Bio Inc.). After the transcription reaction, the reaction liquid in any case was mixed with an equal mixture of TE saturated phenol and chloroform, followed by vigorous mixing, and then the resulting protein-like insolubles were removed. The aqueous layer was extracted with chloroform, then purified using an Amicon Ultra 10K ultrafiltration filter unit, and analyzed by reverse-phase HPLC.

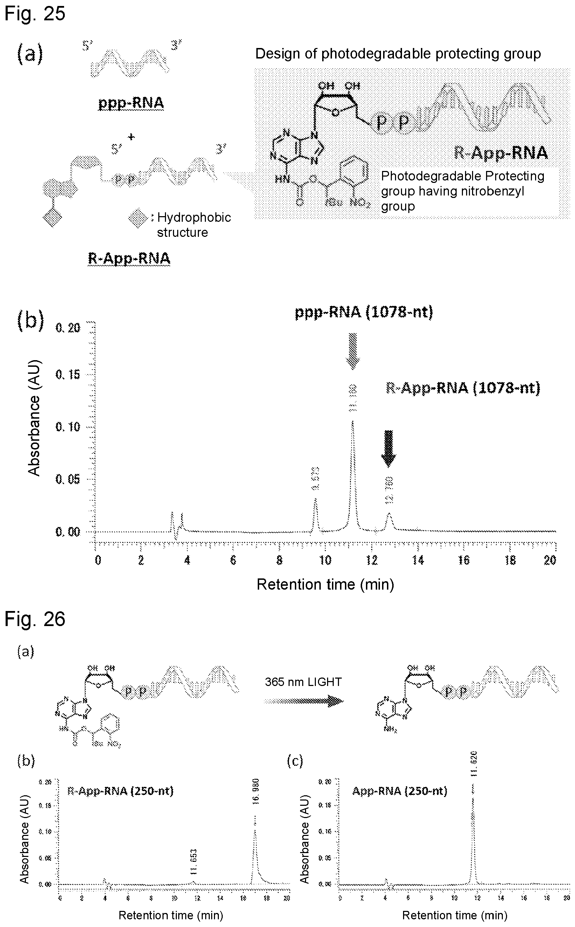

Fig. 25 shows that a protecting group-containing adenylated RNA can be obtained by adding Compound 11 to an in vitro transcription reaction using T7 RNA polymerase.

(a) of the figure illustrates the outline of the experiment. The transcribed RNA is obtained as a mixture of one having a triphosphate structure at the 5'-end (ppp-RNA) and an objective protecting group-containing adenylated structure (R-App-RNA). Since the latter has hydrophobicity that is high and different from that of the former, it can be isolated by reverse-phase HPLC utilizing this property.

(b) of the figure shows the result of reverse-phase HPLC analysis of a 1078-base-long RNA transcription reaction.

Column: YMC-Triart Bio C4 (250 mm × 4.6 mm I.D.); Solvent A: 50 mM triethylammonium acetate (pH 7.0), 5% acetonitrile; Solvent B: acetonitrile; Linear Gradient: 0 to 20% Solvent B (0 to 20 min); Flow rate: 1 mL/min; Detection wavelength: 260 nm; Column temperature: 50°C.

### (10) Deprotection by photoirradiation

A 250-base-long protecting group-containing adenylated RNA was prepared by heating, at 37°C for 2 hours, the following reaction liquid: 5 ng/uL dsDNA transcription template (PCR product of 276-bp containing T7 promoter sequence 5'TAATACGACTCACTATAG3'-(SEQ ID NO: 1)), 2 mM ATP, 2 mM UTP, 2 mM CTP, 0.5 mM GTP, 2 mM Compound 11 (AppG dinucleotide compound having a photodegradable hydrophobic protecting group), 40 mM Tris-HCl (pH 8.0), 8 mM MgCl₂, 2 mM spermidine, 5 mM DTT, 2.5 units/µL T7 RNA polymerase (Takara Bio Inc.). The reaction liquid after the transcription reaction was mixed with an equal mixture of TE saturated phenol and chloroform, followed by vigorous mixing, and then the resulting protein-like insolubles were removed. The aqueous layer was extracted with chloroform, then purified using an Amicon Ultra 10K ultrafiltration filter unit. This was analyzed by reverse-phase HPLC, and a photodegradable hydrophobic protecting group-containing adenylated RNA was fractionated and purified. The eluate containing the objective RNA was desalted using an Amicon Ultra 10K ultrafiltration filter unit. The protecting group-containing adenylated RNA obtained above was deprotected by photoirradiation. The RNA solution (16.5 ng RNA/µL, 100 µL) was added to a transparent 96-multiwell plate, and irradiated with 365 nm light at a quantity of light of 4 mW/cm² for 15 minutes using a MAX-305 light source device (Asahi Spectra Co., Ltd.).

Fig. 26 shows that a protecting group-containing adenylated RNA prepared through a transcription reaction is deprotected by photoirradiation and can be converted into an objective adenylated RNA.

(a) of the figure illustrates the outline of the experiment.

(b) of the figure shows the results of reverse-phase HPLC analysis of a protecting group-containing adenylated RNA (after isolation).

(c) of the figure shows the result of reverse-phase HPLC analysis after irradiation of a protecting group-containing adenylated RNA (after isolation) with 365 nm light.

Column of (b) and (c): YMC-Triart Bio C4 (250 mm × 4.6 mm I.D.); Solvent A: 50 mM triethylammonium acetate (pH 7.0), 5% acetonitrile; Solvent B: acetonitrile; Linear Gradient: 0 to 20% Solvent B (0 to 20 min); Flow rate: 1 mL/min; Detection wavelength: 260 nm; Column temperature: 50°C.

### 5. Cap analog compound having photodegradable hydrophobic protecting group

Novel cap analog compounds having a photodegradable hydrophobic protecting group (Cap Analog_1 and Cap Analog_2) were synthesized.

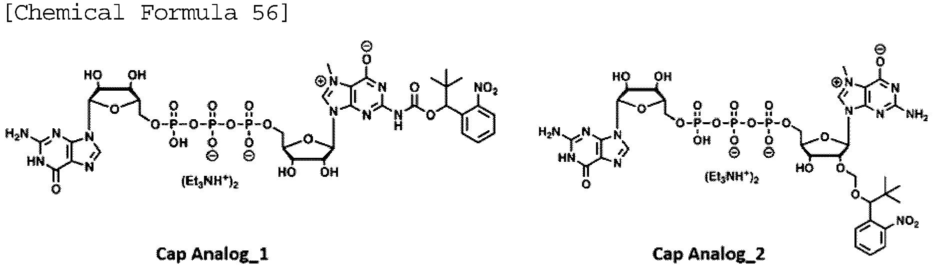

### (1) Synthesis of Cap Analog_1 (N² modification)

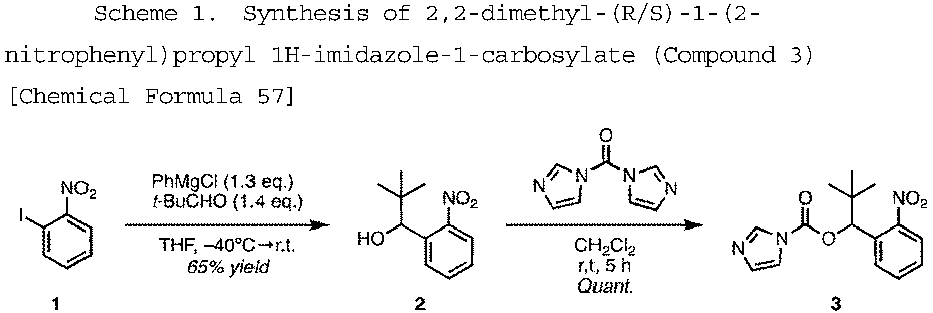

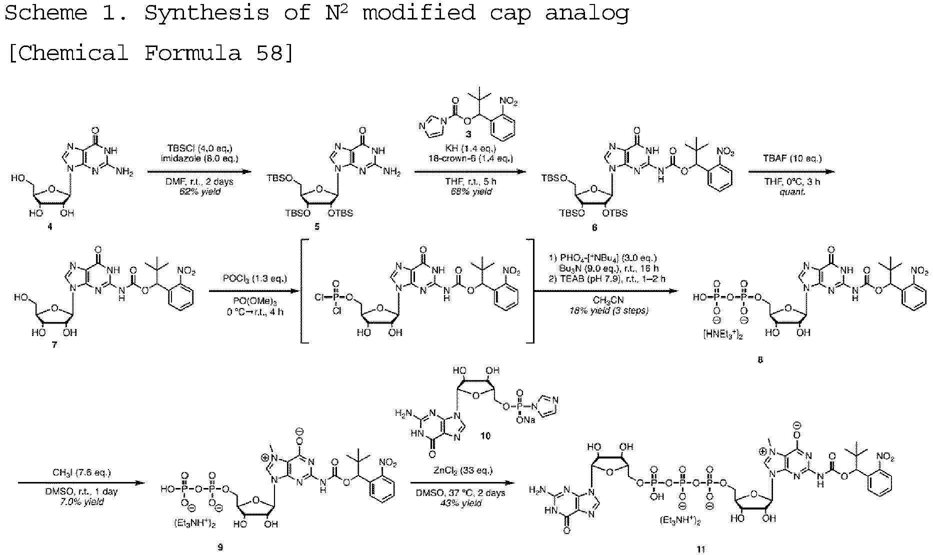

### (a) (R/S)-1-(2-Nitrophenyl)-2,2-dimethyl-1-propanol (Compound 2)

The synthesis of (R/S)-tBu-(2-nitrobenzyl) alcohol (Compound 2) was carried out in accordance with literature. A mixture of 1-iodo-2-nitrobenzene (20 g, 80 mmol) in THF (150 mL) was cooled to -40°C. A solution of phenylmagnesium chloride in THF (2 M, 52 mL, 0.10 mol) was added and stirred at -40°C for 1 hour. Trimethylacetaldehyde (12 mL, 9.6 g, 0.11 mol) was added to the reaction mixture and stirred at -40°C for 36 min. The reaction mixture was gradually warmed to room temperature. After stirring for 4 hours, a saturated aqueous NH₄Cl solution was added to stop the reaction. An aqueous NH₄Cl solution was added to stop the reaction. The mixture was extracted three times with ethyl acetate. The organic layer was dried over Na₂SO₄ and concentrated. The residue was purified by silica gel column chromatography dissolved with hexane and then with 17 → 25% ethyl acetate/hexane, affording Compound 2 as a dark brown solid (11 g, yield 65%). The spectral data of the compound were all consistent with those of the literature.

### (b) 2,2-Dimethyl-(R/S)-1-(2-nitrophenyl)propyl 1H-imidazole-1-carbosylate (Compound 3)

To a solution of (R/S)-1-(2-nitrophenyl)-2,2-dimethyl-1-propanol (Compound 2) (5.8 g, 28 mmol) in dichloromethane (60 mL) was added 1,1'-carbonyldiimidazole (6.8 g, 42 mmol). After stirring at room temperature for 5 hours, the reaction mixture was diluted with dichloromethane and washed with brine. The organic layer was dried over Na₂SO₄ and concentrated. The residue was purified by silica gel column chromatography dissolved with 50 → 67% ethyl acetate/hexane, affording Compound 3 (8.5 g, quant.) as a brown oil.
¹H NMR (400 MHz, CDCl₃) δ8.11 (t, J = 1.1 Hz, 1H), 7.87 (dd, J = 8.2, 1.3 Hz, 1H), 7.59-7.48 (m, 2H), 7.46-7.35 (m, 2H), 7.05-6.98 (m, 1H), 6.62 (s, 1H), 0.96 (s, 9H) ppm
¹³C NMR (101 MHz, CDCl₃) δ149.22, 147.85, 136.97, 132.83, 131.71, 131.01, 129.35, 128.58, 124.97, 117.09, 80.39, 36.51, 25.84. ESI-TOF-MS calcd. for C₁₅H₁₇N₃NaO₄, 326.1111 [M+Na]⁺; found, 326.1076.

### (c) 2',3',5'-O-Tris(tert-butyldimethylsilyl)guanosine (Compound 5)

Synthesis of 2',3',5'-O-trisTBDMS-guanosine (Compound 5) was performed in accordance with literature. To a suspension of guanosine (Compound 4: 10 g, 35 mmol) and imidazole (19 g, 0.28 mol) in N,N-dimethylformamide (70 mL) was added tertbutyldimethylsilyl chloride (21 g, 0.14 mol). After stirring at room temperature for 2 days, the reaction mixture was diluted with ethyl acetate and washed twice with an aqueous NH₄Cl solution and twice with brine. The organic layer was dried over Na₂SO₄ and concentrated. The residue was purified by silica gel column chromatography dissolved with dichloromethane and then with 1 → 3% methanol/dichloromethane, affording Compound 5 as a white solid (14 g, yield 62%). The spectral data of Compound 5 were all consistent with those of the literature.

### (d) N²-{[tBu-(2-nitrobenzyl)oxy]carbonyl}-2' ,3' ,5'-O-tris(tertbutyldimethylsilyl) guanosine (Compound 6)

To a solution of 2' ,3' ,5'-O-trisTBDMS-guanosine (Compound 5) (4.9 g, 7.9 mmol), Compound 3 (2.7 g, 8.7 mmol), and 18-crown-6 (2.8 g, 11 mmol) in THF (40 mL) was added 30% potassium hydride (1.4 g, 11 mmol) in mineral oil. After stirring at room temperature for 5 hours, the reaction mixture was diluted with ethyl acetate and washed with a 1:1 mixed liquid of brine and saturated aqueous NH₄Cl solution. The organic layer was dried over Na₂SO₄ and concentrated. The residue was purified by silica gel column chromatography dissolved with 25 → 50% ethyl acetate/hexane, affording Compound 6 as a brown foam (4.6 g, yield 68%) .

¹H NMR (600 MHz, CDCl₃) δ11.17, 11.15 (2s, 1H), 8.22, 8.15 (2s, 1H), 8.00 (d, J = 2.4 Hz, 1H), 7.90 (d, J = 8.2 Hz, 1H), 7.62-7.50 (m, 2H), 7.49-7.42 (m, 1H), 6.53 (d, J = 6.2 Hz, 1H), 5.83 (dd, J = 5.9, 1.6 Hz, 1H), 4.37 (dd, J = 5.8, 4.3, 1.6 Hz, 1H), 4.22 (dd, J = 4.4, 2.7 Hz, 1H), 4.06-4.03 (m, 1H), 3.87 (dt, J = 11.5, 3.0 Hz, 1H), 3.74 (dd, J = 11.4, 2.5 Hz, 1H), 0.96-0.88 (m, 28H), 0.75 (d, J = 12.9 Hz, 9H), 0.12-0.04 (m, 13H), -0.08 (d, J = 6.9 Hz, 3H), -0.30 (d, J = 14.6 Hz, 3H) ppm.

¹³C NMR (151 MHz, CDCl₃) δ155.39, 152.80, 149.27, 149.23, 148.54, 146.38, 137.13, 132.65, 132.60, 132.11, 129.16, 128.77, 128.73, 124.90, 120.86, 87.24, 86.22, 79.15, 77.02, 72.57, 62.99, 60.52, 36.32, 26.15, 25.92, 25.77, 25.69, 21.17, 18.59, 18.16, 17.91, 14.27, -4.35, -4.50, -4.58, -5.11, -5.30, -5.38 ppm. ESI-TOF-MS calcd. for C₄₀H₆₈N₆NaO₉Si₃, 883.4248 [M+Na]⁺; found, 883.4555.

### (e) N²-{[tBu-(2-nitrobenzyl)oxy]carbonyl}guanosine (Compound 7)

To an ice-cooled solution of fully protected nucleoside 6 (2.0 g, 2.3 mmol) in THF (11 mL) was added 1 M tetrabutylammonium fluoride/THF (23 mL, 23 mmol) in an ice bath. After stirring in an ice bath at 0°C for 3 hours, the reaction mixture was diluted with dichloromethane and washed three times with water. The organic layer was dried over Na₂SO₄ and concentrated. The residue was purified by silica gel column chromatography dissolved with dichloromethane, and then dissolved with 1 → 17% methanol/dichloromethane. The product was suspended in dichloromethane and the solution was added dropwise to diethyl ether with stirring. The resulting precipitate was collected by filtration and washed with diethyl ether, affording Compound 7 (1.2 g, quant) as a white solid.

¹H NMR (400 MHz, DMSO-d₆) δ11.65 (s, 1H), 11.13 (s, 1H), 8.20 (s, 1H), 7.96 (dd, J = 8.0, 1.3 Hz, 1H), 7.77 (td, J = 7.6, 1.3 Hz, 1H), 7.65-7.52 (m, 2H), 6.23 (s, 1H), 5.75 (dd, J = 5.7 Hz, 1H), 5.46 (dd, J = 5.8, 3.1 Hz, 1H), 5.16 (d, J = 4.6 Hz, 1H), 5.01 (t, J = 5.4 Hz, 1H), 4.39 (q, J = 5.5 Hz, 1H), 4.09 (q, J = 4.3 Hz, 1H), 3.86 (q, J = 4.0 Hz, 1H), 3.60 (dt, J = 12.0, 4.8 Hz, 1H), 3.50 (dt, J = 12.0, 4.6 Hz, 1H), 0.88 (s, 9H) ppm.

¹³C NMR (101 MHz, DMSO-d₆) δ155.56, 154.36, 149.53, 149.31, 147.68, 138.15, 133.69, 132.07, 130.01, 129.33, 125.10, 120.42, 87.15, 85.83, 77.67, 74.46, 70.72, 61.67, 36.46, 25.83 ppm. ESI-TOF-MS calcd. for C₂₂H₂₆N₆NaO₉, 541.1654 [M+Na]⁺; found, 541.1887.

### (f) N²-{[tBu-(2-nitrobenzyl)oxy]carbonyl}guanosine-5'-triethylammonium diphosphate salt (Compound 8)

To a solution of guanosine derivative 7 (400 mg, 0.77 mmol) in trimethyl phosphate (7 mL) was added phosphoryl chloride (83 µL, 153 mg, 1.0 mmol) with ice cooling. The mixture was gradually warmed to room temperature and stirred at room temperature. After stirring at room temperature for 4 hours, a mixture of 0.5 M tetrabutylammonium dihydrogen phosphate/CH₃CN (4.6 mL, 2.3 mmol) and tributylamine (1.6 mL, 1.3 g, 6.9 mmol) was added to the reaction mixture in an ice bath. The reaction mixture was warmed to room temperature and stirred at room temperature for 16 hours. Subsequently, a 0.2 M TEAB buffer (pH 7.9, 10 mL) was added to quench the reaction mixture. After stirring at room temperature for 1 to 2 hours, the clear solution was diluted with water and washed five times with dichloromethane. The aqueous layer was concentrated with a rotary evaporator at 50°C. The residue was purified by ion exchange chromatography on QAE-Sephadex in a linear 0 to 1.5 M gradient of TEAB buffer containing 0 to 10% CH₃CN. Guanosine-5'-diphosphate (Compound 8) was obtained in the form of a triethylammonium salt as a white solid (121 mg, 18% yield).

¹H NMR (600 MHz, D₂O) δ8.15 (s, 1H), 7.84 (dd, J = 8.1 Hz, 1H), 7.60 (dd, J = 7.3, 3.6 Hz, 2H), 7.44 (dt, J = 8.4, 4.3 Hz, 1H), 6.25 (s, 1H), 5.92 (dd, J = 6.1, 2.1 Hz, 1H), 4.73-4.68 (m, 1H), 4.43 (dt, J = 6.1, 3.1 Hz, 1H), 4.26-4.22 (m, 1H), 4.09 (q, J = 4.0 Hz, 2H), 3.06 (q, J = 7.4 Hz, 11H), 1.14 (t, J = 7.3 Hz, 17H), 0.82 (s, 9H) ppm.

¹³C NMR (151 MHz, D₂O) δ157.47, 154.72, 149.98, 148.55, 147.48, 139.55, 133.45, 133.41, 131.75, 129.33, 129.05, 124.69, 119.49, 87.22, 87.14, 84.11, 84.05, 79.23, 73.94, 73.85, 70.51, 65.17, 58.68, 46.71, 35.78, 24.80, 8.26 ppm.

³¹P NMR (243 MHz, D₂O) δ-10.25 (1P), -10.69 (1P) ppm. ESI-TOF-MS calcd. for C₂₂H₂₇N₆O₁₅P₂, 677.1015 [M-H]⁻; found, 677.1162.

### (g) N²-{[tBu-(2-nitrobenzyl)oxy]carbonyl}-N⁷-methyl-guanosine-5'-triethylammonium diphosphate salt (Compound 9)

To a solution of guanosine-5'-diphosphate (Compound 8) (72.0 mg, 82.0 µmol) in DMSO (1.82 mL) was added iodomethane (88.0 mg, 39.0 µL, 623 µmol). After stirring at room temperature for 24 hours, the reaction mixture was diluted with water and washed five times with diethyl ether. The aqueous phase was concentrated and the residue was dissolved in water. The crude product was purified by reverse-phase HPLC using a YMC-Triart C8 column (250 × 10.0 mm I.D., S-5 µm, 12 nm, flow rate: 3 mL/min, temperature: 50°C) with a linear gradient of 10 to 80% CH₃CN in a 0.1 M triethylammonium carbonate buffer (pH 7.9) over 25 minutes. The fractions containing the product were collected and acidified to pH 4.0 with the addition of a few drops of acetic acid. After removal of CH₃CN under reduced pressure, the product was desalted with Wakosil (registered trademark) 25C18 (15 to 30 µm, spherical) and freeze-dried, affording N⁷-methyl-guanosine-5'-diphosphate (Compound 9) as a white solid (5.10 mg, 7.0% yield).

¹H NMR (600 MHz, D₂O) δ7.84 (d, J = 8.2 Hz, 1H), 7.60 (s, 2H), 7.44 (d, J = 7.2 Hz, 1H), 6.21 (s, 1H), 6.03 (d, J = 4.9 Hz, 1H), 4.52 (d, J = 15.6 Hz, 1H), 4.37 (s, 1H), 4.27 (s, 1H), 4.19 (d, J = 11.7 Hz, 1H), 4.08 (s, 1H), 4.02 (s, 3H), 3.05 (q, J = 7.1 Hz, 13H), 1.13 (t, J = 7.3 Hz, 20H), 0.81 (s, 9H) ppm.

¹³C NMR (151 MHz, D₂O) δ154.19, 148.63, 148.05, 133.34, 131.98, 129.23, 129.00, 124.64, 111.87, 89.95, 84.20, 78.79, 75.14, 69.16, 63.98, 46.69, 36.06, 35.73, 24.83, 8.25 ppm.

³¹P NMR (243 MHz, D₂O) δ-9.24 (1P), -10.67 (1P) ppm. ESI-TOF-MS calcd. for C₂₃H₂₉N₆O₁₅P₂, 691.1172 [M-H]⁻; found, 691.1395.

### (h) Guanosine 5'-phosphorimidazolide monosodium salt (Compound 10)

The synthesis of guanosine-5'-phosphorimidazolide (Compound 10) was carried out in accordance with literature. To a solution of guanosine 5'-monophosphate (300 mg, 0.53 mmol), imidazole (290 mg, 4.2 mmol), 2,2'-dithiodipyridine (350 mg, 1.6 mmol) in N,N-dimethylformamide (7.5 mL) was added triethylamine (150 µL, 110 mg, 1.1 mmol), and then triphenylphosphine (420 mg, 1.6 mmol) was added. After stirring at room temperature for 2 hours, the reaction mixture was added dropwise to a solution of sodium perchlorate (65 mg, 0.53 mmol) in acetone (60 mL) with stirring. The resulting suspension was transferred to a centrifuge tube and centrifuged at 4,500 rpm for 10 minutes. The supernatant was discarded, and the precipitate was washed five times with acetone. The resulting solid was dried under reduced pressure in a desiccator over P₂O₅, affording guanosine 5'-phosphorimidiazolide monosodium salt (Compound 10) as a white solid (230 mg, quant.).

³¹P NMR (243 MHz, DMSO-d₆) δ1.80 ppm.

### (i) P1-(N²-tBu-nitrobenzyloxycarbonyl-N⁷-methylguanosin-5'-yl) P3-guanosin-5'-yl triphosphate (N²-^{t}Bu-NB-m⁷GpppG) (Compound 11)

N⁷-methyl-guanosine-5'-diphosphate (Compound 9) (1.30 mg, 1.45 µmol) and guanosine 5'-phosphorimidazolide (Compound 10) (2.10 mg, 4.79 µmol) were dissolved in a 0.59 M zinc chloride solution in DMSO (80 µL, ZnCl₂: 47.2 µmol). The mixture was incubated at 37°C for 2 days. The reaction mixture was quenched by the addition of 3 mM aq. EDTA (1.6 mL, 60 µmol) and diluted with a 0.2 M triethylammonium carbonate buffer (pH 7.9, 600 µL, pH adjusted to around 4.0). The mixture was purified by reverse-phase HPLC using a YMC-Triart C8 column (250 × 4.6 mm I.D., S-5 µm, 12 nm, flow rate: 1 mL/min, temperature: 50°C) with a linear gradient of 5 to 80% CH₃CN in a 0.1 M triethylammonium carbonate buffer (pH 7.9) over 25 minutes. The fractions containing the product were collected and acidified to pH 4.0 with the addition of a few drops of acetic acid. After removal of CH₃CN under reduced pressure, the product was desalted with Wakosil (registered trademark) 25C18 (15 to 30 µm, spherical) and freeze-dried, affording N²-^{t}Bu-NB-m⁷GpppG (Compound 11) as a white solid (0.50 mg, 43% yield).

¹H NMR (600 MHz, D₂O) δ7.86-7.81 (m, 2H), 7.60 (dt, J = 5.4 Hz, 2H), 7.43 (dt, J = 8.5, 4.5 Hz, 1H), 6.23 (d, J = 2.8 Hz, 1H), 5.90 (t, J = 2.9 Hz, 1H), 5.64 (t, J = 7.1 Hz, 1H), 4.57-4.51 (m, 1H), 4.44 (dt, J = 13.6, 4.1 Hz, 1H), 4.31 (tt, J = 9.6, 5.1 Hz, 2H), 4.25-4.20 (m, 2H), 4.18-4.04 (m, 5H), 3.95 (s, 3H), 3.06 (q, J = 7.4 Hz, 16H), 1.13 (t, J = 7.3 Hz, 24H), 0.82 (s, 9H) ppm.

¹³C NMR (151 MHz, D₂O) δ158.52, 154.16, 153.87, 151.55, 148.53, 147.63, 137.35, 133.35, 131.77, 129.27, 129.01, 124.60, 115.94, 111.35, 89.88, 87.44, 86.60, 84.06, 83.81, 79.01, 75.14, 73.65, 70.46, 69.16, 65.47, 64.30, 46.67, 36.18, 35.76, 24.82, 8.24 ppm.

³¹P NMR (243 MHz, D₂O) δ-11.02 (2P), -22.60 (1P) ppm. ESI-TOF-MS calcd. for C₃₃H₄₁N₁₁O₂₂P₃, 1036.1646 [M-H]⁻; found, 1036.1643.

### (2) Synthesis of Cap Analog_2 (2'-O-modification)

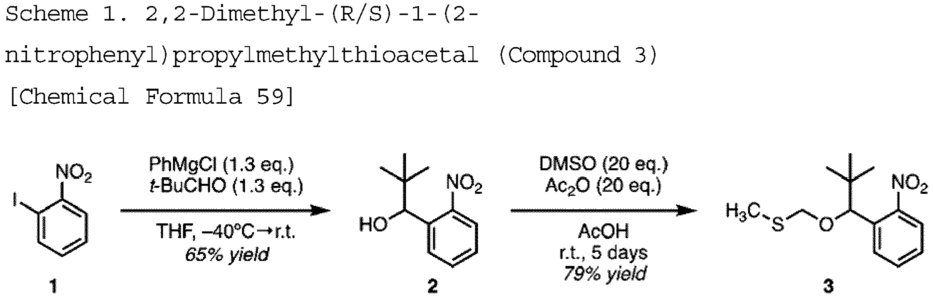

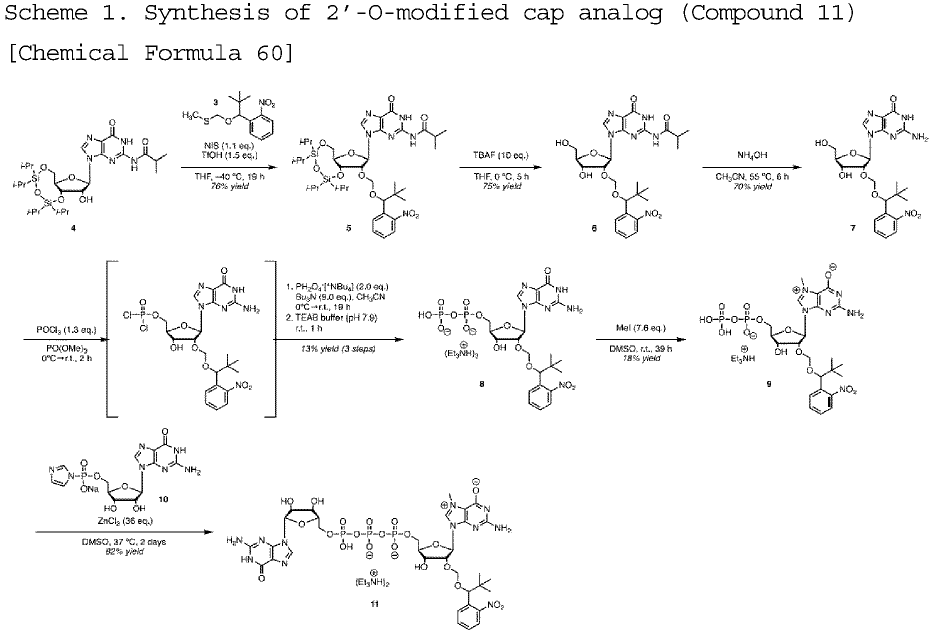

### (a) (R/S)-1-(2-Nitrophenyl)-2,2-dimethyl-1-propanol (Compound 2)

The synthesis of (R/S)-tBu-(2-nitrobenzyl) alcohol (2) was carried out in accordance with literature. A mixture of 1-iodo-2-nitrobenzene (20 g, 80 mmol) in THF (150 mL) was cooled to -40°C. A solution of phenylmagnesium chloride in THF (2 M, 52 mL, 0.10 mol) was added and stirred at -40°C for 1 hour.
Trimethylacetaldehyde (12 mL, 9.6 g, 0.11 mol) was added to the reaction mixture and stirred at -40°C for 36 min. The reaction mixture was gradually warmed to room temperature. After stirring for 4 hours, a saturated aqueous NH₄Cl solution was added to stop the reaction. The mixture was extracted three times with ethyl acetate. The organic layer was dried over Na₂SO₄ and concentrated. The residue was purified by silica gel column chromatography dissolved with hexane and then with 17 → 25% ethyl acetate/hexane, affording Compound 2 as a dark brown solid (11 g, yield 65%). The spectral data of the compound were all consistent with those of the literature.

### (b) 2,2-Dimethyl-(R/S)-1-(2-nitrophenyl)propylmethylthioacetal (Compound 3)

To a solution of (R/S)-1-(2-nitrophenyl)-2,2-dimethyl-1-propanol (Compound 2) (3.00 g, 14.3 mmol) in acetic acid (34.4 g, 33.0 mL, 572 mmol) were added DMSO (22.3 g, 20.0 mL, 286 mmol) and acetic anhydride (29.2 g, 27.0 mL, 286 mmol). After stirring at room temperature for 5 days, a 10 M aqueous KOH solution (130 mL, 1.29 mol) was added dropwise to the reaction mixture in an ice bath. The mixture was stirred at room temperature for 2 hours and then extracted with ethyl acetate. The organic layer was washed with brine and dried over Na₂SO₄. The residue was dissolved in hexane and then subjected to silica gel column chromatography dissolved with 0 to 6.3% ethyl acetate/hexane, affording methylthioacetal 3 as a yellow oil (3.1 g, yield 79%).

¹H NMR (600 MHz, CDCl₃) δ7.71 (dd, J = 8.2, 1.3 Hz, 1H), 7.64 (dd, J = 7.9, 1.5 Hz, 1H), 7.57-7.51 (m, 1H), 7.40-7.33 (m, 1H), 5.28 (s, 1H), 4.62 (d, J = 11.6 Hz, 1H), 4.27 (d, J = 11.6 Hz, 1H), 2.11 (s, 3H), 0.83 (s, 9H) ppm.

¹³C NMR (151 MHz, CDCl₃) δ151.20, 133.97, 131.94, 130.07, 128.35, 123.94, 79.15, 74.16, 36.41, 26.00, 14.58 ppm. ESI-TOF-MS calcd. for C₁₃H₁₉NNaO₃S, 292.0978 [M+Na]⁺; found, 292.0981.

### (c) N²-Isobutyryl-3',5'-O-(1,1,3,3-tetraisopropyldisiloxane-1,3-diyl)-2'-O-[2,2-dimethyl-(R/S)-1-(2-nitrophenyl)propyloxy]methylguanosine (Compound 5)

To a suspension of N²-isobutyryl-3',5'-O-(1,1,3,3-tetraisopropyldisiloxane-1,3-diyl)guanosine (Compound 4) (2.96 g, 4.97 mmol), 2,2-dimethyl-(R/S)-1-(2-nitrophenyl)propylmethylthioacetal (Compound 3) (1.47 g, 5.47 mmol) and powdered molecular sieve 3 angstrom (2.00 g) were charged in tetrahydrofuran (50.0 mL), and N-iodosuccinimide (1.23 g, 5.47 mmol) was added, followed by cooling to -40°C. Trifluoromethanesulfonic acid (1.12 g, 662 µL, 7.46 mmol) was added to the cooled suspension, and the mixture was stirred at - 40°C for 19 hours. The reaction mixture was quenched by addition of triethylamine (24.0 mL), diluted with ethyl acetate, and washed twice with saturated aq. NaHCO³. The organic layer was dried over Na₂SO₄ and concentrated. The residue was purified by silica gel column chromatography dissolved with 0 to 3.2% methanol/dichloromethane, affording Compound 5 as a yellow foam (3.12 g, yield 76%).

¹H NMR (600 MHz, CDCl₃) δ12.08 (s, 1H), 9.04 (s, 1H), 7.94 (s, 1H), 7.74 (dd, J = 8.4 Hz, 1H), 7.61-7.52 (m, 2H), 7.40 (dd, J = 8.3, 4.3 Hz, 1H), 5.92 (s, 1H), 5.19 (d, J = 6.7 Hz, 1H), 4.61 (d, J = 6.7 Hz, 1H), 4.52 (dd, J = 9.4, 4.4 Hz, 1H), 4.28 (d, J = 4.4 Hz, 1H), 4.16 (d, J = 13.4 Hz, 1.5H), 4.02 (dd, J = 9.5, 2.3 Hz, 1.5H), 3.93 (dd, J = 13.4, 2.5 Hz, 2H), 2.73 (p, J = 6.9 Hz, 1H), 1.23 (d, J = 6.8 Hz, 3H), 1.18 (d, J = 6.9 Hz, 3H), 1.12-0.91 (m, 78H), 0.91-0.82 (m, 7H), 0.65 (s, 9H) ppm.

¹³C NMR (151 MHz, CDCl₃) δ179.11, 155.65, 150.82, 147.80, 147.47, 136.43, 133.14, 132.35, 130.10, 128.64, 124.11, 121.15, 91.47, 88.01, 80.98, 77.42, 77.21, 76.99, 76.25, 70.30, 59.48, 50.81, 36.54, 36.45, 25.78, 19.13, 18.92, 17.51, 17.44, 17.39, 17.35, 17.31, 17.27, 17.24, 17.17, 17.13, 17.02, 16.89, 13.47, 13.25, 13.10, 12.94, 12.70, 12.57 ppm. ESI-TOF-MS calcd. for C₃₈H₆₁N₆O₁₀Si₂, 817.3982 [M+H]⁺; found, 817.3921.

### (d) N²-Isobutyryl-2'-O-[2,2-dimethyl-(R/S)-1-(2-nitrophenyl)propyloxy]methylguanosine (Compound 6)

A solution of compound 5 (850 mg, 1.04 mmol) in tetrahydrofuran (4.80 mL) was cooled in an ice bath, and 1 M TBAF/THF (10.0 mL, 10.4 mmol) was added. After stirring at 0°C for 5 hours, the reaction mixture was diluted with dichloromethane and washed with water. The organic layer was dried over Na₂SO₄ and concentrated. The residue was purified by silica gel column chromatography dissolved with 0 to 9.1% methanol/dichloromethane, affording Compound 6 as a colorless amorphous solid (450 mg, yield 75%).

¹H NMR (600 MHz, CDCl₃, mixture of stereoisomers) δ12.40 (s, 0.5H), 12.36 (s, 0.5H), 10.27 (m, 0.5H), 10.21 (m, 0.5H), 8.21 (s, 0.5H), 8.00 (s, 0.5H), 7.66 (d, J = 8.8.21 (s, 0.5H), 8.00 (s, 0.5H), 7.66 (d, J = 8.1 Hz, 0.5H), 7.61 (d, J = 7.9 Hz, 0.5H), 7.53-7.45 (m, 2H), 7.35 (q, J = 8.3 Hz, 1.5H), 7.25 (t, J = 7.7 Hz, 0.5H), 5.98 (d, J = 4.7 Hz, 0.5H), 5.80 (d, J = 4.6 Hz, 0.5H), 5.10 (s, 0.5H), 4.99 (s, 0.5H), 4.84-4.71 (m, 2.5H), 4.67 (t, J = 4.8 Hz, 1H), 4.58 (t, J = 10.7 Hz, 1.5H), 4.13 (dd, J = 10.4, 3.8 Hz, 1H), 3.91-3.84 (m, 1H), 3.76 (t, J = 13.2 Hz, 1H), 3.39 (t, J = 4.1 Hz, 1H), 2.90 (h, J = 6.8 Hz, 1H), 1.24 (td, J = 4.3, 2.0 Hz, 6H), 0.71 (s, 4.5H), 0.59 (s, 4.5H) ppm.

¹³C NMR (151 MHz, CDCl₃, mixture of stereoisomers) δ180.28, 180.22, 155.67, 155.60, 150.52, 149.79, 148.38, 148.27, 148.16, 147.86, 139.26, 133.87, 133.51, 132.14, 131.81, 130.01, 129.92, 128.51, 128.32, 123.95, 123.77, 121.00, 120.94, 95.02, 93.66, 88.27, 86.19, 86.05, 80.47, 79.49, 79.41, 79.23, 77.38, 77.17, 76.96, 70.68, 70.50, 70.25, 62.00, 36.66, 36.21, 36.16, 26.57, 25.65, 25.60, 19.14, 19.06, 19.03 ppm. ESI-TOF-MS calcd. for C₂₆H₃₃N₆O₉, 573.2315 [M-H]⁻; found 573.2333.

### (e) N²-Isobutyryl-2'-O-[2,2-dimethyl-(R/S)-1-(2-nitrophenyl)propyloxy]methylguanosine (Compound 6)

A solution of compound 5 (850 mg, 1.04 mmol) in tetrahydrofuran (4.80 mL) was cooled in an ice bath, and 1 M TBAF/THF (10.0 mL, 10.4 mmol) was added. After stirring at 0°C for 5 hours, the reaction mixture was diluted with dichloromethane and washed with water. The organic layer was dried over Na₂SO₄ and concentrated. The residue was purified by silica gel column chromatography dissolved with 0 to 9.1% methanol/dichloromethane, affording Compound 6 as a colorless amorphous solid (450 mg, yield 75%).

¹H NMR (600 MHz, CDCl₃, mixture of stereoisomers) δ12.40 (s, 0.5H), 12.36 (s, 0.5H), 10.27 (m, 0.5H), 10.21 (m, 0.5H), 8.21 (s, 0.5H), 8.00 (s, 0.5H), 7.66 (d, J = 8.8.21 (s, 0.5H), 8.00 (s, 0.5H), 7.66 (d, J = 8.1 Hz, 0.5H), 7.61 (d, J = 7.9 Hz, 0.5H), 7.53-7.45 (m, 2H), 7.35 (q, J = 8.3 Hz, 1.5H), 7.25 (t, J = 7.7 Hz, 0.5H), 5.98 (d, J = 4.7 Hz, 0.5H), 5.80 (d, J = 4.6 Hz, 0.5H), 5.10 (s, 0.5H), 4.99 (s, 0.5H), 4.84-4.71 (m, 2.5H), 4.67 (t, J = 4.8 Hz, 1H), 4.58 (t, J = 10.7 Hz, 1.5H), 4.13 (dd, J = 10.4, 3.8 Hz, 1H), 3.91-3.84 (m, 1H), 3.76 (t, J = 13.2 Hz, 1H), 3.39 (t, J = 4.1 Hz, 1H), 2.90 (h, J = 6.8 Hz, 1H), 1.24 (td, J = 4.3, 2.0 Hz, 6H), 0.71 (s, 4.5H), 0.59 (s, 4.5H) ppm.

¹³C NMR (151 MHz, CDCl₃, mixture of stereoisomers) δ180.28, 180.22, 155.67, 155.60, 150.52, 149.79, 148.38, 148.27, 148.16, 147.86, 139.26, 133.87, 133.51, 132.14, 131.81, 130.01, 129.92, 128.51, 128.32, 123.95, 123.77, 121.00, 120.94, 95.02, 93.66, 88.27, 86.19, 86.05, 80.47, 79.49, 79.41, 79.23, 77.38, 77.17, 76.96, 70.68, 70.50, 70.25, 62.00, 36.66, 36.21, 36.16, 26.57, 25.65, 25.60, 19.14, 19.06, 19.03 ppm. ESI-TOF-MS calcd. for C₂₆H₃₃N₆O₉, 573.2315 [M-H]⁻; found 573.2333.

### (f) 2'-O-[2,2-Dimethyl-(R/S)-1-(2-nitrophenyl)propyloxy]methylguanosine (Compound 7)

A 28% ammonium hydroxide solution (36.0 mL) was added to a solution of Compound 6 (1.08 g, 1.88 mmol) in acetonitrile (14.4 mL). The mixture was heated to 55°C. After stirring at 55°C for 6 hours, the reaction mixture was concentrated and azeotroped with benzene. The residue was suspended in a 1:3 mixture of dichloromethane and diethyl ether. The resulting precipitate was collected by filtration and washed with a 1:3 mixture of dichloromethane and diethyl ether. The resulting solid was dried in a desiccator on P₂O₅ under vacuum, affording Compound 7 as a white foam (666 mg, 70% yield).

¹H NMR (600 MHz, DMSO-d₆, mixture of stereoisomers) δ10.61, 10.58 (2s, 1H), 7.88 (s, 0.5H), 7.79 (dd, J = 8.0, 1.3 Hz, 0.5H), 7.70-7.62 (m, 1.5H), 7.53-7.46 (m, 1H), 7.41 (m, 1H), 7.31 (ddd, J = 8.6, 6.6, 2.1 Hz, 0.5H), 6.37 (d, J = 10.1 Hz, 2H), 5.81 (d, J = 6.0 Hz, 0.5H), 5.59 (d, J = 5.6 Hz, 0.5H), 5.21 (d, J = 5.4 Hz, 0.5H), 5.15 (d, J = 5.3 Hz, 0.5H), 5.10 (t, J = 5.6 Hz, 0.5H), 5.03 (t, J = 5.5 Hz, 0.5H), 4.95 (s, 0.5H), 4.82 (s, 0.5H), 4.71-4.67 (m, 1H), 4.67-4.61 (m, 1H), 4.47 (t, J = 5.2 Hz, 0.5H), 4.38 (d, J = 7.2 Hz, 0.5H), 4.23 (td, J = 5.1, 3.8 Hz, 1H), 3.87 (q, J = 3.6 Hz, 0.5H), 3.82 (q, J = 3.7 Hz, 0.5H), 3.63-3.52 (m, 1H), 3.52-3.42 (m, 1H), 0.68 (s, 4.5H), 0.53 (s, 4.5H) ppm.

¹³C NMR (151 MHz, DMSO-d₆, mixture of stereoisomers) δ157.25, 154.15, 154.11, 151.61, 151.48, 150.98, 150.00, 136.35, 135.70, 133.46, 133.00, 132.82, 132.39, 130.10, 129.99, 129.45, 128.93, 124.35, 124.11, 117.38, 117.18, 94.57, 92.51, 86.37, 86.04, 85.85, 85.39, 80.21, 78.95, 78.84, 78.72, 70.25, 70.06, 69.75, 62.05, 61.81, 40.46, 40.32, 40.19, 40.04, 39.91, 39.76, 39.62, 36.64, 36.04, 25.88, 25.83 ppm. ESI-TOF-MS calcd. for C₂₂H₂₈N₆NaO₈, 527.1861 [M+Na]⁺; found 527.1758.

### (g) 2'-O-[2,2-Dimethyl-(R/S)-1-(2-nitrophenyl)propyloxy]methylguanosine-5'-triethylammonium diphosphate salt (Compound 8)

To a solution of guanosine derivative 7 (200 mg, 0.396 mmol) in trimethyl phosphate (3.70 mL) was added phosphoryl chloride (48.0 µL, 79.0 mg, 0.515 mmol) with ice cooling. The mixture was gradually warmed to room temperature and stirred at room temperature. After stirring at room temperature for 2 hours, a mixture of 0.44 M tetrabutylammonium dihydrogen phosphate/CH₃CN (1.80 mL, 0.792 mmol) and tributylamine (846 µL, 660 mg, 3.56 mmol) was added to the reaction mixture in an ice bath. The reaction mixture was warmed to room temperature and stirred at room temperature for 19 hours. Subsequently, a 0.2 M triethylammonium carbonate buffer (pH 7.9, 5.0 mL) was added to quench the reaction mixture. After stirring at room temperature for 1 hour, the mixture was diluted with water and washed five times with dichloromethane. The aqueous layer was concentrated with a rotary evaporator at 50°C. The residue was purified by ion exchange chromatography on QAE-Sephadex in a linear 0 to 1.5 M gradient of triethylammonium carbonate buffer containing 0 to 10% CH₃CN. Guanosine-5'-diphosphate (Compound 8) was obtained as a triethylammonium salt in the form of a white solid (51.0 mg, 13% yield).

¹H NMR (600 MHz, D₂O, mixture of stereoisomers) δ7.87 (s, 0.5H), 7.58 (s, 0.5H), 7.52 (d, J = 8.1 Hz, 0.5H), 7.47-7.38 (m, 1H), 7.38-7.29 (m, 1H), 7.28-7.17 (m, 1H), 7.01 (t, J = 7.8 Hz, 0.5H), 5.73 (d, J = 5.8 Hz, 0.5H), 5.48 (d, J = 5.0 Hz, 0.5H), 4.96, 4.94 (2s, 1H), 4.82 (d, J = 7.5 Hz, 0.5H), 4.76 (s, 0.5H), 4.71 (d, J = 7.3 Hz, 0.5H), 4.62 (dd, J = 18.0, 6.6 Hz, 1.5H), 4.52 (t, J = 5.1 Hz, 0-.5H), 4.45 (t, J = 5.0 Hz, 1H), 4.14 (d, J = 4.1 Hz, 0.5H), 4.10-4.03 (m, 1.5H), 3.99 (d, J = 4.8 Hz, 1H), 3.02 (q, J = 7.3 Hz, 15H), 1.10 (t, J = 7.3 Hz, 24H), 0.52 (s, 4.5H), 0.41 (s, 4.5H) ppm.

¹³C NMR (151 MHz, D₂O, mixture of stereoisomers) δ158.95, 158.75, 153.89, 153.59, 151.59, 151.01, 149.74, 148.09, 138.02, 137.37, 133.49, 132.57, 132.47, 132.21, 129.86, 129.66, 128.65, 128.06, 123.80, 123.30, 116.33, 115.96, 95.44, 93.71, 86.20, 84.26, 84.20, 83.73, 83.67, 81.61, 80.72, 78.55, 76.97, 69.07, 68.94, 64.84, 64.81, 64.46, 46.63, 36.55, 35.65, 24.78, 24.68, 8.24 ppm.

³¹P NMR (243 MHz, D₂O, mixture of stereoisomers) δ-8.54 (1P), -10.61 (1P) ppm. ESI-TOF-MS calcd. for C₂₂H₂₉N₆O₁₄P₂, 663.1223 [M-H]-; found 663.1378.

### (h) N⁷-Methyl-2'-O-[2,2-dimethyl-(R/S)-1-(2-nitrophenyl)propyloxy]methylguanosine-5'-triethylammonium diphosphate salt (Compound 9)

To a solution of guanosine-5'-diphosphate (Compound 8) (4.3 mg, 4.4 µmol) in DMSO (100 µL) was added iodomethane (4.7 mg, 2.1 µL, 33 µmol). After stirring at room temperature for 39 hours, the reaction mixture was diluted with water and washed five times with diethyl ether. The aqueous phase was concentrated and the residue was dissolved in water. Crude purification was performed by reverse-phase HPLC using a YMC-Triart C8 column (250 × 4.6 mm I.D., S -5 um, 12 nm, flow rate 1 mL/min, temperature 50°C) with a linear gradient of 5 to 80% in concentration of CH₃CN in a 0.1 M triethylammonium hydrogen carbonate buffer (pH 7.9) over 25 min. The fractions containing the product were collected and acidified to pH 4.0 with the addition of a few drops of acetic acid. After removal of CH₃CN under reduced pressure, the product was desalted with Wakosil (registered trademark) 25C18 (15 to 30 µm, spherical) and freeze-dried, affording N⁷-methyl-guanosine-5'-diphosphate (Compound 9) as a white solid (0.60 mg, 18% yield, a mixture of two stereoisomers).

¹H NMR (600 MHz, D₂O, single stereoisomer isolated by HPLC) δ8.93 (s, 1H), 7.60 (d, J = 8.2 Hz, 1H), 7.49 (dd, J = 15.2, 8.1 Hz, 2H), 7.29 (t, J = 7.6 Hz, 1H), 5.82 (s, 1H), 5.00 (d, J = 4.1 Hz, 1H), 4.93 (d, J = 8.0 Hz, 1H), 4.86 (d, J = 7.2 Hz, 1H), 4.54-4.50 (m, 1H), 4.42 (t, J = 5.2 Hz, 1H), 4.25 (s, 1H), 4.10 (d, J = 11.2 Hz, 1H), 3.94 (d, J = 3.7 Hz, 3H), 3.05 (t, J = 7.3 Hz, 4H), 1.13 (t, J = 7.1 Hz, 6H), 0.61 (s, 10H) ppm.

¹³C NMR (151 MHz, D₂O, single stereoisomer isolated by HPLC) δ155.41, 154.99, 149.37, 148.61, 136.68, 133.90, 132.44, 130.24, 128.15, 123.51, 107.91, 95.16, 87.76, 85.95, 82.16, 79.32, 68.89, 64.78, 46.70, 36.52, 36.07, 24.76, 8.25 ppm.

³¹P NMR (243 MHz, D₂O, single stereoisomer isolated by HPLC) δ-10.03 (1P), -10.71 (1P) ppm. ESI-TOF-MS calcd. for C₂₃H₃₁N₆O₁₄P₂, 677.1379 [M-H]-; found 677.1374.

### (i) Guanosine 5'-phosphorimidazolide monosodium salt (Compound 10)

The synthesis of guanosine-5'-phosphorimidazolide (Compound 10) was carried out in accordance with literature. To a solution of guanosine 5'-monophosphate (300 mg, 0.53 mmol), imidazole (290 mg, 4.2 mmol), 2,2'-dithiodipyridine (350 mg, 1.6 mmol) in N,N-dimethylformamide (7.5 mL) was added triethylamine (150 µL, 110 mg, 1.1 mmol), and then triphenylphosphine (420 mg, 1.6 mmol) was added. After stirring at room temperature for 2 hours, the reaction mixture was added dropwise to a solution of sodium perchlorate (65 mg, 0.53 mmol) in acetone (60 mL) with stirring. The resulting suspension was transferred to a centrifuge tube and centrifuged at 4,500 rpm for 10 minutes. The supernatant was discarded, and the precipitate was washed five times with acetone. The resulting solid was dried under reduced pressure in a desiccator over P₂O₅, affording guanosine 5'-phosphorimidiazolide monosodium salt (Compound 10) as a white solid (230 mg, quant.).

³¹P NMR (243 MHz, DMSO-d₆) δ1.80 ppm.

### (j) P1-{N⁷-Methyl-2'-O-[2,2-dimethyl-(R/S)-1-(2-nitrophenyl)propyloxy]methylguanosin-5'-yl} P3-guanosin-5'-yl triphosphate (2'-O-tBu-NB-m7GpppG) (Compound 11)

N⁷-methyl-guanosine-5'-diphosphate (Compound 9) (1.1 mg, 1.3 µmol) and guanosine 5'-phosphorimidazolide (Compound 10) (1.9 mg, 4.3 µmol) were dissolved in 0.59 M zinc chloride solution in DMSO (80 µL, ZnCl₂: 47 µmol). The mixture was incubated at 37°C for 2 days. The reaction mixture was quenched by the addition of 38 mM aq. EDTA (1.6 mL, 60 µmol) and diluted with a 0.2 M TEAB buffer (pH 7.9, 600 µL, pH adjusted to around 4.0). The mixture was purified by reverse-phase HPLC using a YMC-Triart C8 column (250 × 4.6 mm I.D., S-5 µm, 12 nm, flow rate: 1 mL/min, temperature: 50°C) with a linear gradient of 5 to 80% CH₃CN in a 0.1 M triethylammonium carbonate buffer (pH 7.9) over 25 minutes. The fractions containing the product were collected and acidified to pH 4.0 with the addition of a few drops of acetic acid. After removal of CH₃CN under reduced pressure, the product was desalted with Wakosil (registered trademark) 25C18 (15 to 30 µm, spherical) and freeze-dried, affording 2'-O-^{t}Bu-NB-m⁷GpppG (Compound 11) as a white solid (0.90 mg, 82% yield).

¹H NMR (600 MHz, D₂O, mixture of stereoisomers) δ7.85, 7.82 (2s, 1H), 7.66 (dd, J = 8.1, 1.3 Hz, 0.5H), 7.57-7.51 (m, 1H), 7.49-7.41 (m, 1.5H), 7.38-7.32 (m, 0.5H), 7.24 (dd, J = 8.4, 4.1 Hz, 0.5H), 5.81 (d, J = 4.8 Hz, 0.5H), 5.66-5.59 (m, 1.5H), 4.95 (s, 0.5H), 4.87 (d, J = 7.8 Hz, 1H), 4.80-4.70 (m, 1H), 4.55-4.45 (m, 2H), 4.41 (t, J = 4.9 Hz, 0.5H), 4.35-4.25 (m, 2H), 4.25-4.04 (m, 8.5H), 3.95 (s, 1.5H), 3.90 (s, 1.5H), 3.06 (q, J = 7.4 Hz, 13H), 1.14 (t, J = 7.3 Hz, 20H), 0.56 (s, 5H), 0.53 (s, 6H) ppm.

¹³C NMR (151 MHz, D₂O, mixture of stereoisomers) δ158.25, 155.45, 155.20, 154.66, 154.51, 153.89, 149.73, 149.40, 148.93, 148.54, 137.38, 136.90, 136.48, 133.82, 132.74, 132.41, 130.14, 130.00, 128.82, 128.08, 123.88, 123.40, 107.73, 107.39, 94.91, 93.58, 87.60, 87.04, 86.94, 85.62, 84.87, 83.72, 81.98, 80.56, 79.67, 79.24, 73.84, 73.74, 70.26, 68.68, 68.24, 65.37, 64.96, 64.56, 62.55, 46.68, 36.42, 36.18, 35.74, 24.84, 24.71, 8.25 ppm.

³¹P NMR (243 MHz, D₂O, mixture of stereoisomers) δ-10.96 (2P), -22.54 (1P) ppm. ESI-TOF-MS calcd. for C₃₃H₄₃N₁₁O₂₁P₃, 1022.1853 [M-H]-; found 1022.1851.

### (3) Analyses of synthesized cap analogs

The cap analogs (Cap Analog_1 and Cap Analog_2) synthesized above were subjected to various analyses.

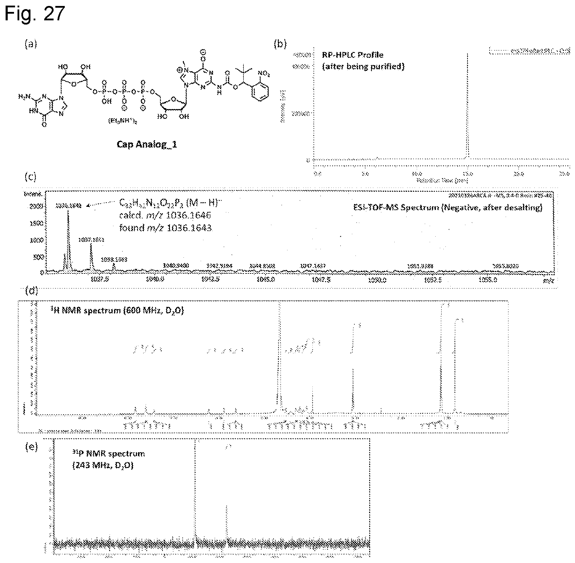

Fig. 27 is a diagram showing the result of various analysis of Cap Analog_1, which is a novel cap analog compound synthesized. These analysis results all showed that the objective compound was obtained with good purity.

(a) of the figure illustrates the chemical structure of cap analog_1.

(b) of the figure shows the result of reverse-phase HPLC analysis after isolation and purification of Cap Analog_1. The analysis conditions are as follows.
Column: YMC-Triart C8 (250 mm × 4.6 mm I.D.); Solvent A: 0.1 M triethylammonium carbonate buffer (pH 7.9); Solvent B: acetonitrile; Linear Gradient: 5 to 80% Solvent B (0 to 25 min); Flow rate: 1 mL/min; Detection wavelength: 280 nm; Column temperature: 50°C

(c) of the figure shows the result of ESI-TOF MS molecular weight measurement.

(d) of the figure shows the result of ¹H-NMR analysis, and (e) of the figure shows the result of ³¹P-NMR analysis.

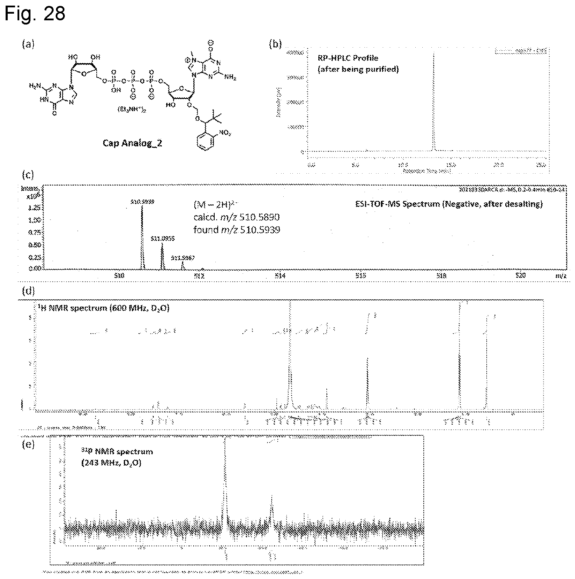

Fig. 28 is a diagram showing the result of various analysis of Cap Analog_2, which is a novel cap analog compound synthesized. These analysis results all showed that the objective compound was obtained with good purity.

(a) of the figure illustrates the chemical structure of the cap analog_2.

(b) of the figure shows the result of reverse-phase HPLC analysis after isolation and purification. The analysis conditions are as follows.

Column: YMC-Triart C8 (250 mm × 4.6 mm I.D.); Solvent A: 0.1 M triethylammonium carbonate buffer (pH 7.9); Solvent B: acetonitrile; Linear Gradient: 5 to 80% Solvent B (0 to 25 min); Flow rate: 1 mL/min; Detection wavelength: 280 nm; Column temperature: 50°C

(c) of the figure shows the result of ESI-TOF MS molecular weight measurement.

(d) of the figure shows the result of ¹H-NMR analysis, and (e) of the figure shows the result of ³¹P-NMR analysis.

### (4) Isolation and purification of RNA with novel cap analogue introduced therein

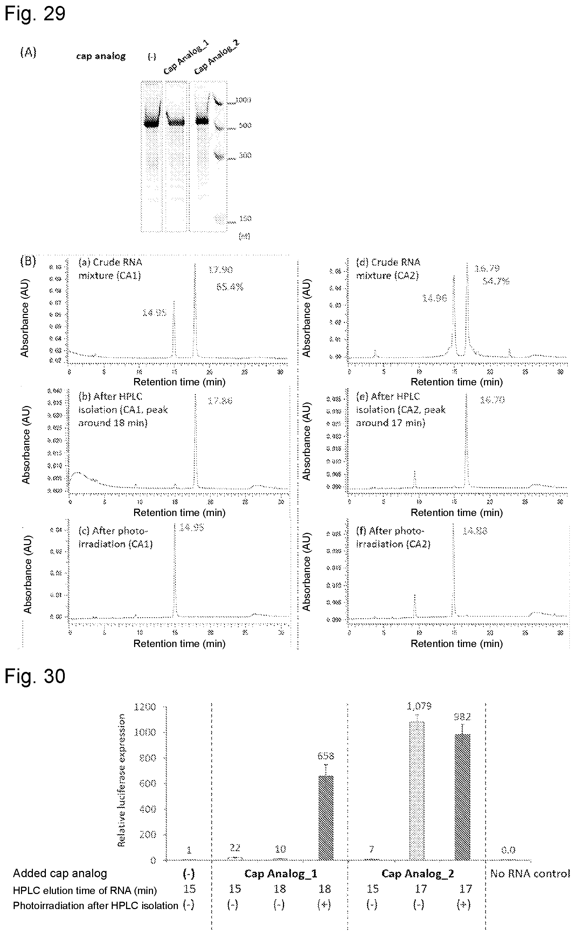

Fig. 29 is a diagram showing that an RNA with a novel cap analog introduced therein could be isolated and purified by reverse-phase HPLC.

In (A) of the figure, a cap analog compound was added, a NanoLuc luciferase mRNA (650-base-long) was transcriptionally synthesized using T7 RNA polymerase, and the transcription reaction was analyzed by denaturing polyacrylamide gel (5%, containing 7.5 M urea as a denaturant) electrophoresis. The composition of the transcription reaction liquid and the reaction conditions are shown below.

15 ng/µL dsDNA transcription template (PCR product of 676-bp containing T7 promoter sequence 5'TAATACGACTCACTATAG3'-(SEQ ID NO: 1) and containing a NanoLuc luciferase gene coding region derived from a pNL1.1 TK vector (Promega)), 2 mM ATP, 2 mM UTP, 2 mM CTP, 2mM GTP or [0.5 mM GTP + 2 mM Cap Analog], 40 mM Tris-HCl (pH 8.0), 8 mM MgCl₂, 2 mM spermidine, 5 mM DTT, 10 units/µL T7 RNA polymerase (Takara Bio Inc.).

The reaction liquid was warmed at 37°C for 2 hours. After electrophoresis, the gel was stained with SYBR Green II stain and photographed with ChemiDoc MP imager (Bio-Rad), whereby the RNA was visualized.

### (B) of the figure is a diagram showing the results of reverse-phase HPLC analysis and purification of a transcribed RNA.

(a) and (d) show the results of performing a transcription reaction by adding (a) Cap Analog_1 (CA1) or (d) Cap Analog_2 (CA2), subjecting an RNA to crude purification (deproteinization, alcohol precipitation, colloquial filtration), and analyzing the RNA by reverse-phase HPLC. A peak appearing around 15 minutes and a peak appearing thereafter were each fractionated and purified.

(b) of the figure shows the result of reanalysis performed by fractionating the eluted RNA around 17.9 minutes shown in (a).

(e) of the figure shows the result of reanalysis performed by fractionating the eluted RNA around 16.8 minutes shown in (d).

(c) of the figure shows the result of RNA analysis after irradiation with 365-nm light at 4 mW/cm² for 10 minutes of the fractionated RNA eluted near 17.9 shown in (a). From the fact that the elution time of the RNA was completely shifted from around 17.9 minutes to around 15 minutes, it can be determined that the hydrophobic protecting group of the cap analog moiety was completely deprotected.

(f) of the figure shows the result of RNA analysis after irradiation with 365-nm light at 4 mW/cm² for 10 minutes of the fractionated RNA eluted near 16.8 shown in (d). From the fact that the elution time of the RNA was completely shifted from around 16.8 minutes to around 15 minutes, it was determined that the hydrophobic protecting group of the cap analog moiety was completely deprotected.

The analytical conditions of HPLC are shown below.

Column: YMC-Triart Bio C4 (250 mm × 4.6 mm I.D.); Solvent A, 50 mM triethylammonium acetate (pH 7.0), 5% acetonitrile; Solvent B, acetonitrile; Linear Gradient 0 to 20% Solvent B (0 to 20 min); Flow rate: 1 mL/min; Detection wavelength: 260 nm; Column temperature: 50°C.

Fig. 30 is a diagram showing the evaluation of the translation activity of a NanoLuc luciferase mRNA after reverse-phase HPLC isolation and purification. The activity was indicated with the expression level of the mRNA prepared without addition of the cap analog being 1. The mRNA (20 ng/well) after HPLC isolation was introduced into HeLa cells (seeded on a 96-multiwell plate in 1 × 10⁴ cells/well the day before) by a lipofection method (Lipofectamine Messenger MAX, 0.3 µL/well), cultivated for 7 hours, and then lysed, and the amount of expressed protein (NanoLuc luciferase) contained in the lysate was measured using a Nano-Glo Luciferase Assay System (Promega). mRNAs capped with a novel cap analog compound showed high translation activity. The mRNA modified with Cap Analog_1 does not show high translation activity unless it is irradiated with light and deprotected, but the mRNA modified with Cap Analog_2 showed similar high translation activity before and after photoirradiation and deprotection.

Cap Analog_1 has a form in which the hydrogen bonding of a base moiety is protected, and can completely inhibit translation activity in the protected state. Cap Analog_1 can be applied, through irradiation with light, to an mRNA that activates translation in vivo.

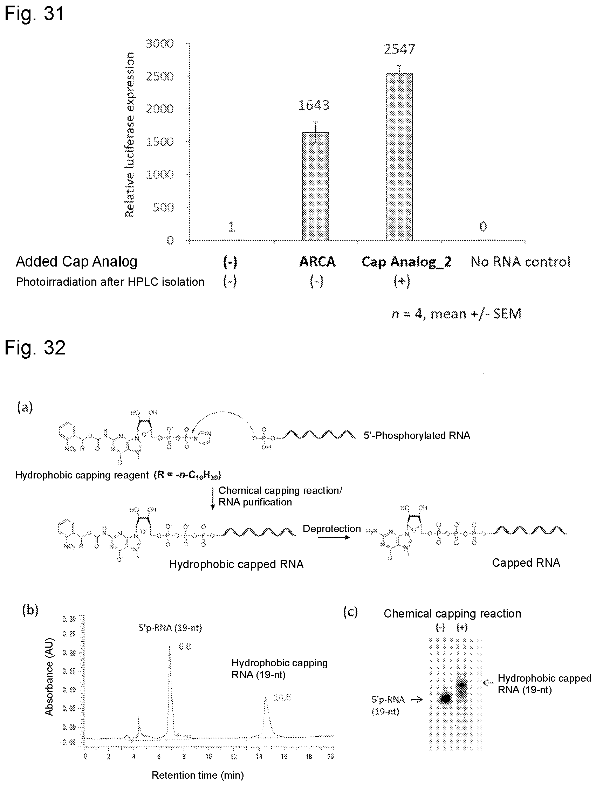

Fig. 31 is a diagram showing the evaluation of the translation activity of a NanoLuc luciferase mRNA after reverse-phase HPLC isolation and purification. The activity was indicated with the expression level of the mRNA prepared without addition of the cap analog being 1. The translation activity was compared to that of the mRNA similarly prepared using a commercially available ARCA cap analog (JENA BIOSCIENCE, cat #78862). The mRNA (5 ng/well) after HPLC isolation was introduced into HeLa cells (seeded on a 96-multiwell plate in 1 × 10⁴ cells/well the day before) by a lipofection method (Lipofectamine Messenger MAX, 0.3 pL/well), cultivated for 23 hours, and then lysed, and the amount of expressed protein (NanoLuc luciferase) contained in the lysate was measured using a Nano-Glo Luciferase Assay System (Promega). The mRNA capped using Cap Analog_2 showed higher translation activity compared to the RNA capped with ARCA. This high translation activity is presumed to be due to the fact that purification utilizing a hydrophobic protecting group yielded an mRNA with a capping rate of 100%.

### 6. Synthesis of hydrophobic chemically capped compounds

### (1) Synthesis of hydrophobic chemically capped compound

A synthesis scheme of a hydrophobic chemically capped compound (Compound 8) is shown below.

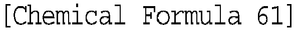

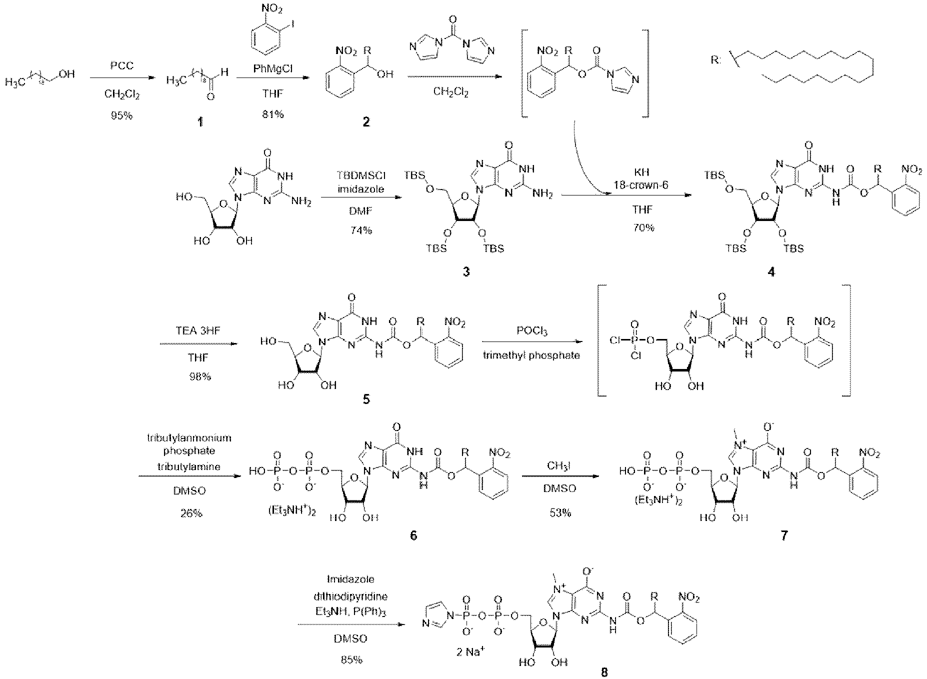

### (a) Synthesis of Compound 1

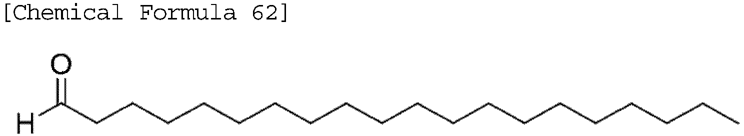

1-Eicosanol (3.00 g, 0.010 mmol, 1.0 equiv.) and Celite (4.0 g) were suspended in dehydrated dichloromethane (20 mL) at 0°C. Pyridinium chlorochromate (3.23 g, 0.015 mmol, 1.5 equiv.) was added thereto, and the mixture was stirred at room temperature for 2 hours. After completion of the reaction, a filtrate was collected by Celite filtration and concentrated. The residue was purified by column chromatography (neutral flash silica, dichloromethane), affording 2.80 g (0.095 mmol, yield 95%) of Compound 1 as a white solid.

¹H-NMR (400 MHz, CDCl₃) δ9.76 (t, J = 1.6 Hz, 1H), 1.29-1.25 (m, 36H), 0.88 (t, J = 7.2 Hz, 3H) ppm.

The other spectral data agreed well with literature values (Gray G. M., Macfarlane M. G., Biochem. J., 1961, 81, 480-488).

### (b) Synthesis of Compound 2

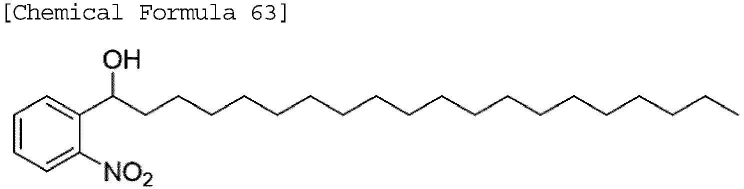

1-Iodo-2-nitrobenzene (2.65 g, 10.7 mmol, 1.0 equiv.) was dissolved in THF (25 mL). Phenylmagnesium chloride (2.0 M solution in THF, 5.86 mL, 11.7 mmol, 1.1 equiv.) was added dropwise at -48°C, and the mixture was stirred at -35°C for 10 minutes. Compound 1 (3.00 g, 10.1 mmol, 0.95 equiv.) dissolved in THF (15 mL) was added dropwise, the reaction temperature was gradually raised to room temperature, and then the mixture was stirred at room temperature for 15 minutes. A saturated aqueous ammonium chloride solution (10 mL) was added thereto, and the resulting mixture was subjected to liquid-liquid separation twice with ethyl acetate (100 mL) and once with saturated saline (50 mL). The organic layer was collected and concentrated. The residue was purified by column chromatography (neutral flash silica, ethyl acetate/hexane = 1/15 → 1/10), affording 3.44 g (8.65 mmol, yield 81%) of Compound 2 as a brown solid.

¹H-NMR (400 MHz, CDCl₃) δ7.89 (dd, J = 8.2 Hz, 1.6 Hz, 1H), 7.80 (dd, J = 6.4 Hz, 1.6 Hz, 1H), 7.63 (td, J = 6.4 Hz, 1.2 Hz, 1H), 7.41 (td, J = 6.0 Hz, 1.6 Hz, 1H), 5.23 (q, J = 4.0 Hz, 1H), 2.09-1.78 (m, 3H), 1.31-1.21 (m, 33H), 0.88 (t, J = 7.2 Hz, 3H) ppm.

¹³C-NMR (100 MHz, CDCl₃) δ148.1, 140.3, 133.5, 128.1, 128.0, 124.4, 69.5, 38.3, 32.0, 29.8-29.4, 26.2, 22.8, 14.2 ppm.

ESI MS-HR calcd. for C₂₆H₄₅NNaO₃⁺ (M+Na)⁺ 442.3297, found 442.3421.

### (c) Synthesis of Compound 3

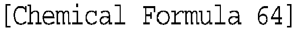

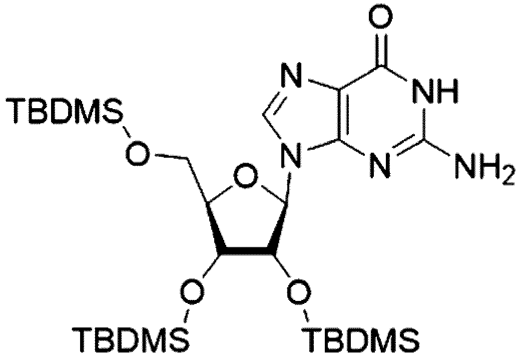

Guanosine (2.00 g, 7.06 mmol, 1.0 equiv.) and tertbutyldimethylsilyl chloride (6.58 g, 43.6 mmol, 6.2 equiv.) were dissolved in dehydrated DMF (25 mL), and the solution was stirred at 0°C for 10 minutes. Imidazole (3.75 g, 55.1 mmol, 7.8 equiv.) was added thereto, and the mixture was stirred at room temperature for 3 hours. Ethyl acetate (200 mL) was added thereto, the mixture was washed twice with water (50 mL), and the organic layer was collected and concentrated. The residue was purified by column chromatography (neutral flash silica, hexane/ethyl acetate = 2/1 → 1/2), affording 3.29 g (5.26 mmol, yield 74%) of Compound 3 as a white solid.

¹H-NMR (400 MHz, DMSO-d₆) δ10.61 (s, 1H), 7.89 (s, 1H), 6.45 (s, 2H), 5.74 (d, J = 6.8 Hz, 1H), 4.59 (dd, J = 4.8 Hz, 2.4 Hz, 1H), 4.17 (dd, J = 1.2 Hz, 3.2 Hz, 1H), 3.95 (t, J = 3.6 Hz, 1H), 3.85 (dd, J = 1.6 Hz, 2.0 Hz, 1H), 3.71 (dd, J = 3.6 Hz, 8.0 Hz, 1H), 0.87 (s, 18H), 0.73 (s, 9H), 0.09-0.11 (m, 12H), -0.08 (s, 3H), -0.28 (s, 3H) ppm.

The other spectral data agreed well with literature values (Ogilvie K. K., Schifman A. L., Penney C. L., Can. J. Chem., 1979, 57(17), 2230-2238).

### (d) Synthesis of Compound 4

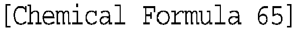

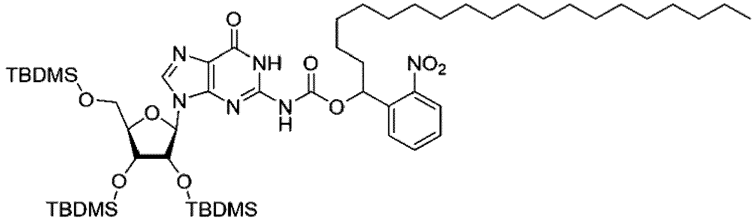

Nitrobenzyl alcohol derivative 2 (1.25 g, 2.98 mmol, 1.0 equiv.) and 1,1'-carbonyldiimidazole (966 mg, 5.96 mmol, 2.0 equiv.) were dissolved in dehydrated dichloromethane (12 mL), and the mixture was stirred at room temperature for 2 hours. The mixture was subjected once to liquid-liquid separation with saturated saline, and the organic layer was collected and concentrated. In the same flask, Compound 3 (2.23 g, 3.58 mmol, 1.2 equiv.) and 18-crown-6 (1.18 g, 4.47 mmol, 1.5 equiv.) were added and dissolved in dehydrated THF (15 mL). Potassium hydride (35% dispersion in mineral oil, 179 mg, 4.47 mmol, 1.5 equiv.) was gradually added thereto at 0°C, and the mixture was stirred at 40°C for 2 hours. After the solvent was distilled off, ethyl acetate was added, and the mixture was washed with saturated saline solution : saturated ammonium chloride aqueous solution : water (2:2:1). The organic layer was collected and concentrated, and then purified by column chromatography (neutral flash silica, ethyl acetate/hexane = 1/5 → 1/2), affording 2.24 g (2.09 mmol, yield 70%) of Compound 4 as a white solid.

¹H-NMR (400 MHz, CDCl₃) δ11.14 (s, 1H), 8.13 (s, 1H), 8.00 (dt, J = 8.4 Hz, 1.6 Hz, 1H), 7.65 (t, J = 7.6 Hz, 1H), 7.58 (d, J = 8.0 Hz, 1H), 7.49 (t, J = 8.8 Hz, 1H), 6.32 (m, 1H), 5.86 (dd, J = 4.8 Hz, 1.2 Hz, 1H), 4.41 (q, J = 4.4 Hz, 1H), 4.24 (t, J = 3.2 Hz, 1H), 4.09 (d, J = 1.6 Hz, 1H), 3.91 (dt, J = 11.2 Hz, 2.8 Hz, 1H), 3.77 (dd, J = 10.0 Hz, 1.6 Hz, 1H), 2.04-1.96 (m, 1H), 1.29-1.25 (m, 36H), 0.94 (s, 18H), 0.87 (t, J = 6.8 Hz, 3H), 0.80 (d, J = 7.2 Hz, 9H), 0.15-0.11 (m, 12H), -0.04 (d, J = 4.0 Hz, 3H), -0.25 (d, J = 10.8 Hz, 3H) ppm.

¹³C-NMR (100 MHz, CDCl₃) δ154.4, 148.1, 137.0, 135.8, 133.5, 129.0, 127.2, 124.8, 87.5, 86.2, 75.1, 72.5, 62.9, 36.1, 32.0, 29.7-29.4, 26.1-25.7, 22.7, 18.6, 18.1, 14.1 ppm.

ESI MS-HR calcd. for C₅₅H₉₈N₆NaO₉Si₃⁺ (M+Na)⁺ 1093.6601, found 1093.6626.

### (e) Synthesis of Compound 5

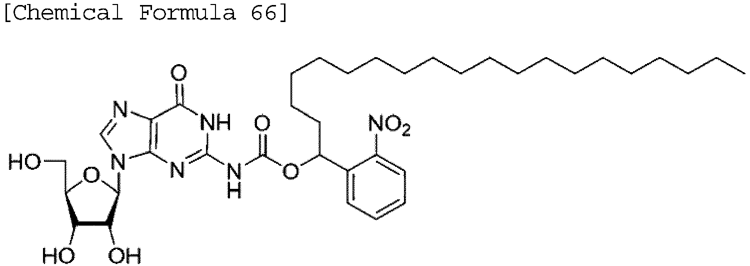

Compound 4 (2.20 g, 2.05 mmol, 1.0 equiv.) was dissolved in dehydrated THF (20 mL). Triethylamine hydrogen trifluoride (2.18 mL, 13.4 mmol, 6.5 equiv.) was added dropwise at 0°C, and the mixture was stirred at room temperature for 2 days. Ethyl acetate (50 mL) was added, and the mixture was washed once with saturated saline (25 mL), and the organic layer was collected and concentrated. The residue was purified by column chromatography (neutral flash silica, methanol/chloroform = 1/15 → 1/8), affording 1.46 g (2.00 mmol, yield 98%) of Compound 5 as a white solid.

¹H-NMR (400 MHz, DMSO-d₆) δ11.56 (s, 1H), 11.10 (s, 1H), 8.18 (s, 1H), 8.00 (d, J = 7.6 Hz, 1H), 7.79 (t, J = 7.2 Hz, 1H), 7.68 (d, J = 7.6 Hz, 1H), 7.57 (t, J = 7.2 Hz, 1H), 6.08 (t, J = 6.0 Hz, 1H), 5.72 (d, J = 5.6 Hz, 1H), 5.42 (br, 1H), 5.14 (br, 1H), 4.96 (br, 1H), 4.40 (q, J = 5.2 Hz, 1H), 4.09 (t, J = 4.0 Hz, 1H), 3.85 (d, J = 4.0 Hz, 1H), 3.54 (dd, J = 22.8 Hz, 7.6 Hz, 2H), 1.22-1.13 (m, 36H), 0.84-0.77 (m, 3H) ppm.

¹³C-NMR (100 MHz, CDCl₃) δ154.4, 148.1, 138.2, 136.0, 133.6, 128.9, 127.2, 124.2, 88.4, 85.5, 75.1, 75.0, 73.7, 70.4, 61.2, 35.7, 31.7, 29.4-29.2, 28.7, 25.4, 22.4, 13.1 ppm.

ESI MS-HR calcd. for C₃₇H₅₆N₆NaO₉⁺ (M+Na)⁺ 751.4001, found 751.4091.

### (f) Synthesis of Compound 6

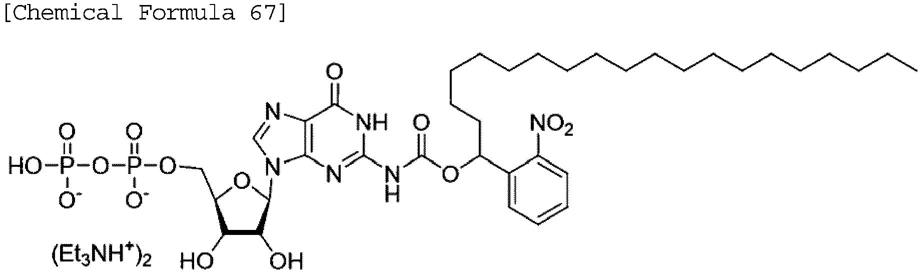

Compound 5 (100 mg, 0.137 mmol, 1.0 equiv.) was dissolved in trimethyl phosphate (2.5 mL). Phosphoryl chloride (23.0 µL, 0.237 mmol, 1.8 equiv.) was added dropwise thereto at 0°C, and the mixture was stirred at -5°C overnight. A mixed solution of a 0.35 m tributylammonium phosphate solution in DMF (2.34 mL, 0.824 mmol, 6.0 equiv.) and tributylamine (294 µL, 1.24 mmol, 9.0 equiv.) was added dropwise, and the mixture was stirred at -10°C for 6 hours. After the reaction, a 0.2 M triethylammonium carbonate buffer (5 mL, pH 7.9) was added, and the mixture was stirred at room temperature for 30 minutes. The mixture was purified by reverse-phase HPLC (YMC Triart C8 (250 × 10 mm l.D., S-5 µm, 12 nm, TO12S05-2510WT), solvent: A) 0.1 M triethylammonium carbonate buffer (pH 7.97), B) acetonitrile, gradient: 40 to 100% B (0 to 35 min), column temperature: room temperature, flow rate: 3 mL/min, detection wavelength: 260 nm), affording 31.1 mg (35.0 µmol, yield 26%, triethylammonium salt) of Compound 6 as a white solid.

¹H-NMR (600 MHz, DMSO-d₆) δ8.11 (d, J = 4.8 Hz, 1H), 8.01 (d, J = 7.8 Hz, 1H), 7.81 (q, J = 7.2 Hz, 1H), 7.57 (q, J = 7.2 Hz, 1H), 6.13 (td, J = 9.0 Hz, 4.2 Hz, 1H), 5.71 (d, J = 6.0 Hz, 1H), 4.77 (dt, J = 34.2 Hz, 6.0 Hz, 1H), 4.32 (dt, J = 13.8 Hz, 3.0 Hz, 1H), 3.97 (t, J = 3.6 Hz, 1H), 3.88 (m, 1H), 1.97-1.80 (m, 2H), 1.26-1.13 (m, 36H), 0.84 (t, J = 6.6 Hz, 3H) ppm.

³¹P-NMR (241 MHz, DMSO-d₆) : δ-9.63 (d), -9.98 (d) ppm.

ESIMS-HR calcd. for C₃₇H₅₇N₆O₁₅P₂⁻ (M-H)- 887.3363, found 887.3563.

### (g) Synthesis of Compound 7

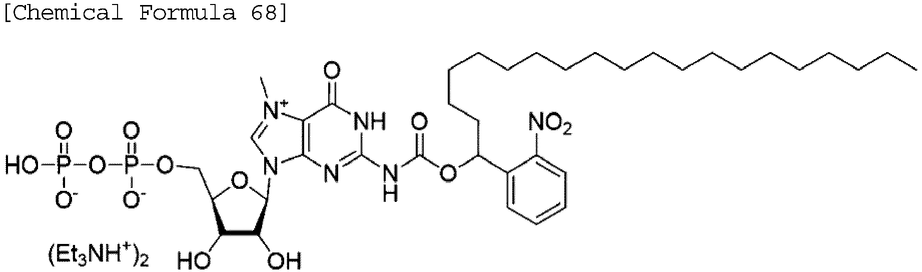

Diphosphate 6 (18.6 mg, 21.0 µmol, 1.0 equiv., triethylammonium salt) was dissolved in dehydrated DMSO (270 µL). Dimethyl sulfate (39.7 µL, 0.419 mmol, 20 equiv.) was added dropwise thereto, and the mixture was stirred at room temperature overnight. After the reaction, a 2 M triethylammonium carbonate buffer (2.0 mL, pH 7.97) was added, and the mixture was stirred at room temperature for 15 minutes. Water was added to the reaction solution, and the mixture was purified by reverse-phase HPLC (YMC Triart C8 (250 × 10 mm l.D., S-5 µm, 12 nm, TO12S05-2510WT), solvent: A) 0.1 M triethylammonium carbonate buffer (pH 7.97), B) acetonitrile, gradient: 40 to 100% B (0 to 35 min), 100% B (35 to 40 min), column temperature: room temperature, flow rate: 3 mL/min, detection wavelength: 260 nm), affording 9.99 mg (11.0 µmol, yield 53%, triethylammonium salt) of a compound 21 as a white solid.

¹H-NMR (600 MHz, DMSO-d₆) δ9.67 (s, 1H), 7.82-7.78 (m, 2H), 7.76 (t, J = 6.6 Hz, 1H), 6.01 (br, 1H), 5.87 (d, J = 3.6 Hz, 1H), 4.24 (s, 1H), 4.27 (q, J = 4.8 Hz, 1H), 4.08 (s, 1H), 4.06 (s, 3H), 4.02 (s, 1H), 1.83-1.82 (m, 2H), 1.22-1.13 (m, 36H), 0.85 (t, J = 7.2 Hz, 3H).

³¹P-NMR (241 MHz, DMSO-d₆) δ-9.90 (d), -10.61 (d) ppm.

ESI MS-HR calcd. for C₃₈H₅₈N₆O₁₅P₂⁻ (M-2H)- 901.3519, found 901.3562.

### (h) Synthesis of Compound 8

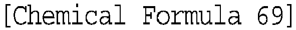

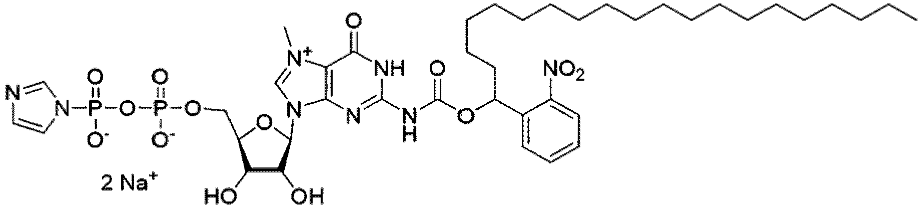

Compound 7 (11.0 mg, 11.1 µmol, 1.0 equiv., triethylammonium salt), imidazole (9.97 mg, 111 µmol, 10 equiv.) and 2,2'-dithiodipyridine (9.47 mg, 38.8 µmol, 3.5 equiv.) were dissolved in dehydrated DMSO (400 µL). Triethylamine (5.99 µL, 38.8 µmol, 3.5 equiv.) and triphenylphosphine (11.28 mg, 38.8 µmol, 3.5 equiv.) were added thereto, and the mixture was stirred at room temperature for 4 hours. After the reaction, sodium perchlorate (5.51 mg, 44.4 µmol, 4.0 equiv.) dissolved in dehydrated acetone (1.5 mL) was added, and the mixture was cooled at 0°C for 15 minutes. The precipitate formed was collected by centrifugation and washed five times with dehydrated acetone (2.0 mL). The precipitate was dried up by freeze-drying, affording 9.90 mg (10.1 µmol, 85% yield, Na salt) of Compound 8 as a white solid.

¹H-NMR (600 MHz, DMSO-d₆) δ9.66 (s, 1H), 7.98 (d, J = 5.6 Hz, 1H), 7.79 (d, J = 4.4 Hz, 1H), 7.71 (s, 1H), 7.54 (t, J = 4.4 Hz, 1H), 7.18 (s, 1H), 6.79 (s, 1H), 6.00 (br, 1H), 5.87 (d, J = 2.4 Hz, 1H), 5.61 (m, 1H), 4.41 (s, 1H), 4.20 (q, J = 2.0 Hz, 1H), 4.05 (s, 3H), 1.81 (q, J = 4.4 Hz, 2H), 1.27-1.14 (m, 36H), 0.85 (t, J = 4.8 Hz, 3H) ppm.

¹³C-NMR (101 MHz, CDCl₃) δ154.8, 153.7, 148.0, 147.9, 138.6, 136.0, 133.7, 128.8, 127.2, 124.5, 74.6, 70.5, 61.5, 36.1, 31.8, 29.8-29.0, 25.7, 22.5, 13.9 ppm.

³¹P-NMR (241 MHz, DMSO-d₆) δ-9.90 (d, J = 16.6 Hz), -10.61 (d, J = 19.8) ppm.

ESI MS-HR calcd. for C₄₁H₆₁N₈O₁₄P₂⁻ (M-2H)- 951.3788, found 951.4272.

### (2) Chemical capping reaction of RNA using hydrophobic chemical capping reagent

Fig. 32 shows a chemical capping reaction of an RNA using a hydrophobic chemical capping reagent.

(a) of the figure shows a conceptual diagram of the reaction.

(b) of the figure shows the result of reverse-phase HPLC analysis of the reaction of a 5'-monophosphorylated 19-base-long RNA with a hydrophobic chemical capping reagent.

(c) of the figure shows the result of denatured PAGE analysis.

### Experimental section

A hydrophobic capping reagent (Compound 8) and a 19-base-long 5'-end phosphorylated RNA (5'-p-GAACGUGCGAAAGUCCACA-3': SEQ ID NO: 15) were reacted as follows. 38 µL of an aqueous solution containing 2 nmol of the RNA and 500 nmol of calcium chloride was frozen and dried up by a freeze-drying method. 18.5 µL of dimethyl sulfoxide (DMSO), 26.5 µL of a hydrophobic capping reagent solution in DMSO (19 mM), and 5 µL of 1-methylimidazole were added thereto and mixed, and the mixture was heated at 55°C for 5 hours. The final concentration of each component in the DMSO solution is as follows. 40 µM RNA, 10 mM hydrophobic capped compound, 10 mM calcium chloride, 1.25 M 1-methylimidazole. After completion of the reaction, the RNA was collected by alcohol precipitation, and analyzed by reverse-phase HPLC and denaturing polyacrylamide gel electrophoresis (PAGE). The HPLC analysis conditions shown in (b) of the figure are as follows. System used: Chromaster (Hitachi High-Tech Corporation); column: YMC Hydrosphere C18 (250 × 4.6 mm I.D., YMC); eluent A: 50 mM triethylammonium acetate containing 5% acetonitrile, pH 7.0; eluent B: acetonitrile; gradient condition: 0 to 100% B (0 to 20 min); flow rate: 1.0 mL/min; column temperature: 50°C; detection wavelength: 260 nm. In (c) of the figure, an RNA was migrated with 15% acrylamide (acrylamide:bis = 19:1) containing 7.5 M urea as a denaturant, the gel was stained with a nucleic acid staining reagent SYBR Green II, and photographed with ChemiDoc XRS + imager (Bio-Rad).

### 7. Improved synthesis of N⁷-methyl-GDP and synthesis of chemical capping reagent with hydrophobic tag

The following shows an improved method of synthesizing 2'-O-modified-N''-methyl-GDP (Compound 4) and a synthetic scheme of chemical capping reagent (5).

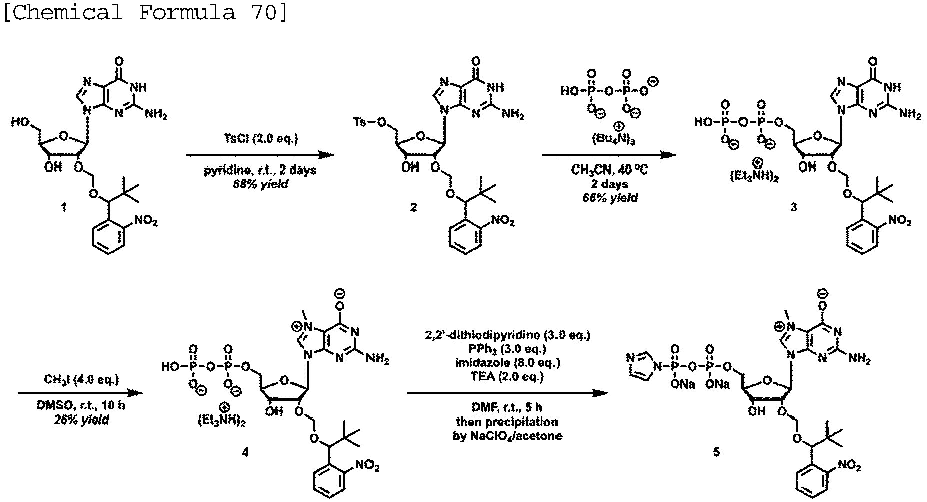

### (1) Synthesis of 2'-O-[2,2-dimethyl-(R/S)-1-(2-nitrophenyl)propyloxy]methyl-5'-O-p-toluenesulfonyl-guanosine (Compound 2)

To a solution of 2'-modified guanosine (1) (294 mg, 0.583 mmol) in pyridine (6.00 mL) was added dropwise a solution of p-toluenesulfonyl chloride (214 mg, 1.12 mmol) in pyridine (1.40 mL) in an ice bath. The mixture was stirred at room temperature for 3 to 4 days. Ice-cooled water was added to the reaction mixture to stop the reaction, and the mixture was extracted three times with dichloromethane. The organic layer was dried over Na2SO4 and distilled off. The residue was subjected to silica gel column chromatography and was eluted with 24 to 91% methanol/dichloromethane, affording Compound 2 (260 mg, 68% yield) as a yellow foam.

¹H NMR (600 MHz, CDCl₃, mixture of stereoisomers) δ11.92 (br, s), 7.76-7.63 (m, 4H), 7.57 (d, J = 11.9 Hz, 1.5H), 7.46-7.35 (m, 2H), 7.27-7.23 (m, 2.5H), 6.03-5.76 (m, 1H), 5.68 (s, 1H), 5.26-5.02 (m, 2H), 4.90 (d, J = 16.1 Hz, 2H), 4.76 (s, 2H), 4.47-4.30 (m, 1.5H), 4.21 (s, 2.5H), 2.37 (s, 3H), 0.77 (s, 4.5H), 0.74 (s, 4.5H) ppm.

¹³C NMR (151 MHz, CDCl₃, mixture of stereoisomers) δ158.75, 154.18, 151.28, 151.01, 150.38, 145.30, 136.63, 133.76, 132.27, 131.92, 130.36, 130.10, 130.06, 129.95, 128.51, 128.02, 123.87, 117.14, 94.78, 81.80, 79.86, 78.56, 77.91, 70.70, 70.13, 69.94, 53.56, 36.68, 36.50, 29.78, 25.73, 25.67, 25.56, 21.72 ppm.

ESI-MS calcd. for C₂₉H₃₃N₆O₁₀S, 657.2 [M-H]-; found 657.4.

### (2) Synthesis of 2'-O-[2,2-dimethyl-(R/S)-1-(2-nitrophenyl)propyloxy]methylguanosine-5'-triethylammonium diphosphate salt (Compound 3)

To a suspension of 5'-O-tosyl-2'-modified guanosine (2) (458 mg, 0.695 mmol) and powdered 3A molecular sieve (500 mg) in CH₃CN (500 µL) was added 0.475 M tris-tetrabutylammonium pyrophosphate. After stirring in CH₃CN (2.63 mL, 1.25 mmol) at 40°C for 2 days, the reaction mixture was diluted with water and filtered through a membrane filter, whereby insolubles were removed. The filtrate was purified by ion exchange chromatography on QAE-Sephadex (ϕ = 3 cm, h = 10 cm, 70 cm³, 6 mL/min) in a linear 0 to 1.5 M gradient of triethylammonium carbonate buffer (pH 7.9) containing 0 to 10% acetonitrile. Guanosine-5'-diphosphate derivative (3) was obtained in the form of a triethylammonium salt (441 mg, 66% yield) as a yellow amorphous solid.

¹H NMR (600 MHz, D₂O, mixture of stereoisomers) δ7.87 (s, 0.5H), 7.58 (s, 0.5H), 7.52 (d, J = 8.1 Hz, 0.5H), 7.47-7.38 (m, 1H), 7.38-7.29 (m, 1H), 7.28-7.17 (m, 1H), 7.01 (t, J = 7.8 Hz, 0.5H), 5.73 (d, J = 5.8 Hz, 0.5H), 5.48 (d, J = 5.0 Hz, 0.5H), 4.96, 4.94 (2s, 1H), 4.82 (d, J = 7.5 Hz, 0.5H), 4.76 (s, 0.5H), 4.71 (d, J = 7.3 Hz, 0.5H), 4.62 (dd, J = 18.0, 6.6 Hz, 1.5H), 4.52 (t, J = 5.1 Hz, 0-.5H), 4.45 (t, J = 5.0 Hz, 1H), 4.14 (d, J = 4.1 Hz, 0.5H), 4.10-4.03 (m, 1.5H), 3.99 (d, J = 4.8 Hz, 1H), 3.02 (q, J = 7.3 Hz, 15H), 1.10 (t, J = 7.3 Hz, 24H), 0.52 (s, 4.5H), 0.41 (s, 4.5H) ppm.

¹³C NMR (151 MHz, D₂O, mixture of stereoisomers) δ158.95, 158.75, 153.89, 153.59, 151.59, 151.01, 149.74, 148.09, 138.02, 137.37, 133.49, 132.57, 132.47, 132.21, 129.86, 129.66, 128.65, 128.06, 123.80, 123.30, 116.33, 115.96, 95.44, 93.71, 86.20, 84.26, 84.20, 83.73, 83.67, 81.61, 80.72, 78.55, 76.97, 69.07, 68.94, 64.84, 64.81, 64.46, 46.63, 36.55, 35.65, 24.78, 24.68, 8.24 ppm.

³¹P NMR (243 MHz, D₂O, mixture of stereoisomers) δ-9.09 (d, J = 20.8 Hz, 1P), -10.29 (d, J = 22.4 Hz, 1P) ppm.

ESI-TOF-MS calcd. for C₂₂H₂₉N₆O₁₄P₂, 663.1223 [M-H]-; found 663.1378.

### (3) N⁷-Methyl-2'-O-[2,2-dimethyl-(R/S)-1-(2-nitrophenyl)propyloxy]methylguanosine-5'-triethylammonium diphosphate salt (Compound 4)

To a solution of guanosine-5'-diphosphate derivative (3) (441 mg, 0.509 mmol) in DMSO (4.24 mL) was added iodomethane (290 mg, 127 µL, 2.04 mmol) . After stirring at room temperature for 10 hours, the reaction mixture was diluted with water and washed four times with dichloromethane. The aqueous layer was purified with a linear gradient of DEAE-Sephadex^{™} A-25 (ϕ = 3 cm, h = 10 cm, 70 cm³, 6 mL/min) in a 0 to 1.0 M triethylammonium acetate buffer (pH 6.0) containing 0 to 10% CH₃CN. After removal of CH₃CN under reduced pressure, the residue was desalted with Wakosil^{™} 25C18 (15 to 30 µm, spherical) and freeze-dried, affording N7-methyl-guanosine-5'-diphosphate derivative (4) (118 mg, 26% yield, a mixture of two stereoisomers) as a white solid.

¹H NMR (600 MHz, D₂O, single stereoisomer isolated by HPLC) δ8.93 (s, 1H), 7.60 (d, J = 8.2 Hz, 1H), 7.49 (dd, J = 15.2, 8.1 Hz, 2H), 7.29 (t, J = 7.6 Hz, 1H), 5.82 (s, 1H), 5.00 (d, J = 4.1 Hz, 1H), 4.93 (d, J = 8.0 Hz, 1H), 4.86 (d, J = 7.2 Hz, 1H), 4.54-4.50 (m, 1H), 4.42 (t, J = 5.2 Hz, 1H), 4.25 (s, 1H), 4.10 (d, J = 11.2 Hz, 1H), 3.94 (d, J = 3.7 Hz, 3H), 3.05 (t, J = 7.3 Hz, 4H), 1.13 (t, J = 7.1 Hz, 6H), 0.61 (s, 10H) ppm.

¹³C NMR (151 MHz, D₂O, single stereoisomer isolated by HPLC) δ155.41, 154.99, 149.37, 148.61, 136.68, 133.90, 132.44, 130.24, 128.15, 123.51, 107.91, 95.16, 87.76, 85.95, 82.16, 79.32, 68.89, 64.78, 46.70, 36.52, 36.07, 24.76, 8.25 ppm.

³¹P NMR (243 MHz, D₂O, single stereoisomer isolated by HPLC) δ-10.03 (1P), -10.71 (1P) ppm.

ESI-TOF-MS calcd. for C₂₃H₃₁N₆O₁₄P₂, 677.1379 [M-H]-; found 677.1374.

### (4) N⁷-Methyl-2'-O-[2,2-dimethyl-(R/S)-1-(2-nitrophenyl)propyloxy]methylguanosine-5'-diphosphorimidazolide disodium salt (Compound 5)

To a solution of N7-methyl-GDP derivative (4) (118 mg, 0.134 mmol), imidazole (72.8 mg, 1.07 mmol), and 2,2'-dithiodipyridine (88.6 mg, 0.402 mmol) in N,N-dimethylformamide (1.60 mL) was added triethylamine (37.5 µL, 27.1 mg, 0.268 mmol), and subsequently triphenylphosphine (105 mg, 0.402 mmol) was added. After stirring at room temperature for 5 hours, the reaction mixture was added dropwise to a solution of sodium perchlorate (39.4 mg, 0.322 mmol) in acetone (15 mL) with stirring. The resulting suspension was transferred to a centrifuge tube and centrifuged at 3,500 rpm for 10 minutes. The supernatant was removed and the precipitate was washed five times with acetone. The solid was dried under reduced pressure on P2O5 in a desiccator, affording guanosine 5'-diphosphate derivative imidazolide disodium salt (5) as a white solid.

¹H NMR (600 MHz, D₂O, mixture of stereoisomers) δ8.10 (s, 1H), 7.82 (d, J = 8.0 Hz, 1H), 7.68-7.01 (m, 6H), 6.88 (d, J = 10.1 Hz, 1H), 5.98-5.63 (m, 1H), 5.03-4.71 (m, 3H), 4.52-4.08 (m, 3H), 3.95 (s, 1H), 3.93-3.80 (m, 3H), 0.70-0.19 (m, 9H) ppm.

¹³C NMR (151 MHz, D₂O, mixtureofstereoisomers) δ159.27, 149.76, 149.32, 148.44, 139.84, 134.64, 133.79, 132.74, 132.18, 130.00, 128.79, 128.33, 127.97, 123.79, 123.52, 120.43, 120.25, 108.52, 108.26, 95.13, 87.30, 85.53, 85.01, 81.91, 80.60, 79.17, 68.99, 68.59, 65.00, 36.45, 36.41, 35.88, 35.72, 30.25, 24.85, 24.75 ppm.

³¹P NMR (243 MHz, D₂O, mixture of stereoisomers) δ-11.18 (d, J = 19.7 Hz, 1P), -19.39 (d, J = 19.7 Hz, 1P) ppm.

ESI-MS calcd. for C₂₆H₃₃N₈O₁₃P₂, 727.2 [M-H]-; found 727.3.

### 8. Synthesis of novel cap compound

The following shows a synthetic scheme of a novel cap compound (Compound 10).

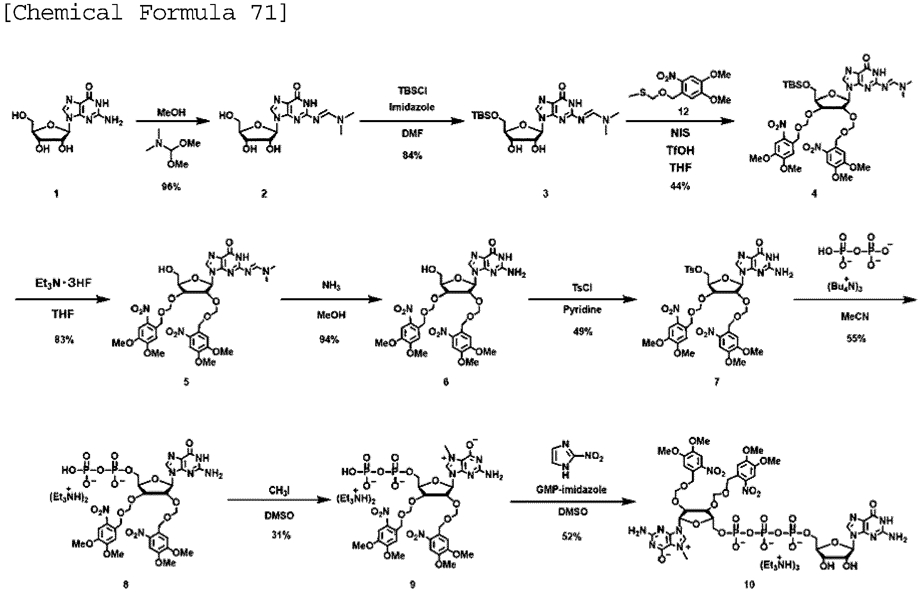

### (1) Synthesis of Compound 2

Methanol (80.0 mL) was added to guanosine (Compound 1) (6.00 g, 21.2 mmol) to suspend the guanosine, and then N,N-dimethylformamide dimethyl acetal (18.9 mL, 159 mmol) was added thereto. After stirring at room temperature for 22 hours, the reaction solution was filtered and the solid formed was washed with ice-cooled methanol (80.0 mL). The solid collected by filtration was dried with a freeze-dryer, affording Compound 2 as a white solid. (6.81 g, 20.1 mmol, yield 95%)
¹H NMR (400 MHz, DMSO-d₆) δ11.35 (s, 1H), 8.53 (s, 1H), 8.04 (s, 1H), 5.79 (d, J = 6.1 Hz, 1H), 5.43 (s, 1H), 5.19 (s, 1H), 5.04 (t, J = 5.4 Hz, 1H), 4.48 (t, J = 5.4 Hz, 1H), 4.11 (m, 1H), 3.90 (q, J = 3.7 Hz, 1H), 3.58 (m, 2H)3.15 (s, 3H), 3.03 (s, 3H) ppm
¹³C NMR (101 MHz, DMSO-d₆) δ157.96, 157.63, 157.32, 150.02, 136.97, 119.79, 86.72, 85.45, 73.80, 70.51, 61.52, 40.68, 34.67 ppm
HRMS (ESI) m/z calcd. for C₁₃H₁₈N₆NaO₅⁺ (M+Na)⁺ = 361.1231, found 361.1221

### (2) Synthesis of Compound 3

Dimethylformamide (90.0 mL) was added to Compound 2 (3.00 g, 8.87 mmol) and the compound is dissolved, and then imidazole (1.40 g, 21.3 mmol) and tert-butylmethylchlorosilane (1.60 g, 10.6 mmol) were added thereto. After stirring at room temperature for 20 hours, the reaction solution was diluted with chloroform and washed with water. The organic layer was dried over anhydrous sodium sulfate and then concentrated. The residue was purified by silica gel column chromatography (eluent: chloroform/methanol = 10/1), affording Compound 3 as a white solid. (3.38 g, 7.47 mmol, yield 84%)
¹H NMR (400 MHz, DMSO-d₆) δ11.33 (s, 1H), 8.55 (s, 1H), 7.96 (s, 1H), 5.82 (d, J = 5.2 Hz, 1H), 5.51 (d, J = 3.6 Hz, 1H), 5.22 (d, J = 3.4 Hz, 1H), 4.42 (d, J = 3.6 Hz, 1H), 4.12 (s, 1H), 3.91 (d, J = 3.9 Hz, 1H), 3.77 (m, 2H), 3.16 (s, 3H), 3.03 (s, 3H), 0.87 (s, 9H), 0.04 (s, 6H) ppm
¹³C NMR (101 MHz, DMSO-d₆) δ157.91, 157.62, 157.31, 150.07, 136.28, 119.59, 86.45, 84.51, 73.94, 70.07, 63.09, 40.70, 34.71, 25.85, 18.08, -5.40, -5.43 ppm
HRMS (ESI) m/z calcd. for C₁₉H₃₂N₆NaO₅Si⁺ (M+Na)⁺ = 475.2096, found 475.2097

### (3) Synthesis of Compound 4

Tetrahydrofuran (45 mL) was added to Compound 3 (2.00 g, 4.35 mmol) and Compound 12 (2.62 g, 9.57 mmol), and these compounds were dissolved. Thereafter, powdered molecular sieve 3 Å (2.10 g) and N-iodosuccinimide (2.15 g, 9.57 mmol) were added, and the mixture was cooled to -40°C. To this reaction solution was added trifluoromethanesulfonic acid (0.85 mL, 9.57 mmol), and the mixture was stirred at -40°C for 2 hours. Thereafter, triethylamine (2.89 mL, 15.2 mmol) was added to stop the reaction. The resulting reaction solution was filtered with Celite to remove the powdery molecular sieve 3Å. The filtrate was diluted with ethyl acetate and washed with water. The organic layer was dried over anhydrous sodium sulfate and then concentrated. The residue was purified by silica gel column chromatography (eluent: ethyl acetate/methanol = 100/3 → 20/1), affording Compound 4 as a brown solid. (1.72 g, 1.90 mmol, yield 44%)
¹H NMR (400 MHz, DMSO-d₆) δ11.15 (s, 1H), 8.46 (s, 1H), 7.99 (s, 1H), 7.63 (s, 1H), 7.56 (s, 1H), 7.19 (s, 1H), 6.79 (s, 1H), 6.01 (d, J = 5.7 Hz, 1H), 4.99 (s, 1H), 4.91 (m, 6H), 4.71 (s, 2H), 4.49 (d, J = 4.3 Hz, 1H), 4.11 (t, J = 4.1 Hz, 1H), 3.81 (m, 16H), 3.08 (s, 3H), 2.98 (s, 3H), 0.82 (s, 9H), 0.01 (d, J = 1.6 Hz, 6H) ppm
¹³C NMR (101 MHz, DMSO-d₆) δ157.73, 157.16, 153.37, 153.24, 147.42, 147.20, 139.00, 138.13, 129.03, 128.92, 110.13, 109.93, 108.90, 108.78, 107.92, 107.78, 94.88, 94.41, 94.26, 85.40, 85.20, 83.87, 83.01, 79.06, 74.26, 66.50, 66.33, 66.22, 61.00, 59.75, 55.98, 55.93, 55.78, 40.58, 34.64, 25.81, 25.67, 20.76, 17.89, 14.09, -3.20, -5.61, -5.64 ppm
HRMS (ESI) m/z calcd. for C₃₉H₅₄N₈NaO₁₅Si⁺ (M+Na)⁺ = 925.3370, found 925.3337

### (4) Synthesis of Compound 5

Tetrahydrofuran (10.0 mL) was added to Compound 4 (0.69 g, 0.76 mmol), and the compound was dissolved. Thereafter, triethylamine (0.32 mL, 2.29 mmol) and triethylamine hydrotrifluoride (0.37 mL, 2.29 mmol) were added, and the mixture was stirred at room temperature for 17 hours. The resulting reaction solution was diluted with ethyl acetate and washed with water. The organic layer was dried over anhydrous sodium sulfate and then concentrated. The residue was purified by silica gel column chromatography (eluent: ethyl acetate/methanol = 20/1 → 10/1), affording Compound 5 as a yellow solid. (0.50 g, 0.63 mmol, yield 83%)
¹H NMR (400 MHz, DMSO-d₆) δ11.10 (s, 1H), 8.44 (s, 1H), 8.08 (s, 1H), 7.63 (s, 1H), 7.56 (s, 1H), 7.21 (s, 1H), 6.78 (s, 1H), 5.99 (d, J = 5.9 Hz, 1H), 5.22 (t, J = 5.3 Hz, 1H), 4.98 (d, J = 8.6 Hz, 1H), 4.89 (m, 6H), 4.67 (dd, J1 = 20.3 Hz, J2 = 15.3 Hz, 2H), 4.51 (m, 1H), 4.10 (q, J = 3.5 Hz, 1H), 3.86 (s, 3H), 3.85 (s, 3H), 3.84 (s, 3H), 3.77 (s, 3H), 3.63 (m, 2H), 3.08 (s, 3H), 2.98 (s, 3H) ppm
¹³C NMR (101 MHz, DMSO-d₆) δ157.77, 157.25, 157.14, 153.42, 153.24, 149.61, 147.40, 147.17, 139.05, 138.06, 136.41, 129.05, 128.97, 119.63, 110.12, 108.80, 107.89, 107.79, 94.89, 94.41, 85.29, 83.84, 79.34, 79.19, 74.99, 66.42, 66.22, 61.06, 56.04, 55.99, 55.93, 55.79, 40.53, 34.55 ppm
HRMS (ESI) m/z calcd. for C₃₃H₄₀N₈NaO₁₅⁺ (M+Na)⁺ = 811.2505, found 811.2489

### (5) Synthesis of Compound 6

To Compound 5 (0.50 g, 0.63 mmol) was added dichloromethane (6.00 mL), and the compound was dissolved. To this reaction solution was added a 7 M ammonia solution in methanol (6.30 mL, 44.4 mmol), and the mixture was stirred at 50°C for 15 hours. The resulting reaction solution was diluted with chloroform and washed with water. The organic layer was dried over anhydrous sodium sulfate and concentrated. The residue was purified by silica gel column chromatography (eluent: chloroform/methanol = 20/1 → 10/1), affording Compound 6 as a pale yellow solid. (0.43 g, 0.59 mmol, yield 94%)
¹H NMR (400 MHz, DMSO-d₆) δ10.41 (s, 1H), 7.94 (s, 1H), 7.63 (s, 1H), 7.57 (s, 1H), 7.21 (s, 1H), 6.85 (s, 1H), 6.35 (s, 2H), 5.88 (d, J = 7.2 Hz, 1H), 5.24 (t, J = 5.5 Hz, 1H), 4.96 (m, 4H), 4.88 (d, J = 7.0 Hz, 1H), 4.77 (d, J = 6.7 Hz, 2H), 4.61 (dd, J1 = 35.9 Hz, J2 = 15.3 Hz, 2H), 4.45 (dd, J1 = 4.9 Hz, J2 = 2.0 Hz, 1H), 4.09 (dd, J1 = 5.9 Hz, J2 = 3.7 Hz, 1H), 3.87 (s, 3H), 3.85 (s, 3H), 3.84 (s, 6H), 3.61 (m, 2H) ppm
¹³C NMR (101 MHz, DMSO-d₆) δ156.36, 153.62, 153.38, 153.25, 151.03, 147.38, 147.17, 139.03, 138.16, 135.04, 129.04, 128.97, 116.53, 110.11, 108.99, 107.90, 107.80, 94.65, 94.44, 84.40, 84.13, 79.18, 79.03, 75.30, 66.24, 61.27, 56.06, 55.99, 55.92 ppm
HRMS (ESI) m/z calcd. for C₃₀H₃₅N₇NaO₁₅⁺ (M+Na)⁺ = 756.2083, found 756.2067

### (6) Synthesis of Compound 7

Pyridine (2.00 mL) was added to Compound 6 (0.10 g, 0.14 mmol) and the compound was dissolved therein, and p-toluenesulfonyl chloride (0.04 g, 0.21 mmol) was added thereto under ice-cooling. After stirring at room temperature for 3 days, the mixture was diluted with chloroform and washed with water. The organic layer was dried over anhydrous sodium sulfate and concentrated. The residue was purified by silica gel column chromatography (eluent: chloroform/methanol = 100/3), affording Compound 7 as a yellow solid. (61.2 mg, 68.9 µmol, yield 49%)
¹H NMR (400 MHz, DMSO-d₆) δ10.48 (s, 1H), 7.73 (s, 1H), 7.70 (s, 1H), 7.68 (s, 1H), 7.65 (s, 1H), 7.58 (s, 1H), 7.35 (s, 1H), 7.33 (s, 1H), 7.19 (s, 1H), 6.86 (s, 1H), 6.36 (s, 2H), 5.84 (d, J = 6.3 Hz, 1H), 4.92 (m, 4H), 4.86 (d, J = 7.0 Hz, 1H), 4.79 (m, 2H), 4.64 (dd, J1 = 33.8 Hz, J2 = 15.1 Hz, 2H), 4.47 (m, 1H), 4.31 (m, 2H), 4.23 (m, 1H), 3.87 (s, 3H), 3.86 (s, 3H), 3.84 (s, 3H), 3.82 (s, 3H), 2.35 (s, 3H) ppm
¹³C NMR (101 MHz, DMSO-d₆) δ156.40, 153.61, 153.33, 153.26, 150.95, 147.42, 147.21, 145.11, 139.01, 138.28, 134.95, 131.91, 129.98, 128.89, 128.82, 127.49, 116.61, 110.05, 109.14, 107.87, 94.81, 84.92, 80.08, 79.18, 78.01, 74.86, 69.77, 66.43, 56.03, 55.91, 21.06 ppm
HRMS (ESI) m/z calcd. for C₃₇H₄₁N₇NaO₁₇S⁺ (M+Na)⁺ = 910.2172, found 910.2181

### (7) Synthesis of Compound 8

To Compound 7 (50.0 mg, 56.3 µmol) was added acetonitrile (0.13 mL), and the compound was dissolved therein. Powdered molecular sieve 3 Å (50.0 mg) was added thereto, then a solution of bistetraammonium pyrophosphate in acetonitrile (0.475 M, 0.21 mL) was added, and the mixture was stirred at room temperature for 21 hours. After dilution with water, the precipitate was removed by centrifugation (3500 rpm, 15 min) and the supernatant was purified by ion exchange chromatography using QAE-Sephadex (eluent: 0 to 1.5 M triethylammonium carbonate buffer). Thereafter, the purified product was dried with a freeze dryer, affording Compound 8, which was a triethylammonium salt, as a yellow solid. (34.1 mg, 31.1 µmol, yield 55%)
¹H NMR (600 MHz, D₂O) δ8.01 (s, 1H), 7.44 (s, 1H), 7.42 (s, 1H), 7.01 (s, 1H), 6.53 (s, 1H), 5.82 (d, J = 6.3 Hz, 1H), 5.04 (d, J = 6.5 Hz, 1H), 4.92 (m, 6H), 4.66 (s, 1H), 4.53 (d, J = 15.9 Hz, 1H), 4.43 (s, 1H), 4.33 (d, J = 15.0 Hz, 1H), 4.18 (s, 2H), 3.85 (s, 3H), 3.83 (s, 3H), 3.78 (s, 3H), 3.75 (s, 3H), 3.46 (d, J = 6.5 Hz, 2H), 3.16 (q, J = 7.0 Hz, 10H), 1.24 (t, J = 7.2 Hz, 18H) ppm
¹³C NMR (151 MHz, D₂O) δ157.70, 153.27, 153.13, 151.10, 146.90, 146.50, 138.65, 137.00, 129.75, 129.20, 115.51, 109.94, 107.59, 107.29, 95.05, 94.94, 85.57, 82.89, 79.89, 76.06, 66.95, 66.38, 65.20, 56.14, 56.03, 55.87, 46.62, 8.22 ppm
³¹P NMR (243 MHz, D₂O) δ-10.12 (d, J = 21.2 Hz, 1P), -10.86 (d, J = 21.2 Hz, 1P) ppm
HRMS (ESI) m/z calcd. for C₃₀H₃₆N₇O₂₁P₂⁺ (M-H)⁻ = 892.1445, found 892.1431

### (8) Synthesis of Compound 9

Dimethyl sulfoxide (0.76 mL) was added to Compound 8 (0.10 g, 91.2 µmol), and the compound was dissolved therein. Thereafter, iodomethane (46.0 µL, 0.73 mmol) was added, and the mixture was stirred at room temperature for 2 days. The reaction was stopped by adding a triethylammonium acetate buffer (pH 7.0) to the reaction solution, and the reaction solution was diluted with water. Thereafter, the mixture was washed with chloroform, and the aqueous layer was purified by ion exchange chromatography (eluent: 0 to 1.0 M triethylammonium acetate buffer) using QAE-Sephadex. Thereafter, the purified product was dried with a freeze dryer, affording Compound 9, which was a triethylammonium salt, as a pale yellow solid. (31.8 mg, 28.6 µmol, yield 31%)
¹H NMR (600 MHz, D₂O) δ7.28 (s, 1H), 7.20 (s, 1H), 6.80 (s, 1H), 6.68 (s, 1H), 6.01 (s, 1H), 4.95 (d, J = 24.1 Hz, 4H), 4.74 (s, 1H), 4.65 (d, J = 16.0 Hz, 1H), 4.56 (m, 4H), 4.47 (s, 1H), 4.26 (dd, J1 = 69.2 Hz, J2 = 8.0 Hz, 2H), 3.95 (s, 3H), 3.75 (s, 3H), 3.74 (s, 3H), 3.73 (s, 3H), 3.71 (s, 3H), 3.16 (q, J = 7.3 Hz, 10H), 1.24 (t, J = 7.3 Hz, 15H) ppm
¹³C NMR (151 MHz, D₂O) δ155.49, 154.46, 153.08, 152.99, 149.40, 146.77, 146.65, 138.27, 137.51, 129.46, 129.02, 109.63, 107.88, 107.48, 107.30, 95.66, 94.63, 87.40, 83.35, 80.01, 75.17, 66.97, 66.58, 56.13, 56.06, 55.85, 55.78, 46.64, 35.91, 23.18, 8.24 ppm
³¹P NMR (243 MHz, D₂O) δ-9.26 (d, J = 19.7 Hz, 1P), -10.72 (d, J = 26.3 Hz, 1P) ppm
HRMS (ESI) m/z calcd. for C₃₁H₃ₛN₇O₂₁P₂⁻ (M-H)- = 906.1601, found 906.1628

### (9) Synthesis of Compound 10

To Compound 9 (30.0 mg, 27 µmol) and guanosine 5'-phosphorimidazolide (35.0 mg, 81.1 µmol) was added dimethyl sulfoxide (0.27 mL), and those compounds were dissolved. Thereafter, 2-nitroimidazole (9.20 mg, 81.1 µmol) was added to the reaction solution, and the mixture was stirred at 37°C for 2 days. After dilution with water, the mixture was washed with chloroform and the aqueous layer was purified by reverse phase chromatography (Chromatorex C8 MB 100-40/75). (Eluent: 0 to 60% acetonitrile/100 to 40% 50 mM triethylammonium acetate buffer) Thereafter, the purified product was dried with a freeze dryer, affording Compound 10, which was a triethylammonium salt, as a pale yellow solid. (21.5 mg, 13.8 µmol, yield 51%)
¹H NMR (600 MHz, D₂O) δ7.88 (s, 1H), 7.25 (s, 1H), 7.17 (s, 1H), 6.66 (d, J = 8.8 Hz, 1H), 5.92 (s, 1H), 5.62 (d, J = 5.4 Hz, 1H), 5.11 (d, J = 12.9 Hz, 1H), 4.98 (s, 1H), 4.90 (s, 2H), 4.64 (s, 2H), 4.52 (s, 2H), 4.49 (d, J = 5.1 Hz, 1H), 4.47 (d, J = 9.2 Hz, 1H), 4.41 (d, J = 3.7 Hz, 4H), 4.23 (d, J = 10.5 Hz, 4H), 3.97 (s, 3H), 3.72 (s, 3H), 3.70 (s, 3H), 3.69 (s, 3H), 3.68 (s, 3H), 3.16 (q, J = 7.3 Hz, 15H), 1.23 (t, J = 7.5 Hz, 23H) ppm
¹³C NMR (151 MHz, D₂O) δ158.05, 155.10, 153.52, 153.03, 152.86, 150.97, 149.08, 146.63, 146.47, 138.08, 137.40, 136.85, 129.67, 128.87, 115.63, 109.50, 107.77, 107.59, 107.24, 96.03, 94.13, 87.53, 86.80, 83.49, 83.43, 82.50, 79.10, 74.55, 74.09, 70.11, 67.09, 66.75, 65.27, 64.01, 56.03, 55.94, 55.74, 46.65, 36.02, 23.25, 8.24 ppm
³¹P NMR (243 MHz, D₂O) δ-10.87 (d, J = 19.7 Hz, 1P), -11.09 (d, J = 19.7 Hz, 1P), -22.41 (t, J = 19.7 Hz, 1P) ppm
HRMS (ESI) m/z calcd. for C₄₁H₅₀N₁₂O₂₈P₃⁻ (M-H)⁻ = 1251.2081, found 1251.2123

### (10) Synthesis of Compound 12

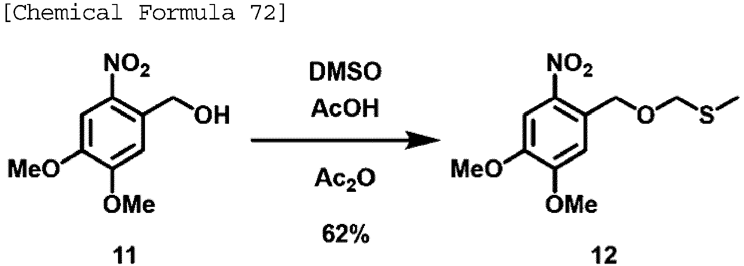

To Compound 11 (1.50 g, 7.04 mmol) were added acetic acid (16.1 mL, 281 mmol) and dimethyl sulfoxide (10.0 mL, 141 mmol), and the compound was dissolved. To the reaction solution was added acetic anhydride (13.3 mL, 141 mmol), and the mixture was stirred at room temperature for 3 days. Then, potassium hydroxide (10.0 M, 63.3 mL, 633 mmol) was added thereto at 0°C, and the mixture was stirred for 2 hours to stop the reaction. The reaction solution was dissolved in diethyl ether and washed with water. The organic layer was dried over anhydrous sodium sulfate, and the solvent was distilled off under reduced pressure. The residue obtained was suspended in a 1:3 mixture of ethyl acetate and hexane, and the suspension was filtered. The resulting solid was dried in a desiccator on P₂O₅ under vacuum, affording Compound 12 as a pale yellow solid. (1.20 g, 4.39 mmol, yield 62%)
¹H NMR (400 MHz, CHLOROFORM-D) δ7.71 (s, 1H), 7.25 (s, 1H), 5.01 (s, 2H), 4.82 (s, 2H), 4.00 (s, 3H), 3.95 (s, 3H), 2.22 (s, 3H) ppm
¹³C NMR (101 MHz, CHLOROFORM-D) δ153.80, 147.76, 130.22, 109.71, 108.04, 77.48, 76.84, 75.68, 67.15, 56.57, 56.49, 14.54 ppm
HRMS (ESI) m/z calcd. for C₁₁H₁₅NNaO₅S⁺ (M+Na)⁺ = 296.0563, found 296.0567

### 9. Synthesis of 2'-O-methylated dinucleotide cap compound

The following shows a synthesis scheme of a nitrobenzyl derivative to be used for the synthesis of a 2'-O-methylated dinucleotide cap compound.

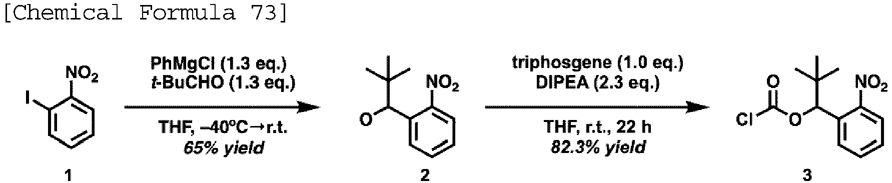

The following shows a synthesis scheme of the 2'-O-methylated dinucleotide cap compound.

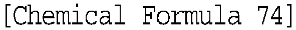

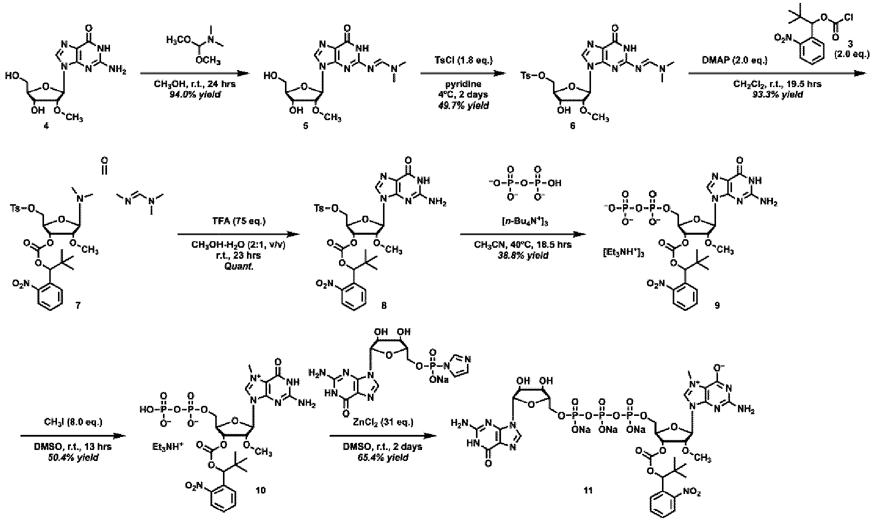

### (1) Synthesis of Compound 2

The synthesis of Compound (2) was carried out in accordance with literature. In THF (150 mL), 1-iodo-2-nitrobenzene (1) (20 g, 80 mmol) was cooled to -40°C. To the mixture was added a solution of phenylmagnesium chloride in THF (2 M, 52 mL, 0.10 mol), and the resulting mixture was stirred at -40°C for 1 hour. Trimethylacetaldehyde (12 mL, 9.6 g, 0.11 mol) was added to the reaction mixture, followed by stirring at -40°C for 36 minutes. The reaction mixture was gradually warmed to room temperature. After stirring for 4 hours, the reaction was quenched by the addition of a saturated aqueous ammonium chloride solution. The mixture was extracted three times with ethyl acetate. The organic layer was dried over Na₂SO₄ and concentrated. The residue was purified by silica gel column chromatography (17 → 25% ethyl acetate/hexane), affording Compound 2 (11 g, yield 65%) as a dark brown solid. All spectral data of the product agreed with those disclosed in literature (V. A. Litosh, et al., Nucleic Acids Res., 2011, 29(6), e39).

### (2) Synthesis of Compound 3

To a solution of Compound 2 (639 mg, 3.05 mmol) in THF (10.2 mL) were added N,N-diisopropylethylamine (986 mg, 1.30 mL, 7.63 mmol) and triphosgene (997 mg, 3.36 mmol) continuously. After stirring at room temperature for 23 hours, triphosgene (200 mg) and N,N-diisopropylethylamine (1.00 mL) were added to the mixture, and the resulting mixture was stirred at room temperature for 24 hours. The reaction mixture was diluted with dichloromethane and washed with water. The organic layer was dried over Na₂SO₄ and concentrated with a rotary evaporator, affording a chloroformate (3) as a dark brown oil (1.64 g, crude product). The crude product was purified by silica gel column chromatography (hexane : AcOEt = 1.0, 6 : 1), affording Compound 3 (601 mg, 72.6% yield) as a yellow oil.

¹H NMR (600 MHz, CDCl₃) δ 7.90 (ddd, J = 8.2, 3.3, 1.4 Hz, 1H), 7.70-7.54 (m, 2H), 7.46 (dddd, J = 9.8, 7.1, 2.6, 1.5 Hz, 1H), 6.56 (d, J = 1.5 Hz, 1H), 0.94 (d, J = 2.5 Hz, 9H) ppm.

¹³C NMR (151 MHz, CDCl₃) δ 149.97, 149.95, 149.04, 132.99, 131.13, 129.52, 128.80, 124.93, 84.30, 36.60, 25.56 ppm.

### (3) Synthesis of Compound 5

To a suspension of 2'-O-methylguanosine (4) (3.63 g, 12.2 mmol) in methanol (61.0 mL) was added N,N-dimethylformamide dimethyl acetal (7.27 g, 8.17 mL, 61.0 mmol). After stirring at room temperature for 21 hours, the reaction mixture was concentrated with a rotary evaporator. The oily residue was dissolved in methanol (10.0 mL) and a few drops of ether were added. The mixture was sonicated and then suspended by the addition of ether (150 mL). The resulting precipitate was collected by filtration and washed with ether. The solid was dried under reduced pressure, affording objective Compound 5 (4.08 g, 94.0) as a white powder. All spectral data agreed with those previous reported (T. Mukobata et al., Bioorg. Med. Chem. Lett., 2010, 20, 129-131).

### (4) Synthesis of Compound 6

To a cooled solution of Compound (5) (1.00 g, 2.84 mmol) in pyridine (22.7 mL) was added a solution of p-toluenesulfonyl chloride (812 mg, 4.26 mmol) in pyridine (5.70 mL) dropwise on an ice bath. The mixture was allowed to stand in a refrigerator at 4°C for 17 hours, and then a solution of p-toluenesulfonyl chloride (271 mg, 1.42 mmol) in pyridine (1.00 mL) was added thereto. The mixture was placed in a refrigerator at 4°C for 17 hours. The reaction mixture was quenched by pouring into water. The resulting mixture was extracted twice with dichloromethane. The organic layer was dried over Na₂SO₄ and evaporated. The residue was subjected to silica gel column chromatography eluted with 1.6 to 9.1% methanol/dichloromethane, affording 5'-O-tosylated compound 6 (682 mg, 47.4% yield) as a white foam.

¹H NMR (400 MHz, CDCl₃) δ 10.21-10.00 (m, 1H), 8.46 (s, 1H), 7.70 (s, 1H), 7.63 (d, J = 8.4 Hz, 2H), 7.17 (d, J = 8.4 Hz, 2H), 5.93 (d, J = 4.1 Hz, 1H), 5.24 (s, 1H), 4.60 (t, J = 5.4 Hz, 1H), 4.33-4.15 (m, 4H), 3.44 (s, 3H), 3.08 (s, 3H), 2.99 (s, 3H), 2.30 (s, 3H) ppm.

¹³C NMR (101 MHz, CDCl₃) δ 158.44, 158.38, 157.14, 150.29, 145.36, 136.69, 132.27, 129.94, 127.88, 120.31, 86.32, 83.03, 81.66, 69.41, 69.02, 58.82, 58.59, 53.62, 41.66, 41.48, 35.27, 31.00, 21.75, 21.65 ppm.

ESI-TOF-MS calcd for C₂₁H₂₇N₆O₇S, 507.1656 [M+H]+; found 507.1675, calcd for C₂₁H₂₆N₆NaO₇S, 529.1476 [M+Na]⁺; found 529.1495.

### (5) Synthesis of Compound 7

To a cooled solution of nitrobenzyl derivative (3) (107 mg, 0.394 mmol) in dichloromethane (985 µL) were added Compound 6 (100 mg, 0.197 mmol) and DMAP (48.1 mg, 0.394 mmol) in ice. After stirring at room temperature for 19.5 hours, the reaction mixture was diluted with dichloromethane and washed with a saturated aqueous NH₄Cl solution. The organic layer was dried over Na₂SO₄ and concentrated. The residue was subjected to silica gel column chromatography eluted with 2.0 to 4.8% methanol/dichloromethane, affording target Compound 7 (126 mg, yield 93.3%) as a white solid.

¹H NMR (600 MHz, CDCl₃) δ 9.73-9.60 (m, 1H), 8.61 (s, 0.5H), 8.58 (s, 0.5H), 7.88 (ddd, J = 8.8, 4.0, 1.2 Hz, 1H), 7.67-7.49 (m, 7H), 7.49-7.40 (m, 1H), 7.40-7.27 (m, 1H), 7.21-7.09 (m, 3H), 6.41 (s, 0.5H), 6.32 (s, 0.5H), 5.71 (td, J = 11.0, 4.5 Hz, 1H), 5.67-5.61 (m, 1H), 4.68 (dd, J = 5.7, 3.1 Hz, 0.5H), 4.58 (dd, J = 5.7, 3.5 Hz, 0.5H), 4.33-4.13 (m, 4H), 3.37 (s, 2H), 3.21 (s, 1.5H), 3.08 (d, J = 2.1 Hz, 3H), 3.03 (s, 1.5H), 2.92 (s, 1.5H), 2.33 (d, J = 9.0 Hz, 3H), 0.95 (s, 4.5H), 0.93 (s, 4.5H) ppm.

¹³C NMR (151 MHz, CDCl₃) δ 158.71, 158.66, 158.47, 158.15, 158.09, 157.24, 154.28, 154.02, 149.90, 149.31, 149.09, 145.91, 145.62, 145.51, 137.64, 132.93, 132.72, 132.59, 132.37, 132.13, 130.19, 129.93, 129.80, 129.76, 129.68, 129.26, 129.04, 129.00, 128.77, 128.01, 127.86, 127.80, 127.74, 124.81, 124.58, 121.22, 120.92, 88.19, 88.01, 87.36, 81.02, 80.93, 80.83, 80.78, 80.69, 80.60, 80.49, 80.16, 79.10, 78.94, 78.52, 78.20, 78.11, 75.84, 75.59, 75.20, 74.58, 74.07, 73.89, 67.70, 67.61, 67.55, 66.08, 65.30, 59.58, 59.40, 59.20, 58.94, 58.88, 53.58, 41.56, 41.43, 41.36, 41.05, 36.59, 36.34, 36.29, 36.25, 35.40, 35.34, 25.78, 25.74, 25.71, 25.60, 21.79, 21.68 ppm.

ESI-TOF-MS calcd for C₃₃H₄₀N₇O₁₁S, 742.2502 [M+H]+; found 742.2562, calcd for C₃₃H₃₉N₇NaO₁₁S, 764.2321 [M+Na]⁺; found 764.2313.

### (6) Synthesis of Compound 8

To a suspension of Compound 7 (591 mg, 0.797 mmol) in methanol (13.3 µL) and water (6.63 mL) was added trifluoroacetic acid (6.82 g, 4.68 mL, 59.8 mmol). After stirring at room temperature for 23 hours, the reaction mixture was concentrated to remove methanol. The residue was diluted with dichloromethane and washed with water. The organic layer was dried over Na₂SO₄ and then concentrated, affording objective Compound 8 (576 mg, quantitative) as a gray foam.

¹H NMR (600 MHz, CDCl₃) δ 7.91-7.86 (m, 1H), 7.75-7.66 (m, 3H), 7.66-7.60 (m, 2H), 7.29-7.21 (m, 3H), 6.43 (s, 0.5H), 6.30 (s, 0.5H), 5.75 (dd, J = 6.3, 3.7 Hz, 1H), 5.29-5.21 (m, 1H), 4.73 (t, J = 5.8 Hz, 0.5H), 4.63 (t, J = 5.7 Hz, 0.5H), 4.40-4.35 (m, 1H), 4.31 (dd, J = 7.9, 3.9 Hz, 1H), 4.29-4.20 (m, 1H), 3.31 (s, 1.5H), 2.97 (s, 1.5H), 2.36 (d, J = 9.7 Hz, 3H), 0.95 (d, J = 11.2 Hz, 9H) ppm.

¹³C NMR (151 MHz, CDCl₃) δ 159.21, 154.10, 153.77, 151.66, 149.39, 149.25, 145.58, 136.77, 132.81, 132.70, 132.56, 132.41, 132.09, 130.17, 130.13, 130.10, 129.69, 129.27, 128.97, 128.89, 128.82, 128.07, 128.02, 127.99, 127.96, 126.45, 124.66, 120.91, 117.56, 86.95, 86.86, 80.88, 80.67, 80.54, 80.16, 80.10, 79.75, 79.69, 74.36, 74.21, 68.40, 59.66, 59.39, 59.21, 36.59, 36.32, 36.24, 35.44, 25.81, 25.73, 25.70, 25.61, 21.80, 21.71, 21.68 ppm.

ESI-TOF-MS calcd for C₃₀H₃₅N₆O₁₁S, 687.2079 [M+H]+; found 687.2091.

### (7) Synthesis of Compound 9

Compound 8 (53.3 mg, 71.9 µmol) and powdered 3A molecular sieve (55.0 mg) were suspended in acetonitrile (80.0 µL), and a 616 mM solution of tris-tetrabutylammonium pyrophosphate in acetonitrile (175 µL, 108 µmol) was added. After stirring at 40°C for 18.5 hours, the reaction mixture was diluted with water. The resulting suspension was centrifuged at 4,500 rpm for 30 minutes. The supernatant was collected and the precipitate was resuspended in water. The resulting suspension was centrifuged at 4,500 rpm for 30 minutes. The supernatant was collected and purified by ion exchange chromatography (A: water, B: 1.5 M TEAB buffer (pH 7.9, containing 10% CH₃CN), gradient: 0 to 100% B (270 min)) using QAE-Sephadex (ϕ = 3.0 cm, h = 10 cm, 70 cm³, 6 mL/min) . The fractions containing the product were collected and concentrated, affording modified guanosine diphosphate 9 (56.9 mg, 57.1 mol, 79.7% yield) in the form of a triethylammonium salt. Quantification of the product by UV at 260 nm: 27.9 µmol (yield: 38.8%). The absorption coefficient (ε₂₆₀) = 15,780 M⁻¹·cm⁻¹ was used for the calculation.

¹H NMR (400 MHz, CD₃OD) δ 8.23-8.02 (m, 1H), 7.92 (d, J = 8.2 Hz, 1H), 7.69 (q, J = 6.3 Hz, 2H), 7.62-7.47 (m, 1H), 6.36 (s, 0.5H), 6.23 (s, 0.5H), 5.83 (dd, J = 7.6, 2.9 Hz, 1H), 5.49 (dd, J = 15.8, 5.0 Hz, 1H), 4.48-4.08 (m, 4H), 3.32 (s, 3H), 3.15 (q, J = 7.3 Hz, 18H), 1.28 (q, J = 11.2 Hz, 40H), 0.97 (d, J = 8.8 Hz, 9H) ppm.

¹³C NMR (101 MHz, CD₃OD) δ 158.15, 154.23, 153.93, 153.86, 149.52, 132.55, 132.16, 129.13, 129.05, 128.85, 126.03, 125.62, 124.27, 122.87, 85.84, 81.96, 80.80, 80.70, 80.23, 79.94, 75.54, 64.94, 58.41, 58.14, 58.03, 45.98, 42.24, 41.75, 36.10, 35.71, 30.37, 24.94, 24.77, 19.24, 10.25, 10.05, 7.75, 6.88 ppm.

³¹P NMR (162 MHz, CD₃OD) δ -9.04 (1P), -9.44 (0.5P), -10.75 (0.5P) ppm.

ESI-TOF-MS calcd for C₂₃H₂₉N₆O₁₅P₂, 691.1172 [M-H]-; found 691.1270.

### (8) Synthesis of Compound 10

To a solution of Compound 9 (180 µmol) in DMSO (1.50 mL) was added iodomethane (204 mg, 89.6 µL, 1.44 mmol). After stirring at room temperature for 13 hours, the reaction mixture was diluted with water and washed three times with dichloromethane. The crude product was purified by ion exchange chromatography (gradient, 0 → 0.8 M TEAA buffer (pH 6.0, 0 → 8% CH₃CN (270 min)) using QAE-Sephadex TM A-25 (ϕ = 4.5 cm, h = 8.4 cm, 140 cm³, 12 mL/min) . After removal of CH₃CN under reduced pressure, the product was desalted with Wakosil (registered trademark) 25C18 (particle size: 15 to 30 um (spherical), column size: ϕ = 4.80 cm, h = 10.2 cm, 184 cm³ (12 mL/min), solvent A: water (20 min), solvent B: 90% CH₃CN/water (20 min)). The fractions containing the product were concentrated, affording the objective Compound 10 (73.3 mg, 90.8 µmol, 50.4% yield) in the form of triethylammonium. The yield was calculated from the absorption at 260 nm. An absorption coefficient (ε260) = 15,100 M⁻¹·cm⁻¹ was used for the calculation.

¹H NMR (600 MHz, D₂O) δ 7.89-7.70 (m, 1H), 7.66-7.33 (m, 3H), 6.18 (s, 0.5H), 6.00 (d, J = 11.2 Hz, 1.5H), 5.47 (s, 0.5H), 4.62 (d, J = 5.6 Hz, 0.5), 4.67 (m, 0.5H), 4.55 (s, 0.5H), 4.50-4.38 (m, 1H), 4.18 (s, 0.5H), 4.06 (s, 1.5H), 3.99 (s, 3H), 3.30 (s, 1.5H), 3.07 (qd, J = 7.4, 2.1 Hz, 6H), 2.99 (s, 1.5H), 1.14 (td, J = 7.3, 2.2 Hz, 9H), 0.74 (d, J = 12.1 Hz, 9H) ppm.

¹³CNMR (151 MHz, D₂O) δ155.70, 155.63, 155.09, 154.94, 154.35, 153.94, 150.05, 149.87, 148.91, 148.65, 133.38, 133.05, 131.87, 131.18, 129.43, 129.22, 128.59, 124.70, 124.63, 108.16, 108.01, 87.55, 86.87, 83.55, 83.02, 82.51, 82.20, 81.55, 81.15, 75.27, 74.81, 69.65, 64.65, 64.44, 59.06, 58.99, 46.71, 36.20, 36.13, 35.58, 24.95, 24.92, 8.28 ppm. ³¹PNMR (243 MHz, D₂O) δ-9.64 (1P), -10.72 (1P) ppm.

ESI-TOF-MS calcd for C₂₄H₃₁N₆O₁₅P₂, 705.1328 [M-H]-; found 705.1404.

### (9) Synthesis of Compound 11

Compound 10 (7.60 mg, 9.40 µmol) and guanosine 5'-monophosphorimidazolide (12.3 mg, 28.2 µmol) were dissolved in a 0.59 M zinc chloride solution in DMSO (493 µL, ZnCl₂: 291 µmol). After stirring at 37°C for 2 days, 200 mM aqueous EDTA solution (pH 7.0, 1.60 mL) was added to quench the reaction mixture. After dilution with a 2.0 M TEAB buffer (pH 7.9, 100 µL), the mixture was purified by reverse-phase HPLC using a YMC-Triart-C8 column (250 × 4.6 mm I.D., S-5 µm, 12 nm, flow rate 1.0 mL/min, temperature: 50°C). The fractions containing the product were concentrated, affording target Compound 11 (8.33 mg, 6.15 µmol, 65.4% yield) in the form of a triethylammonium salt. The product was freeze-dried and the resulting solid was re-dissolved in methanol (2.0 mL). A 190 mM NaClO₄ (10 mL) solution in acetone was added to the mixture, and the resulting suspension was centrifuged (4,000 rpm, 20 min). The supernatant was discarded and the precipitate was resuspended in acetone. The suspension centrifugation process was repeated four more times. The precipitate was dried under reduced pressure, affording a target cap compound 11 (3.46 mg, 3.10 µmol, 33.0% yield) in the form of a sodium salt. The yield was calculated using the absorbance of the product at 260 nm measured by NanoDrop. An absorption coefficient (ε₂₆₀) = 26,300 M⁻¹·cm⁻¹ was used for the calculation. Triethylammonium salt

¹H NMR (600 MHz, CD₃OD, triethylammonium salt) δ 7.99 (d, J = 5.4 Hz, 1H), 7.92 (q, J = 8.0 Hz, 1H), 7.77-7.61 (m, 2H), 7.59-7.49 (m, 1H), 6.41-6.29 (m, 0.5H), 6.27-6.17 (m, 0.5H), 6.04-5.93 (m, 1H), 5.76 (s, 1H), 5.30 (s, 1H), 4.76-4.67 (m, 1H), 4.61 (s, 1H), 4.58-4.38 (m, 4H), 4.27 (s, 2H), 4.23-4.13 (m, 3H), 4.13-3.99 (m, 3H), 3.53-3.43 (m, 1.5H), 3.08 (d, J = 7.9 Hz, 1.5H), 0.96 (d, J = 8.4 Hz, 9H) ppm.

¹³C NMR (151 MHz, CD₃OD, triethylammonium salt) δ 159.34, 157.66, 155.50, 155.17, 155.02, 153.03, 150.94, 150.78, 138.22, 133.97, 133.82, 133.27, 130.47, 130.35, 130.12, 125.63, 117.77, 108.69, 89.71, 89.29, 85.29, 84.35, 84.11, 84.02, 81.73, 81.48, 75.66, 75.20, 71.92, 66.63, 65.32, 60.04, 59.78, 47.43, 37.51, 37.09, 36.97, 26.28, 26.13, 23.46, 9.14 ppm.

³¹P NMR (243 MHz, CD₃OD, triethylammonium salt) δ -10.53~-11.77 (m, 2P), -22.19 (s, 1P) ppm.

### Sodium salt

¹H NMR (600 MHz, D₂O, sodium salt) δ 7.87-7.80 (m, 2H), 7.68-7.54 (m, 2H), 7.45 (t, J = 7.7 Hz, 1H), 6.19 (s, 0.5H), 6.04 (s, 0.5H), 5.78 (d, J = 5.4 Hz, 1H), 5.62 (t, J = 6.4 Hz, 1H), 5.27-5.22 (m, 0.5H), 5.17 (t, J = 4.3 Hz, 0.5H), 4.54-4.47 (m, 1H), 4.45-4.38 (m, 1H), 4.35 (t, J = 5.4 Hz, 0.5H), 4.31 (q, J = 5.4 Hz, 1H), 4.25-4.04 (m, 5.5H), 3.95 (d, J = 5.4 Hz, 3H), 3.21 (s, 1.5H), 2.95 (s, 1.5H), 0.82 (d, J = 13.4 Hz, 9H) ppm.

³¹P NMR (243 MHz, D₂O, sodium salt) δ -10.97 (dd, J = 36.2, 19.7 Hz), -22.56 (t, J = 19.7 Hz) ppm.

### 10. Synthesis of 3'-O-methylated dinucleotide cap compound

The following shows a synthetic scheme of a 3'-O-methylated dinucleotide cap compound.

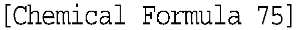

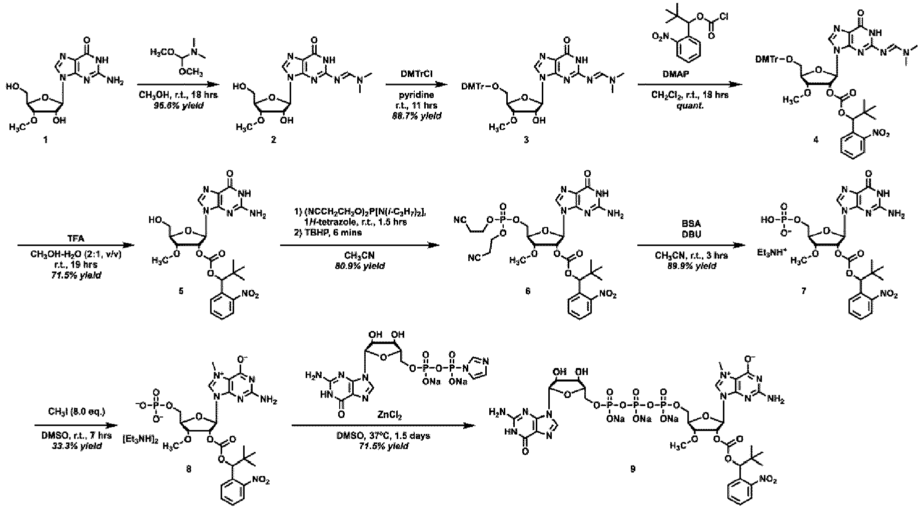

### (1) Synthesis of Compound 2

To a suspension of 2'-O-methylguanosine (1.50 g, 5.05 mmol) in methanol (25.3 mL) was added N,N-dimethylformamide dimethyl acetal (3.01 g, 3.39 mL, 25.3 mmol). After stirring at room temperature for 18 hours, the reaction mixture was concentrated with a rotary evaporator. The oily residue was dissolved in dichloromethane (10.0 mL) and suspended by the addition of ether. The resulting precipitate was collected by filtration and washed with ether. The solid was dried under reduced pressure, affording objective Compound 2 (1.72 g, yield 96.6%) as a white powder.

¹H NMR (600 MHz, DMSO-d₆) δ 11.35 (s, 1H), 8.54 (s, 1H), 8.04 (s, 1H), 5.78 (d, J = 6.3 Hz, 1H), 5.52 (s, 1H), 5.15 (s, 1H), 4.68-4.63 (m, 1H), 3.99 (q, J = 4.0 Hz, 1H), 3.84 (dd, J = 5.0, 3.3 Hz, 1H), 3.66-3.60 (m, 1H), 3.53 (d, J = 13.3 Hz, 1H), 3.41 (s, 3H), 3.16 (s, 3H), 3.03 (s, 3H) ppm.

¹³C NMR (151 MHz, DMSO-d₆) δ 157.97, 157.45, 150.02, 136.87, 119.85, 86.88, 82.96, 79.86, 73.02, 64.93, 61.47, 57.61, 40.64, 34.65 ppm.

### (2) Synthesis of Compound 3

To a solution of N2-protected-3'-O-methylguanosine 2 (195 mg, 0.553 mmol) in pyridine (2.77 mL) was added DMTrCl (244 mg, 0.719 mmol). After stirring at room temperature for 12 hours, DMTrCl (93.9 mg, 0.277 mmol) was added. The reaction mixture was stirred at room temperature for 3.5 hours and then quenched by the addition of methanol (1.00 mL). The resulting mixture was diluted with dichloromethane and washed with a saturated aqueous sodium bicarbonate solution. The organic layer was dried over Na₂SO₄ and concentrated with a rotary evaporator. The crude product was subjected to silica gel column chromatography eluted with 1.6 to 4.8% methanol/dichloromethane containing 1.0% triethylamine, affording target Compound 3 (337 mg, 93.1% yield) as a white foam.

¹H NMR (400 MHz, CDCl₃, containing triethylamine) δ 8.37 (s, 1H), 7.66 (s, 1H), 7.36-7.29 (m, 2H), 7.25-7.04 (m, 7H), 6.75-6.67 (m, 4H), 5.91 (d, J = 5.9 Hz, 1H), 4.73 (t, J = 5.7 Hz, 1H), 4.17 (q, J = 4.1 Hz, 1H), 3.93-3.87 (m, 1H), 3.66 (d, J = 1.2 Hz, 6H), 3.37 (s, 3H), 3.32 (dd, J = 10.6, 4.0 Hz, 1H), 3.22 (dd, J = 10.6, 4.5 Hz, 1H), 2.87 (d, J = 8.6 Hz, 6H), 2.43 (q, J = 7.2 Hz, 15H), 0.92 (t, J = 7.2 Hz, 22H) ppm.

¹³C NMR (101 MHz, CDCl₃, containing triethylamine) δ 158.56, 158.16, 156.99, 150.69, 144.65, 136.21, 135.69, 130.04, 128.10, 127.95, 126.93, 120.26, 113.21, 87.65, 86.46, 81.54, 80.25, 73.84, 63.68, 58.29, 55.20, 46.15, 41.27, 35.09, 11.51 ppm.

### (3) Synthesis of Compound 4

To a cooled solution of protected guanosine 3 (531 mg, 0.811 mmol) and chloroformate (439 mg, 1.62 mmol) in dichloromethane (4.06 mL) was added DMAP (198 mg, 1.62 mmol) in an ice bath. After stirring at room temperature for 18 hours, the reaction mixture was diluted with dichloromethane and washed with a saturated aqueous ammonium chloride solution. The organic layer was dried over Na2SO4 and basified by the addition of triethylamine (4.00 mL, 4%). After distilling off the solvent, the residue was subjected to silica gel column chromatography eluted with 2.4 to 6.3% methanol/dichloromethane containing triethylamine, affording a target compound (745 mg, quantitative) as a white amorphous solid.

¹H NMR (600 MHz, CDCl₃) δ 10.20 (s, 1H), 8.56 (s, 0.4H), 8.47 (s, 0.6H), 7.87-7.80 (m, 1H), 7.70 (s, 0.4H), 7.66 (s, 0.6H), 7.60-7.53 (m, 0.8H), 7.52-7.49 (m, 1.2H), 7.42-7.34 (m, 3H), 7.28-7.24 (m, 4H), 7.23-7.19 (m, 2H), 7.17-7.12 (m, 1H), 6.79-6.73 (m, 4H), 6.45 (s, 0.6H), 6.30 (s, 0.4H), 6.03 (dd, J = 6.8, 4.3 Hz, 1H), 6.02-5.94 (m, 1H), 4.23 (dt, J = 5.6, 3.7 Hz, 1H), 4.13 (t, J = 5.1 Hz, 0.6H), 4.00 (dd, J = 6.1, 4.9 Hz, 0.4H), 3.72 (d, J = 1.1 Hz, 6H), 3.45-3.40 (m, 1H), 3.33 (s, 1.8H), 3.27 (td, J = 11.3, 4.1 Hz, 1H), 3.09 (s, 1.2H), 3.06-3.05 (m, 1.2H), 3.01-2.99 (m, 1.8H), 2.97 (s, 1.2H), 2.94 (s, 1.8H), 0.91 (d, J = 5.8 Hz, 9H) ppm.

¹³C NMR (151 MHz, CDCl₃) δ 158.75, 158.63, 158.51, 158.29, 158.26, 157.13, 157.08, 153.75, 153.56, 150.03, 149.80, 149.10, 148.91, 144.28, 136.42, 136.19, 135.48, 132.67, 132.45, 132.05, 129.96, 129.74, 129.08, 128.95, 128.71, 128.56, 128.07, 127.88, 126.90, 124.55, 124.35, 120.84, 120.76, 113.16, 86.47, 86.44, 86.15, 85.55, 81.74, 81.58, 80.77, 80.41, 78.23, 77.94, 76.31, 76.21, 62.84, 62.68, 58.83, 58.65, 55.17, 46.12, 41.33, 41.14, 36.51, 36.11, 35.12, 35.05, 25.72, 25.64, 25.44, 11.47 ppm.

ESI-TOF-MS calcd for C₄₇H₅₁N₇NaO₁₁, 912.3539 [M+Na]⁺; found 912.3644.

### (4) Synthesis of Compound 5

To a suspension of guanosine derivative 4 (56.9 mg, 63.9 µmol) in methanol (1.07 mL) and water (533 µL) was added trifluoroacetic acid (546 mg, 367 µL, 4.79 mmol). After stirring at room temperature for 19 hours, the reaction mixture was diluted with a mixture of methanol/water (2:1, v/v, 5.00 mL). The mixture was concentrated and purified by silica gel column chromatography with elution using 1.9 to 9.1% methanol/dichloromethane, affording the objective compound (24.3 mg, 71.5% yield) as a white solid.

¹H NMR (600 MHz, DMSO-d₆) δ 10.73 (d, J = 9.5 Hz, 1H), 8.01-7.93 (m, 2H), 7.81-7.68 (m, 1H), 7.64-7.52 (m, 2H), 6.54 (s, 2H), 6.18 (s, 0.6H), 6.02 (s, 0.4H), 5.96 (d, J = 5.5 Hz, 1H), 5.48 (q, J = 5.1 Hz, 1H), 4.22 (t, J = 4.7 Hz, 0.5H), 4.08-3.95 (m, 1.5H), 3.68-3.60 (m, 1H), 3.58-3.50 (m, 2H), 3.35 (s, 2H), 2.98 (s, 1H), 0.87 (d, J = 3.7 Hz, 9H) ppm.

¹³C NMR (151 MHz, DMSO-d₆) δ 156.61, 156.57, 153.91, 153.88, 153.10, 152.99, 150.98, 150.92, 149.08, 148.86, 135.66, 135.55, 133.05, 131.36, 130.78, 129.60, 129.47, 128.89, 128.51, 124.51, 124.48, 116.54, 116.47, 84.03, 82.96, 82.89, 79.93, 79.65, 77.90, 77.75, 77.27, 77.23, 60.91, 60.86, 58.05, 57.71, 36.02, 35.59, 25.44, 25.21 ppm.

### (5) Synthesis of Compound 6

To Compound 5 (290 mg, 0.545 mmol) was added a solution of bis(2-cyanoethyl) N,N-diisopropylphosphoramidite (222 mg, 0.818 mmol) in acetonitrile (5.45 mL). 1H-Tetrazole (57.3 mg, 0.818 mmol) was added to the mixture, followed by stirring at room temperature for 1.5 hours, and subsequently, to the reaction mixture was added 5 to 6 M tert-butyl hydroperoxide in a decane solution (218 µL, 1.09 to 1.31 mmol). The reaction mixture was further stirred at room temperature for 6 minutes and diluted with dichloromethane. The mixture was washed with a saturated aqueous sodium bicarbonate solution. The organic layer was dried over Na2SO4 and concentrated. The residue was purified by silica gel column chromatography eluted with 4.8 to 17% methanol/dichloromethane, affording the objective compound (317 mg, yield 80.9%) as a brown amorphous solid.

¹H NMR (600 MHz, CDCl₃) δ 12.25 (s, 0.5H), 7.88 (d, J = 8.2 Hz, 0.5H), 7.81 (d, J = 8.0 Hz, 0.5H), 7.74 (d, J = 16.2 Hz, 1H), 7.66-7.60 (m, 1H), 7.59-7.50 (m, 1H), 7.46 (ddd, J = 8.5, 5.9, 2.9 Hz, 0.5H), 7.40 (t, J = 7.8 Hz, 0.6H), 6.60 (s, 1.5H), 6.35 (s, 0.5H), 6.22 (s, 0.5H), 5.95 (d, J = 4.4 Hz, 1H), 5.73 (dd, J = 10.3, 5.4 Hz, 1H), 4.53-4.32 (m, 2.5H), 4.25 (tq, J = 11.5, 5.4 Hz, 5.5H), 3.44 (s, 1.5H), 3.03 (s, 1.5H), 2.75 (dq, J = 12.6, 6.8 Hz, 4H), 0.94 (s, 9H) ppm.

¹³C NMR (151 MHz, CDCl₃) δ 158.89, 154.01, 153.97, 153.81, 153.60, 151.23, 149.48, 149.26, 137.08, 132.74, 132.58, 131.98, 129.35, 129.10, 128.90, 124.62, 124.52, 117.55, 117.50, 117.12, 117.00, 86.69, 86.57, 80.98, 80.74, 78.01, 77.77, 76.37, 67.07, 62.85, 62.81, 59.18, 58.94, 36.52, 36.16, 25.83, 25.63, 19.69, 19.63 ppm.

³¹P NMR (243 MHz, CDCl₃) δ -1.83 and 1.87 ppm.

HRESIMS calcd. for C₂₉H₃₅N₈NaO₁₂P, 741.2005 [M+Na]⁺; found 741.1980.

### (6) Synthesis of Compound 7

To a solution of nucleoside monophosphate 6 (317 mg, 0.441 mmol) in acetonitrile (4.41 mL) were added continuously N,O-bis(trimethylsilyl)acetamide (897 mg, 1.08 mL, 4.41 mmol) and 1,8-diazabicyclo[5.4. 0]undec-7-ene (268 mg, 263 µL, 1.76 mmol). After stirring at room temperature for 3 hours, the reaction mixture was quenched by the addition of a 1 M TEAA buffer (pH 6.0, 1.50 mL). The mixture was diluted with water (25.0 mL) and washed three times with dichloromethane (20.0 mL). The aqueous layer was purified using Wakosil (registered trademark) 25C18 (particle size: 15 to 30 µm (spherical), column size: ϕ = 4.80 cm, h = 10.2 cm, 184 cm³ (12 mL/min)) (solvent A: 50 mM TEAA + 0.5% CH₃CN (pH 6.0), solvent B: CH₃CN, 0 to 80% B gradient in 60 min). The fractions containing the objective product were combined, concentrated, and freeze-dried, affording the objective Compound 7 (347 mg, 89.9% yield; monotriethylammonium salt contained 3 equivalents of TEAA salt) as a brown powder.

¹H NMR (600 MHz, CD₃OD) δ 8.11-8.01 (m, 1H), 7.96-7.85 (m, 1H), 7.73 (t, J = 7.7 Hz, 0.5H), 7.65 (dt, J = 8.0, 5.8 Hz, 1H), 7.60-7.49 (m, 1.5H), 6.26 (s, 0.5H), 6.17 (s, 0.5H), 6.07 (dd, J = 11.3, 5.6 Hz, 1H), 5.61 (q, J = 5.1 Hz, 1H), 4.37 (t, J = 4.4 Hz, 0.5H), 4.33-4.21 (m, 1.5H), 4.16 (q, J = 7.3 Hz, 1H), 4.12-4.00 (m, 1H), 3.47 (s, 1.5H), 3.15 (q, J = 7.4 Hz, 24H, triethylammonium), 3.11 (s, 1.5H), 1.93 (s, 9H, Acetate), 1.27 (t, J = 7.4 Hz, 36H, triethylammonium), 0.92 (d, J = 6.3 Hz, 9H) ppm.

¹³C NMR (151 MHz, CD₃OD) δ 179.20, 159.53, 159.46, 155.91, 155.84, 154.88, 154.79, 152.96, 152.92, 150.76, 150.55, 138.06, 137.93, 133.90, 133.52, 133.15, 130.40, 130.34, 130.05, 130.03, 125.56, 125.51, 117.74, 117.53, 86.77, 86.50, 83.77, 83.72, 81.68, 81.53, 80.21, 79.86, 78.97, 78.80, 65.80, 65.77, 65.64, 59.17, 59.01, 47.10, 47.07, 37.24, 36.99, 26.19, 26.09, 23.81, 23.79, 9.05 ppm.

³¹P NMR (243 MHz, CD₃OD) δ -1.53 and 1.48 ppm.

HRESIMS calcd. for C₂₃H₂₈N₆O₁₂P, 611.1508 [M-H]-; found 611.1504.

### (7) Synthesis of Compound 8

To a solution of 2'-modified guanosine monophosphate 7 (61.6 mg, 75.6 µmol) in DMSO (630 µL) was added iodomethane (85.9 mg, 37.7 µL, 605 µmol). After stirring at room temperature for 7 hours, the reaction mixture was diluted with water and washed three times with dichloromethane. The aqueous layer was purified using Wakosil (registered trademark) 25C18 (particle size: 15 to 30 um (spherical), column size: ϕ = 4.80 cm, h = 10.2 cm, 184 cm³ (12 mL/min)) (solvent A: 50 mM TEAA + 0.5% CH₃CN (pH 6.0), solvent B: CH₃CN, 0 to 80% B gradient in 60 min). The fractions containing the objective product were combined, concentrated, and freeze-dried, affording the objective Compound 8 (18.3 mg, 33.3% yield) in the form of a triethylammonium salt.

¹H NMR (600 MHz, D₂O, triethylammonium salt) δ 7.86 (dd, J = 8.2, 1.3 Hz, 0.4H), 7.79 (dd, J = 8.2, 1.4 Hz, 0.6H), 7.73-7.67 (m, 0.4H), 7.64-7.59 (m, 1H), 7.57-7.44 (m, 1.6H), 6.19 (d, J = 3.7 Hz, 0.4H), 6.09 (d, J = 5.7 Hz, 0.6H), 6.04 (s, 0.5H), 5.96 (s, 0.5H), 5.62-5.55 (m, 1H), 4.44 (dt, J = 8.8, 3.1 Hz, 1H), 4.35 (dd, J = 5.0, 3.0 Hz, 1H), 4.27 (t, J = 5.1 Hz, 0.5H), 4.16-4.10 (m, 0.5H), 4.08-4.02 (m, 3H), 3.98-3.92 (m, 1H), 3.49 (s, 2H), 3.17 (q, J = 7.4 Hz, 18H triethylammonium), 3.14 (s, 1H), 1.89 (s, 3H, acetate), 1.25 (t, J = 7.4 Hz, 27H, triethylammonium), 0.86 (d, J = 7.0 Hz, 9H) ppm.

¹³C NMR (151 MHz, D₂O, triethylammonium salt) δ 181.47, 160.00, 159.51, 154.23, 153.75, 149.74, 149.55, 149.03, 148.80, 133.35, 132."8, 1'1.59, 131.05, 129.58, 129.39, 128.73, 128.50, 124.58, 124.16, 108.63, 108.02, 86.78, 85.62, 83.82, 83.76, 83.25, 81.89, 81.60, 78.86, 78.80, 78.09, 77.64, 63.23, 62.59, 58.52, 58.39, 46.68, 36.01, 35.95, 35.62, 24.92, 24.87, 23.32, 8.26 ppm.

³¹P NMR (243 MHz, D₂O, triethylammonium salt) δ 4.17, 4.13 ppm.

HRESIMS calcd. for C₂₄H₃₀N₆O₁₂P, 625.1664 [M-H]-; found 625.1693.

### (8) Synthesis of Compound 9

To a solution of 2'-Nb-3'-OMe-N7m-guanosine monophosphate 8 (18.4 mg, 18.6 µmol) in DMSO (372 µL) were added guanosine 5'-phosphorimidazolide diphosphate (30.0 mg, 55.8 µmol) and zinc chloride (50.7 mg, 372 pmol). After stirring at 37°C for 1.5 days, a 500 mM aqueous EDTA solution (pH 8.0, 968 µL, EDTA: 484 mmol) was added to quench the reaction mixture. The mixture was diluted with water (14.0 mL) and purified by reverse-phase HPLC (instrument: Shimadzu Prep, column: YMC-Actus Triart C8 (Preparative, 250 × 20.0 mm I.D., solvent A: 50 mM TEAA buffer (pH 6.0, containing 0.5% CH₃CN), solvent B: CH₃CN, 5 to 80% B gradient (25 min), flow rate: 10 ml/min, detection: 254 nm). The fractions containing the target product were collected, concentrated, and freeze-dried, affording a PureCap analog in the form of a triethylammonium salt. The product was re-dissolved in methanol (2.00 mL). 190 mM NaClO₄ (12.0 mL) in acetone was added to the mixture and the resulting suspension was centrifuged (4,000 rpm, 20 min). The supernatant was discarded and the precipitate was resuspended in acetone. The suspension centrifugation process was repeated four more times. The precipitate was dried under reduced pressure, affording Cap Analog 9 (14.9 mg, 13.3 µmol, 71.5% yield) in the form of a sodium salt. The yield was calculated using the absorbance of the product at 260 nm measured by NanoDrop. For the calculation, an absorption coefficient (ε₂₆₀) = 26,300 M⁻¹·cm⁻¹ was used.

¹H NMR (600 MHz, D₂O) δ 7.93 (s, 1H), 7.80 (d, J = 8.2 Hz, 1H), 7.68-7.40 (m, 3H), 5.99 (s, 1H), 5.92 (d, J = 5.7 Hz, 0.5H), 5.79 (s, 0.5H), 5.72 (d, J = 5.9 Hz, 0.5H), 5.43 (s, 0.5H), 4.60 (s, 1H), 4.48-4.41 (m, 1H), 4.39-4.14 (m, 8H), 4.08 (s, 1H), 4.01 (s, 2H), 3.51-3.39 (m, 2H), 0.93-0.77 (m, 9H) ppm.

¹³C NMR (151 MHz, D₂O) δ 158.67, 154.06, 153.84, 151.39, 149.23, 148.87, 137.33, 133.00, 131.59, 130.94, 129.55, 129.27, 128.77, 124.21, 115.98, 108.52, 107.89, 86.67, 85.49, 83.68, 83.62, 82.51, 81.65, 78.20, 77.97, 73.79, 70.33, 65.53, 64.94, 58.55, 36.05, 35.67, 30.29, 24.80 ppm.

³¹P NMR (243 MHz, D₂O) δ -10.96 (2P, dt, J = 88.8, 23.0 Hz), -22.44 (1P, t, J = 19.7 Hz) ppm.

### 11. Synthesis of long-chain-alkyl-modified nitrobenzyl dinucleotide cap compound

The following shows a synthesis scheme of a nitrobenzyl derivative to be used for the synthesis of a long-chain-alkyl-modified nitrobenzyl dinucleotide cap compound.

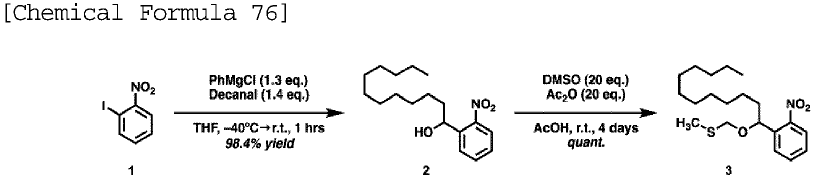

The following shows a synthetic scheme of a long-chain-alkyl-modified nitrobenzyl dinucleotide cap compound.

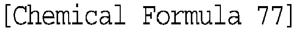

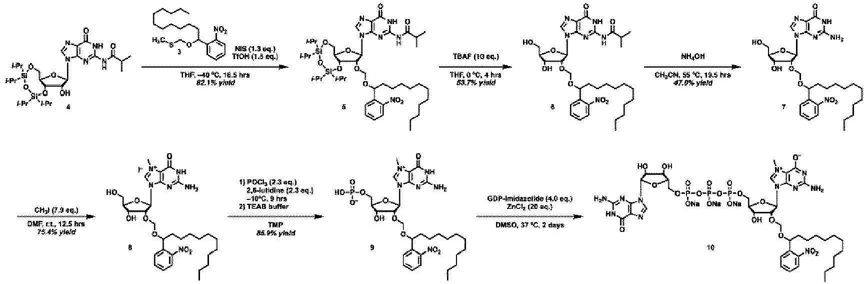

### (1) Synthesis of Compound 2

A mixture of 1-iodo-2-nitrobenzene 1 (5.00 g, 20.1 mmol) in THF (41.9 mL) was cooled to -40°C. A solution of phenylmagnesium chloride in THF (2 M, 13.1 mL, 26.1 mmol) was added to the mixture, followed by stirring at -40°C for 1 hour. Decanal (5.18 g, 6.24 mL, 28.1 mmol) was added to the reaction mixture, followed by stirring at -40°C for 1 hour. The reaction mixture was quenched by the addition of a saturated aqueous ammonium chloride solution (20.0 mL). The mixture was extracted three times with dichloromethane (20.0 mL). The organic layer was dried over Na2SO4 and concentrated. The residue was purified by silica gel column chromatography eluted with 17 → 50% ethyl acetate/hexane, affording the objective Compound 2 (6.08 g, yield 98.4%) as a brown oil.

¹H NMR (60 MHz, CDCl₃) δ 8.09-6.79 (m, 5H, overlappingwiththesignalofCHCl₃), 5.59-4.86 (m, 1H), 1.34 (s, 20H), 0.99 (d, J = 4.8 Hz, 3H) ppm.

¹³C NMR (15 MHz, CDCl₃) δ 147.46, 140.76, 137.30, 133.11, 130.05, 129.25, 127.92, 127.57, 123.94, 119.79, 115.41, 68.96, 60.50, 38.59, 31.88, 29.63, 29.33, 26.10, 22.62, 13.97, 13.88 ppm.

### (2) Synthesis of Compound 3

Acetic acid (47.6 g, 45.3 mL, 792 mmol), DMSO (30.9 g, 28.1 mL, 396 mmol) and acetic anhydride (40.4 g, 37.4 mL, 396 mmol) were added to a nitrobenzyl alcohol derivative 2 (6.08 g, 19.8 mmol). After stirring at room temperature for 4 days, the reaction mixture was added dropwise to a 10 M aqueous KOH solution. (178 mL, 1.78 mol) In an ice bath. The mixture was stirred at room temperature for 4 hours and then extracted with ethyl acetate. The organic layer was washed with brine and dried over Na2SO4. The residue was dissolved in hexane and subjected to silica gel column chromatography eluted with 4.8 to 17% ethyl acetate/hexane, affording methyl thioacetal 3 (10.6 g, > 100% yield) as a yellow oil.

¹H NMR (600 MHz, CDCl₃) δ 7.88 (dd, J = 8.2, 1.3 Hz, 1H), 7.70 (dd, J = 7.9, 1.5 Hz, 1H), 7.64-7.58 (m, 1H), 7.39 (ddd, J = 8.2, 7.3, 1.5 Hz, 1H), 5.26 (dd, J = 8.9, 3.4 Hz, 1H), 4.60 (d, J = 11.6 Hz, 1H), 4.23 (d, J = 11.6 Hz, 1H), 2.10 (s, 3H), 1.81-1.67 (m, 2H), 1.58-1.41 (m, 2H), 1.39-1.33 (m, 1H), 1.29-1.21 (m, 15H), 0.85 (t, J = 7.1 Hz, 3H) ppm.

¹³C NMR (151 MHz, CDCl₃) δ 148.81, 138.49, 133.35, 128.35, 128.10, 124.33, 73.92, 73.60, 37.92, 31.96, 29.70, 29.66, 29.64, 29.61, 29.38, 29.30, 26.18, 22.73, 14.23, 14.15 ppm.

### (3) Synthesis of Compound 5

To a suspension of N2-isobutyryl-3',5'-O-(1,1,3,3-tetrahydrofurandisiloxane-1,3-diyl) guanosine 4 (2.00 g, 3.36 mmol), a methylthioacetal derivative 3 (1.61 g, 4.37 mmol) and powdered molecular sieve 3 Å (1.50 g) in tetrahydrofuran (33.6 mL) was added N-iodosuccinimide (983 mg, 4.37 mmol), followed by cooling to -40°C. After 20 minutes at -40°C, trifluoromethanesulfonic acid (756 mg, 448 µL, 5.04 mmol) was added to the suspension, and the mixture was stirred at -40°C for 16.5 hours. The reaction mixture was quenched by the addition of triethylamine (16.8 mL), and then diluted with ethyl acetate (100 mL). The mixture was washed with a saturated aqueous sodium bicarbonate solution (100 mL × 2), sodium thiosulfate (100 mL × 2), and brine (100 mL × 1). The organic layer was dried over Na2SO4 and concentrated. The residue was purified by silica gel column chromatography with elution using 0 to 4.8% methanol/dichloromethane, affording objective Compound 5 (2.53 g, 82.1% yield) as a brown foam.

¹H NMR (600 MHz, CDCl₃, mixture of the target compound and the starting material) δ 12.32-12.08 (m, 1.5H), 10.79 (s, 0.7H), 9.70 (s, 0.5H), 9.39 (s, 0.3H), 7.99 (s, 0.3H), 7.93-7.88 (m, 1H), 7.82 (s, 0.3H), 7.70 (dd, J = 8.2, 1.4 Hz, 0.4H), 7.63 (dd, J = 5.4, 2.0 Hz, 0.6H), 7.54 (dd, J = 7.9, 1.5 Hz, 0.3H), 7.41 (dddd, J = 10.6, 9.0, 6.9, 2.1 Hz, 0.6H), 7.23 (td, J = 7.9, 1.5 Hz, 0.3H), 5.79 (s, 0.4H), 5.73 (s, 0.9H), 5.58 (s, 0.4H), 5.29 (dd, J = 8.6, 3.3 Hz, 0.4H), 5.24 (s, 1.5H), 5.21 (dd, J = 7.6, 4.6 Hz, 0.5H), 5.13 (d, J = 6.5 Hz, 0.3H), 5.03 (d, J = 6.6 Hz, 0.4H), 4.63 (d, J = 6.6 Hz, 0.4H), 4.58 (d, J = 6.5 Hz, 0.3H), 4.48 (dd, J = 9.4, 4.2 Hz, 0.3H), 4.38 (dt, J = 10.5, 5.4 Hz, 1.2H), 4.25 (d, J = 5.2 Hz, 0.8H), 4.20-4.05 (m, 3.3H), 4.03-3.87 (m, 3.3H), 3.44 (s, 0.4H), 3.36 (d, J = 5.9 Hz, 0.4H), 2.92 (p, J = 6.8 Hz, 0.8H), 2.81 (p, J = 6.9 Hz, 0.2H), 2.69 (p, J = 6.8 Hz, 0.4H), 1.78-1.59 (m, 2H), 1.25-1.14 (m, 24H), 1.13-1.09 (m, 3H), 1.08-0.84 (m, 54H), 0.79 (t, J = 7.1 Hz, 4H) ppm.

¹³C NMR (151 MHz, CDCl₃, mixture of the target compound and the starting material) δ 180.13, 179.64, 179.13, 156.03, 155.80, 155.72, 148.50, 148.38, 148.02, 147.97, 147.58, 147.45, 138.31, 137.95, 136.56, 136.43, 136.37, 133.94, 133.27, 128.57, 128.51, 128.45, 128.07, 124.52, 124.00, 121.45, 121.35, 121.23, 93.62, 91.30, 89.08, 88.14, 87.38, 81.66, 81.25, 81.07, 79.04, 76.60, 75.30, 74.74, 72.28, 70.63, 70.03, 69.50, 69.37, 60.55, 59.57, 59.50, 53.55, 50.39, 37.93, 37.87, 36.45, 36.24, 36.05, 31.92, 29.68, 29.65, 29.62, 29.59, 29.55, 29.51, 29.49, 29.46, 29.43, 29.37, 29.33, 26.54, 25.81, 25.64, 22.69, 19.21, 19.08, 18.97, 18.95, 18.90, 17.49, 17.46, 17.40, 17.33, 17.26, 17.20, 17.17, 17.11, 17.08, 17.05, 17.00, 16.98, 16.96, 16.88, 16.84, 14.12, 13.44, 13.39, 13.08, 13.00, 12.93, 12.67, 12.54 ppm

### (4) Synthesis of Compound 6

A solution of a starting material 5 (2.53 g, 2.76 mmol) in tetrahydrofuran (13.8 mL) was cooled in an ice bath and 1 M TBAF/THF (27.6 mL, 27.6 mmol) was added thereto. After stirring at 0°C for 4 hours, the reaction mixture was diluted with dichloromethane (120 mL) and washed with water (120 mL). The aqueous layer was extracted with dichloromethane (120 mL). The combined organic layers were dried over Na2SO4 and concentrated. The residue was purified by silica gel column chromatography eluted with 0 to 9.1% methanol/dichloromethane, affording target Compound 6 (1.00 g, 53.7% yield) as a white foam.

¹H NMR (600 MHz, CDCl₃) δ 12.15 (d, J = 17.5 Hz, 1H), 10.71-10.47 (m, 1H), 7.81 (s, 0.5H), 7.69 (d, J = 8.0 Hz, 0.5H), 7.62 (dd, J = 8.2, 1.4 Hz, 0.5H), 7.49-7.42 (m, 2H), 7.28-7.19 (m, 1H), 7.16-7.10 (m, 0.5H), 5.71 (d, J = 3.2 Hz, 0.5H), 5.30 (d, J = 2.9 Hz, 0.5H), 5.10 (t, J = 6.4 Hz, 0.5H), 5.02-4.92 (m, 1.5H), 4.82 (d, J = 6.8 Hz, 0.5H), 4.76 (d, J = 6.9 Hz, 0.5H), 4.72 (d, J = 7.1 Hz, 0.5H), 4.60 (t, J = 4.2 Hz, 0.5H), 4.50 (dd, J = 4.9, 3.0 Hz, 0.5H), 4.44 (d, J = 7.2 Hz, 0.5H), 4.23 (s, 1H), 3.92 (dt, J = 6.5, 3.3 Hz, 0.5H), 3.87 (dt, J = 7.0, 3.5 Hz, 0.5H), 3.78-3.59 (m, 2H), 3.27 (d, J = 5.5 Hz, 0.5H), 3.24-3.17 (m, 6.5H), 3.05-2.93 (m, 1H), 1.56 (dq, J = 16.3, 7.9 Hz, 8H), 1.50-1.39 (m, 1.5H), 1.30 (h, J = 6.9 Hz, 7H), 1.17-1.01 (m, 23H), 1.01-0.92 (m, 3H), 0.92-0.88 (m, 1H), 0.85 (td, J = 7.4, 1.3 Hz, 10H), 0.71 (td, J = 7.1, 1.4 Hz, 3H) ppm.

¹³C NMR (151 MHz, CDCl₃) δ 180.47, 180.28, 155.49, 155.47, 148.15, 147.87, 147.77, 147.73, 147.55, 147.51, 147.10, 138.97, 138.76, 138.44, 137.71, 133.25, 132.77, 128.26, 128.18, 127.97, 127.74, 123.89, 123.56, 121.42, 121.21, 94.72, 93.07, 88.61, 88.47, 84.22, 83.86, 78.84, 78.60, 74.87, 73.51, 70.30, 69.29, 69.11, 61.01, 60.87, 58.63, 37.49, 37.37, 35.53, 35.42, 31.59, 29.31, 29.29, 29.17, 29.08, 29.01, 26.22, 25.41, 25.26, 23.76, 22.37, 19.46, 18.97, 18.94, 18.83, 18.79, 13.84, 13.40 ppm.

### (5) Synthesis of Compound 7

A solution of a starting material 6 (970 mg, 1.44 mmol) in acetonitrile (9.40 mL) was added to a 28% ammonium hydroxide solution (18.8 mL). The mixture was heated to 55°C. After stirring at 55°C for 19.5 hours, the reaction mixture was concentrated and azeotroped with benzene (x 1). The residue was dissolved in methanol (6.00 mL) and suspended by the addition of diethyl ether (200 mL). The resulting precipitate was collected by filtration and rinsed with ether. The resulting solid was dried under vacuum, affording objective Compound 7 (408 mg, yield 47.0%) as a white solid.

¹H NMR (600 MHz, DMSO-d₆) δ 10.23 (s, 0.5H), 7.93 (s, 0.5H), 7.88 (dd, J = 8.2, 1.3 Hz, 0.5H), 7.84 (dt, J = 8.1, 0.9 Hz, 0.5H), 7.78 (s, 0.5H), 7.70 (td, J = 7.6, 1.4 Hz, 0.5H), 7.57 (dd, J = 7.9, 1.5 Hz, 0.5H), 7.55-7.50 (m, 1.5H), 7.40 (ddd, J = 8.1, 5.2, 3.6 Hz, 0.5H), 6.43 (s, 1.5H), 5.82 (d, J = 6.3 Hz, 0.5H), 5.68 (d, J = 5.9 Hz, 0.5H), 5.23 (d, J = 5.3 Hz, 0.5H), 5.16 (d, J = 5.0 Hz, 0.5H), 5.11 (t, J = 5.5 Hz, 0.5H), 5.08 (t, J = 5.4 Hz, 0.5H), 5.04 (dd, J = 7.8, 4.4 Hz, 0.5H), 4.78 (dd, J = 7.6, 4.9 Hz, 0.5H), 4.69-4.62 (m, 1.5H), 4.57 (d, J = 7.0 Hz, 0.5H), 4.52-4.47 (m, 0.5H), 4.34 (d, J = 7.3 Hz, 0.5H), 4.22 (dtd, J = 16.7, 5.0, 3.4 Hz, 1H), 3.89 (q, J = 3.7 Hz, 0.5H), 3.85 (q, J = 3.7 Hz, 0.5H), 3.61 (tdd, J = 12.5, 5.1, 3.6 Hz, 1H), 3.51 (dtd, J = 12.0, 6.3, 3.9 Hz, 1H), 3.20-3.14 (m, 2H), 1.60-1.53 (m, 2H), 1.31 (p, J = 7.4 Hz, 2H), 1.28-1.15 (m, 13H), 0.93 (t, J = 7.4 Hz, 3H), 0.85 (td, J = 7.1, 0.9 Hz, 3H) ppm.

¹³C NMR (151 MHz, DMSO-d₆) δ 156.71, 153.69, 153.64, 151.13, 151.02, 148.51, 147.69, 137.19, 136.43, 135.59, 135.17, 133.42, 133.24, 128.74, 128.34, 128.10, 123.97, 123.91, 116.89, 116.67, 93.20, 91.53, 85.92, 85.60, 85.05, 84.77, 78.82, 77.97, 74.07, 73.01, 69.60, 69.09, 61.49, 61.27, 57.54, 37.09, 36.76, 31.31, 31.28, 29.01, 28.96, 28.93, 28.83, 28.72, 28.69, 28.63, 24.98, 24.92, 23.06, 22.10, 19.21, 13.96, 13.49 ppm.

### (6) Synthesis of Compound 8

To a solution of 2'-modified guanosine 7 (100 mg, 0.166 mmol) in DMF (1.33 mL) was added iodomethane (186 mg, 81.6 µL, 1.31 mmol). After stirring at room temperature for 12.5 hours, water (15.0 mL) was added to the reaction mixture, and the mixture was extracted four times with dichloromethane (15.0 mL). The organic layer was concentrated and the residue was dissolved in acetone (2.00 mL). The solution was added dropwise to ether (150 mL) and the resulting precipitate was collected by filtration. The solid was dried under reduced pressure, affording objective Compound 8 (107 mg, yield 75.4%) as a yellow solid.

¹H NMR (600 MHz, CDCl₃) δ 9.56 (d, J = 7.3 Hz, 0.5H), 9.45 (d, J = 7.6 Hz, 0.5H), 7.98 (d, J = 7.1 Hz, 0.2H), 7.82 (q, J = 8.7 Hz, 0.5H), 7.75 (t, J = 7.8 Hz, 0.5H), 7.64 (dq, J = 10.5, 5.5 Hz, 2H), 7.40 (dtd, J = 14.8, 8.7, 5.4 Hz, 0.8H), 7.31-7.23 (m, 0.5H), 6.72 (s, 1H), 6.04 (q, J = 3.6 Hz, 0.5H), 5.89 (q, J = 3.4 Hz, 0.5H), 5.18-5.12 (m, 0.5H), 5.06 (q, J = 6.6 Hz, 0.5H), 4.96 (t, J = 7.0 Hz, 0.5H), 4.89 (t, J = 7.1 Hz, 0.5H), 4.78 (t, J = 7.1 Hz, 0.5H), 4.73 (t, J = 7.0 Hz, 0.5H), 4.56 (qt, J = 8.5, 4.8 Hz, 1H), 4.45 (dt, J = 10.2, 5.0 Hz, 0.5H), 4.23-4.11 (m, 3H), 4.00-3.91 (m, 1H), 3.79 (dt, J = 12.7, 5.9 Hz, 1H), 3.28 (dd, J = 10.8, 6.2 Hz, 1.5H), 3.02-2.81 (m, 1H), 1.76-1.62 (m, 4H), 1.44 (hept, J = 7.4 Hz, 2H), 1.27-1.13 (m, 14H), 1.00 (q, J = 7.4 Hz, 2.5H), 0.82 (q, J = 7.1 Hz, 2.5H) ppm.

¹³C NMR (151 MHz, CDCl₃) δ 162.91, 156.24, 155.98, 154.16, 153.87, 148.90, 148.65, 148.53, 147.91, 137.62, 137.37, 136.28, 136.15, 133.73, 133.56, 128.68, 128.60, 128.45, 124.30, 124.02, 108.76, 108.55, 94.32, 94.12, 88.95, 86.85, 86.38, 80.38, 80.27, 75.20, 74.77, 69.30, 68.87, 60.50, 60.23, 59.26, 50.06, 49.91, 49.77, 49.63, 49.48, 37.63, 37.55, 37.04, 36.97, 36.74, 31.98, 29.73, 29.58, 29.54, 29.41, 25.70, 25.43, 24.29, 22.75, 19.88, 14.19, 13.80 ppm.

### (7) Synthesis of Compound 9

A suspension of 2'-C11Nb-N 7-methyl-guanosine (8) (53.2 mg, 62.3 µmol) in trimethyl phosphate (312 µL) was cooled to -10°C. 2,6-Lutidine (15.3 mg, 16.7 µL, 143 µmol) and phosphoryl chloride (21.9 mg, 13.4 µL, 143 µmol) were added continuously to the mixture. After stirring at -10°C for 9 hours, a 0.2 M TEAB buffer (pH 7.9, 500 µL) was added at -10°C to quench the reaction mixture. After warming to room temperature, the mixture was diluted with methanol (9.00 mL). The mixture was further diluted with 50% methanol/water (to 50 mL) and purified using a CHRMATREX C8 MB-100-40/75 column (column size: ϕ = 3.0 cm, h = 10 cm, 70 cm³ (7 mL/min) (solvent A: 50 mM TEAA buffer (pH 6.0, containing 0.5% CH₃CN), solvent B: CH₃CN, 0 to 90% B gradient over 60 minutes) . The fractions containing the target product were combined, concentrated, and freeze-dried, affording the target Compound 9 (37.3 mg, yield 85.9%) as a white solid.

¹H NMR (600 MHz, CD₃OD) δ 7.88-7.79 (m, 1H), 7.74-7.62 (m, 2H), 7.49-7.41 (m, 1H), 6.12 (d, J = 3.5 Hz, 0.5H), 5.96 (d, J = 3.5 Hz, 0.5H), 5.22 (dd, J = 7.6, 4.5 Hz, 0.5H), 5.12 (dd, J = 7.6, 4.8 Hz, 0.5H), 5.01 (d, J = 7.1 Hz, 0.5H), 4.77 (d, J = 7.1 Hz, 0.5H), 4.70 (d, J = 7.0 Hz, 0.5H), 4.61 (ddd, J = 16.9, 4.8, 3.4 Hz, 1H), 4.47 (t, J = 5.1 Hz, 0.5H), 4.41-4.34 (m, 0.5H), 4.24-4.16 (m, 1.5H), 4.13 (d, J = 4.6 Hz, 2H), 4.06 (d, J = 1.4 Hz, 1H), 4.04 (dd, J = 4.7, 1.7 Hz, 0.5H), 4.02 (dt, J = 4.7, 1.4 Hz, 0.5H), 3.24-3.19 (m, 3H), 3.16 (q, J = 7.4 Hz, 16H, triethylammonium), 1.91 (s, 10H, acetate), 1.67-1.61 (m, 4H), 1.39 (h, J = 7.4 Hz, 4H), 1.28 (t, J = 7.4 Hz, 25H, triethylammonium), 1.22 (d, J = 13.0 Hz, 14H), 1.00 (t, J = 7.4 Hz, 5H), 0.86 (dd, J = 7.6, 6.5 Hz, 3H) ppm.

¹³C NMR (151 MHz, CD₃OD) δ 178.42 (acetate), 158.23, 158.05, 156.05, 155.91, 151.05, 150.87, 150.18, 149.47, 139.09, 138.56, 138.40, 134.49, 134.37, 129.79, 129.73, 129.67, 129.62, 129.37, 125.14, 125.06, 109.30, 109.10, 94.72, 94.62, 89.68, 89.63, 86.64, 86.40, 86.35, 81.47, 81.33, 80.84, 79.51, 79.29, 79.07, 75.88, 75.39, 69.89, 69.76, 64.26, 64.08, 59.49, 47.48, 38.63, 38.54, 36.70, 36.64, 33.04, 30.73, 30.70, 30.68, 30.64, 30.54, 30.49, 30.44, 26.64, 26.51, 24.76, 23.71, 22.96, 20.70, 14.45, 13.93, 9.10 ppm.

³¹P NMR (243 MHz, CD₃OD) δ 1.65 ppm.

### (8) Synthesis of Compound 10

To a solution of 2'-Nb-N7-methyl-guanosine-5'-monophosphate (9) (37.3 mg, 53.5 µmol) in DMSO (1.07 mL) were added guanosine 5'-diphosphorimidazolide (115 mg, 214 µmol) and zinc chloride (146 mg, 1.07 mmol) . The mixture was incubated at 37°C for 2 days. The reaction mixture was quenched by the addition of a 500 mM aqueous EDTA solution (pH 8.0, 2.78 mL, EDTA: 1.39 mmol) and then diluted with water (10.0 mL). The crude product was purified using a CHRMATREX C8 MB-100-40/75 column (column size: ϕ = 3.0 cm, h = 10 cm, 70 cm³ (7 mL/min), solvent A: 50 mM TEAA buffer (pH 6.0, containing 0.5% CH₃CN), solvent B: CH₃CN, 0 to 90% B gradient over 60 min). The fractions containing the target product were combined, concentrated, and freeze-dried, affording the target compound in a triethylammonium form. The triethylammonium salt of the object was dissolved in methanol (1.00 mL). A solution of 0.19 M NaClO₄ in acetone (12.0 mL) was added to the methanol solution, affording a white precipitate. The suspension was centrifuged at 4,500 rpm for 15 minutes. The supernatant was discarded and the precipitate was resuspended in acetone. The suspension centrifugation process was repeated three more times. The precipitate was dried under reduced pressure, affording target Cap Compound 10 as a sodium salt.
ESI-TOF-MS C₄₀H₅₆N₁₁O₂₁P₃ (M-2H)²⁻ calcd. m/z 559.6438, found m/z 559.6

### 12. Synthesis of nitrobenzyl-modified dinucleotide cap compound removable by reducing conditions

The following shows a synthesis scheme of a nitrobenzyl derivative used for synthesis of a nitrobenzyl-modified dinucleotide cap compound that can be removed by reduction conditions.

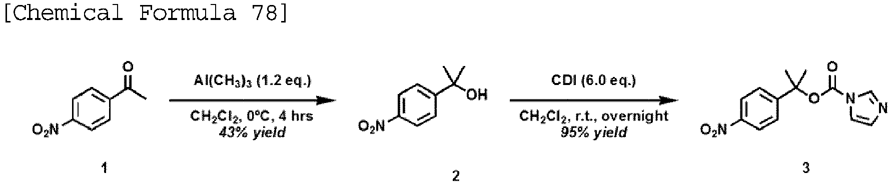

The following shows a synthesis scheme of the nitrobenzyl-modified dinucleotide cap compound that can be removed by reduction conditions.

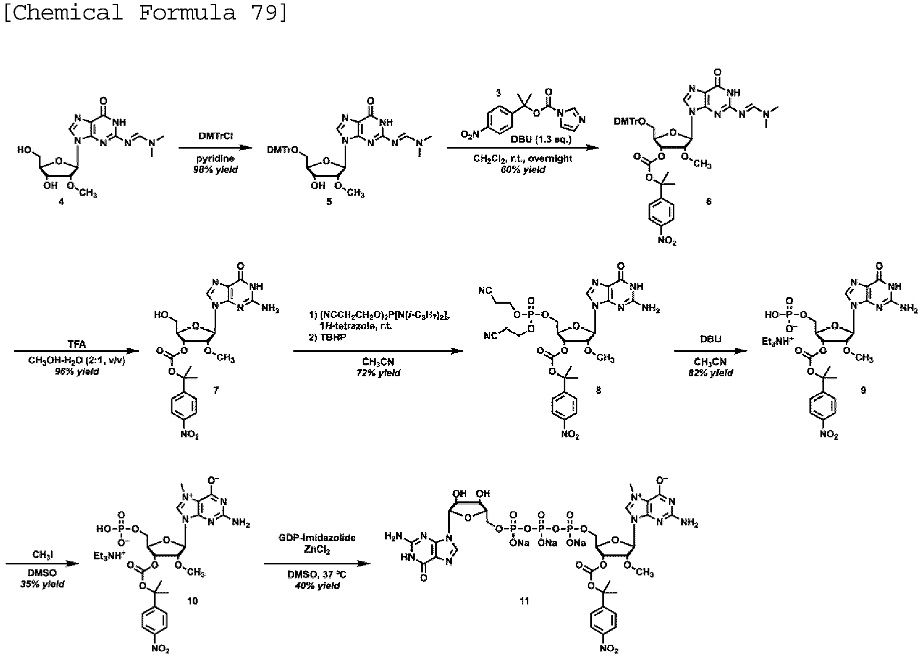

### (1) Synthesis of Compound 2

1-(4-Nitrophenyl)ethan-1-one (1) was azeotroped three times with toluene (25.0 mL) and the residue was dissolved in dichloromethane (100 mL). To the mixture was added Al(CH3)3 (15.6 mL, 21.8 mmol) at 0°C. After stirring at 0°C for 4 hours, the reaction mixture was quenched by the addition of acetone (15.0 mL) and washed with a saturated aqueous ammonium chloride solution (100 mL). The mixture was diluted with dichloromethane and washed with water, a saturated aqueous NaHCO₃ solution, and brine. The organic layer was dried over Na₂SO₄ and concentrated. The crude product was purified by silica gel column chromatography eluted with 1 → 3% methanol/chloroform, affording the target Compound 2 (1.41 g, 7.78 mmol, 43% yield).
¹H NMR (600 MHz, CDCl₃) δ 8.19 (d, J = 8.8 Hz, 2H), 7.67-7.64 (2H), 1.61 (s, 6H) ppm

### (2) Synthesis of Compound 3

To nitrobenzyl alcohol 2 (740 mg, 4.08 mmol) in dichloromethane (40.8 mL) was added 1,1'-carbonyldiimidazole (3.97 g, 24.5 mmol). After stirring overnight at room temperature, the reaction mixture was diluted with dichloromethane and washed with water and a saturated aqueous ammonium chloride solution. The organic layer was dried over Na2SO4 and concentrated, affording the target Compound 3 (1.08 g, 3.80 mmol, 95% yield).

¹H NMR (400 MHz, CDCl₃) δ 8.25 (dd, J = 6.8, 2.0 Hz, 2H), 8.13 (d, J = 2.0 Hz, 1H), 7.59 (dd, J = 7.1, 2.0 Hz, 2H), 7.39 (t, J = 1.4 Hz, 1H), 7.09 (q, J = 0.8 Hz, 1H), 1.98 (s, 6H) ppm.

### (3) Synthesis of Compound 5

To a solution of N2 protected-2'-O-methylguanosine (4) (1.20 g, 3.50 mmol) in pyridine (34.6 mL) was added DMTrCl (1.40 g, 4.20 mmol). After stirring overnight at room temperature, the reaction mixture was diluted with dichloromethane and washed with water and brine. The organic layer was dried over Na2SO4 and concentrated with a rotary evaporator. The crude material was subjected to silica gel column chromatography eluted with 4.7% methanol/chloroform containing 1.0% triethylamine, affording the target Compound 5 (2.22 g, 3.40 mmol, 98% yield).
¹H NMR (600 MHz, DMSO-d₆) δ 11.39 (s, 1H), 8.50 (s, 1H), 7.94 (s, 1H), 7.19-7.37 (m, 9H), 6.82-6.86 (m, 4H), 5.96 (d, J = 4.7 Hz, 1H), 5.29 (d, J = 5.8 Hz, 1H), 4.33 (t, J = 5.4 Hz, 1H), 4.26 (t, J = 5.0 Hz, 1H), 4.01-4.03 (m, 1H), 3.73 (s, 6H), 3.38 (s, 3H), 3.24 (dd, J = 10.4, 5.9 Hz, 1H), 3.15 (dd, J = 7.4, 2.7 Hz, 1H), 3.10 (s, 3H), 3.03 (s, 3H) ppm

### (4) Synthesis of Compound 6

Protected nucleoside 5 (2.0 g, 3.1 mmol) was added to a solution of a carbonyl imidazole compound (1.1 g, 4.0 mmol) in dichloromethane (31 mL) and DBU (0.59 mL, 4.0 mmol). After stirring overnight at room temperature, the reaction mixture was diluted with dichloromethane and washed with water and a saturated aqueous ammonium chloride solution. The organic layer was dried over Na2SO4 and concentrated. The crude product was purified by silica gel column chromatography eluted with 4.8% methanol/ethyl acetate, affording the target Compound 6 (1.6 g, 1.9 mmol, 60% yield).

¹H NMR (600 MHz, DMSO-d₆) δ 11.43 (s, 1H), 8.47 (s, 1H), 8.17-8.18 (m, 2H), 7.98 (s, 1H), 7.65-7.61 (2H), 7.27 (dd, J = 8.3, 1.5 Hz, 2H), 7.19-7.23 (m, 3H), 7.13-7.15 (m, 4H), 6.77-6.80 (m, 4H), 5.98 (d, J = 4.1 Hz, 1H), 5.44 (t, J = 5.6 Hz, 1H), 4.74 (t, J = 4.9 Hz, 1H), 4.17 (d, J = 4.1 Hz, 1H), 3.72 (d, J = 6.1 Hz, 6H), 3.28 (s, 3H), 3.13-3.21 (m, 2H), 2.96 (s, 3H), 2.89-2.82 (3H), 1.82-1.78 (3H), 1.75 (s, 3H) ppm.

### (5) Synthesis of Compound 7

To a mixture of protected guanosine 6 (2.1 g, 2.4 mmol) in THF (17.8 mL) and water (8.9 mL) was added trifluoroacetic acid (12.3 L, 160 mmol). After stirring at room temperature for 5 hours, the reaction mixture was diluted with chloroform and washed with water, and with saturated aqueous sodium bicarbonate solution. The organic layer was concentrated and purified by silica gel column chromatography eluted with 7.4% methanol/chloroform, affording the target Compound 7 (1.1 g, 2.2 mmol, 92% yield).
¹H NMR (600 MHz, DMSO-d₆) δ 10.76 (s, 1H), 8.24 (dt, J = 9.5, 2.3 Hz, 2H), 8.08 (s, 1H), 7.66 (dt, J = 9.5, 2.3 Hz, 2H), 6.57 (s, 2H), 5.79 (d, J = 7.1 Hz, 1H), 5.17 (q, J = 2.3 Hz, 1H), 4.51 (dd, J = 7.3, 4.9 Hz, 1H), 4.12 (td, J = 4.0, 1.9 Hz, 1H), 3.56-3.61 (m, 2H), 3.18 (s, 3H), 1.82 (s, 3H), 1.75 (s, 3H) ppm

### (6) Synthesis of Compound 8

To a guanosine derivative (7) (290 mg, 570 µmol) was added a solution of bis(2-cyanoethyl) N,N-diisopropylphosphoramidite (230 mg, 850 µmol) in CH₃CN (5.70 mL). 1H-Tetrazole (60.0 mg, 850 µmol) was added to the mixture, followed by stirring at room temperature for 3 hours, and then, to the reaction mixture was added 5 to 6 M of tert-butyl hydroperoxide in a decane solution (320 µL, 1.6 to 1.9 mmol). The reaction mixture was further stirred at room temperature for 1 hour and diluted with dichloromethane. The mixture was washed with a saturated aqueous sodium bicarbonate solution. The organic layer was dried over Na2SO4 and concentrated. The residue was purified by silica gel column chromatography eluted with 9.1% methanol/chloroform, affording the objective Compound 8 (280 mg, 410 µmol, 72% yield).

¹H NMR (400 MHz, DMSO-d₆) δ 10.73 (s, 1H), 8.23-8.25 (m, 2H), 7.94 (s, 1H), 7.67 (dd, J = 7.0, 2.0 Hz, 2H), 6.53 (s, 2H), 5.83 (d, J = 7.2 Hz, 1H), 5.25 (q, J = 2.5 Hz, 1H), 4.54 (dd, J = 7.2, 5.2 Hz, 1H), 4.34-4.37 (m, 1H), 4.28-4.31 (m, 2H), 4.14-4.21 (m, 4H), 3.21-3.16 (3H), 2.90 (ddd, J = 16.4, 5.8, 0.9 Hz, 4H), 1.83 (s, 3H), 1.76 (s, 3H) ppm.
³¹P NMR (161 MHz, DMSO-d₆) δ -1.60 (s, 1P) ppm

### (7) Synthesis of Compound 9

To a solution of a nucleoside monophosphate derivative (8) (250 mg, 0.360 mmol) in acetonitrile (3.60 mL), N,O-bis(trimethylsilyl)acetamide (0.890 mL, 3.60 mmol) and 1,8-diazabicyclo[5.4.0]undec-7-ene (0.220 mL, 1.45 mmol) were added continuously. After stirring at room temperature for 1 hour, the reaction mixture was quenched by the addition of a 2M TEAB buffer (pH 7.7, 0.730 mL). The mixture was diluted with water and washed three times with chloroform. The aqueous layer was purified using DEAE Sephadex A-25, Φ = 4.5 cm, h = 8.4 cm (140 cm³), flow rate: 12 mL/min, solvent A: water, solvent B: 1.5 MTEAB buffer (pH 7.7, containing 10% CH₃CN). The fractions containing the objective product were collected and desalted with Wakosil (registered trademark) 25C18 (particle size: 15 to 30 µm (spherical), column size: ϕ = 1.3 cm, h = 4.0 cm, 21 cm³), solvent A: water, solvent B: methanol). The fractions containing the product were combined and concentrated, affording the target Compound 9 (221 mg, 0.310 mmol, yield 86%).

¹H NMR (400 MHz, D₂O) δ 8.25 (dd, J = 7.0, 2.0 Hz, 2H), 8.12 (s, 1H), 7.68 (dd, J = 7.1, 2.1 Hz, 2H), 5.84 (d, J = 7.2 Hz, 1H), 5.40 (dd, J = 5.0, 1.9 Hz, 1H), 4.66 (dd, J = 7.3, 5.0 Hz, 1H), 4.46 (d, J = 3.8 Hz, 1H), 4.04 (dd, J = 4.7, 3.4 Hz, 2H), 3.25 (s, 3H), 3.19 (q, J = 7.3 Hz, 8H), 1.90 (s, 3H), 1.81 (s, 3H), 1.27 (t, J = 7.3 Hz, 12H) ppm.

³¹P NMR (161 MHz, D₂O) δ 1.66 ppm.

### (8) Synthesis of Compound 10

Iodomethane (73.0 µL, 1.17 mmol) was added to a solution of a modified guanosine monophosphoric acid triethylammonium salt 9 (100 mg, 0.150 mmol) in DMSO (1.22 mL). After stirring overnight at room temperature, the reaction mixture was diluted with water and washed three times with chloroform. The aqueous layer was purified using DEAE Sephadex A-25, Φ = 3.0 cm, h = 10 cm (70 cm³), flow rate: 6 mL/min, solvent A: water, solvent B: 1.0 M TEAA buffer (pH 7.0, containing 10% CH₃CN). The fractions containing the objective product were collected and desalted with Wakosil (registered trademark) 25C18 (particle size: 15 to 30 um (spherical), column size: ϕ = 1.3 cm, h = 4.0 cm, 21 cm³), solvent A: water, solvent B: methanol). The fractions containing the product were combined and concentrated, affording the target Compound 10 (28.6 mg, 43.0 µmol, 33% yield).

¹H NMR (600 MHz, D₂O) δ 8.27 (d, J = 8.8 Hz, 2H), 7.69 (d, J = 8.5 Hz, 2H), 6.07 (d, J = 5.4 Hz, 1H), 5.32 (s, 1H), 4.55 (t, J = 5.1 Hz, 2H), 4.12 (s, 1H), 4.10 (s, 3H), 3.99 (d, J = 10.5 Hz, 1H), 3.34 (s, 3H), 3.19 (q, J = 7.4 Hz, 4H), 1.89 (s, 3H), 1.80 (s, 3H), 1.27 (t, J = 7.5 Hz, 6H) ppm.

³¹P NMR (243 MHz, D₂O) δ 2.49 (s, 1P) ppm

### (9) Synthesis of Compound 11

2-Nitroimidazole (10 mg, 90 µmol) was added to 2'-OMe-3'-Nb-N7m-guanosine monophosphate 10 (20 mg, 30 µmol) and GDP-imidazolide (48 mg, 90 µmol) in DMSO (300 µL). After stirring at 37°C for 2 days, the reaction mixture was diluted with water and washed with chloroform. The aqueous layer was purified using DEAE Sephadex A-25, Φ = 3.0 cm, h = 10 cm (70 cm³), flow rate: 6 mL/min, solvent A: water, solvent B: 1.0 M TEAA buffer (pH 7.0, containing 10% CH₃CN). The fractions containing the objective product were collected and desalted with Wakosil (registered trademark) 25C18 (particle size: 15 to 30 um (spherical), column size: ϕ = 1.3 cm, h = 4.0 cm, 21 cm³), solvent A: water, solvent B: methanol).The fractions containing the product were combined and concentrated, affording the target compound as a triethylammonium salt. An aqueous solution of the triethylammonium type as the objective compound was passed through a column filled with an H-type Dowex (registered trademark) 50W-X2 resin, and was added dropwise to a mixture of triethylamine (10 equivalents) and ethanol (0.5 M triethylamine). After washing with water until the pH of the eluate was neutral, the mixture was concentrated and freeze-dried, affording the target Compound 11 in a sodium form (11.4 mg, 11.0 µmol, 37% yield). The yield was calculated using the absorbance of the product at 260 nm measured by NanoDrop. For the calculation, an absorption coefficient (ε₂₆₀) = 28,293 M⁻¹·cm⁻¹ was used.

¹H NMR (600 MHz, D₂O) δ 8.25 (d, J = 9.0 Hz, 2H), 7.94 (s, 1H), 7.67 (d, J = 9.0 Hz, 2H), 5.86 (d, J = 5.4 Hz, 1H), 5.73 (d, J = 6.1 Hz, 1H), 5.24 (dd, J = 4.9, 3.6 Hz, 1H), 4.62 (t, J = 5.6 Hz, 1H), 4.52 (t, J = 2.8 Hz, 1H), 4.43 (dd, J = 5.2, 3.6 Hz, 1H), 4.38 (t, J = 5.3 Hz, 1H), 4.31-4.35 (m, 1H), 4.28 (t, J = 4.2 Hz, 1H), 4.23-4.25 (m, 1H), 4.21 (t, J = 2.9 Hz, 1H), 4.17-4.19 (m, 1H), 4.07 (s, 3H), 3.29 (s, 3H), 1.88 (s, 3H), 1.79 (d, J = 7.6 Hz, 3H) ppm.
³¹P NMR (161 MHz, D₂O) δ -10.97 (t, J = 23.3 Hz, 2P), -22.56 (t, J = 21.2 Hz, 1P) ppm

### 13. Synthesis of novel nitrobenzyl-modified nucleotide phosphorimidazolide to be used in chemical capping

The following shows the structure of the novel nitrobenzyl-modified nucleotide phosphorimidazolide compound.

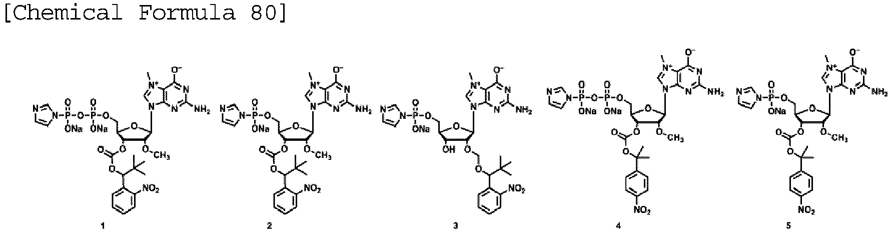

### (1) Synthesis of Compound 1

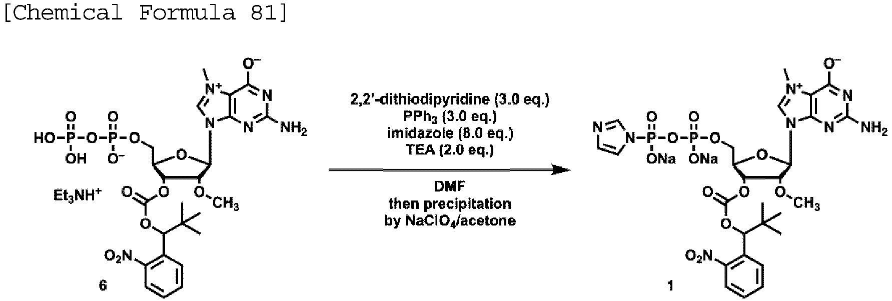

To a solution (1.08 mL) of N7-methyl-guanosine-5'-diphosphate 6 (73.3 mg, 90.8 µmol), imidazole (49.4 mg, 726 µmol), and 2,2'-dithiodipyridine (59.9 mg, 272 µmol) in N,N-dimethylformamide was added triethylamine (25.4 µL, 18.4 mg, 182 µmol), and subsequently, triphenylphosphine (71.3 mg, 272 µmol) was added. After stirring at room temperature for 23 hours, a solution of sodium perchlorate (66.7 mg, 545 µmol) in 4% triethylamine/dry acetone (27.3 mL) was added dropwise while stirring the reaction mixture. The resulting suspension was centrifuged at 4,000 rpm for 20 minutes. The supernatant was discarded and the precipitate was rinsed four times with dry acetone. The solid was dried under reduced pressure, affording N7-methyl-guanosine 5'-diphosphorimidazolide disodium salt 1 (65.0 mg, 89.4% yield) as a white solid.

¹H NMR (600 MHz, DMSO-d₆) δ 9.82 (d, J = 16.7 Hz, 1H), 7.98 (dd, J = 8.4, 3.9 Hz, 1H), 7.81 (q, J = 7.8 Hz, 1H), 7.69 (d, J = 11.5 Hz, 1.5H), 7.62 (q, J = 8.1 Hz, 1.5H), 7.17 (s, 1H), 6.78 (d, J = 6.5 Hz, 1H), 6.25 (s, 0.5H), 6.10 (s, 0.5H), 5.95 (dd, J = 14.7, 6.2 Hz, 1H), 5.36-5.23 (m, 1H), 4.76 (t, J = 4.9 Hz, 0.2H), 4.66-4.55 (m, 0.8H), 4.41-4.31 (m, 1H), 4.02 (d, J = 6.3 Hz, 3H), 3.92-3.75 (m, 2H), 3.45 (s, 1.5H), 3.00 (s, 1.5H), 0.92 (d, J = 16.3 Hz, 9H) ppm.

¹³C NMR (151 MHz, DMSO-d₆) δ 160.96, 153.28, 153.19, 149.91, 149.08, 148.90, 139.29, 134.79, 133.15, 131.57, 130.98, 129.62, 129.46, 129.12, 128.41, 127.94, 127.88, 124.61, 124.52, 120.14, 107.50, 85.74, 82.94, 82.08, 81.78, 79.82, 79.51, 75.71, 75.46, 68.54, 63.74, 58.67, 58.35, 55.85, 36.07, 35.67, 35.44, 32.14, 30.72, 29.62, 29.57, 25.53, 25.26 ppm.

³¹P NMR (243 MHz, DMSO-d₆) δ -10.50 (d, 1P), -19.02 (d, 1P) ppm.

### (2) Synthesis of Compound 2

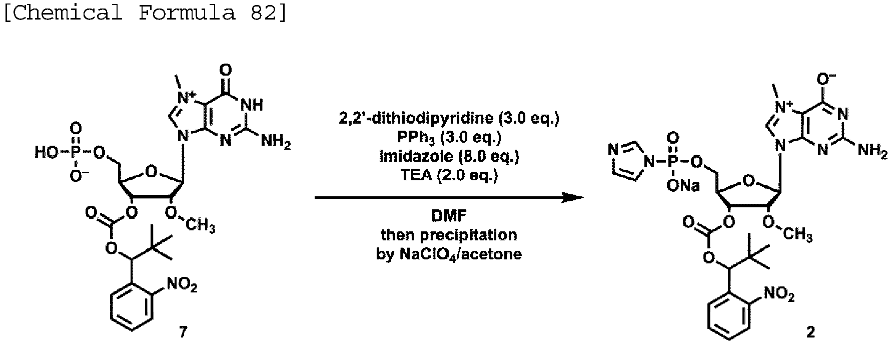

To a solution (233 µL) of N7-methyl-guanosine-5'-monophosphate 7 (12.3 mg, 19.6 µmol), imidazole (10.7 mg, 157 µmol), and 2,2'-dithiodipyridine (13.0 mg, 58.8 µmol) in N,N-dimethylformamide was added triethylamine (5.48 µL, 3.97 mg, 39.2 µmol), and subsequently, triphenylphosphine (15.4 mg, 58.8 µmol) was added. After stirring at room temperature for 23 hours, a solution of sodium perchlorate (14.4 mg, 118 µmol) in 4% triethylamine/dry acetone (5.90 mL) was added dropwise while stirring the reaction mixture. The resulting suspension was centrifuged at 4,000 rpm for 20 minutes. The supernatant was discarded and the precipitate was rinsed five times with dry acetone. The solid was dried under reduced pressure, affording N7-methyl-guanosine 5'-diphosphorimidazolide disodium salt 2 (11.6 mg, 85.0% yield) as a white solid.

¹H NMR (600 MHz, DMSO-d₆) δ 9.47-9.31 (m, 1H), 7.94 (d, J = 8.2 Hz, 1H), 7.77 (dt, J = 15.4, 7.8 Hz, 1H), 7.69 (d, J = 9.9 Hz, 1H), 7.65-7.51 (m, 3H), 7.14-7.00 (m, 1H), 6.87-6.77 (m, 1H), 6.18 (s, 0.5H), 6.03 (s, 0.5H), 5.91 (dd, J = 16.4, 6.0 Hz, 1H), 5.13-5.02 (m, 1H), 4.54 (d, J = 11.6 Hz, 1H), 4.41 (t, J = 5.3 Hz, 1H), 4.32-4.21 (m, 1H), 4.13-4.05 (m, 1H), 4.01-3.95 (m, 3H), 3.72 (s, 1H), 3.37 (s, 1.5H), 2.92 (s, 1.5H), 0.92-0.82 (2s, 6H), 0.75-0.62 (2s, 3H) ppm.

¹³C NMR (151 MHz, DMSO-d₆) δ 161.78, 153.56, 149.58, 139.69, 133.64, 130.14, 129.04, 128.92, 125.03, 120.34, 108.52, 83.59, 81.93, 81.65, 80.36, 69.05, 59.28, 58.50, 56.37, 36.56, 36.16, 35.90, 32.65, 31.24, 30.14, 26.03, 25.92, 25.76 ppm.

³¹P NMR (243 MHz, DMSO-d₆) δ -9.63 ppm.

### (3) Synthesis of Compound 3

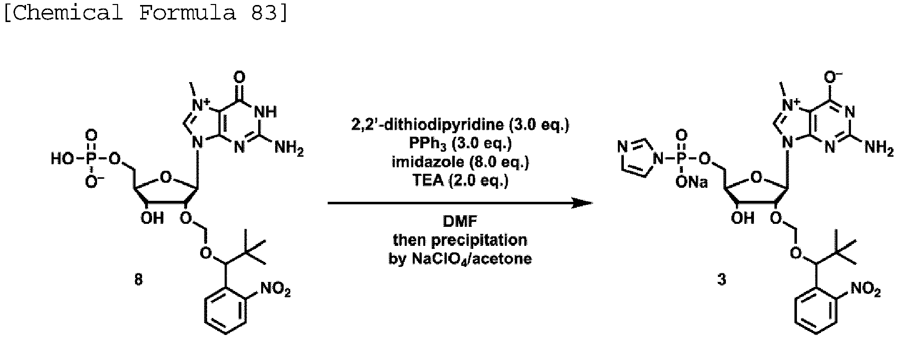

Triethylamine (9.00 µL, 6.50 mg, 64.2 µmol) was added to a solution of N7-methyl-guanosine-5'-monophosphate 8 (19.2 mg, 32.1 µmol), imidazole (17.5 mg, 257 µmol), and 2,2'-dithiodipyridine (21.2 mg, 96.3 µmol) in N,N-dimethylformamide (382 µL), and subsequently, triphenylphosphine (25.3 mg, 96.3 µmol) was added. After stirring at room temperature for 16.5 hours, a solution of sodium perchlorate (23.6 mg, 193 µmol) in 4% triethylamine/dry acetone (9.65 mL) was added dropwise while stirring the reaction mixture. The resulting suspension was centrifuged at 4,500 rpm for 20 minutes. The supernatant was discarded and the precipitate was rinsed four times with dry acetone. The solid was dried under reduced pressure, affording N7-methyl-guanosine 5'-monophosphorimidazolide monosodium salt 3 (18.5 mg, 86.0% yield) as a pale yellow solid.

¹H NMR (600 MHz, DMSO-d₆) δ 9.50 (s, 0.5H), 9.28 (s, 0.5H), 7.80 (dd, J = 8.1, 1.4 Hz, 0.5H), 7.73-7.62 (m, 1H), 7.60 (dt, J = 9.5, 1.1 Hz, 1.5H), 7.60-7.49 (m, 2.5H), 7.50 (ddd, J = 8.5, 7.4, 1.5 Hz, 1H), 7.39 (ddd, J = 8.4, 6.6, 2.1 Hz, 0.5H), 7.07 (dq, J = 17.3, 1.4 Hz, 1H), 6.82 (dq, J = 9.4, 1.3 Hz, 1H), 6.46 (s, 2.5H), 5.95 (d, J = 5.4 Hz, 0.5H), 5.72 (d, J = 4.3 Hz, 0.5H), 5.41 (d, J = 5.9 Hz, 0.5H), 5.34 (d, J = 4.8 Hz, 0.5H), 4.99 (s, 0.5H), 4.87-4.78 (m, 1H), 4.73-4.61 (m, 1.5H), 4.45 (d, J = 7.1 Hz, 0.5H), 4.41 (t, J = 4.5 Hz, 0.5H), 4.17 (q, J = 5.0 Hz, 0.5H), 4.08-3.85 (m, 6.5H), 3.74-3.62 (m, 1H), 0.70 (s, 4H), 0.62 (s, 5H) ppm.

³¹P NMR (243 MHz, DMSO-d₆) δ -9.53, -9.57 ppm.

HRESIMS calcd. for C₂₆H₃₂N₈O₁₀P, 647.1984 [M-H]-; found 647.2036.

The following shows a synthesis scheme of a nitrobenzyl-modified nucleotide phosphorimidazolide compound that can be removed by reduction conditions.

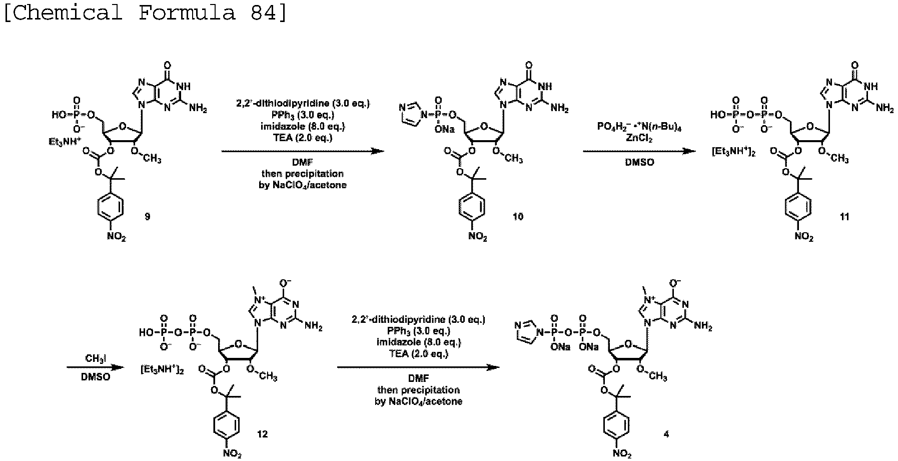

### (4) Synthesis of Compound 10

Triethylamine (41 µL, 70.29 mmol) was added to a solution of 3'-Nb-guanosine-5'-monophosphoric acid triethylammonium salt 9 (85 mg, 0.14 mmol), imidazole (79 mg, 1.16 mmol) and 2,2'-dithiodipyridine (96 mg, 0.47 mmol) in N,N-dimethylformamide (1.45 mL), and subsequently, triphenylphosphine (110 mg, 0.47 mmol) was added. After stirring at room temperature overnight, a solution of sodium perchlorate (110 mg, 0.87 mmol) in 4% triethylamine/dry acetone (44 mL) was added dropwise while stirring the reaction mixture. The suspension was centrifuged at 4,500 rpm for 20 minutes. The supernatant was discarded and the precipitate was resuspended in acetone. The suspension centrifugation process was repeated three more times. The solid was dried under reduced pressure, affording 3'-Nb-guanosine 5'-monophosphorphosphoimidazolide disodium salt 10 (70 mg, 0.11 mmol, yield of 79%).

¹H NMR (600 MHz, DMSO-d₆) δ 10.71 (s, 1H), 8.23 (d, J = 9.2 Hz, 2H), 8.00 (s, 1H), 7.66 (d, J = 2.0 Hz, 1H), 7.63-7.65 (m, 2H), 7.08 (s, 1H), 6.85 (s, 1H), 6.67 (s, 2H), 5.75-5.76 (m, 1H), 5.08 (dd, J = 4.8, 1.0 Hz, 1H), 4.60 (dd, J = 7.5, 4.8 Hz, 1H), 4.13 (t, J = 4.1 Hz, 1H), 3.73-3.86 (m, 2H), 3.12 (s, 3H), 1.81 (s, 3H), 1.74 (s, 3H) ppm.

³¹P NMR (243 MHz, DMSO-d₆) δ -9.90 ppm.

### (5) Synthesis of Compound 11

Triethylamine (58 µL, 0.41 mmol), zinc chloride (56 mg, 0.41 mmol), and a solution of tetrabutylammonium dihydrogen phosphate in CH₃CN (0.1 M, 5.1 mL, 0.51 mmol) were added continuously to a solution of 3'-Nb-guanosine-monophosphrphosphoimidazolide 10 (34 mg, 51 µmol) in DMSO (0.51 mL). After stirring at room temperature for 2 days, the reaction mixture was quenched by the addition of 50 mM EDTA, and diluted with water. The mixture was washed with chloroform, and the aqueous layer was purified using DEAE Sephadex A-25, Φ = 4.5 cm, h = 8.4 cm (140 cm³), flow rate: 12 mL/min, solvent A: water, solvent B: 1.5 M TEAB buffer (pH 7.7, containing 10% CH₃CN). The fractions containing the objective product were collected and desalted with Wakosil (registered trademark) 25C18 (particle size: 15 to 30 µm (spherical), column size: ϕ = 1.3 cm, h = 4.0 cm, 21 cm³), solvent A: water, solvent B: methanol). The fractions containing the product were combined and concentrated, affording the target compound as triethylammonium salt 11 (20 mg, 23 µmol, 46% yield).

¹H NMR (600 MHz, D₂O) δ 8.19 (s, 1H), 8.16 (d, J = 8.8 Hz, 2H), 7.62 (d, J = 8.8 Hz, 2H), 5.81 (d, J = 7.5 Hz, 1H), 5.41 (dd, J = 4.9, 1.5 Hz, 1H), 4.63 (dd, J = 7.3, 4.9 Hz, 1H), 4.48 (s, 1H), 4.16 (d, J = 3.7 Hz, 2H), 3.22 (s, 3H), 3.16 (q, J = 7.4 Hz, 12H), 1.86 (s, 3H), 1.76 (s, 3H), 1.24 (t, J = 7.5 Hz, 18H) ppm.

³¹P NMR (241 MHz, D₂O) δ -10.19 (s, 1P), -10.92 (d, J = 19.7 Hz, 1P) ppm.

### (6) Synthesis of Compound 12

Iodomethane (43 µL, 0.69 mmol) was added to a solution of modified guanosine diphosphoric acid triethylammonium salt 11 (60 mg, 69 µmol) in DMSO (0.58 mL). After stirring overnight at room temperature, the reaction mixture was diluted with water and washed three times with chloroform. The aqueous layer was purified using QAE Sephadex A-25, Φ = 3.0 cm, h = 10 cm (70 cm³), flow rate: 6 mL/min, solvent A: water, solvent B: 1.0 M TEAA buffer (pH 7.0, containing 10% CH₃CN). The fractions containing the objective product were collected and desalted with Wakosil (registered trademark) 25C18 (particle size: 15 to 30 um (spherical), column size: ϕ = 1.3 cm, h = 4.0 cm, 21 cm³), solvent A: water, solvent B: methanol). The fractions containing the product were combined and concentrated, affording the target Compound 12 (30.5 mg, 39.0 µmol, 56% yield).

¹H NMR (400 MHz, D₂O) δ 8.26 (d, J = 9.0 Hz, 2H), 7.68 (d, J = 9.0 Hz, 2H), 6.06 (d, J = 5.4 Hz, 1H), 5.34 (dd, J = 4.8, 3.3 Hz, 1H), 4.58 (d, J = 3.8 Hz, 1H), 4.56 (d, J = 5.2 Hz, 1H), 4.14-4.30 (m, 2H), 4.10 (s, 3H), 3.34 (s, 3H), 3.19 (q, J = 7.3 Hz, 7H), 1.89 (s, 3H), 1.79 (s, 3H), 1.26 (t, J = 7.3 Hz, 10H) ppm.

³¹P NMR (161 MHz, D₂O) δ -9.96 (d, J = 21.2 Hz, 1P), -10.84 (d, J = 21.2 Hz, 1P) ppm.

### (7) Synthesis of Compound 4

Triethylamine (6.6 µL, 47 µmol) was added to 3'-Nb-N7-methyl-guanosine-5'-diphosphoric acid triethylammonium salt 12 (19 mg, 24 µmol), imidazole (13 mg, 0.19 mmol), and 2,2'-dithiodipyridine (16 mg, 71 µmol) in N, N-dimethylformamide (0.24 mL), and subsequently, triphenylphosphine (19 mg, 71 µmol) was added. After stirring at room temperature overnight, a solution of sodium perchlorate (17 mg, 71 µmol) in 4% triethylamine/dry acetone (7.1 mL) was added dropwise while stirring the reaction mixture. The suspension was centrifuged at 4,500 rpm for 20 minutes. The supernatant was discarded and the precipitate was resuspended in acetone. The suspension centrifugation process was repeated three more times. The solid was dried under reduced pressure, affording 3'-Nb-N7-methyl-guanosine 5'-diphosphorphosphoimidazolide disodium salt 4 (20 mg, 24 µmol, quantitative).

¹H NMR (600 MHz, DMSO-d₆) δ 9.78 (s, 1H), 8.24 (d, J = 8.8 Hz, 2H), 7.71 (s, 1H), 7.68 (d, J = 8.8 Hz, 2H), 7.17 (s, 1H), 6.85 (s, 2H), 6.80 (s, 1H), 5.97 (d, J = 5.8 Hz, 1H), 5.17-5.19 (m, 1H), 4.60 (t, J = 5.3 Hz, 1H), 4.34 (s, 1H), 4.02 (s, 3H), 3.77-3.92 (m, 2H), 3.28 (s, 3H), 1.83 (s, 3H), 1.76 (s, 3H) ppm.

³¹P NMR (243 MHz, DMSO-d₆) δ -10.44 (d, J = 21.2 Hz, 1P), - 18.99 (d, J = 21.2 Hz, 1P) ppm.

### (7) Synthesis of Compound 5

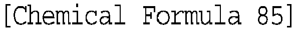

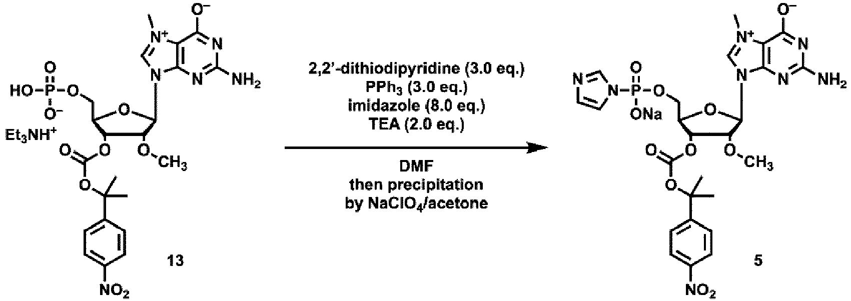

Triethylamine (10 µL, 72 µmol) was added to a solution of 3'-Nb-N7-methyl-guanosine-5'-monophosphoric acid triethylammonium salt 13 (22 mg, 36 µmol), imidazole (20 mg, 0.11 mmol), and 2,2'-dithiodipyridine (24 mg, 0.11 mmol) in N,N-dimethylformamide (0.37 mL), and subsequently, triphenylphosphine (29 mg, 0.11 mmol) was added. After stirring at room temperature overnight, a solution of sodium perchlorate (27 mg, 0.22 mmol) in 4% triethylamine/dry acetone (11 mL) was added dropwise while stirring the reaction mixture. The suspension was centrifuged at 4,500 rpm for 20 minutes. The supernatant was discarded and the precipitate was resuspended in acetone. The suspension centrifugation process was repeated three more times. The solid was dried under reduced pressure, affording 3'-Nb-N7-methyl-guanosine 5'-monophosphorphosphoimidazolide disodium salt 5 (21.4 mg, 32 µmol, 89% yield).

¹H NMR (600 MHz, DMSO-d₆) δ 9.42 (s, 1H), 8.23 (d, J = 8.8 Hz, 2H), 7.65 (d, J = 8.5 Hz, 3H), 7.12-7.09 (1H), 6.86 (s, 1H), 6.71 (s, 2H), 5.97 (d, J = 5.4 Hz, 1H), 5.02 (t, J = 4.1 Hz, 1H), 4.50 (d, J = 10.2 Hz, 1H), 4.29 (d, J = 3.4 Hz, 1H), 4.03 (s, 3H), 3.99 (dd, J = 7.1, 4.4 Hz, 1H), 3.76-3.79 (m, 1H), 3.26-3.24 (3H), 1.81 (s, 3H), 1.74 (s, 3H) ppm.

³¹P NMR (243 MHz, DMSO-d₆) δ -9.64 (s, 1P) ppm.

### 14. Synthesis of trinucleotide/tetranucleotide-type cap compound

The following shows the structure of a nitrobenzyl-modified trinucleotide-type cap compound.

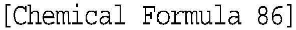

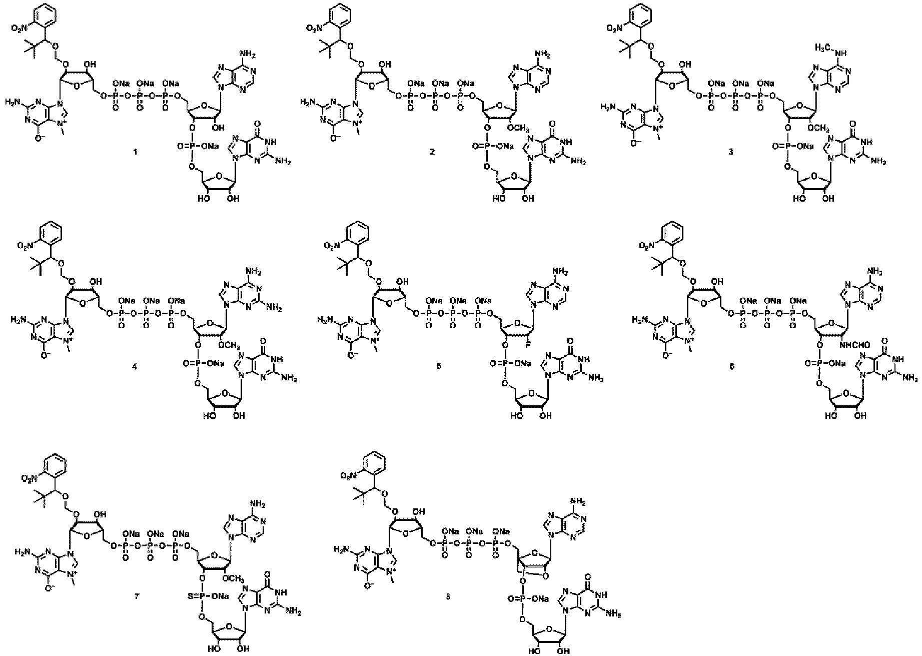

The following shows the structure of a nitrobenzyl-modified tetranucleotide-type cap compound.

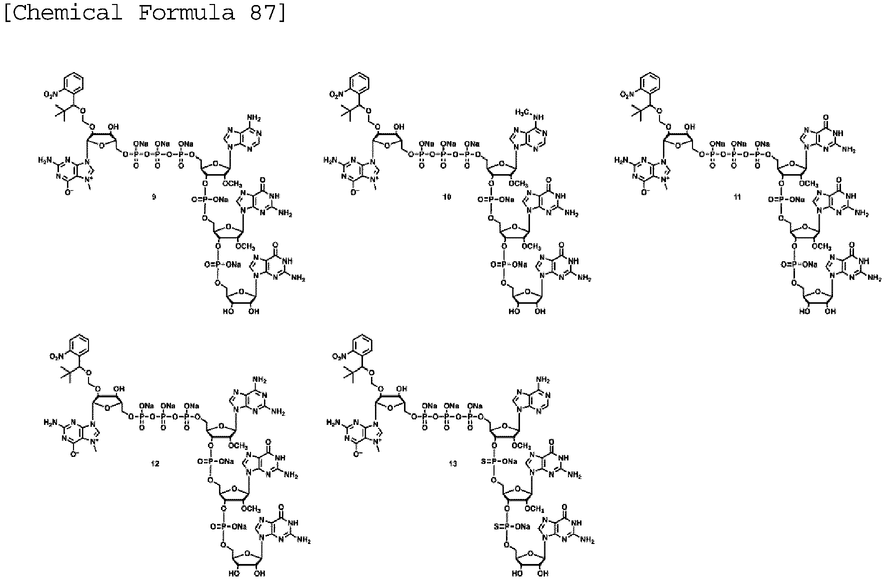

The following shows the structure of dinucleotide/trinucleotide required for the synthesis of the nitrobenzyl-modified trinucleotide/tetranucleotide-type cap compound.

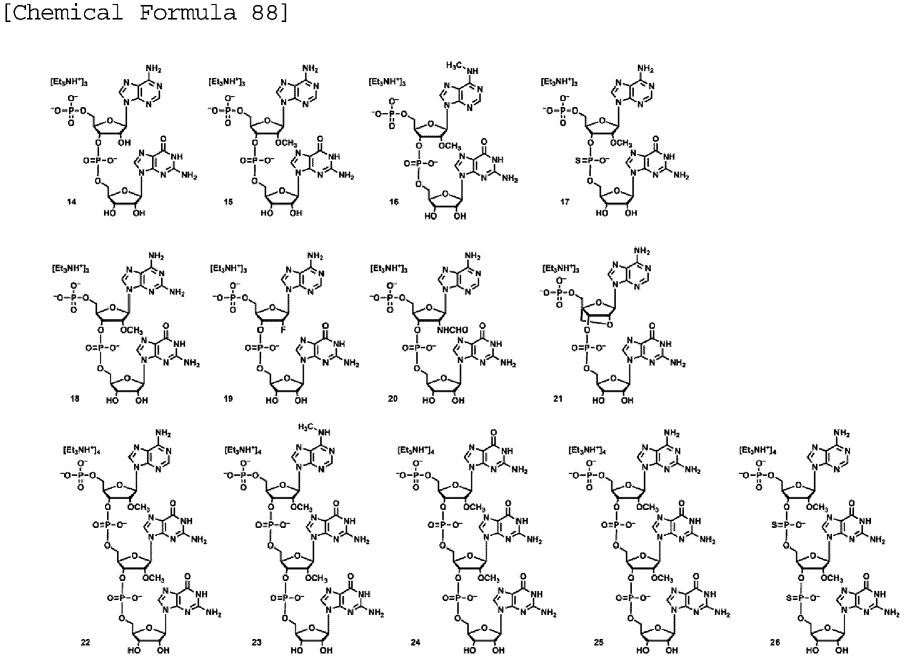

### (1) Synthesis of dinucleotide/trinucleotide (Compounds 14 to 26)

The following shows the synthesis scheme of dinucleotides/trinucleotides (Compounds 14 to 26). R includes various chemical modifications.

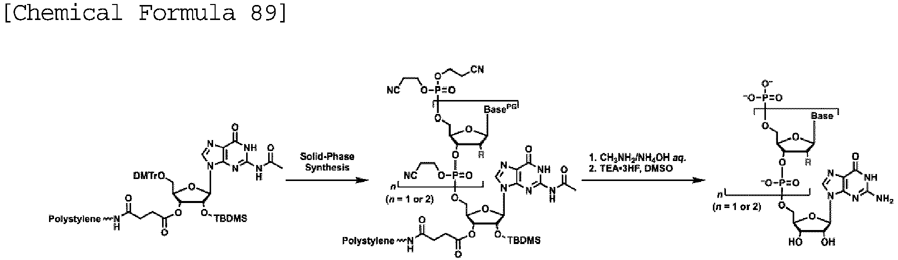

Synthesis of dinucleotides and trinucleotides was performed using a DNA/RNA synthesizer NR-2A_7MX (Nihon Techno Service Co., Ltd., Ushiku, Ibaraki, Japan) with Primer Support 5G ribo G 300 (Cytiva, synthesis in 15.0 µmol scale). In the coupling step, 100 mM 2'-O-methyl-adenosine (n-benzoyl)-CE-phosphoramidite (ChemGenes), 100 mM 2'-O-methyl-N6-methyl-adenosine (n-benzoyl)-CE-phosphoramidite (synthesized in accordance with literature: Nucleic acid res., 2020, 48(4), 1607-1626) in CH₃CN, 100 mM 2'-O-methyl-Guanosine (n-i-Bu)-CE-phosphoramidite (ChemGenes) in CH₃CN, 10 mM 2'-F-adenosine (n-benzoyl)-CE-phosphoramidite (ChemGenes) in CH₃CN, 100 mM LNA (MBN) adenosine (n-benzoyl)-CE-phosphoramidite (ChemGenes) in CH₃CN, 100 mM 2'-formamide-adenosine (n-benzoyl)-CE-phosphoramidite in CH₃CN, 100 mM 2'-OMe-2,6-diaminopurine-CE-phosphoramidite (Glen Research) in CH₃CN, 50 to 70 mM 5'-DMTr-2'-TOM-ribo Adenosine (n-acetyl) OP (ChemGenes) in CH₃CN, 150 mM bis(2-cyanoethyl)-N,N-diisopropylphosphoramidite in CH₃CN, and 0.3 M 5-(benzylthio)-1H-tetrazole (activator) in CH₃CN were circulated through a column. A solution of 184 mM trichloroacetic acid/dichloromethane was used as a detritylation reagent, and 0.05 M 12 in pyridine/H2O (9:1, v/v), 10% Ac2O in THF/pyridine (8:1, v/v) as Cap A, and 10% 1-methylimidazole in THF as Cap B were used for oxidation. The synthesis was performed by applying a standard RNA synthesis protocol in a 10 µmol scale. After the last cycle of the synthesis, a solid support was treated with a 1:1 mixture of 28% ammonium hydroxide/40% aqueous methylamine solution at 65°C for 1 to 2 hours (1.00 mL/15.0 µmol of solid support), whereby the nucleotide was cleaved from the solid support and the protecting group was removed. The resulting nucleotide was concentrated and dried up, and then re-dissolved in DMSO (1.00 mL/60.0 µmol). To this solution was added TEA·3HF (1.00 mL/60.0 µmol), and the mixture was incubated at 65°C for 3 to 5 hours to remove a 2'-O-silyl protecting group. The reaction mixture was neutralized by adding a 250 mM aqueous Na2CO3 solution (10.0 mL/60.0 µmol). The crude product was purified by DEAE-Sephadex (ϕ = 4.5 cm, h = 8.4 cm, 140 cm³, 12.0 mL/min, solvent: 0 to 8 M TEAB buffer (pH 7.9, containing 0 to 8% CH₃CN)). The fractions containing the product were collected and concentrated, affording di-/tri-nucleotides. The yield was calculated using the absorbance of the product at 260 nm measured by NanoDrop. For the calculations, the absorption coefficient (ε260) = 25,000 or 18,100 M⁻¹·cm⁻¹ (for dinucleotides), and 35,100, 31,700 or 28,200 M⁻¹·cm⁻¹ (for trinucleotides) were used.

### (2) Method for synthesis of trinucleotide-type cap compound (Compounds 1 to 8)

The following shows a synthesis scheme of a trinucleotide-type cap compound (Compounds 1 to 8). R₁ and R₂ include various chemical modifications.

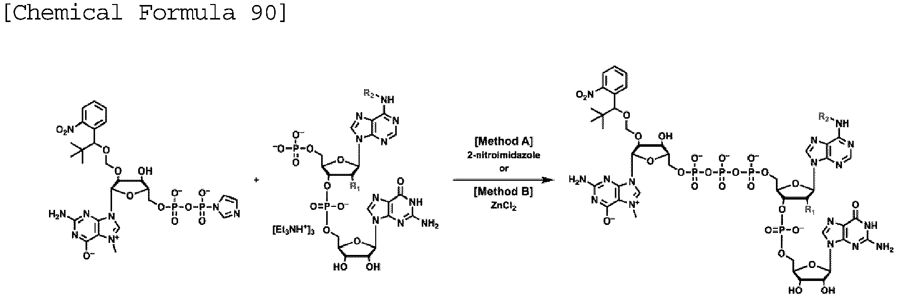

### (3) Method for synthesis of tetranucleotide-type cap compound (Compounds 9 to 13)

The following shows a synthesis scheme of a tetranucleotide-type cap compound (Compounds 9 to 13). R₁ and R₂ include various chemical modifications.

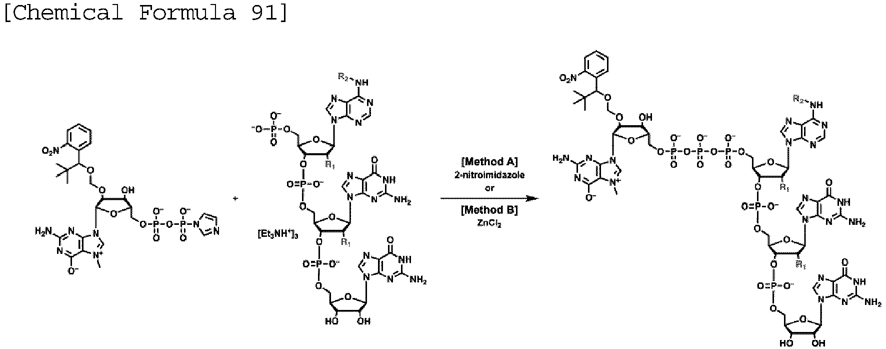

### (4) Synthesis of trinucleotide/tetranucleotide-type cap compounds (Method A)

2'-Nb-N7-methylguanosine diphosphorimidazolide disodium salt (2.00 equiv.) and 2-nitroimidazole (3.00 equiv.) were added to a solution of di-/trinucleotide triethylammonium salt (17.2 to 43.6 µmol) in DMSO (79.5 mM). After incubation at 37°C for 1 to 2 days, 2'-Nb-N7-methylguanosine diphosphorimidazolide disodium salt (2.00 equiv.) and 2-nitroimidazole (3.00 equiv.) were added. The mixture was incubated at 37°C for 2 to 3 days, and the reaction mixture was diluted with 18.5 to 38.6-fold amount of water. The crude product was purified by ion exchange chromatography on QAE-Sephadex (ϕ = 3.0 cm, h = 10 cm, 70 cm³, 6 mL/min). The fractions containing the product were collected and concentrated. The product was desalted with Wakosil (registered trademark) 25C18 (particle size: 15 to 30 um (spherical), column size: ϕ = 4.80 cm, h = 10.2 cm, 184 cm³ (12 mL/min)). The fractions containing the product were concentrated, affording the objective tri/tetranucleotide cap analog in the form of a triethylammonium salt. The product was re-dissolved in methanol (2.0 mL). A 190 mM NaClO₄ (12 mL) solution in acetone was added to the mixture, and the resulting suspension was centrifuged (4,500 rpm, 20 min). The supernatant was discarded and the precipitate was resuspended in acetone. The suspension centrifugation process was repeated three or four more times. The precipitate was dried under reduced pressure, affording the target tri-/tetranucleotide cap analog as a sodium salt. The yield was calculated using the absorbance of the product at 260 nm measured by NanoDrop. For the calculations, the absorption coefficient (ε260) = 40,100 or 33,200 M⁻¹·cm⁻¹ (for trinucleotide cap analogs) and 50,200, 46,800 or 43,300 M⁻¹·cm⁻¹ (for tetranucleotide cap analogs) were used.

### (5) Synthesis of trinucleotide-tetranucleotide-type cap compound (Method B)

2'-Nb-N7-methylguanosine diphosphorimidazolide disodium salt (3.00 equiv.) and zinc chloride (20.0 equiv.) were added to a solution of di-/trinucleotide triethylammonium salt (up to 106 µmol) in DMSO (50 mM). After stirring at 37°C for 3 days, a 500 mM aqueous EDTA solution (pH 8.0, EDTA: 1.30 equiv. per molar amount of ZnCl₂) was added to quench the reaction mixture. The mixture was diluted with 5.24 volumes of water, and purified by reverse-phase HPLC. The fractions containing the target compound were collected, concentrated, and freeze-dried, affording the target tri/tetranucleotide cap analog in the form of a triethylammonium salt. The product was re-dissolved in methanol (2.00 mL). A 190 mM NaClO₄ (24.0 mL) solution in acetone was added to the mixture, and the resulting suspension was centrifuged (4,000 rpm, 20 min). The supernatant was discarded and the precipitate was resuspended in acetone. The suspension centrifugation process was repeated three or four more times. The precipitate was dried under reduced pressure, affording the target tri-/tetranucleotide cap analog as a sodium salt. The yield was calculated using the absorbance of the product at 260 nm measured by NanoDrop. For the calculations, the absorption coefficient (ε260) = 40,100 or 33,200 M⁻¹·cm⁻¹ (for trinucleotide cap analogs) and 50,200, 46,800 or 43,300 M⁻¹·cm⁻¹ (for tetranucleotide cap analogs) were used.

### (6) Synthesis of trinucleotide type cap compound Nb-^{m7}GpppApG (Method A)

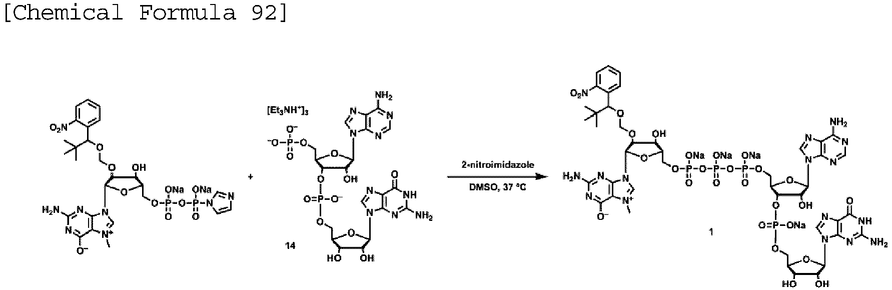

2'-Nitrobenzyl-modified N7-methylguanosine diphosphorimidazolide disodium salt (40.6 mg, 52.6 µmol) and 2-nitroimidazole (8.92 mg, 78.9 µmol) were added to a solution of dinucleotide triethylammonium salt 14 (26.2 mg, 26.3 µmol) in DMSO (331 µL). After incubation at 37°C for 1 day, 2'-nitrobenzyl-modified N7-methylguanosine diphosphorimidazolide disodium salt (40.6 mg, 52.6 µmol) and 2-nitroimidazole (8.92 mg, 78.9 µmol) were added. The mixture was incubated at 37°C for 3 days and the reaction mixture was diluted with water (10.0 mL). The crude product was purified by ion exchange chromatography on QAE-Sephadex (ϕ = 3.0 cm, h = 10 cm, 70 cm³, 6 mL/min) . The fractions containing the product were collected and concentrated. The product was desalted with Wakosil (registered trademark) 25C18 (particle size: 15 to 30 um (spherical), column size: ϕ = 4.80 cm, h = 10.2 cm, 184 cm³ (12 mL/min) . The fractions containing the product were concentrated, affording the target compound as a triethylammonium salt. The product was re-dissolved in methanol (2.0 mL). To the mixture was added 190 mM NaClO₄ (12 mL) in acetone, and the resulting suspension was centrifuged (4,500 rpm, 20 minutes) . The supernatant was discarded and the precipitate was resuspended in acetone. The suspension centrifugation process was repeated four more times. The precipitate was dried under reduced pressure, affording the target Cap analog 1 (23.6 mg, 16.4 µmol, 62.3% yield) as a sodium salt. The yield was calculated using the absorbance of the product at 260 nm measured by NanoDrop. For the calculation, an absorption coefficient (ε260) = 40,100 M⁻¹·cm⁻¹ was used.

¹H NMR (600 MHz, D₂O) δ 8.36 (s, 1H), 8.14-7.85 (m, 2H), 7.62-7.36 (m, 4H), 7.22 (s, 1H), 5.98-5.87 (m, 1.5H), 5.77 (s, 1H), 5.71 (s, 0.5H), 5.04-4.83 (m, 3H), 4.71 (s, 3H), 4.57 (s, 1H), 4.49 (d, J = 14.2 Hz, 3H), 4.39-4.21 (m, 10H), 4.17 (s, 1H), 4.10 (t, J = 7.0 Hz, 1H), 4.05-3.96 (m, 3H), 0.49 (s, 9H) ppm.

¹³C NMR (151 MHz, D₂O) δ 158.30, 155.43, 155.24, 154.54, 154.35, 153.63, 151.85, 151.26, 149.67, 149.25, 149.06, 148.85, 148.72, 148.50, 139.66, 137.26, 133.59, 132.70, 132.51, 132.21, 129.97, 129.80, 128.71, 128.09, 123.75, 123.34, 118.19, 116.01, 107.57, 107.29, 94.61, 93.70, 87.76, 87.40, 87.10, 86.99, 85.08, 84.43, 83.41, 82.79, 81.24, 80.16, 79.56, 79.10, 74.53, 73.82, 70.19, 68.48, 68.13, 65.21, 64.98, 64.71, 64.37, 61.76, 48.96, 36.24, 36.20, 36.15, 35.68, 30.31, 25.10, 24.95, 24.84, 20.58, 13.32, 8.30 ppm.

³¹P NMR (243 MHz, D₂O) δ 0.07 (1P), -10.70 (2P), -21.98 (1P) ppm.

### (7) Synthesis of trinucleotide type cap compound Nb-^{m7}GpppA(2'OCH₃)pG (Method A)

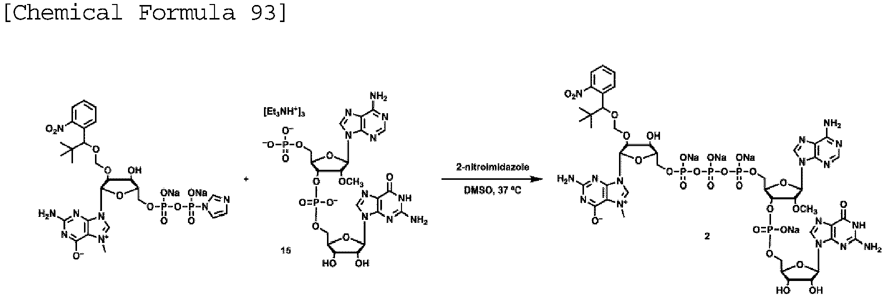

2'-Nitrobenzyl-modified N7-methylguanosine diphosphorimidazolide disodium salt (26.6 mg, 34.4 µmol) and 2-nitroimidazole (5.83 mg, 51.6 µmol) were added to a solution of 2'-O-methyl-modified dinucleotide triethylammonium salt 15 (17.4 mg, 17.2 µmol) in DMSO (216 µL). After incubating at 37°C for 2 days, the reaction mixture was diluted with water (4.00 mL). The crude product was purified by ion exchange chromatography on DEAE-Sephadex (ϕ = 4.5 cm, h = 8.4 cm, 140 cm³, 12 mL/min) . % CH₃CN (270 min). The fractions containing the product were collected and concentrated. After removal of CH₃CN under reduced pressure, the product was desalted with Wakosil (registered trademark) 25C18 (particle size: 15 to 30 um (spherical), column size: ϕ = 4.80 cm, h = 10.2 cm, 184 cm³ (12 mL/min)). The fractions containing the product were concentrated, affording the objective compound as a triethylammonium salt. The product was re-dissolved in methanol (2.0 mL). To the mixture was added 190 mM NaClO₄ (12 mL) in acetone, and the resulting suspension was centrifuged (4,500 rpm, 20 minutes). The supernatant was discarded and the precipitate was resuspended in acetone. The suspension centrifugation process was repeated four more times. The precipitate was dried under reduced pressure, affording the target Cap analog 2 (8.02 mg, 5.51 µmol, 32.0% yield) as a sodium salt. The yield was calculated using the absorbance of the product at 260 nm measured by NanoDrop. For the calculation, an absorption coefficient (ε260) = 40,100 M⁻¹·cm⁻¹ was used.

¹H NMR (600 MHz, D₂O) δ 8.51-8.28 (m, 1H), 8.17-7.88 (m, 2H), 7.63 (s, 0.5H), 7.54 (d, J = 7.1 Hz, 1H), 7.44 (s, 1.5H), 7.37 (s, 0.5H), 7.28 (s, 0.5H), 5.98 (dd, J = 17.3, 6.4 Hz, 1H), 5.88 (s, 1H), 5.79 (d, J = 6.6 Hz, 1H), 5.70 (s, 1H), 5.00-4.87 (m, 3H), 4.72 (s, 1H), 4.61-4.55 (m, 0.5H), 4.49 (s, 2.5H), 4.42-4.14 (m, 10H), 4.11 (dt, J = 15.6, 7.5 Hz, 1H), 4.08-3.94 (m, 3H), 3.39 (d, J = 10.7 Hz, 3H), 0.72-0.46 (2s, 9H) ppm.

¹³C NMR (151 MHz, D₂O) δ 158.52, 155.75, 155.19, 154.97, 153.66, 152.74, 149.69, 149.27, 148.83, 148.62, 139.49, 133.63, 132.61, 132.25, 129.99, 129.86, 128.77, 128.08, 123.78, 123.35, 118.27, 116.26, 107.62, 107.30, 94.76, 93.54, 87.62, 87.37, 85.31, 85.03, 84.92, 84.67, 83.59, 83.53, 83.13, 82.16, 82.03, 81.66, 80.38, 79.49, 79.16, 73.53, 72.84, 70.32, 68.60, 68.24, 65.11, 64.84, 64.46, 61.77, 57.97, 36.31, 36.15, 36.12, 35.67, 35.54, 30.29, 24.87, 24.82, 24.74, 24.66, 20.56, 13.29 ppm.

³¹P NMR (243 MHz, D₂O) δ -0.23 (1P), -10.81 (2P), -22.01 (1P) ppm.

### (8) Synthesis of trinucleotide type cap compound Nb-^{m7}Gppp^{m6}A(2'OCH₃)pG (Method A)

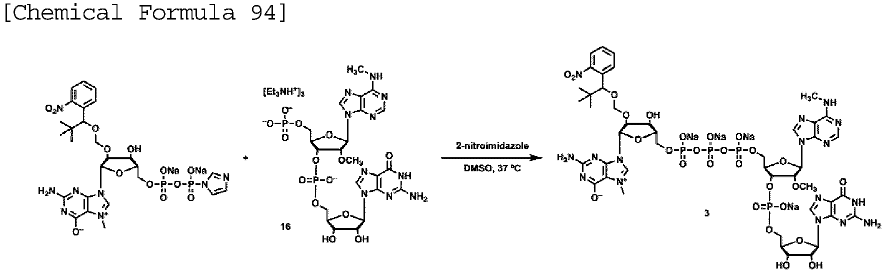

N7-methylguanosine diphosphorimidazolide disodium salt (67.4 mg, 87.2 µmol) and 2-nitroimidazole (14.8 mg, 131 µmol) were added to a solution of dinucleotide triethylammonium salt 16 (43.6 µmol) in DMSO (548 µL). After incubation at 37°C for 1.5 days, N7-methylguanosine diphosphorimidazolide disodium salt (67.4 mg, 87.2 µmol) and 2-nitroimidazole (14.8 mg, 131 µmol) were added. The mixture was incubated at 37°C for 3 days and the reaction mixture was diluted with water (14.0 mL). The crude product was purified by ion exchange chromatography on QAE-Sephadex (ϕ = 3.0 cm, h = 10 cm, 70 cm³, 6 mL/min) . The fractions containing the product were collected and concentrated. The product was desalted with Wakosil (registered trademark) 25C18 (particle size: 15 to 30 um (spherical), column size: ϕ = 4.80 cm, h = 10.2 cm, 184 cm³ (12 mL/min)). The fractions containing the product were concentrated, affording the target compound as a triethylammonium salt. The product was re-dissolved in methanol (2.0 mL). To the mixture was added 190 mM NaClO₄ (12 mL) in acetone, and the resulting suspension was centrifuged (4,500 rpm, 20 minutes). The supernatant was discarded and the precipitate was resuspended in acetone. The suspension centrifugation process was repeated four more times. The precipitate was dried under reduced pressure, affording the target Cap analog 3 (37.9 mg, 25.8 µmol, 59.2% yield) as a sodium salt. The yield was calculated using the absorbance of the product at 260 nm measured by NanoDrop. For the calculation, an absorption coefficient (ε260) = 40,100 M⁻¹·cm⁻¹ was used.

¹H NMR (600 MHz, D₂O) δ 9.14 (s, 0.6H), 8.94 (s, 0.3H), 8.35 (d, J = 17.1 Hz, 1H), 8.17-7.85 (m, 2H), 7.64-7.35 (m, 4H), 6.09-5.68 (m, 3H), 4.95 (s, 3H), 4.82 (s, 3H), 4.76 (s, 1H), 4.68 (s, 1H), 4.61-3.96 (m, 16H), 3.41 (d, J = 8.6 Hz, 2H), 3.05-2.91 (m, 1H), 0.59-0.38 (m, 9H) ppm. ¹³CNMR (151 MHz, D₂O) δ158.30, 155.42, 155.22, 154.51, 154.39, 154.30, 153.64, 151.31, 149.89, 149.70, 149.26, 148.88, 148.80, 147.14, 139.20, 137.29, 136.32, 133.57, 132.75, 132.54, 132.25, 129.98, 128.72, 128.16, 123.75, 123.38, 118.63, 116.03, 107.89, 107.57, 107.28, 94.62, 93.76, 87.74, 87.49, 85.08, 84.44, 83.52, 83.09, 82.12, 81.26, 80.26, 79.62, 79.21, 73.71, 72.73, 70.28, 68.45, 68.13, 65.09, 64.66, 64.27, 61.76, 57.99, 57.49, 36.24, 36.20, 35.79, 35.70, 25.24, 25.14, 24.96, 24.86, 20.57, 16.85, 13.31 ppm.

³¹P NMR (243 MHz, D₂O) δ -0.26 (1P), -10.84 (2P), -21.96 (1P) ppm.

ESI-TOF-MS calcd for C₄₅H₅₇N₁₆O₂₇P₄, 459.0849 [M-3H]³⁻; found 459.0837.

### (9) Synthesis of trinucleotide type cap compound Nb-^{m7}GpppD(2'OCH₃)pG (Method A)

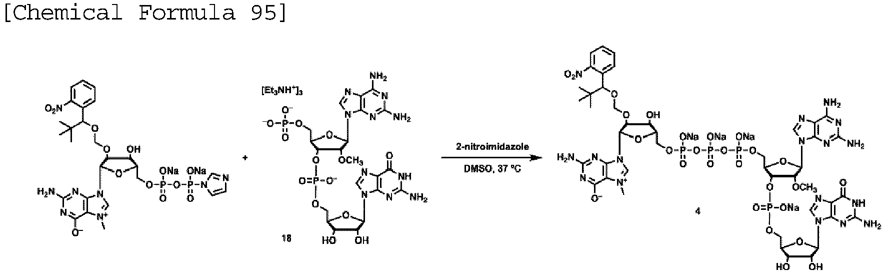

N7-methylguanosine diphosphorimidazolide disodium salt (11.7 mg, 15.1 µmol) and 2-nitroimidazole (2.57 mg, 22.7 µmol) were added to a solution of dinucleotide triethylammonium salt 18 (7.74 mg, 7.55 µmol) in DMSO (95.0 µL). After incubation at 37°C for 4 days, N7-methylguanosine diphosphorimidazolide disodium salt (11.7 mg, 15.1 µmol) and 2-nitroimidazole (2.57 mg, 22.7 µmol) were added. The mixture was incubated at 37°C for 2.5 days and the reaction mixture was diluted with water (14.0 mL). The crude product was purified by ion exchange chromatography on QAE-Sephadex (ϕ = 3.0 cm, h = 10 cm, 70 cm³, 6 mL/min) . The fractions containing the product were collected and concentrated. The product was desalted with Wakosil (registered trademark) 25C18 (particle size: 15 to 30 um (spherical), column size: ϕ = 4.80 cm, h = 10.2 cm, 184 cm³ (12 mL/min)). The fractions containing the target compound were collected and freeze-dried, affording the target compound as a triethylammonium salt. The product was re-dissolved in methanol (1.0 mL). To the mixture was added 190 mM NaClO4 (12 mL) in acetone, and the resulting suspension was centrifuged (4,000 rpm, 20 minutes). The supernatant was discarded and the precipitate was resuspended in acetone. The suspension centrifugation process was repeated four more times. The precipitate was dried under reduced pressure, affording the target Cap analog 4 as a sodium salt.

### (10) Synthesis of trinucleotide type cap compound Nb-^{m7}GpppA(2'F)pG (Method B)

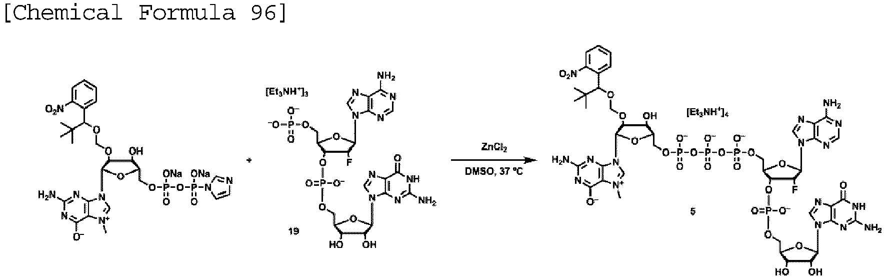

2'-Nb-Guanosine 5'-diphosphorphosphoimidazolide (46.4 mg, 60.0 µmol) and zinc chloride (54.5 mg, 400 µmol) were added to a solution of 5'-p-A(2'F)PG dinucleotide 19 (20.0 mg, 20.0 µmol, crude) in DMSO (400 µL). After stirring at 37°C for 1.5 days, a 500 mM aqueous EDTA solution (pH 8.0, 1.04 mL, EDTA: 520 µmol) was added to quench the reaction mixture. The mixture was diluted with water (13.0 mL) and purified by reverse-phase HPLC (instrument: Shimadzu Prep, column: YMC-Actus Triart C8 (Preparative, 250 × 20.0 mm I.D., solvent A: 50 mM TEAA buffer (pH 6.0, 0.5% CH₃CN), solvent B: CH₃CN, 5 to 80% B linear gradient (25 min), flow rate: 10 ml/min, detection: 254 nm). The fractions containing the objective product were collected, concentrated, and freeze-dried, affording Pure Cap analog 5 as a triethylammonium salt (1.58 mg, 0.900 µmol, 4.50% yield, ε260 = 40,100 M⁻¹·cm⁻¹).

¹H NMR (600 MHz, D₂O) δ 8.15 (s, 1H), 7.92 (s, 1H), 7.73 (s, 1H), 7.54-7.40 (m, 4H), 7.25 (t, J = 7.6 Hz, 1H), 5.98 (d, J = 16.3 Hz, 1H), 5.69-5.66 (m, 1H), 5.63 (d, J = 5.8 Hz, 1H), 5.39 (s, 0.5H), 5.30 (s, 0.5H), 5.02 (s, 1H), 4.94 (d, J = 7.6 Hz, 1H), 4.90 (d, J = 7.6 Hz, 1H), 4.69 (t, J = 5.8 Hz, 1H), 4.61 (s, 1H), 4.46 (d, J = 12.7 Hz, 2H), 4.39 (s, 1H), 4.34-4.25 (m, 4.5H), 4.21 (d, J = 10.5 Hz, 3.5H), 4.11 (d, J = 10.5 Hz, 1H), 4.01 (s, 3H), 3.19 (qd, J = 7.3, 1.2 Hz, 52H, triethylammonium), 1.89 (t, J = 1.2 Hz, 12H, acetate), 1.26 (tt, J = 7.3, 1.1 Hz, 80H, triethylammonium), 0.61 (s, 9H) ppm.

³¹P NMR (243 MHz, D₂O) δ -0.63 (1P), -10.98 (2P), -22.33 (1P) ppm.

### (11) Synthesis of trinucleotide type cap compound Nb-^{m7}GpppA(NHCHO)pG (Method A)

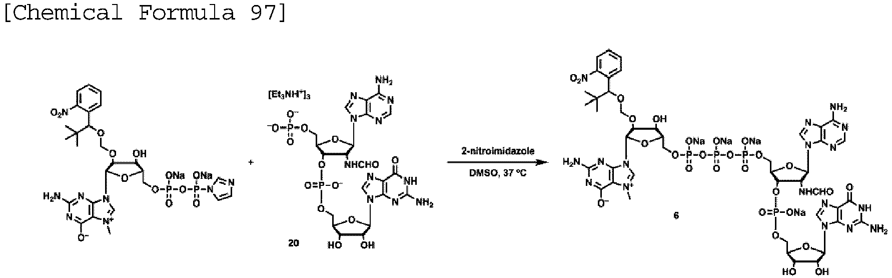

N7-methylguanosine diphosphorimidazolide disodium salt (13.6 mg, 17.6 µmol) and 2-nitroimidazole (3.00 mg, 26.5 µmol) were added to a solution of dinucleotide triethylammonium salt 20 (8.82 µmol) in DMSO (111 µL). After 1.5 days of incubation at 37°C, N7-methylguanosine diphosphorimidazolide disodium salt (13.6 mg, 17.6 µmol) and 2-nitroimidazole (3.00 mg, 26.5 µmol) were added. The mixture was incubated at 37°C for 2.5 days and the reaction mixture was diluted with water (10.0 mL). The crude product was purified by ion exchange chromatography on QAE-Sephadex (ϕ = 3.0 cm, h = 10 cm, 70 cm³, 6 mL/min) . The fractions containing the product were collected and concentrated. The product was desalted with Wakosil (registered trademark) 25C18 (particle size: 15 to 30 um (spherical), column size: ϕ = 4.80 cm, h = 10.2 cm, 184 cm³ (12 mL/min)). The fractions containing the product were collected, and purified by reverse-phase HPLC using a YMC-Triart C8 column (250 × 4.6 mm I.D., S-5 µm, 12 nm, flow rate: 1 mL/min, temperature: 50°C). The fractions containing the target compound were collected and freeze-dried, affording the target compound as a triethylammonium salt. The product was re-dissolved in methanol (1.0 mL). To the mixture was added 190 mM NaClO4 (12 mL) in acetone, and the resulting suspension was centrifuged (4,000 rpm, 20 minutes). The supernatant was discarded and the precipitate was resuspended in acetone. The suspension centrifugation process was repeated four more times. The precipitate was dried under reduced pressure, affording the target Cap analog 6 (2.42 mg, 1.65 µmol, 18.7% yield) as a sodium salt. The yield was calculated using the absorbance of the product at 260 nm measured by NanoDrop. For the calculation, an absorption coefficient (ε260) = 40,100 M⁻¹·cm⁻¹ was used.

¹H NMR (600 MHz, D₂O) δ 8.25 (s, 0.5H), 8.20 (s, 0.5H), 7.95 (s, 0.5H), 7.90 (s, 0.5H), 7.85 (s, 1H), 7.71 (d, J = 5.6 Hz, 1H), 7.51 (s, 0.5H), 7.44 (s, 0.5H), 7.36 (s, 1H), 7.33-7.30 (m, 1H), 7.26 (s, 0.5H), 7.11 (s, 0.5H), 5.73 (s, 3.5H), 5.53 (s, 0.5H), 4.89 (s, 1.5H), 4.82 (s, 1.5H), 4.77 (s, 3H), 4.46 (s, 1H), 4.40 (s, 1H), 4.34 (s, 0.5H), 4.28 (s, 0.5H), 4.19 (s, 2.6H), 4.12 (s, 2.4H), 4.09-4.06 (m, 3H), 3.91 (d, J = 5.1 Hz, 1.5H), 3.86 (d, J = 5.1 Hz, 1.5H), 0.47 (s, 5H), 0.39 (s, 4H) ppm.

³¹P NMR (243 MHz, D₂O) δ -0.30 (1P), -10.81 (2P), -21.96 (1P) ppm.

¹³C NMR (151 MHz, D₂O) δ 163.92, 160.09, 158.75, 155.35, 153.79, 152.81, 151.62, 149.63, 148.84, 148.17, 139.75, 137.70, 133.82, 132.53, 132.02, 129.91, 128.72, 127.74, 123.70, 123.42, 118.15, 116.39, 108.30, 107.99, 95.24, 93.61, 87.41, 86.98, 86.80, 85.06, 84.04, 83.36, 82.28, 80.68, 79.98, 79.23, 73.32, 70.25, 69.08, 68.71, 65.63, 65.42, 65.22, 54.24, 36.43, 35.93, 35.61, 30.29, 24.67, 24.61, 23.32 ppm.

### (12) Synthesis of trinucleotide type cap compound Nb-^{m7}GpppA(2'OCH₃)psG (Method B)

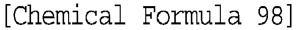

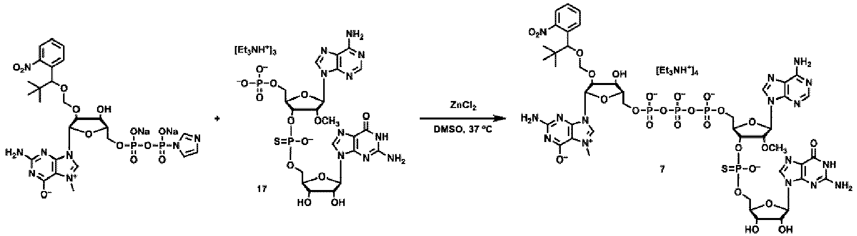

2'-Nb-guanosine 5'-diphosphorimidazolide (34.1 mg, 44.1 µmol) and zinc chloride (40.1 mg, 294 µmol) were added to a solution of 5'-p-A(2'OMe)PSG dinucleotide 17 (14.7 µmol) in DMSO (294 µL). After stirring at 37°C for 1.5 days, a 500 mM aqueous EDTA solution (pH 8.0, 764 µL, EDTA: 382 µmol) was added to quench the reaction mixture. The mixture was diluted with water (14.0 mL), and purified by reverse-phase HPLC (instrument: Shimadzu Prep., column: YMC-Actus Triart C8 (Preparative, 250 × 20.0 mm I.D.)). The fractions containing the objective product were collected, concentrated, and freeze-dried, affording Cap analog 7 (3.18 mg, 1.78 µmol, 12.1% yield) as a triethylammonium salt. The yield was calculated using the absorbance of the product at 260 nm measured by NanoDrop. For the calculation, an absorption coefficient (ε260) = 40,100 M⁻¹·cm⁻¹ was used.

¹H NMR (600 MHz, D₂O) δ 8.24 (s, 1H), 8.01-7.85 (m, 2H), 7.43 (d, J = 7.8 Hz, 1H), 7.35 (s, 2H), 7.16 (s, 1H), 5.84 (d, J = 12.2 Hz, 1H), 5.73-5.65 (m, 1H), 5.58 (s, 1H), 4.92 (d, J = 9.4 Hz, 1H), 4.89 (s, 1H), 4.83 (d, J = 7.1 Hz, 1H), 4.76 (d, J = 7.6 Hz, 1H), 4.58 (s, 1H), 4.49-4.33 (m, 3H), 4.32-4.25 (m, 1H), 4.24-4.16 (m, 4H), 4.15-4.05 (m, 5H), 3.88 (d, J = 8.8 Hz, 3H), 3.27 (d, J = 15.4 Hz, 3H), 3.10-3.02 (m, 33H, triethylammonium), 1.80-1.74 (m, 7H, CH₃COO⁻), 1.18-1.09 (m, 51H, triethylammonium), 0.54-0.47 (m, 9H) ppm.

³¹P NMR (243 MHz, D₂O) δ 56.60 (1P, d, J = 131.5 Hz), -10.81 (2P), -21.86 (1P) ppm.

### (13) Synthesis of trinucleotide type cap compound Nb-^{m7}GpppA(LNA)pG (Method B)

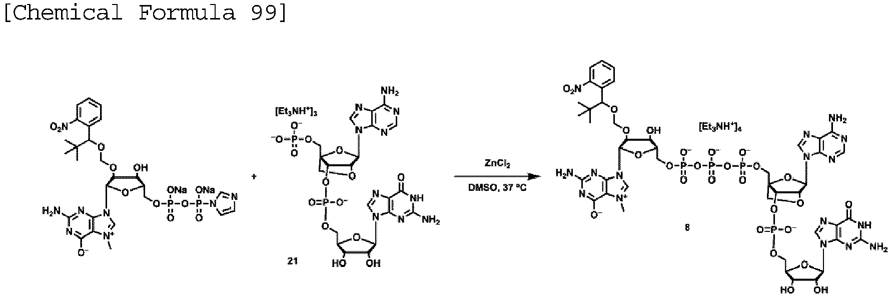

2'-Nb-Guanosine 5'-diphosphorphosphoimidazolide (60.7 mg, 78.6 µmol) and zinc chloride (71.4 mg, 524 µmol) were added to a solution of 5'-p-A(LNA)PG dinucleotide 21 (26.4 mg, 26.2 µmol) in DMSO (524 µL). After stirring at 37°C for 2 days, a 500 mM aqueous EDTA solution (pH 8.0, 1.36 mL, EDTA: 681 µmol) was added to quench the reaction mixture. The mixture was diluted with water (13.0 mL), and purified by reverse-phase HPLC (instrument: Shimadzu Prep., column: YMC-Actus Triart C8 (Preparative, 250 × 20.0 mm I.D.)). The fractions containing the objective product were collected, concentrated, and freeze-dried, affording Pure Cap analog 8 as a triethylammonium salt (2.44 mg, 1.38 µmol, 5.27% yield, ε260 = 40,100 M⁻¹·cm⁻¹).

¹H NMR (600 MHz, D₂O) δ 8.78 (s, 1H), 7.97-7.90 (m, 1H), 7.84 (d, J = 4.6 Hz, 1H), 7.63 (s, 1H), 7.48 (dd, J = 8.3, 4.0 Hz, 1H), 7.41 (s, 2H), 7.22 (s, 1H), 5.60-5.54 (m, 2H), 5.52 (d, J = 3.9 Hz, 1H), 4.90 (d, J = 4.4 Hz, 1H), 4.83 (d, J = 4.0 Hz, 1.5H), 4.77 (dd, J = 7.7, 3.8 Hz, 1.5H), 4.74 (d, J = 4.6 Hz, 1.5H), 4.55 (s, 0.5H), 4.48 (d, J = 3.6 Hz, 0.5H), 4.38 (s, 1H), 4.35 (s, 1H), 4.28 (d, J = 4.0 Hz, 2.5H), 4.20 (t, J = 4.9 Hz, 2H), 4.17-4.08 (m, 4H), 4.00-3.91 (m, 5H), 3.06 (dt, J = 11.5, 7.4 Hz, 15H), 1.14 (td, J = 7.4, 3.9 Hz, 23H), 0.53 (d, J = 4.2 Hz, 9H) ppm.³¹PNMR (243 MHz, D₂O) δ-1.37 (1P), -11.02 (2P), -22.30 (1P) ppm.

### (14) Synthesis of tetranucleotide-type cap compound Nb-^{m7}GpppA(2'OCH₃)pG(2'OCH₃)pG (Method A)

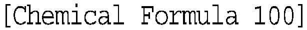

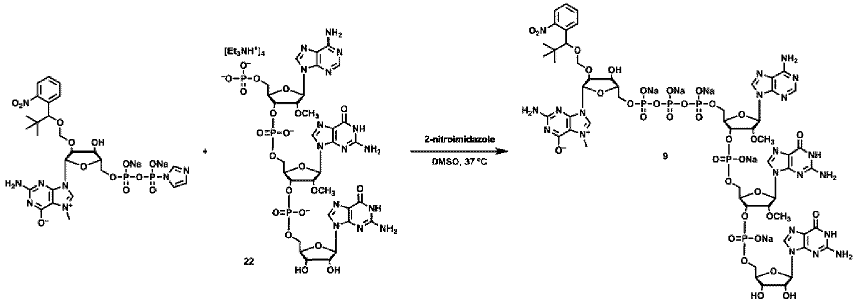

2'-Nitrobenzyl-modified N7-methylguanosine diphosphorimidazolide disodium salt (31.8 mg, 41.2 µmol) and 2-nitroimidazole (6.99 mg, 61.8 µmol) were added to a solution of 2'-O-methyl-modified dinucleotide triethylammonium salt 22 (30.3 mg, 20.6 µmol) in DMSO (259 µL). After incubation at 37°C for 1 day, 2'-nitrobenzyl-modified N7-methylguanosine diphosphorimidazolide disodium salt (31.8 mg, 41.2 µmol) and 2-nitroimidazole (6.99 mg, 61.8 µmol) were added. The mixture was incubated at 37°C for 3 days and the reaction mixture was diluted with water (10.0 mL). The crude product was purified by ion exchange chromatography on QAE-Sephadex (ϕ = 3.0 cm, h = 10 cm, 70 cm³, 6 mL/min) . The product was desalted with Wakosil (registered trademark) 25C18 (particle size: 15 to 30 µm (spherical), column size: ϕ = 4.80 cm, h = 10.2 cm, 184 cm³ (12 mL/min). The fractions containing the product were concentrated, affording the target compound as a triethylammonium salt. The product was re-dissolved in methanol (2.0 mL). To the mixture was added 190 mM NaClO4 (12 mL) in acetone, and the resulting suspension was centrifuged (4,500 rpm, 20 minutes). The supernatant was discarded and the precipitate was resuspended in acetone. The suspension centrifugation process was repeated four more times. The precipitate was dried under reduced pressure, affording the target Cap analog 9 (16.0 mg, 8.70 µmol, 42.2% yield) as a sodium salt. The yield was calculated using the absorbance of the product at 260 nm measured by NanoDrop. For the calculation, an absorption coefficient (ε260) = 50,200 M⁻¹·cm⁻¹ was used.

¹H NMR (600 MHz, D₂O) δ 8.42-8.34 (m, 1H), 8.06 (d, J = 12.4 Hz, 1.5H), 7.97 (s, 1H), 7.84 (s, 1H), 7.67 (s, 0.5H), 7.57 (d, J = 7.6 Hz, 1H), 7.51-7.36 (m, 2H), 7.29 (s, 1H), 6.88 (s, 1H), 5.94 (dd, J = 11.6, 5.1 Hz, 2H), 5.88-5.80 (m, 1.5H), 5.73 (s, 1H), 5.68 (d, J = 4.3 Hz, 0.5H), 4.89 (s, 4H), 4.58 (s, 2.5H), 4.52 (s, 0.5H), 4.46 (s, 3H), 4.38 (s, 3H), 4.34-4.24 (m, 8H), 4.21-4.08 (m, 11H), 4.05 (s, 1.5H), 4.00 (s, 1.5H), 3.83-3.47 (m, 6H), 0.64-0.46 (m, 9H) ppm.

¹³C NMR (151 MHz, D₂O) δ 159.70-158.2 (m), 153.71-150.8 (m), 149.70-148.00 (m), 132.07, 129.36, 128.17, 123.14, 117.26, 114.91, 107.66, 87.66, 84.99, 82.62, 80.56, 79.37, 75.68, 72.44, 68.13, 65.01, 61.77, 58.27, 57.91, 56.82, 48.94, 46.69, 36.13, 35.36, 30.30, 28.25, 24.76, 20.57, 16.84, 13.30, 8.32 ppm.

³¹P NMR (243 MHz, D₂O) δ -0.26 (2P), -10.84 (2P), -22.09 (1P) ppm.

### (15) Synthesis of tetranucleotide-type cap compound Nb-^{m7}Gppp^{m6}A(2'OCH₃)pG(2'OCH₃)pG (Method B)

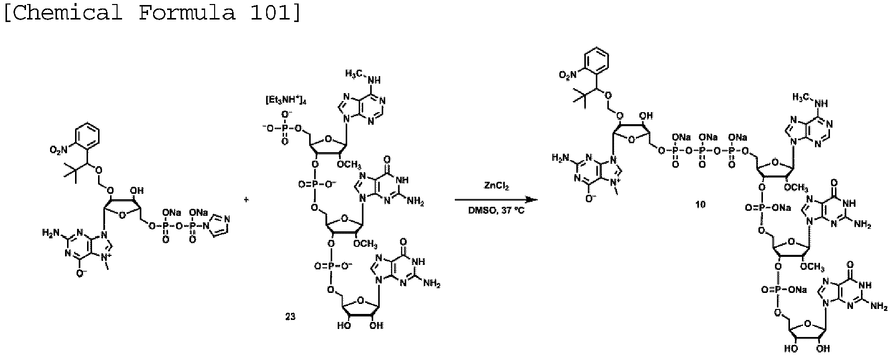

Guanosine 5'-diphosphorimidazolide (246 mg, 0.318 mmol) and zinc chloride (289 mg, 2.12 mmol) were added to a solution of 5'-p-m6A(2'OMe)PG(2'OMe)PG trinucleotide 23 (158 mg, 0.106 mmol) in DMSO (2.12 mL). After stirring at 37°C for 3 days, a 500 mM aqueous EDTA solution (pH 8.0, 5.52 mL, EDTA: 2.76 mmol) was added to quench the reaction mixture. The mixture was diluted with water (40.0 mL), and purified by reverse-phase HPLC (instrument: Shimadzu Prep., column: YMC-Actus Triart C8 (Preparative, 250 × 20.0 mm I.D.)). The fractions containing the target product were collected, concentrated, and freeze-dried, affording Pure Cap analog 10 as a triethylammonium salt. The product was re-dissolved in methanol (2.00 mL). A 190 mM NaClO₄ (24.0 mL) solution in acetone was added to the mixture, and the resulting suspension was centrifuged (4,000 rpm, 20 min). The supernatant was discarded and the precipitate was resuspended in acetone. The suspension centrifugation process was repeated four more times. The precipitate was dried under reduced pressure, affording Cap analog 10 (70.7 mg, 38.2 µmol, 36.0% yield) as a sodium salt. The yield was calculated using the absorbance of the product at 260 nm measured by NanoDrop. For the calculation, an absorption coefficient (ε260) = 50,200 M⁻¹·cm⁻¹ was used.

³¹P NMR (243 MHz, D₂O) δ -0.38, -10.60, -21.60 ppm.

### (16) Synthesis of tetranucleotide-type cap compound Nb-^{m7}GpppG(2'OCH₃)pG(2'OCH₃)pG (Method B)

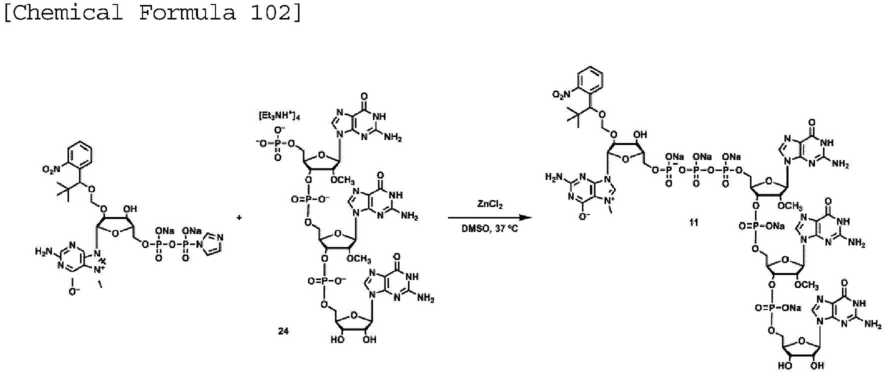

2'-Nb-Guanosine 5'-diphosphorphosphoimidazolide (35.0 mg, 45.5 µmol) and zinc chloride (41.0 mg, 300 µmol) were added to a solution of 5'-p-G(2'OMe)PG(2'OMe)PG trinucleotide 24 (21.0 mg, 15.0 µmol) in DMSO (300 µL). After stirring at 37°C for 2 days, a 38 mM aqueous EDTA-NaOH solution (pH 8.0, 10.3 mL, EDTA: 391 µmol) was added to quench the reaction mixture. The mixture was purified by ion exchange chromatography using QAE-Sephadex (ϕ = 3.0 cm, h = 10 cm, 70 cm³, 6 mL/min) . The fractions containing the product were collected and concentrated. The product was desalted with Wakosil (registered trademark) 25C18 (particle size: 15 to 30 µm (spherical), column size: ϕ = 4.80 cm, h = 10.2 cm, 184 cm³ (12 mL/min) . The fractions containing the product were concentrated, affording the target compound as a triethylammonium salt. The product was re-dissolved in methanol (2.0 mL). To the mixture was added 190 mM NaClO₄ (12 mL) in acetone, and the resulting suspension was centrifuged (4,500 rpm, 20 minutes). The supernatant was discarded and the precipitate was resuspended in acetone. The suspension centrifugation process was repeated four more times. The precipitate was dried under reduced pressure, affording the target Cap analog 11 (1.87 µmol, 12.5% yield) as a sodium salt. The yield was calculated using the absorbance of the product at 260 nm measured by NanoDrop. For the calculation, an absorption coefficient (ε₂₆₀) = 46,800 M⁻¹·cm⁻¹ was used.

¹H NMR (600 MHz, D₂O) δ 7.95-7.81 (m, 3H), 7.55 (d, J = 6.8 Hz, 0.5H), 7.47 (t, J = 7.0 Hz, 0.5H), 7.43-7.27 (m, 2.5H), 7.14 (t, J = 7.4 Hz, 0.5H), 5.84-5.73 (m, 2.5H), 5.66 (dd, J = 13.9, 6.5 Hz, 1H), 5.58 (d, J = 5.8 Hz, 0.5H), 4.94 (s, 0.5H), 4.86 (s, 1.5H), 4.61 (s, 1H), 4.55 (s, 1H), 4.42-4.26 (m, 5.5H), 4.25-4.02 (m, 12H), 3.99 (t, J = 1.6 Hz, 1H), 3.94-3.92 (m, 1.5H), 3.26 (dt, J = 13.7, 1.7 Hz, 3H), 3.20 (dt, J = 9.0, 1.7 Hz, 3H), 2.99 (q, J = 7.4 Hz, triethylammonium), 1.12 (t, J = 7.3 Hz, triethylammonium), 0.52-0.49 (m, 4.5H), 0.42-0.40 (m, 4.5H) ppm.

³¹P NMR (243 MHz, D₂O, triethylammonium form) δ -0.22 (d, J = 32.9 Hz), -11.00 (d, J = 16.4 Hz), -22.43 (t, J = 18.1 Hz) ppm.

### (17) Synthesis of tetranucleotide-type cap compound Nb-^{m7}GpppD(2'OCH₃)pG(2'OCH₃)pG (Method A)

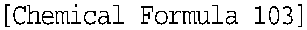

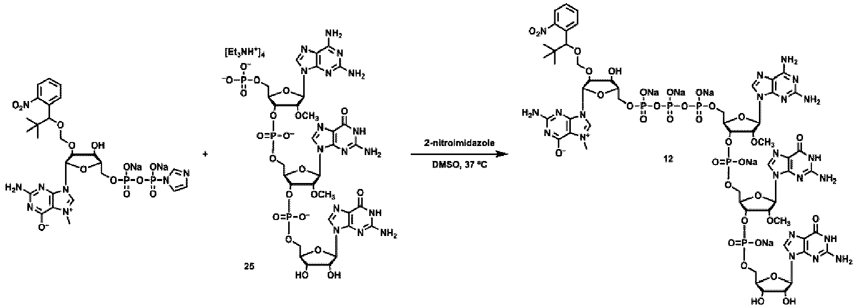

N7-methylguanosine diphosphorimidazolide disodium salt (12.1 mg, 15.7 µmol) and 2-nitroimidazole (2.67 mg, 23.6 µmol) were added to a solution of trinucleotide triethylammonium salt 25 (11.7 mg, 7.85 µmol) in DMSO (98.7 µL). After incubation at 37°C for 4 days, N7-methylguanosine diphosphorimidazolide disodium salt (12.1 mg, 15.7 µmol) and 2-nitroimidazole (2.67 mg, 23.6 µmol) were added. The mixture was incubated at 37°C for 2.5 days and the reaction mixture was diluted with water (14.0 mL). The crude product was purified by ion exchange chromatography on QAE-Sephadex (ϕ = 3.0 cm, h = 10 cm, 70 cm³, 6 mL/min) . The fractions containing the product were collected and concentrated. The product was desalted with Wakosil (registered trademark) 25C18 (particle size: 15 to 30 um (spherical), column size: ϕ = 4.80 cm, h = 10.2 cm, 184 cm³ (12 mL/min) . The fractions containing the target compound were collected and freeze-dried, affording the target compound as a triethylammonium salt. The product was re-dissolved in methanol (1.0 mL). To the mixture was added 190 mM NaClO₄ (12 mL) in acetone, and the resulting suspension was centrifuged (4,000 rpm, 20 minutes). The supernatant was discarded and the precipitate was resuspended in acetone. The suspension centrifugation process was repeated four more times. The precipitate was dried under reduced pressure, affording the target Cap analog 12 as a sodium salt.

### (18) Synthesis of tetranucleotide-type cap compound Nb-^{m7}GpppA(2'OCH₃)psG(2'OCH₃)psG (Method B)

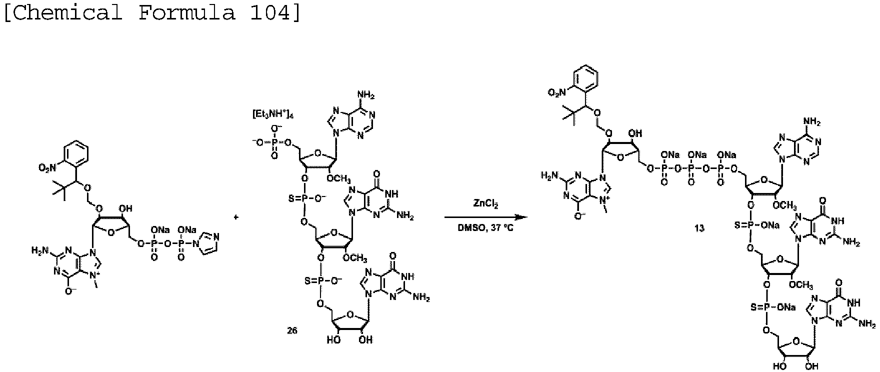

2'-Nb-Guanosine 5'-diphosphorphosphoimidazolide (46.6 mg, 60.3 µmol) and zinc chloride (54.8 mg, 402 µmol) were added to a solution of 5'-p-A(2'OMe)PSG(2'OMe)PSG trinucleotide 26 (30.1 mg, 20.1 µmol) in DMSO (402 µL). After stirring at 37°C for 2 days, a 500 mM aqueous EDTA solution (pH 8.0, 1.05 mL, EDTA: 523 µmol) was added to quench the reaction mixture. The mixture was diluted with water (13.0 mL), and purified by reverse-phase HPLC (instrument: Shimadzu Prep., column: YMC-Actus Triart C8 (Preparative, 250 × 20.0 mm I.D.)). The fractions containing the objective product were collected, concentrated, and freeze-dried, affording Cap analog 13 as a triethylammonium salt (3.31 mg, 1.46 µmol, 7.26% yield, ε₂₆₀ = 50,200 M⁻¹·cm⁻¹).

¹H NMR (600 MHz, D₂O) δ 8.97 (d, J = 7.5 Hz, 1H), 8.51-8.40 (m, 1H), 8.09 (d, J = 2.7 Hz, 1H), 8.06-8.02 (m, 1H), 7.99-7.94 (m, 1H), 7.59 (q, J = 7.0 Hz, 1H), 7.54-7.43 (m, 2H), 7.30 (t, J = 6.9 Hz, 1H), 6.06-5.94 (m, 1H), 5.88-5.67 (m, 3H), 5.20-5.04 (m, 2H), 5.01 (t, J = 5.7 Hz, 1H), 4.94 (d, J = 7.1 Hz, 1H), 4.89-4.83 (m, 1.5H), 4.75-4.72 (m, 1H), 4.63-4.55 (m, 3H), 4.53-4.40 (m, 4.5H), 4.35-4.12 (m, 11H), 4.03 (dd, J = 6.4, 3.4 Hz, 3H), 3.42-3.34 (m, 5H), 3.32 (d, J = 5.5 Hz, 1H), 3.18 (qd, J = 7.3, 2.1 Hz, 23H), 1.26 (td, J = 7.3, 2.3 Hz, 36H), 0.66-0.52 (m, 9H) ppm.

³¹P NMR (243 MHz, D₂O) δ 57.92-55.14 (2P), -10.98 (2P), - 22.24 (1P) ppm.

### 15. mRNA synthesis by in vitro transcription method using synthesized novel cap analog and evaluation of its translation activity

### (1) Transcription reaction by T7 RNA polymerase

SEQ ID NO: 7 is a DNA sequence of a transcription synthesis template of Luc2 mRNA. SEQ ID NO: 8 is a sequence of Luc2 mRNA transcribed using the template DNA of SEQ ID NO: 7.

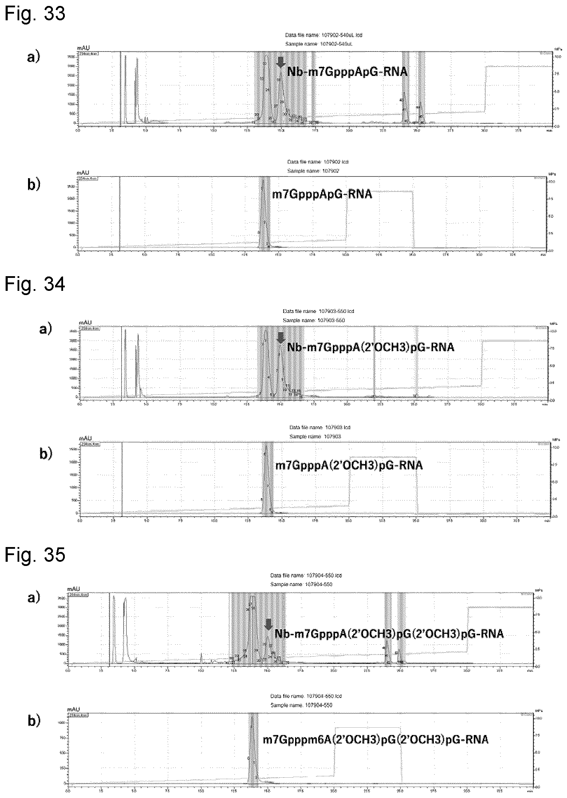

Fig. 33 is a diagram showing the result of HPLC purification of a co-transcription reaction product mRNA using a cap analog synthesized. (a) of this figure shows the HPLC purification results after the co-transcription reaction using a trinucleotide-type cap analog (Nb-m7GpppApG). An uncapped product was eluted at a retention time of 14 minutes, and a capped product was eluted at 15 minutes. (b) of this figure is the HPLC purification result after deprotection by photoirradiation. The fact that the peak was shifted to a retention time of 14 minutes as a result of the deprotection reaction by photoirradiation has shown that the photodegradable protecting group was removed and a capped mRNA was obtained with a high purity.

Fig. 34 is a diagram showing the result of HPLC purification of a co-transcription reaction product mRNA using a cap analog synthesized. (a) of this figure shows the HPLC purification results after the co-transcription reaction using a trinucleotide-type cap analog (Nb-m7GpppA(2'OCH3)pG). An uncapped product was eluted at a retention time of 14 minutes, and a capped product was eluted at 15 minutes. (b) of this figure is the HPLC purification result after deprotection by photoirradiation. The fact that the peak was shifted to a retention time of 14 minutes as a result of the deprotection reaction by photoirradiation has shown that the photodegradable protecting group was removed and a capped mRNA was obtained with a high purity.

Fig. 35 is a diagram showing the result of HPLC purification of a co-transcription reaction product mRNA using a cap analog synthesized. (a) of this figure shows the HPLC purification results after the co-transcription reaction using a tetranucleotide-type cap analog (Nb-m7GpppA(2'OCH3)pG(2'OCH3)pG). An uncapped product was eluted at a retention time of 14 minutes, and a capped product was eluted at 15 minutes. (b) of this figure is the HPLC purification result after deprotection by photoirradiation. The fact that the peak was shifted to a retention time of 14 minutes as a result of the deprotection reaction by photoirradiation has shown that the photodegradable protecting group was removed and a capped mRNA was obtained with a high purity.

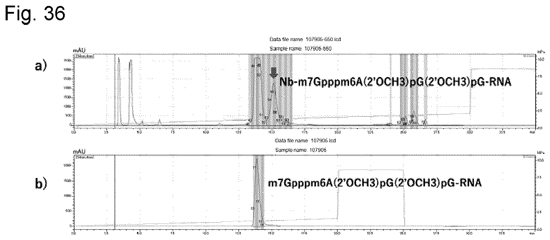

Fig. 36 is a diagram showing the result of HPLC purification of a co-transcription reaction product mRNA using a cap analog synthesized. (a) of this figure shows the HPLC purification results after the co-transcription reaction using a tetranucleotide-type cap analog (Nb-m7Gpppm6A(2'OCH3)pG(2'OCH3)pG). An uncapped product was eluted at a retention time of 14 minutes, and a capped product was eluted at 15 minutes. (b) of this figure is the HPLC purification result after deprotection by photoirradiation. The fact that the peak was shifted to a retention time of 14 minutes as a result of the deprotection reaction by photoirradiation has shown that the photodegradable protecting group was removed and a capped mRNA was obtained with a high purity.

An RNA was transcriptionally synthesized with T7 RNA polymerase using a double-stranded DNA as a template. The double-stranded DNA of the template was obtained by amplifying pSP73-a-luc2 (described at the end of the experimental section) encoding the Luc2 gene by a PCR reaction using KOD-Plus-Neo (TOYOBO), Fw primer (cgcgttggccgattcatt: SEQ ID NO: 3), Rev primer (tttttttttttttttttttttttttttttttttttttttttttttttttttttttttttttttt ttttttttttttttttcagttagacgttgatcctgg: SEQ ID NO: 4) (1.0 ng/pL DNA vector, 1 × KOD-Plus-Neo buffer, 0.2 mM dNTPs, 0.3 µM primers, 1.5 mM MgSO₄, 0.02 units/µL polymerase).

A transcription reaction liquid (template DNA 15 ng/µL, 1 × T7 RNA Polymerase buffer (Takara) (40 mM Tris-HCl (pH 8.0), 8 mM MgCl₂, 2 mM spermidine), 10 U/µL T7 RNA polymerase (Takara), 5 mM DTT, 2 mM ATP, 2 mM UTP, 2 mM CTP, 2 mM GTP, 2 mM Nb-^{m7}GpppApG or Nb-^{m7}GpppA(2'OCH₃)pG or Nb-^{m7}GpppA(2'OCH₃)pG(2'OCH₃)pG or Nb-^{m7}Gppp^{m6}A(2'OCH₃)pG(2'OCH₃)pG) was prepared and incubated at 37°C for 2 hours. Recombinant DNase (Takara) was added to the reaction liquid to 0.1 U/µL, and the mixture was incubated at 37°C for 30 minutes to degrade the template DNA. After the reaction, phenol/chloroform extraction was carried out to perform alcohol precipitation. The product was purified by reverse-phase HPLC.

Column: YMC-Triart Bio C4 (250 mm × 4.6 mm I.D.); Solvent A: 50 mM triethylammonium acetate (pH 7.0), 5% acetonitrile; Solvent B: acetonitrile; Linear Gradient: 5 to 15% Solvent B (0 to 20 min); Flow rate: 1 mL/min; Detection wavelength: 260 nm; Column temperature: 50°C.

Deprotection by photoirradiation: The RNA solution was added to a transparent 96-multiwell plate, and irradiated with 365 nm light at a quantity of light of 4 mW/cm² for 15 minutes using a MAX-305 light source device (Asahi Spectra Co., Ltd.). HPLC purification was performed. Column: YMC-Triart Bio C4 (250 mm × 4.6 mm I.D.); Solvent A: 50 mM triethylammonium acetate (pH 7.0), 5% acetonitrile; Solvent B: acetonitrile; Linear gradient: 5 to 15% Solvent B (0 to 20 min); Flow rate: 1 mL/min; Detection wavelength: 260 nm; Column temperature: 50°C

The sequence information of pSP73-a-luc2 is as shown in SEQ ID NO: 5.

### (2) Evaluation of translation activity of Luc2-expressing mRNA

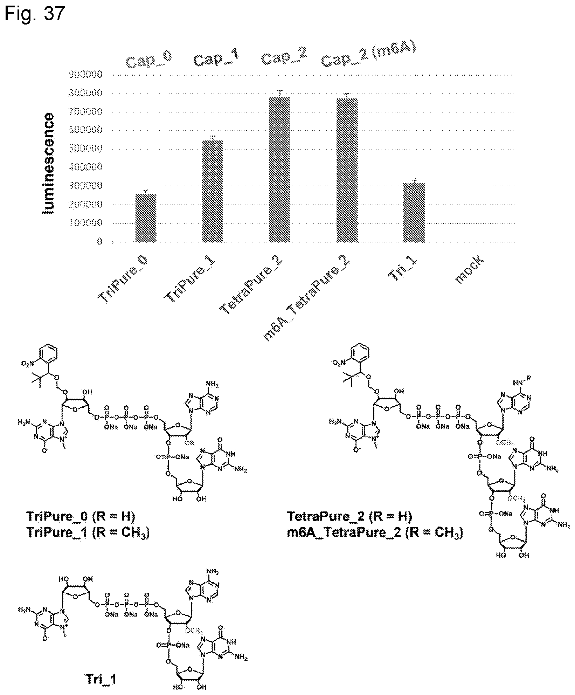

Fig. 37 is a diagram showing the result of evaluation of translation activity in a HeLa cell of RNAs transcriptionally synthesized using a cap analog. An RNA was transfected into HeLa cells by the lipofection method. After 24 hours, the cells were lysed and the luminescence of firefly luciferase was measured. TriPure_0 is Nb-m7GpppApG, TriPure_1 is Nb-m7GpppA(2'OCH3)pG, TetraPure_2 is Nb-m7GpppA(2'OCH3)pG(2'OCH3)pG, m6A_TetraPurte_2 is Nb-m7Gpppm6A(2'OCH3)pG(2'OCH3)pG, and Tri_1 is an mRNA synthesized by co-transcription of a compound having the same structure as CleanCap (registered trademark) Rreagent AG(Trilink). The translation activity was higher in the order of Cap2 > Cap1 > Cap0. TriPure_1 purified with an Nb tag showed about 2 times higher activity than Tri_1 using the existing CleanCap (registered trademark) Rreagent AG.

HeLa cells (RIKEN Cell Bank) were cultivated (37°C, 5% CO₂) in Dulbecco's modified Eagle's medium (DMEM; WAKO) with addition of 10% fetal bovine serum (FBS; Invitrogen). HeLa cells were seeded in a 96-well plate the day before transfection (1.0 × 10⁴ cells/well) . The next day, the medium was removed and replaced with 90 µL/well Opti-MEM (registered trademark) (Thermo Fisher SCIENTIFIC). 0.15 µL Lipofectamine (registered trademark) Messenger MAX (registered trademark) and 25 ng mRNA were diluted in 10 µL Opti-MEM (registered trademark) and introduced into cells. After incubation at 37°C for 5 hours, 20 µL/well 1 × Cell Lysis Buffer (Promega) was added to dissolve the cells. Luminescence was measured using ONE-Glo (registered trademark) luciferase assay (registered trademark) (Promega).

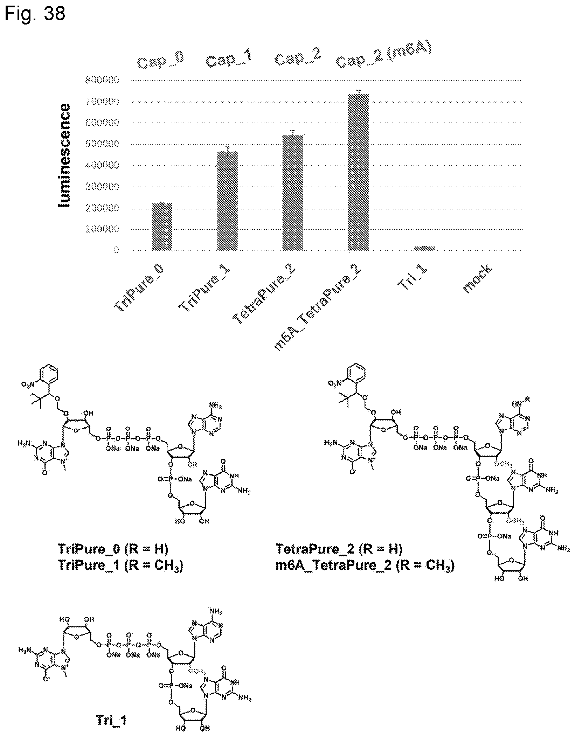

Fig. 38 is a diagram showing the result of evaluation of translation activity in JAWS II cells of an RNA transcriptionally synthesized using a cap analog. An RNA was transfected into JAWS II cells by the lipofection method. After 24 hours, the cells were lysed and the luminescence of firefly luciferase was measured. TriPure_0 is Nb-m7GpppApG, TriPure_1 is Nb-m7GpppA(2'OCH₃)pG, TetraPure_2 is Nb-m7GpppA(2'OCH₃)pG(2'OCH₃)pG, m6A_TetraPurte_2 is Nb-m7Gpppm6A(2'OCH₃)pG(2'OCH₃)pG, and Tri_1 is an mRNA synthesized by co-transcription of a compound having the same structure as CleanCap (registered trademark) Rreagent AG(Trilink). The translation activity was higher in the order of Cap_2 > Cap_1 > Cap_0. TriPure_1 purified with an Nb tag showed about 24 times higher activity than Tri_1 using the existing CleanCap (registered trademark) Rreagent AG.

Evaluation of intracellular translation activity (JAWS II cells): JAWS II cells (ATCC) were cultivated (37°C, 5% CO₂) in Minimum Essential Medium α(MEM α; Thermo Fisher SCIENTIFIC) with addition of 10% fetal bovine serum (FBS; Invitrogen) and GM-CSF (5 ng/mL). JAWS II cells were seeded in a 96-well plate the day before transfection (3.0 × 10⁴ cells/well). The next day, the medium was removed and replaced with 90 µL/well Opti-MEM (registered trademark) (Thermo Fisher SCIENTIFIC). 0.15 µL Lipofectamine (registered trademark) Messenger MAX (registered trademark) and 50 ng mRNA were diluted in 10 µL Opti-MEM (registered trademark) and introduced into cells. After incubation at 37°C for 5 hours, 20 µL/well 1 × Cell Lysis Buffer (Promega) was added to lyse the cells. Luminescence was measured using ONE-Glo (registered trademark) luciferase assay (registered trademark) (Promega).

The amino acid sequence information of Luc2 is as shown in SEQ ID NO: 6.

### (3) Comparison of translation activity of NanoLuc luciferase (Nluc) mRNA synthesized using dinucleotide-type cap analog

Fig. 39 is a diagram showing a comparison of translation activities of 650-base-long NanoLuc luciferase (Nluc) mRNAs prepared using hydrophobic protecting group-containing dinucleotide-type cap analogs (DiPure, DiPure/3'ome, DiPure/2'-ome) and a conventional cap analog (ARCA). This is an experimental result showing that the former translation activity is high. A of this figure shows a chemical structure and an abbreviation of the cap analog used. B of this figure shows the 5'-end analysis result of an mRNA after purification using a hydrophobic protecting group. The full-length mRNA was cleaved at the position of 23 bases from the 5'-end by DNAzyme, and the cleavage product was analyzed by denaturing polyacrylamide electrophoresis. As a result, it was found that all mRNAs purified using a hydrophobic protecting group-containing cap derivative contained only a capped 5'-end. C of this figure shows the result of activity comparison of Nluc mRNAs using HeLa cells. D of this figure shows the result of activity comparison of Nluc mRNAs using JAWS II cells. The mRNAs prepared using the hydrophobic protecting group-containing cap analogs DiPure, DiPure/3'ome, and DiPure/2'ome showed higher translation activities than the mRNA prepared using the conventional cap analog ARCA. ARCA/AP represents a product obtained by dephosphorylating the 5'-end of a coexisting uncapped RNA by acting Antarctic phosphatase on an ARCA-containing mRNA.

The template DNA sequence of NLuc mRNA is as shown in SEQ ID NO: 9. The sequence of NLuc mRNA (transcript) is as shown in SEQ ID NO: 10.

The capped mRNA used in Fig. 39 was transcriptionally synthesized in the following reaction liquid from a DNA template using T7 RNA polymerase. 15 ng/uL dsDNA transcription template (PCR product of 676-bp containing T7 promoter sequence 5'TAATACGACTCACTATAG3'-(SEQ ID NO: 1) and containing a NanoLuc luciferase gene coding region derived from a pNL1.1.TK vector (Promega)), 2 mM ATP, 2 mM UTP, 2 mM CTP, 2mM GTP, 2 mM Cap Analog, 40 mM Tris-HCl (pH 8.0), 8 mM MgCl₂, 2 mM spermidine, 5 mM DTT, 10 units/µL T7 RNA polymerase (Takara Bio Inc.). In the transcription reaction of the ARCA cap analog (Jena Bio-Sciences) containing no hydrophobic protecting group, the final concentration of GTP was 0.5 mM with respect to the final concentration of ARCA of 2 mM. The reaction liquid was warmed at 37°C for 2 hours, then DNase (Takara Bio Inc.) was added thereto such that a final concentration was 0.1 units/µL, and the mixture was warmed at 37°C for 20 minutes. An equal volume mixed liquid of TE saturated phenol and chloroform was added in the same volume as the reaction liquid, and the mixture was vigorously stirred, then centrifuged, and the aqueous layer was taken. An equal amount of chloroform was added thereto, and the mixture was vigorously stirred, then centrifuged, and the aqueous layer was taken. A 1/10 amount of a 3 M sodium acetate buffer (pH 5.2) and an equal amount of 2-propanol were added to the aqueous layer, and the mixture was cooled at minus 30°C for 1 hour and then centrifuged, affording a crude purified product of an RNA. The crude purified product of the RNA was purified by reverse-phase HPLC under the following conditions. Column: YMC-Triart Bio C4 (250 mm × 4.6 mm I.D.); Solvent A: 50 mM triethylammonium acetate (pH 7.0), 5% acetonitrile; Solvent B: acetonitrile; Linear gradient: 0 to 20% Solvent B (0 to 20 min); Flow rate: 1 mL/min; Detection wavelength: 260 nm; Column temperature: 50°C.

The elution time of the RNA transcript containing the hydrophobic protecting group-containing cap analog was 13.7 minutes to 14.0 minutes, whereas the elution time of the RNA transcript not containing the hydrophobic protecting group-containing cap analog was 15.5 minutes to 15.8 minutes, and both were clearly different. As a result, it was possible to separate and purify the mRNA containing the hydrophobic protecting group-containing cap analog. An RNA containing dephosphorylated ARCA (ARCA_AP) was prepared as follows. The reaction liquid (250 ng/pL ARCA-mRNA, 50 mM Bis-Tris-Propane HCl, 1 mM MgCl₂, 0.1 mM ZnCl₂, pH 6.0 @ 25°C, 0.25 U/uL Antarctic phosphatase (New England Biolabs), reaction liquid volume: 20 µL) was warmed at 37°C for 30 minutes, and then extracted with an equal volume mixed solution of TE saturated phenol and chloroform, then extracted with chloroform, and then 1/10 amount of 3 M NaOAc (pH 5.2) buffer and an equal amount of 2-propanol were added. After cooling at -30°C for 1 hour, the RNA was collected in the form of a pellet by centrifugation.

Deprotection by photoirradiation: After HPLC purification, an aqueous solution containing the RNA was added to a transparent 96-multiwell plate, and irradiated with 365 nm light at a quantity of light of 4 mW/cm² for 10 minutes with a MAX-305 light source device (Asahi Spectra Co., Ltd.). Subsequently, the deprotected mRNA was isolated and purified by reverse-phase HPLC. Column: YMC-Triart Bio C4 (250 mm × 4.6 mm I.D.); Solvent A: 50 mM triethylammonium acetate (pH 7.0), 5% acetonitrile; Solvent B: acetonitrile; Linear gradient: 0 to 20% Solvent B (0 to 20 min); Flow rate: 1 mL/min; Detection wavelength: 260 nm; Column temperature: 50°C.

End analysis experiment: Nluc mRNA after purification was cleaved with DNAzyme (35-base-long DNA oligonucleotide) and converted into a mixture containing a 23-base-long 5'-end fragment, which was then analyzed by denaturing PAGE. The DNAzyme sequence used is as follows: 5'-TTCGAGGCCAGGCTAGCTACAACGAACGCGTCACC 3'-(SEQ ID NO: 11). The reaction liquid composition of the cleavage reaction is as follows. 104 ng/uL (0.5 µM) Nluc mRNA, 1 µM DNA, 5 mM Tris-HCl (pH 8.0), 5 mM magnesium chloride, reaction liquid volume 35 µL. The reaction liquid was warmed at 37°C for 1 hour, then a 1/10 amount of a 3 M sodium acetate buffer (pH 5.2), an equal amount of 2-propanol, and 14 ng of glycogen (coprecipitation agent) were added, the mixture was cooled at -30°C for 1 hour, and the RNA was collected in the form of a pellet by centrifugation. The resulting RNA was dissolved in 3.5 µL of water, and a 2.5 µL portion of the solution was analyzed by electrophoresis with 15% denaturing acrylamide gel containing 7.5 M urea as a denaturant. The gel after electrophoresis was stained with a nucleic acid staining reagent SYBR Gold, photographed by ChemiDoc MP gel imaging system (BioRad), and RNA bands were visualized. The molecular weight of the 5'-end RNA cleavage product was calculated using 1 µL of the RNA solution after alcohol precipitation as a substrate and using an MALDI-TOF mass spectrometer ultrafleXtreme (Bruker Corporation). Measurement was performed using 3-Hydroxy picolinic acid as a matrix and a Linear positive mode.

Translation experiment (HeLa cells): HeLa cells (RIKEN Cell Bank) were cultivated (37°C, 5% CO₂) in a Dulbecco's modified Eagle's medium (DMEM; WAKO) with addition of 10% fetal bovine serum (FBS; Invitrogen). On the day before transfection, the culture medium was removed from the HeLa cells (5 × 10³ cells/well) seeded in a 96-well plate and replaced with 40 µL/well Opti-MEM (registered trademark) (Thermo Fisher SCIENTIFIC), to which was added an mRNA (5 ng/well) mixed with Lipofectamine (registered trademark) MessengerMAX (registered trademark) (0.15 µL/well) and Opti-MEM (registered trademark) (10 µL/well). After incubation at 37°C for 3 hours, DMEM culture medium containing 10% FBS was added at 100 µL/well, followed by further cultivation. After 12, 24, and 48 hours, a cell lysate was prepared using NanoGlo (registered trademark) Luciferase Assay System (Promega), and the amount of the NanoLuc luciferase protein contained in the lysate was evaluated by a luminescence measurement method.

Translation experiment (JAWS II cells): JAWS II cells (ATCC) were cultivated (37°C, 5% CO₂) in an MEM α, nucleosides (Gibco) medium with addition of 10% fetal bovine serum (FBS; Invitrogen), 5 ng/mL. On the day before transfection, the culture medium was removed from the JAWS II cells (5 × 10³ cells/well) seeded in a 96-well plate and replaced with 40 µL/well Opti-MEM (registered trademark) (Thermo Fisher SCIENTIFIC), to which was then added an mRNA (5 ng/well) mixed with Lipofectamine (registered trademark) MessengerMAX (registered trademark) (0.15 µL/well) and Opti-MEM (registered trademark) (10 µL). After incubation at 37°C for 3 hours, an MEM α, nucleosides culture medium containing 10% FBS and 5 ng/mL GM-CSF was added at 100 µL/well, followed by further cultivation. After 24, 48, 72, and 120 hours, a cell lysate was prepared using NanoGlo (registered trademark) Luciferase Assay System (Promega), and the amount of the NanoLuc luciferase protein contained in the lysate was evaluated by a luminescence measurement method.

The amino acid sequence information of NLuc is as shown in SEQ ID NO: 12.

### (3) Comparison of translation activity of NanoLuc luciferase (Nluc) mRNAs synthesized using trinucleotide-type cap analog and tetranucleotide-type cap analog

Fig. 40 is a diagram showing a comparison of translation activities of 650-base-long NanoLuc luciferase (Nluc) mRNAs prepared using hydrophobic protecting group-containing trinucleotide type cap analogs (TriPure_0, TriPure_1), tetranucleotide-type cap analogs (TetraPure_2, TetraPure_2/m6A, TetraPure_2/G) and conventional cap analogs (Tri_1, Tetra_2). This is an experimental result showing that the former translation activity is high. A of this figure shows a chemical structure and an abbreviation of the cap analog used. B of this figure is an analysis result showing a purification process of a capped mRNA by reverse-phase HPLC using a hydrophobic protecting group. In the figure, i), ii), and iii) sequentially represent the result of analysis of the post-transcriptional reaction liquid, the result of analysis after HPLC isolation from the post-transcriptional reaction liquid, and the result of analysis after deprotection by irradiation with 365-nm light of the objective RNA isolated from the post-transcriptional reaction liquid. C of Fig. 40 shows the result of comparison of the activities of Nluc mRNAs using HeLa cells. D of this figure is the result of comparison of the activities of Nluc mRNAs using JAWS II cells. From the results of C and D of the figure, the mRNAs prepared using the hydrophobic protecting group-containing cap analogs (TetraPure_2, TetraPure_2/m6A, and TetraPure_2/G) showed higher translation activities than the mRNAs prepared using the conventional cap analogs (Tri_1, a compound having the same structure as CleanCap AG- sold by TriLink, and Tetra_2). Tri_1/AP and Tetra_2/AP represent mRNAs obtained by dephosphorylating the 5'-end of a coexisting uncapped RNA by acting Antarctic phosphatase on the mRNAs prepared using Tri_1 and Tetra_2, respectively. These dephosphorylated RNAs were prepared as follows. The reaction liquid (250 ng/µL mRNA, 50 mM Bis-Tris-Propane HCl, 1 mM MgCl₂, 0.1 mM ZnCl₂, pH 6.0 @ 25°C, 0.25 U/µL Antarctic phosphatase (New England Biolabs), reaction liquid volume: 20 µL) was warmed at 37°C for 30 minutes, and then extracted with an equal volume mixed solution of TE saturated phenol and chloroform, then extracted with chloroform, and then 1/10 amount of 3 M NaOAc (pH 5.2) buffer and an equal amount of 2-propanol were added. After cooling at -30°C for 1 hour, the RNA was collected in the form of a pellet by centrifugation.

The capped mRNA used in Fig. 40 was prepared as follows. An mRNA was transcriptionally synthesized from a DNA template using a T7 RNA polymerase reaction under the following conditions. 15 ng/uL dsDNA transcription template (PCR product of 676-bp containing T7 promoter sequence 5'TAATACGACTCACTATAG3'-(SEQ ID NO: 1) and containing a NanoLuc luciferase gene coding region derived from a pNL1.1 TK vector (Promega)), 2 mM ATP, 2 mM UTP, 2 mM CTP, 2mM GTP or [0.5 mM GTP + 2 mM Cap Analog], 40 mM Tris-HCl (pH 8.0), 8 mM MgCl₂, 2 mM spermidine, 5 mM DTT, 10 units/µL T7 RNA polymerase (Takara Bio Inc.). For the transcription reaction of the cap analogs (Tri_1, Tetra_2) containing no hydrophobic protecting group, different DNA templates having modified promoter sequences (DNA templates being PCR products of 677-bp containing a T7 promoter sequence 5'TAATACGACTCACTATAAG3'), which are known to be higher in the introduction efficiency of the cap analogs, were used. The reaction liquid was warmed at 37°C for 2 hours, then DNase (Takara Bio Inc.) was added thereto such that a final concentration was 0.1 units/µL, and the mixture was warmed at 37°C for 20 minutes. An equal volume mixed liquid of TE saturated phenol and chloroform was added to the reaction liquid in the same volume as the reaction liquid, and the mixture was vigorously stirred, then centrifuged, and the aqueous layer was taken. An equal amount of chloroform was added thereto, and the mixture was vigorously stirred again, then centrifuged, and the aqueous layer was taken. A 1/10 amount of a 3 M sodium acetate buffer (pH 5.2) and an equal amount of 2-propanol were added to the aqueous layer, and the mixture was cooled at minus 30°C for 1 hour and then centrifuged, affording a crude purified product of an RNA. The crude purified product of the RNA was purified by reverse-phase HPLC under the following conditions. Column: YMC-Triart Bio C4 (250 mm × 4.6 mm I.D.); Solvent A: 50 mM triethylammonium acetate (pH 7.0), 5% acetonitrile; Solvent B: acetonitrile; Linear gradient: 0 to 20% Solvent B (0 to 20 min); Flow rate: 1 mL/min; Detection wavelength: 260 nm; Column temperature: 50°C.

The elution time of the RNA transcript containing the hydrophobic protecting group-containing cap analog was 13.7 minutes to 14.0 minutes, whereas the elution time of the RNA transcript not containing the hydrophobic protecting group-containing cap analog was 15.5 minutes to 15.8 minutes, and both were different. As a result, it was possible to separate and purify the mRNA containing the hydrophobic protecting group-containing cap analog.

Deprotection by photoirradiation: An aqueous solution of the RNA containing the hydrophobic protecting group-containing cap analog isolated by reverse-phase HPLC was added to a transparent 96-multiwell plate, and irradiated with 365 nm light at a quantity of light of 4 mW/cm² for 10 minutes using a MAX-305 light source device (Asahi Spectra Co., Ltd.). Subsequently, the product was purified by reverse-phase HPLC under the following conditions. Column: YMC-Triart Bio C4 (250 mm × 4.6 mm I.D.); Solvent A: 50 mM triethylammonium acetate (pH 7.0), 5% acetonitrile; Solvent B: acetonitrile; Linear gradient: 0 to 20% Solvent B (0 to 20 min); Flow rate: 1 mL/min; Detection wavelength: 260 nm; Column temperature: 50°C.

End analysis: Nluc mRNA after purification was cleaved with DNAzyme (35-base-long DNA oligonucleotide) and converted into a mixture containing a 23-base-long 5'-end fragment, which was then analyzed by denaturing PAGE. The DNAzyme sequence used is as follows: 5'-TTCGAGGCCAGGCTAGCTACAACGAACGCGTCACC 3'-(SEQ ID NO: 11). The reaction liquid composition of the cleavage reaction is as follows. 104 ng/µL (0.5 µM) Nluc mRNA, 1 µM DNAzyme, 5 mM Tris-HCl (pH 8.0), 5 mM magnesium chloride, reaction liquid volume 40 µL. The reaction liquid was warmed at 37°C for 1 hour, then a 1/10 amount of a 3 M sodium acetate buffer (pH 5.2), an equal amount of 2-propanol, and 14 ng of glycogen (coprecipitation agent) were added, the mixture was cooled at - 30°C for 1 hour, and the RNA was collected in the form of a pellet by centrifugation. The resulting RNA was dissolved in 4 µL of water, and a 3 µL portion of the solution was analyzed by electrophoresis with 15% denaturing acrylamide gel containing 7.5 M urea as a denaturant. The gel after electrophoresis was stained with a nucleic acid staining reagent SYBR Gold, photographed by ChemiDoc MP gel imaging system (BioRad), and RNA bands were visualized.

Translation experiment (HeLa cells): HeLa cells (RIKEN Cell Bank) were cultivated (37°C, 5% CO₂) in a Dulbecco's modified Eagle's medium (DMEM; WAKO) with addition of 10% fetal bovine serum (FBS; Invitrogen) . On the day before transfection, the culture medium was removed from the HeLa cells (5 × 10³ cells/well) seeded in a 96-well plate and replaced with 40 µL/well Opti-MEM (registered trademark) (Thermo Fisher SCIENTIFIC), to which was added an mRNA (5 ng/well) mixed with Lipofectamine (registered trademark) MessengerMAX (registered trademark) (0.15 µL/well) and Opti-MEM (registered trademark) (10 µL/well). After incubation at 37°C for 3 hours, DMEM culture medium containing 10% FBS was added at 100 µL/well, followed by further cultivation. After 12, 24, and 48 hours, a cell lysate was prepared using NanoGlo (registered trademark) Luciferase Assay System (Promega), and the amount of the NanoLuc luciferase protein contained in the lysate was evaluated by a luminescence measurement method.

Translation experiment (JAWS II cells): JAWS II cells (ATCC) were cultivated (37°C, 5% CO₂) in an MEM α, nucleosides (Gibco) medium with addition of 10% fetal bovine serum (FBS; Invitrogen), 5 ng/mL. On the day before transfection, the culture medium was removed from the JAWS II cells (5 × 10³ cells/well) seeded in a 96-well plate and replaced with 40 µL/well Opti-MEM (registered trademark) (Thermo Fisher SCIENTIFIC), to which was then added an mRNA (5 ng/well) mixed with Lipofectamine (registered trademark) MessengerMAX (registered trademark) (0.15 µL/well) and Opti-MEM (registered trademark) (10 µL). After incubation at 37°C for 3 hours, an MEM α, nucleosides culture medium containing 10% FBS and 5 ng/mL GM-CSF was added at 100 µL/well, followed by further cultivation. After 24, 48, 72, and 120 hours, a cell lysate was prepared using NanoGlo (registered trademark) Luciferase Assay System (Promega), and the amount of the NanoLuc luciferase protein contained in the lysate was evaluated by a luminescence measurement method.

### (4) Isolation and purification of longer chain capped mRNA by reverse-phase HPLC

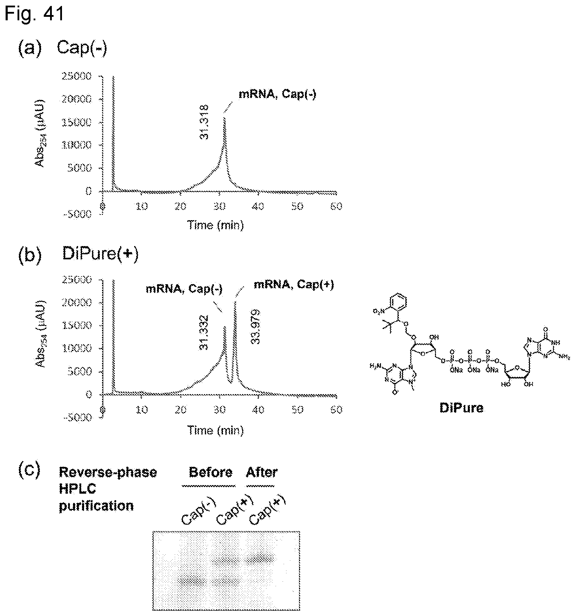

Fig. 41 shows the result of analysis by reverse-phase HPLC of a capped mRNA prepared by adding a hydrophobic protecting group-containing cap analog (DiPure) at the time of transcription of a 4247-base-long RNA strand. Comparison between the case (a) where the present cap analog is not added and the case (b) where the present cap analog is added shows that the elution time of the uncapped form is 31.3 minutes whereas the elution time of the capped RNA is 34.0 minutes. It can be seen that these two have different elution times, whereby the capped RNA and the uncapped RNA can be separated. The mRNA separated was cut at the position of 23 bases from the 5'-end of the mRNA using DNAzyme, and the cut product was analyzed by denaturing PAGE. The result of the analysis was shown in (c). It can be seen that the capped mRNA was isolated well by purification utilizing the hydrophobic protecting group of the cap structure.

The template DNA sequence (4375-bp) of Spike protein mRNA is as shown in SEQ ID NO: 13. The sequence (transcript) 4247 base of Spike protein mRNA is as shown in SEQ ID NO: 14.

The capped mRNA used in Fig. 41 was transcriptionally synthesized in the following reaction liquid from a DNA template using T7 RNA polymerase. 5 ng/uL dsDNA transcription template (PCR product of 4375-bp containing T7 promoter sequence 5'TAATACGACTCACTATAG3'-(SEQ ID NO: 1)), 2 mM ATP, 2 mM UTP, 2 mM CTP, 2 mM GTP, 2 mM PureCap Analog (DiPure), 40 mM Tris-HCl (pH 8.0), 8 mM MgCl₂, 2 mM spermidine, 5 mM DTT, 10 units/µL T7 RNA polymerase (Takara Bio Inc.). The reaction liquid was warmed at 37°C for 2 hours, then DNase (Takara Bio Inc.) was added thereto such that a final concentration was 0.1 units/µL, and the mixture was warmed at 37°C for 20 minutes. An equal volume mixed liquid of TE saturated phenol and chloroform was added in the same volume as the reaction liquid, and the mixture was vigorously stirred, then centrifuged, and the aqueous layer was taken. An equal amount of chloroform was added thereto, and the mixture was vigorously stirred, then centrifuged, and the aqueous layer was taken. A 1/10 amount of a 3 M sodium acetate buffer (pH 5.2) and an equal amount of 2-propanol were added to the aqueous layer, and the mixture was cooled at minus 30°C for 1 hour and then centrifuged, affording a crude purified product of an RNA. The crude purified product of the RNA obtained was analyzed by reverse-phase HPLC under the following conditions. Column: Nacalai Tesque Cosmosil RNA RP-1 (200 mm × 4.6 mm I.D.); Solvent A: 100 mM triethylammonium acetate (pH 7.0), 5% acetonitrile; Solvent B: 100 mM triethylammonium acetate (pH 7.0), 50% acetonitrile; Linear gradient: 10 to 25% Solvent B (0 to 60 min); Flow rate: 1 mL/min; Detection wavelength: 254 nm; Column temperature: 50°C.

The mRNA after purification was cleaved with DNAzyme (39-base-long DNA oligonucleotide) and converted into a mixture containing a 23-base-long 5'-end fragment, which was then analyzed by denaturing PAGE. The DNAzyme sequences used are as follows: 5'-TCTGTGGGGAGGCTAGCTACAACGACAGAAGAATACTAG 3'-(SEQ ID NO: 19). The reaction liquid composition of the cleavage reaction is as follows. 688 ng/µL mRNA (DiPure), 1 µM DNAzyme, 5 mM Tris-HCl (pH 8.0), 5 mM magnesium chloride, reaction liquid volume 9 µL. The reaction liquid was warmed at 37°C for 1 hour, then 2 µL of a 3 M sodium acetate buffer (pH 5.2), 20 µL of 2-propanol, and 20 ng of glycogen (coprecipitation agent) were added, the mixture was cooled at -30°C for 1 hour, and the RNA was collected in the form of a pellet by centrifugation. The resulting RNA was dissolved in 3 µL of water, and a 3 µL portion of the solution was analyzed by electrophoresis with 15% denaturing acrylamide gel containing 7.5 M urea as a denaturant. The gel after electrophoresis was stained with a nucleic acid staining reagent SYBR Gold, photographed by ChemiDoc MP gel imaging system (BioRad), and RNA bands were visualized.

### (5) Advantages of PureCap type mRNA in comparison with conventional mRNA

Fig. 42 shows the result that a PureCap type mRNA exhibits a high amount of protein synthesized without introducing methylpseudouridine (see JP2021-35979A). With conventional ARCA (Cap_0) and CleanCap (Cap_1), introduction of methylpseudouridine increases the amount of protein synthesized. On the other hand, it was shown that with PureCap, introduction of methylpseudouridine reduces the amount of protein synthesized. It was shown throughout that the protein synthesis amount of PureCap type mRNA was high.

A 1Methylpseudouridine-modified mRNA and an unmodified mRNA were prepared as follows.

An mRNA was transcriptionally synthesized from a DNA template using a T7 RNA polymerase reaction under the following conditions. 15 ng/uL dsDNA transcription template (PCR product of 676-bp containing T7 promoter sequence 5'TAATACGACTCACTATAG3'-(SEQ ID NO: 1) and containing a NanoLuc luciferase gene coding region derived from a pNL1.1 TK vector (Promega)), 2 mM ATP, 2 mM 1 methylpseudo UTP or UTP, 2 mM CTP, 2 mM GTP, 2 mM PureCap-type cap analog or [0.5 mM GTP + 2 mM ARCA cap analog (Jena Biosciences)], 40 mM Tris-HCl (pH 8.0), 8 mM MgCl₂, 2 mM spermidine, 5 mM DTT, 10 units/µL T7 RNA polymerase (Takara Bio Inc.). The reaction liquid was warmed at 37°C for 2 hours, then DNase (Takara Bio Inc.) was added thereto such that a final concentration was 0.1 units/µL, and the mixture was warmed at 37°C for 20 minutes. An equal volume mixed liquid of TE saturated phenol and chloroform was added to the reaction liquid in the same volume as the reaction liquid, and the mixture was vigorously stirred, then centrifuged, and the aqueous layer was taken. An equal amount of chloroform was added thereto, and the mixture was vigorously stirred again, then centrifuged, and the aqueous layer was taken. A 1/10 amount of a 3 M sodium acetate buffer (pH 5.2) and an equal amount of 2-propanol were added to the aqueous layer, and the mixture was cooled at minus 30°C for 1 hour and then centrifuged, affording a crude purified product of an RNA. The crude purified product of the RNA was purified by reverse-phase HPLC under the following conditions. Column: YMC-Triart Bio C4 (250 mm × 4.6 mm I.D.); Solvent A: 50 mM triethylammonium acetate (pH 7.0), 5% acetonitrile; Solvent B: acetonitrile; Linear gradient: 0 to 20% Solvent B (0 to 20 min); Flow rate: 1 mL/min; Detection wavelength: 260 nm; Column temperature: 50°C.

The elution time of the RNA transcript containing the hydrophobic protecting group-containing cap analog was 16.0 minutes to 16.8 minutes, whereas the elution time of the RNA transcript not containing the hydrophobic protecting group-containing cap analog was 13.4 minutes to 14.0 minutes, and both were different. As a result, it was possible to separate and purify the mRNA containing the hydrophobic protecting group-containing cap analog.

The translation activity of the mRNA prepared as described above was evaluated as follows. JAWS II cells (ATCC) were cultivated (37°C, 5% CO₂) in an MEM α, nucleosides (Gibco) medium with addition of 10% fetal bovine serum (FBS; Invitrogen), 5 ng/mL. On the day before transfection, the culture medium was removed from the JAWS II cells (5 × 10³ cells/well) seeded in a 96-well plate and replaced with 40 µL/well Opti-MEM (registered trademark) (Thermo Fisher SCIENTIFIC), to which was then added an mRNA (5 ng/well) mixed with Lipofectamine (registered trademark) MessengerMAX (registered trademark) (0.15 µL/well) and Opti-MEM (registered trademark) (10 µL). After incubation at 37°C for 3 hours, an MEM α, nucleosides culture medium containing 10% FBS and 5 ng/mL GM-CSF was added at 100 µL/well, followed by further cultivation. After 24, 48, 72, and 120 hours, a cell lysate was prepared using NanoGlo (registered trademark) Luciferase Assay System (Promega), and the amount of the NanoLuc luciferase protein contained in the lysate was evaluated by a luminescence measurement method.

Fig. 43 shows the result of the evaluation of an intracellular immune response (HEK293 NF-kB cells), and it was revealed that PureCap type mRNAs exhibit lower immune response than conventional mRNAs such as ARCA.

NF-κB reporter (Luc)-HEK 293 cells (BPS Bioscience Inc.) were cultivated (37°C, 5% CO₂) in a Dulbecco's modified Eagle's medium (DMEM; WAKO) with addition of 10% fetal bovine serum (FBS; Invitrogen), 100 µg/ml of Hygromycin B. The day before transfection, NF-κB reporter (Luc)-HEK 293 cells were seeded in a 48-well plate (7.5 × 10⁴ cells/well). The next day, the medium was removed and replaced with 225 µL/well Opti-MEM (registered trademark) (Thermo Fisher SCIENTIFIC). 0.375 µι. Lipofectamine (registered trademark) Messenger MAX (registered trademark) and 12.5 ng mRNA (DiPure) were diluted in 25 µL Opti-MEM (registered trademark) and introduced into cells. After incubation at 37°C for 5 hours, 50 µL/well 1 × Cell Lysis Buffer (Promega) was added to lyse the cells. Luminescence was measured using ONE-Glo (registered trademark) luciferase assay (registered trademark) (Promega). The protein amount was quantified using Pierce (registered trademark) BCA Protein Assay Kit (Thermo Fisher SCIENTIFIC), and the luminescence value was corrected by the number of cells.

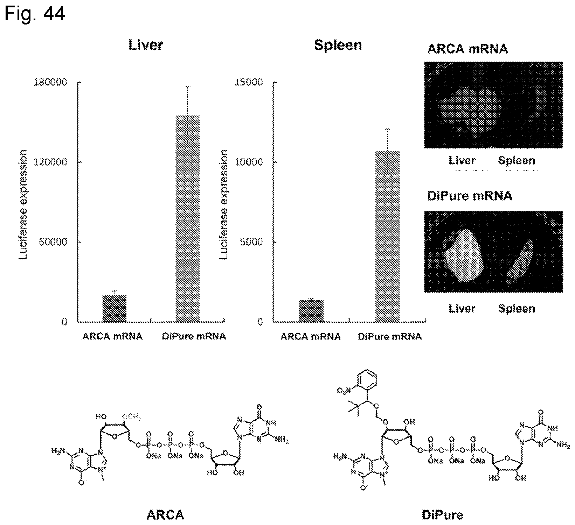

Fig. 44 shows the result that a PureCap mRNA with high purity exhibited higher protein expression ability than an ARCA mRNA in an animal individual. As a result of introducing a luc mRNA into a mouse using delivery alone, it was revealed that the PureCap type mRNA has high protein expression ability. As a method for administering the mRNA to a mouse, the mRNA was encapsulated in lipid nanoparticles (LNP). The mRNA-encapsulating LNP was prepared using an ultra high-speed nanomedicament producing apparatus (nanoAssemblr Spark) (nitrogen/phosphate ratio (N/P ratio) = 5, D-Lin-MC3-DMA:DSPC:Cholesterol:DMG-PEG2000 = 50:10:38.5:1.5 (molar ratio)). The mRNA-encapsulating LNP prepared was administered to balb/c female 7w from the tail vein so as to be 2 µg mRNA/mouse. After 4 hours, the mouse was sacrificed and organs were homogenized in Passive Lysis Buffer (Promega). Luminescence was detected using NanoLuc (registered trademark) Luciferase Technology (Promega).

Fig. 45 shows the result of evaluation of an intracellular immune response (HEK293 NF-kB cell). Fig. 45(a) shows the result of the immune response when 50 ng and 100 ng of an mRNA were administered to HEK 293 NF-kB cells, and Fig. 45(b) shows the result of the immune response when 100 ng of an mRNA was administered to HEK 293 NF-kB cells. It was revealed that PureCap type mRNAs (DiPure, TriPure_0, TriPure_1, TetraPure_2, and TetraPure_2/m6A) showed lower immune response than conventional mRNAs synthesized using ARCA.

NF-κB reporter (Luc)-HEK 293 cells (BPS Bioscience Inc.) were cultivated (37°C, 5% CO₂) in a Dulbecco's modified Eagle's medium (DMEM; WAKO) with addition of 10% fetal bovine serum (FBS; Invitrogen), 100 µg/ml of Hygromycin B. The day before transfection, NF-κB reporter (Luc)-HEK 293 cells were seeded in a 48-well plate (1.0 × 10⁵ cells/well). The next day, the medium was removed and replaced with 240 µL/well Opti-MEM (registered trademark) (Thermo Fisher SCIENTIFIC). 0.15 µL Lipofectamine (registered trademark) Messenger MAX (registered trademark) and 100 ng mRNA were diluted in 10 µL Opti-MEM (registered trademark) and introduced into cells. After incubation at 37°C for 24 hours, 50 µL/well 1 × Cell Lysis Buffer (Promega) was added to lyse the cells. Luminescence was measured using ONE-Glo (registered trademark) luciferase assay (registered trademark) (Promega). The protein amount was quantified using Pierce (registered trademark) BCA Protein Assay Kit (Thermo Fisher SCIENTIFIC), and the luminescence value was corrected by the number of cells.

Sequence Listing

## Claims

1. A method for purifying a nucleotide-based substance having at least one nucleotide and/or a derivative thereof as a constituent unit, the method comprising:
a protecting group introduction step of introducing a hydrophobic protecting group represented by the following formula (P1) or (P2) into the nucleotide-based substance to produce a hydrophobic nucleotide-based substance;
an isolation and purification step of isolating and purifying the hydrophobic nucleotide-based substance under a hydrophobic environment; and
a deprotection step of deprotecting the hydrophobic protecting group from the hydrophobic nucleotide-based substance to produce the nucleotide-based substance, 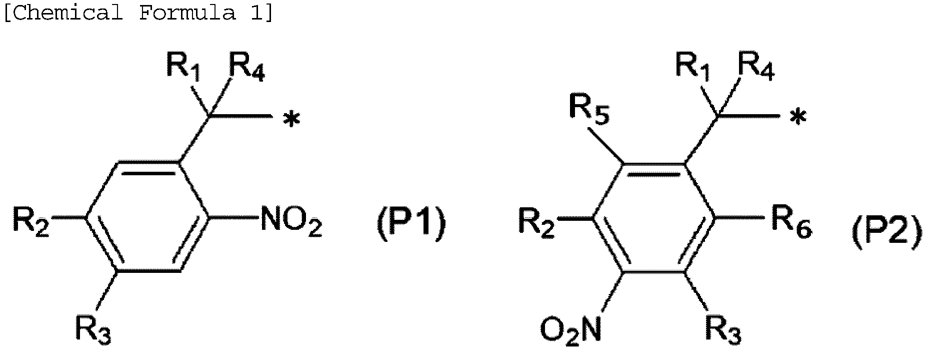
wherein R₁ represents a linear or branched alkyl group having 1 to 30 carbon atoms, R₄ represents hydrogen or a linear or branched alkyl group having 1 to 10 carbon atoms, R₂, R₃, R₅ and R₆ each represent hydrogen, a linear or branched alkyl group having 1 to 10 carbon atoms, or a linear or branched alkoxy group having 1 to 10 carbon atoms, and R₂, R₃, R₅ and R₆ may be the same or different; and * means a bond with a nucleotide-based substance, and is bonded to an atom constituting the nucleotide-based substance directly or with a linking group selected from -O-C- and -O-C(=O)- interposed therebetween.

2. The method for purifying a nucleotide-based substance according to claim 1, wherein the hydrophobic protecting group is represented by the following formula (P3), 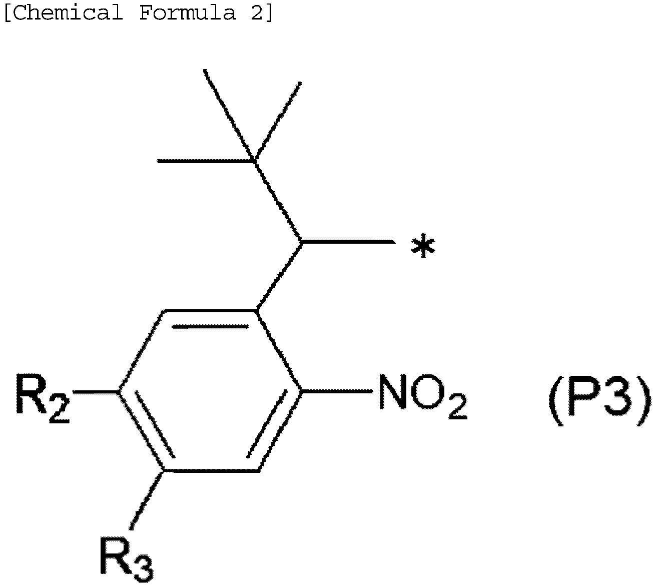 wherein R₂ and R₃ are as defined in claim 1.

3. The method for purifying a nucleotide-based substance according to claim 1, wherein the protecting group introduction step introduces the hydrophobic protecting group into a nucleotide located at a 5'-end of the nucleotide-based substance.

4. The method for purifying a nucleotide-based substance according to claim 3, wherein the protecting group introduction step introduces the hydrophobic protecting group into a 5'-phosphate group, a 2'-hydroxyl group, or a base of a sugar constituting the nucleotide.

5. The method for purifying a nucleotide-based substance according to claim 1, wherein in the protecting group introduction step, a protecting group-introduced nucleotide is synthesized by introducing an amidite reagent represented by the following formula (CR1) into a nucleotide, and a mononucleotide is sequentially linked to a 3'-end side of the protecting group-introduced nucleotide by solid phase synthesis to synthesize the hydrophobic nucleotide-based substance, 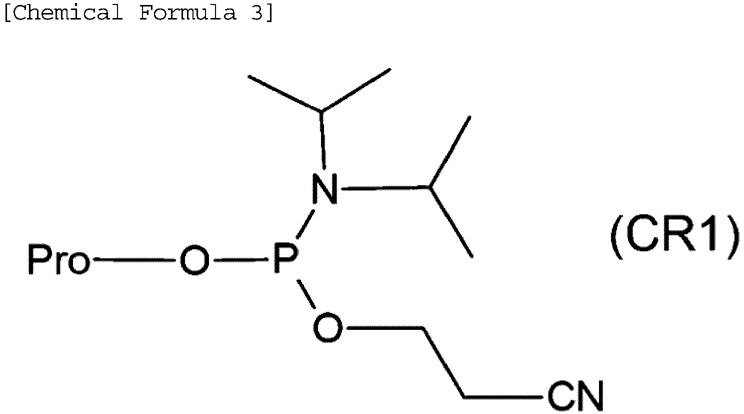 wherein Pro represents the hydrophobic protecting group.

6. The method for purifying a nucleotide-based substance according to claim 1, wherein in the protecting group introduction step, the hydrophobic nucleotide-based substance is synthesized by employing, as a template, a nucleic acid having a sequence complementary to the nucleotide-based substance, employing, as substrates, a hydrophobic substrate represented by the following formula (ER1) and a nucleoside triphosphate, and transcribing the template by an RNA polymerase, 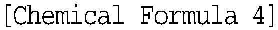 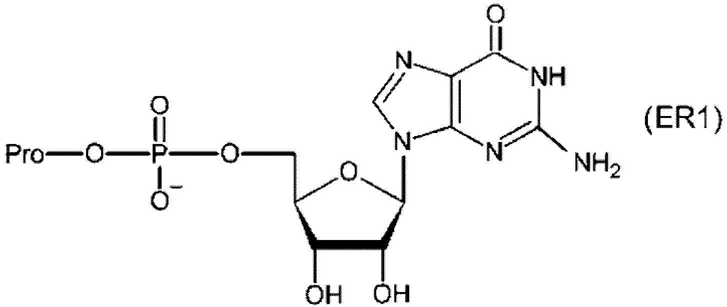 wherein Pro represents the hydrophobic protecting group.

7. The method for purifying a nucleotide-based substance according to claim 1, wherein in the protecting group introduction step, an adenylation reagent represented by the following formula (CA1) is reacted with a phosphate group at a 5'-end of an oligonucleotide containing the nucleotide-based substance to synthesize the hydrophobic nucleotide-based substance, 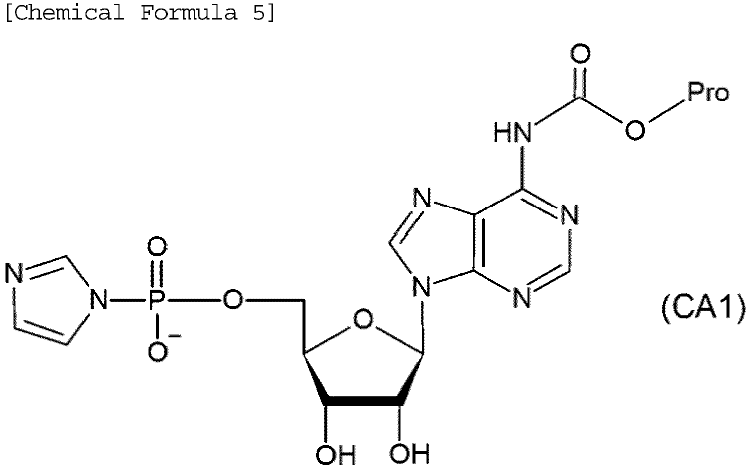 wherein Pro represents the hydrophobic protecting group.

8. The method for purifying a nucleotide-based substance according to claim 1, wherein in the protecting group introduction step, the hydrophobic nucleotide-based substance is synthesized by employing, as a template, a nucleic acid having a sequence complementary to the nucleotide-based substance, employing, as substrates, a hydrophobic substrate represented by the following formula (EA1) and a nucleoside triphosphate, and transcribing the template by an RNA polymerase, 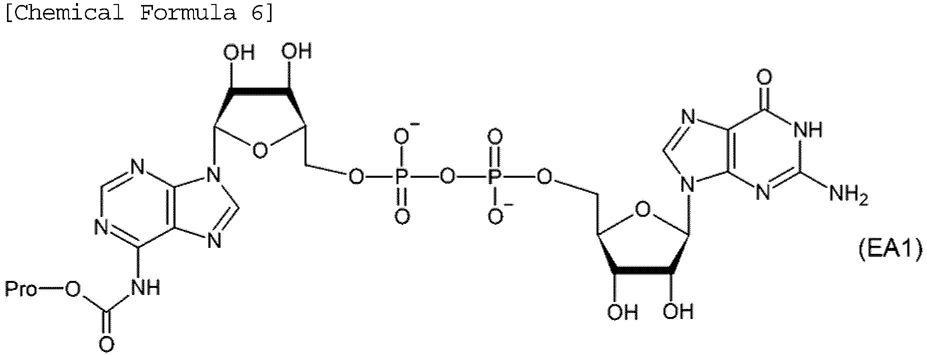 wherein Pro represents the hydrophobic protecting group.

9. The method for purifying a nucleotide-based substance according to claim 1, wherein in the protecting group introduction step, a capping reagent selected from the group consisting of the following formulas (CC1) to (CC4) is reacted with a phosphate group at a 5'-end of an oligonucleotide containing the nucleotide-based substance to synthesize the hydrophobic nucleotide-based substance, 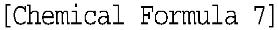 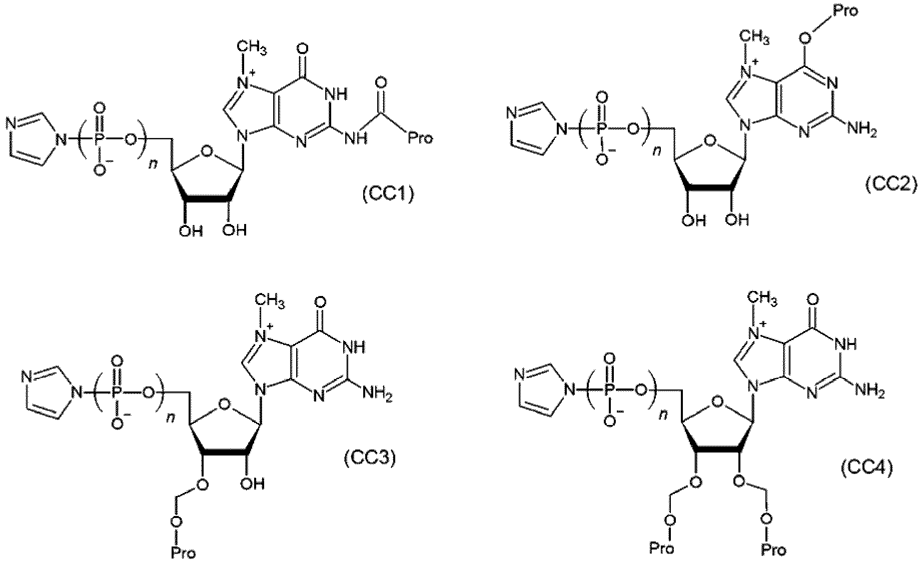 wherein Pro represents the hydrophobic protecting group, and n represents an integer of 1 or 2.

10. The method for purifying a nucleotide-based substance according to claim 1, wherein in the protecting group introduction step, the hydrophobic nucleotide-based substance is synthesized by employing, as a template, a nucleic acid having a sequence complementary to the nucleotide-based substance, employing, as substrates, a hydrophobic substrate containing a structure of the following formula (ECO) and a nucleoside triphosphate, and transcribing the template by an RNA polymerase, 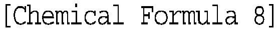 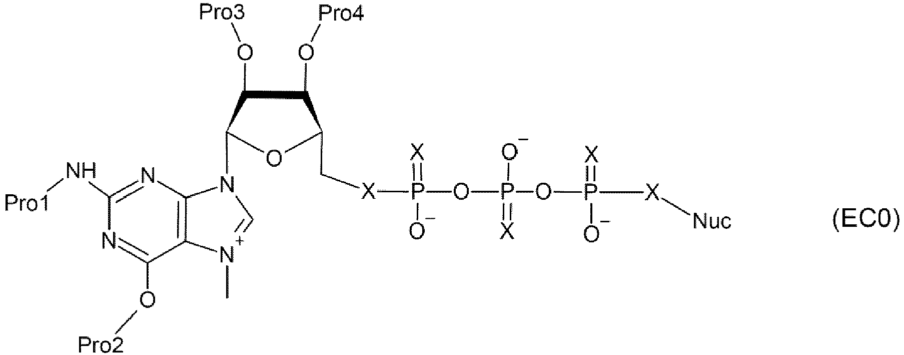 wherein Pro1 to Pro4 each represent the hydrophobic protecting group or hydrogen, and Pro1 to Pro4 may be the same or different from each other; X is selected from the group consisting of oxygen, sulfur and selenium atoms; and Nuc represents a natural or non-natural nucleoside or one or more natural or non-natural nucleotides on carbon at a 3'-position thereof.

11. The method for purifying a nucleotide-based substance according to claim 10, wherein the Nuc is a natural or non-natural nucleoside or a natural or non-natural nucleoside in which one or two natural or non-natural nucleotides are linked to the carbon at the 3'-position thereof.

12. The method for purifying a nucleotide-based substance according to claim 10, wherein the hydrophobic substrate having the structure of the formula (ECO) is selected from the group consisting of the following formulas (EC1) to (EC4) and the following formulas (EC5) to (EC12), 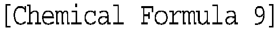 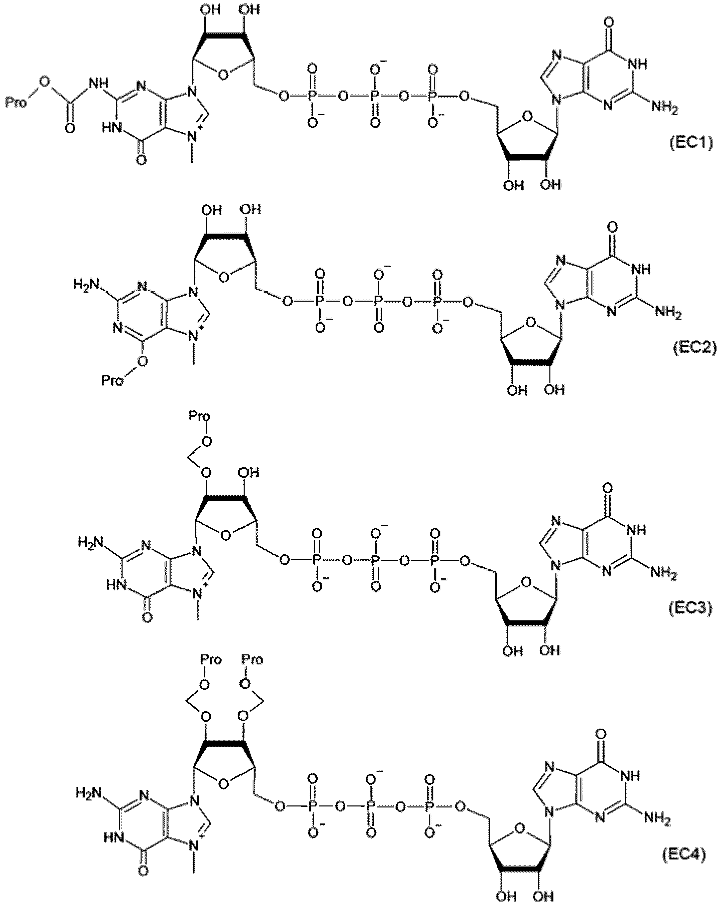
wherein Pro represents the hydrophobic protecting group, 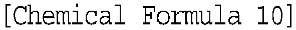 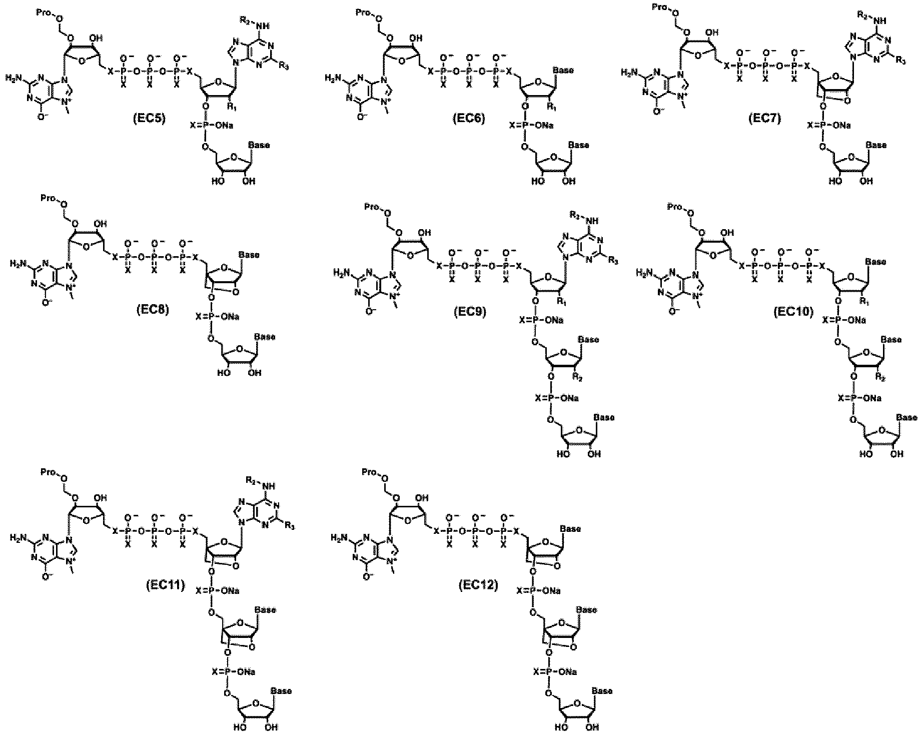
wherein R₁ represents a substituent selected from among a hydroxy group (OH), a methoxy group (OCH₃), methoxyethyl (-OCH₂OCH₃), fluorine (F), and a formamide group (-NHCHO), R₂ represents hydrogen, a linear or branched alkyl group having 1 to 10 carbon atoms, and R₃ represents hydrogen or an amino group (-NH₂); wherein Base is a natural nucleobase selected from among adenine, cytosine, thymine, uracil, and guanine, or represents a non-natural base; wherein X is selected from the group consisting of oxygen, sulfur, and selenium atoms; and wherein Pro represents a hydrophobic protecting group represented by the formula (P1) or (P2) .

13. The method for purifying a nucleotide-based substance according to claim 10, wherein the hydrophobic substrate is a cap derivative represented by the formula (ECO), and
before the deprotection step, translation is performed using the hydrophobic nucleotide-based substance to which the hydrophobic protecting group is bonded.

14. The method for purifying a nucleotide-based substance according to claim 1, wherein in the isolation and purification step, the hydrophobic nucleotide-based substance is isolated and purified by high-performance liquid chromatography.

15. The method for purifying a nucleotide-based substance according to claim 1, wherein in the deprotection step, the hydrophobic protecting group is deprotected by photoirradiation.

16. A device for purifying a nucleotide-based substance having at least one nucleotide and/or a derivative thereof as a constituent unit, the device comprising:
a protecting group introduction means for introducing a hydrophobic protecting group represented by the following formula (P1) or (P2) into a nucleotide-based substance to produce a hydrophobic nucleotide-based substance;
an isolation and purification means for isolating and purifying the hydrophobic nucleotide-based substance under a hydrophobic environment; and
a deprotection means for deprotecting the hydrophobic protecting group from the hydrophobic nucleotide-based substance to produce the nucleotide-based substance, 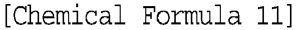 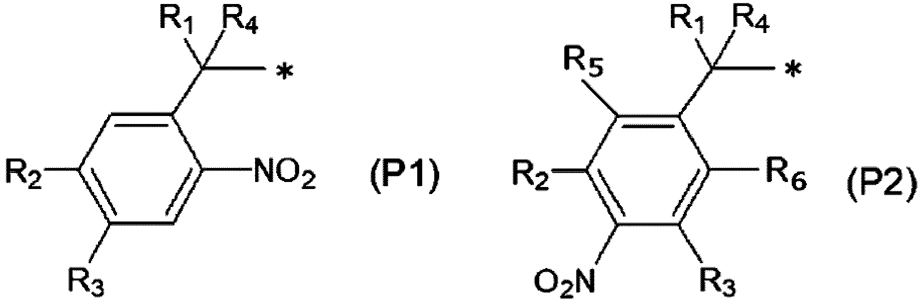
wherein R₁ represents a linear or branched alkyl group having 1 to 30 carbon atoms, R₄ represents hydrogen or a linear or branched alkyl group having 1 to 10 carbon atoms, R₂, R₃, R₅ and R₆ each represent hydrogen, a linear or branched alkyl group having 1 to 10 carbon atoms, or a linear or branched alkoxy group having 1 to 10 carbon atoms, and R₂, R₃, R₅ and R₆ may be the same or different; and * means a bond with a nucleotide-based substance, and is bonded to an atom constituting the nucleotide-based substance directly or with a linking group selected from -O-C- and -O-C(=O)- interposed therebetween.

17. A hydrophobic reagent for synthesizing a hydrophobic nucleotide-based substance by chemical synthesis, wherein the hydrophobic reagent is selected from the group consisting of the following formulas (CR1), (CA1), and (CC1) to (CC4), 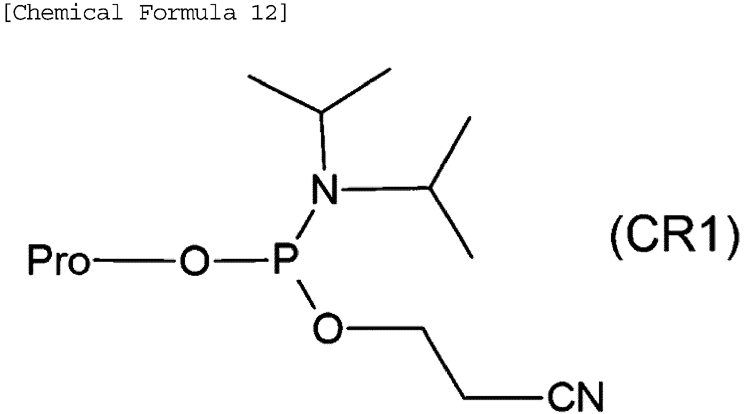 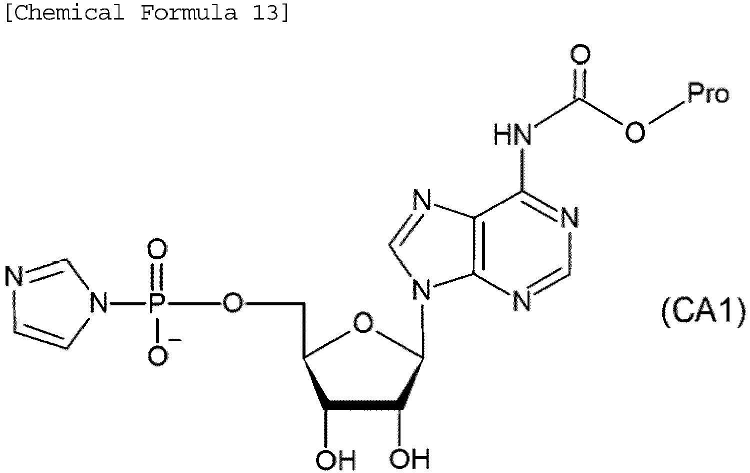 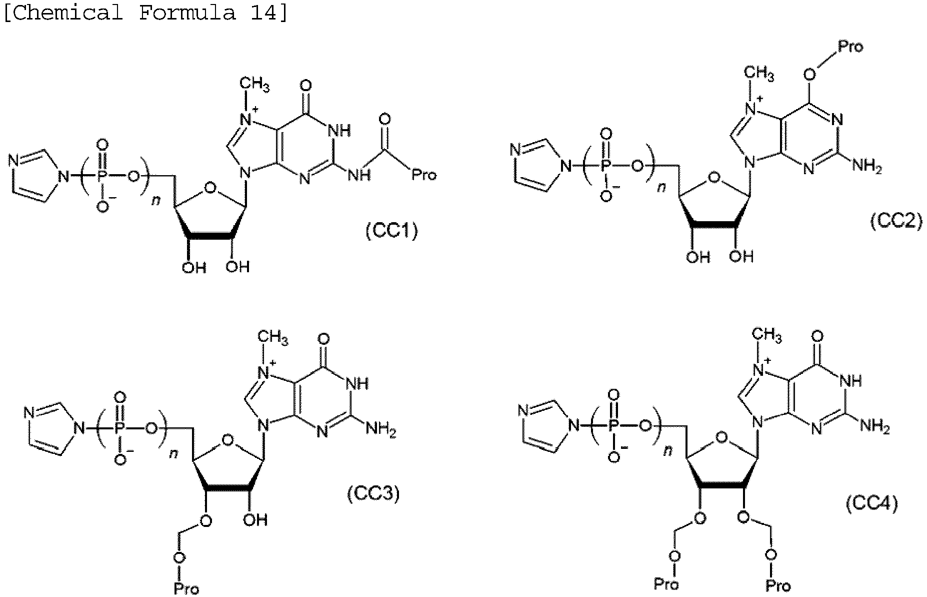
wherein Pro represents a hydrophobic protecting group represented by the following formula (P1) or (P2), and n represents an integer of 1 or 2, 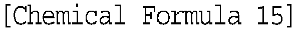 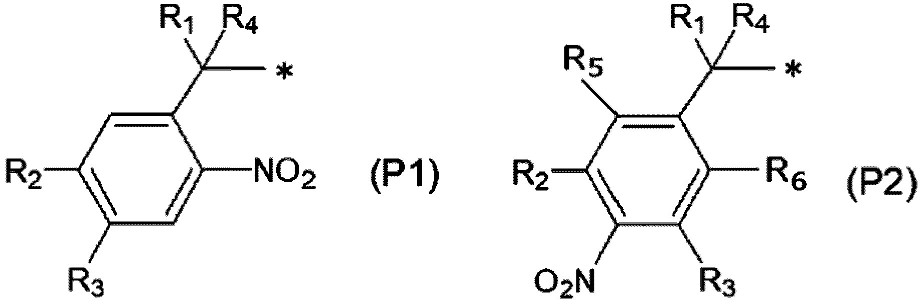
wherein R₁ represents a linear or branched alkyl group having 1 to 30 carbon atoms, R₄ represents hydrogen or a linear or branched alkyl group having 1 to 10 carbon atoms, R₂, R₃, R₅ and R₆ each represent hydrogen, a linear or branched alkyl group having 1 to 10 carbon atoms, or a linear or branched alkoxy group having 1 to 10 carbon atoms, and R₂, R₃, R₅ and R₆ may be the same or different; and * means a bond.

18. A hydrophobic substrate for producing a hydrophobic nucleotide-based substance by an RNA polymerase, wherein the hydrophobic substrate is selected from the group consisting of the following formulas (ER1), (EA1), and (ECO), 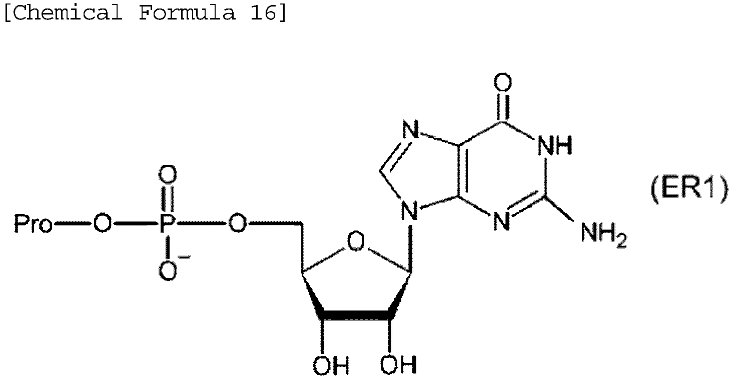
wherein Pro represents the hydrophobic protecting group represented by the following formula (P1) or (P2), 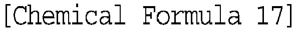 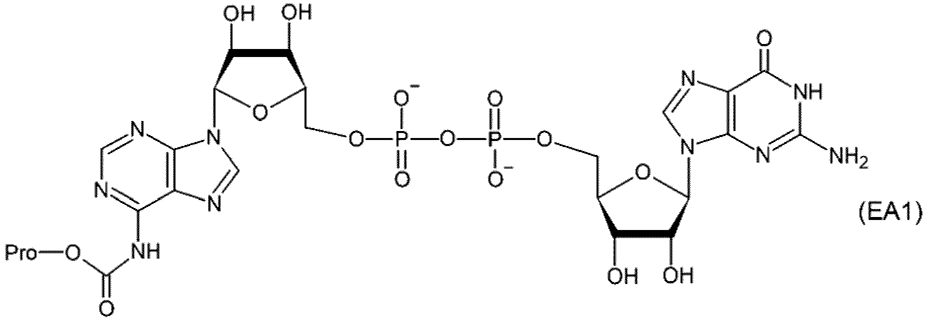
wherein Pro represents the hydrophobic protecting group represented by the following formula (P1) or (P2), 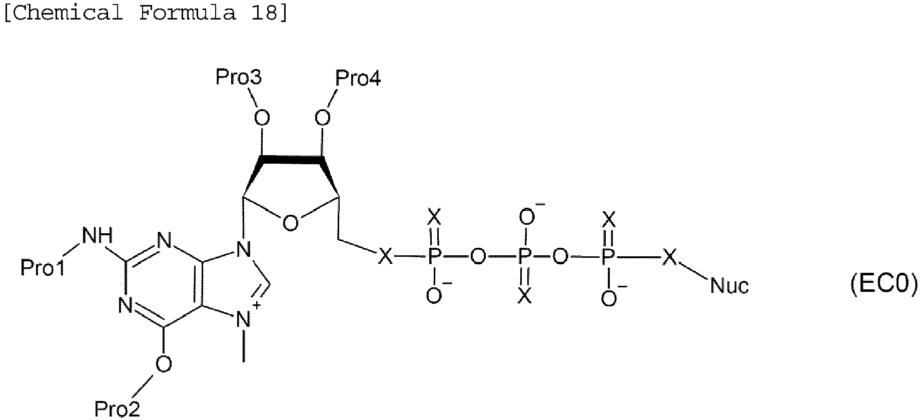
wherein Pro1 to Pro4 each represent a hydrophobic protecting group represented by the following formula (P1) or (P2) or hydrogen, provided that at least one of Pro1 to Pro4 is a hydrophobic protecting group, and Pro1 to Pro4 may be the same or different from each other; X is selected from the group consisting of oxygen, sulfur and selenium atoms; and Nuc represents a natural or non-natural nucleoside or one or more natural or non-natural nucleotides on carbon at a 3'-position thereof, 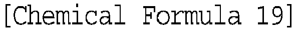 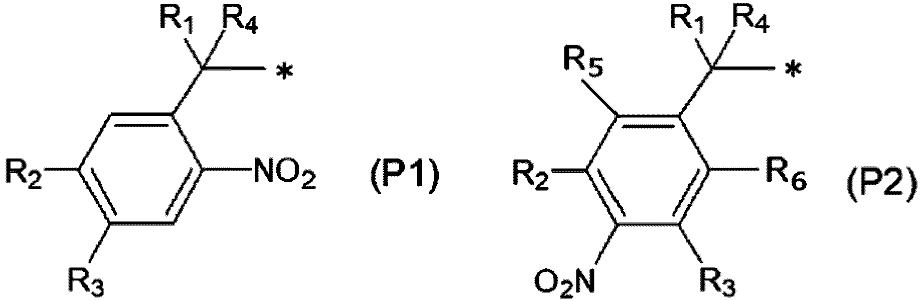
wherein R₁ represents a linear or branched alkyl group having 1 to 30 carbon atoms, R₄ represents hydrogen or a linear or branched alkyl group having 1 to 10 carbon atoms, R₂, R₃, R₅ and R₆ each represent hydrogen, a linear or branched alkyl group having 1 to 10 carbon atoms, or a linear or branched alkoxy group having 1 to 10 carbon atoms, and R₂, R₃, R₅ and R₆ may be the same or different; and * means a bond.

19. The hydrophobic substrate according to claim 18, wherein the Nuc is a natural or non-natural nucleoside or a natural or non-natural nucleoside in which one or two natural or non-natural nucleotides are linked to the carbon at the 3'-position thereof.

20. An mRNA drug comprising a hydrophobic nucleotide-based substance in which a hydrophobic protecting group represented by the following formula (P1) or (P2) is introduced into a nucleotide-based substance having at least one nucleotide and/or a derivative thereof as a constituent unit, 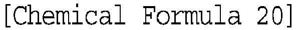 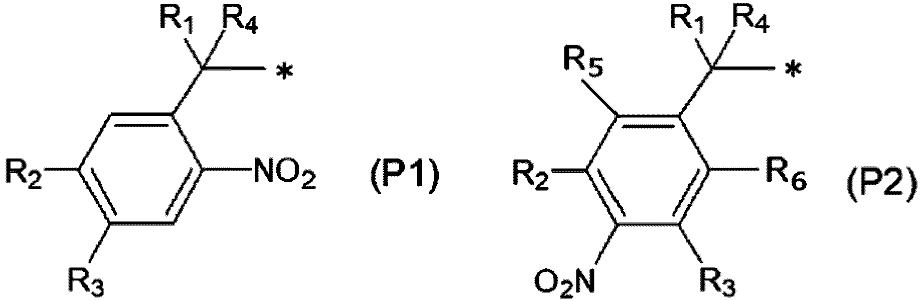 wherein R₁ represents a linear or branched alkyl group having 1 to 30 carbon atoms, R₄ represents hydrogen or a linear or branched alkyl group having 1 to 10 carbon atoms, R₂, R₃, R₅ and R₆ each represent hydrogen, a linear or branched alkyl group having 1 to 10 carbon atoms, or a linear or branched alkoxy group having 1 to 10 carbon atoms, and R₂, R₃, R₅ and R₆ may be the same or different; and * means a bond with a nucleotide-based substance, and is bonded to an atom constituting the nucleotide-based substance directly or with a linking group selected from -O-C- and -O-C(=O)- interposed therebetween.

21. The mRNA drug according to claim 20, having a structure in which RNA is linked to carbon at a 3'-position of a nucleotide on a 3'-end side of a hydrophobic substrate containing a structure of the following formula (ECO), 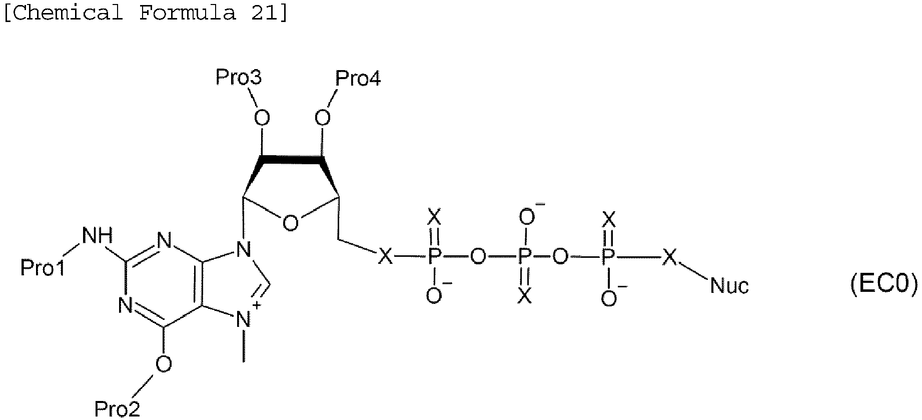 wherein Pro1 to Pro4 each represent the hydrophobic protecting group or hydrogen, provided that at least one of Pro1 to Pro4 is a hydrophobic protecting group, and Pro1 to Pro4 may be the same or different from each other; X is selected from the group consisting of oxygen, sulfur and selenium atoms; and Nuc represents a natural or non-natural nucleoside or one or more natural or non-natural nucleotides on carbon at a 3'-position thereof.

22. The mRNA drug according to claim 20, wherein the Nuc is a natural or non-natural nucleoside or a natural or non-natural nucleoside in which one or two natural or non-natural nucleotides are linked to carbon at a 3'-position thereof.

23. The mRNA drug according to claim 21, wherein the hydrophobic substrate containing the structure of the formula (ECO) is selected from the group consisting of the following formulas (EC1) to (EC12), 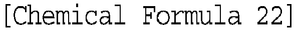 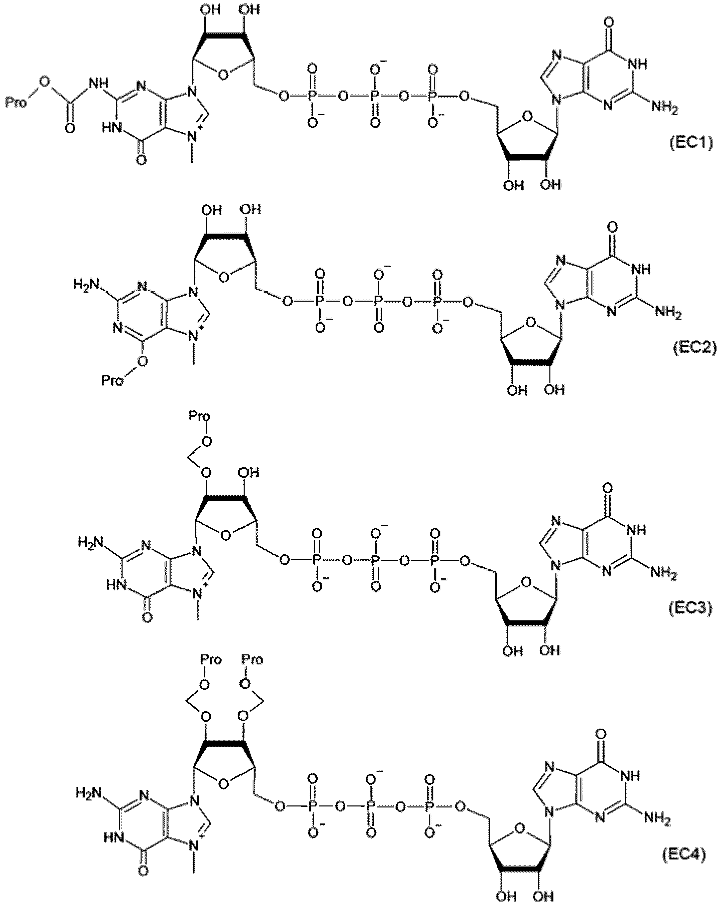
wherein Pro represents the hydrophobic protecting group, 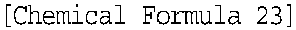 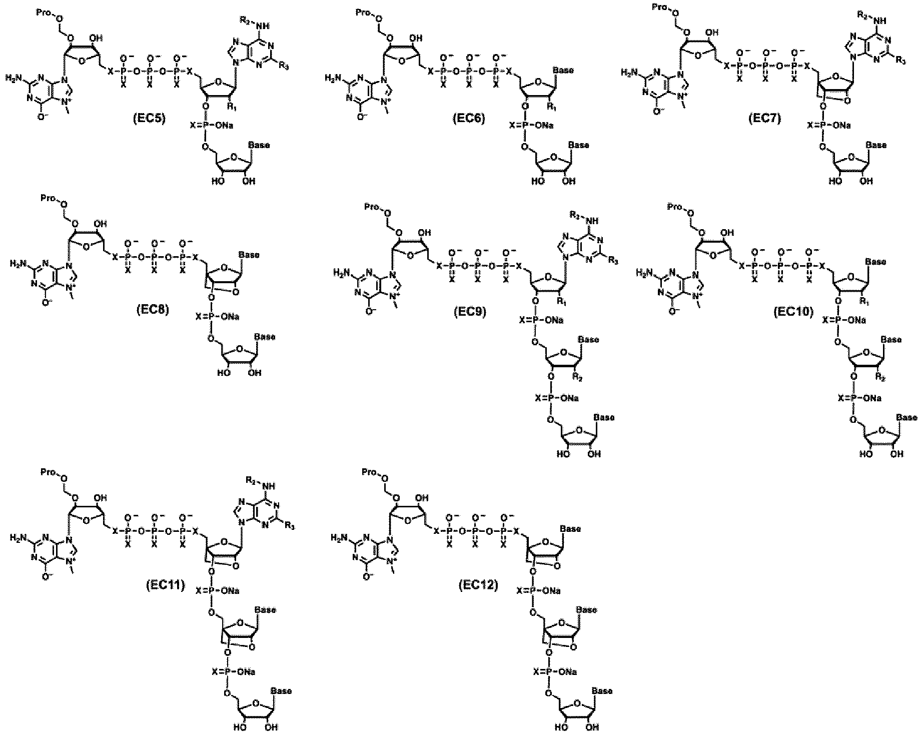
wherein R₁ represents a substituent selected from among a hydroxy group (OH), a methoxy group (OCH₃), methoxyethyl (-OCH₂OCH₃), fluorine (F), and a formamide group (-NHCHO), R₂ represents hydrogen, a linear or branched alkyl group having 1 to 10 carbon atoms, and R₃ represents hydrogen or an amino group (-NH₂); wherein Base is a natural nucleobase selected from among adenine, cytosine, thymine, uracil, and guanine, or represents a non-natural base; wherein X is selected from the group consisting of oxygen, sulfur, and selenium atoms; and wherein Pro represents a hydrophobic protecting group represented by the formula (P1) or (P2) .

24. A method for producing the mRNA drug according to claim 20, the method comprising:
a protecting group introduction step of introducing the hydrophobic protecting group represented by the formula (P1) or (P2) into the nucleotide-based substance to generate a hydrophobic nucleotide-based substance.

25. A method for purifying the mRNA drug according to claim 21, wherein in the protecting group introduction step, the hydrophobic nucleotide-based substance is synthesized by employing, as a template, a nucleic acid having a sequence complementary to the nucleotide-based substance, employing, as substrates, a hydrophobic substrate having a structure of the formula (ECO) and a nucleoside triphosphate, and transcribing the template by an RNA polymerase.
